(19) **Europäisches Patentamt**

**European Patent Office**

**Office européen des brevets**

(11) **EP 4 741 014 A2**

(12) **EUROPEAN PATENT APPLICATION**

(43) Date of publication:
**13.05.2026 Bulletin 2026/20**

(21) Application number: **26168675.2**

(22) Date of filing: **29.07.2019**

(51) International Patent Classification (IPC):
***A61P 25/28*** *(2006.01)*

(52) Cooperative Patent Classification (CPC):
**A61B 5/055; A61B 5/0042; A61B 5/02007;**
**A61B 5/4088; A61P 25/28;** A61B 5/7275;
A61B 2576/026; G01R 33/4816; G01R 33/5601;
G01R 33/56366; G16H 30/40

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **27.07.2018 US 201862711251 P**

(62) Document number(s) of the earlier application(s) in
accordance with Art. 76 EPC:
**19841904.6 / 3 829 432**

(71) Applicant: **Northeastern University**
**Boston, MA 02115 (US)**

(72) Inventors:
• **GHARAGOUZLOO, Codi Amir**
  **Medford, 02155 (US)**
• **FERRIS, Craig**
  **Holden, 01520 (US)**

(74) Representative: **V.O.**
**P.O. Box 87930**
**2508 DH Den Haag (NL)**

Remarks:
This application was filed on 17.03.2026 as a
divisional application to the application mentioned
under INID code 62.

(54) **DIAGNOSIS OF DEMENTIA BY VASCULAR MAGNETIC RESONANCE IMAGING**

(57)      A method of diagnosing a likelihood of onset or progression of Alzheimer's disease and related dementias (ADRD) in a subject is provided. The method requires determining vascularization changes in different regions of the brain on the basis of a quantitative cerebral blood volume (qCBV) map of the subject's brain. The qCBV is obtained from one or more quantitative ultrashort time-to-echo contrast-enhanced (QUTE-CE) MRI images of the brain. A method of treating a subject for ADRD is provided. Diagnostic markers for onset and progression of Alzheimer's disease are also provided.

**EP 4 741 014 A2**

**Description**

CROSS REFERENCE TO RELATED APPLICATIONS

This application claims priority under 35 U.S.C. § 119(e) of U.S. Provisional Application No. 62/711,251, filed on July 27, 2018, entitled "Diagnosis of Dementia by Vascular Magnetic Resonance Imaging," the disclosure of which is hereby incorporated by reference.

STATEMENT REGARDING FEDERALLY SPONSORED RESEARCH OR DEVELOPMENT

This invention was made with government support under Grant No. EB013180 awarded by the National Institutes of Health. The government has certain rights in the invention.

BACKGROUND

Age related dementia is a global health problem affecting over 50 million people worldwide at an estimated cost of over $818 billion (WHO Fact Sheet, Dementia, 2017). Dementia affects more than 8 million seniors in the US alone at a total cost $277 billion (Alzheimer's Disease Facts and Figures, 2018). With aging comes the risk of Alzheimer's Disease (AD), a degenerative brain disorder that is rated as the number one cause of dementia (Wilson, R. S. et al., 2012; Barker, W. W. et al., 2002). Aging also carries the risk of cerebrovascular disease, traditionally considered to be the second leading cause of dementia (Banerjee, G et al., 2015; Roman, G. C. et al., 2004). However, there is accumulating evidence that cerebrovascular disease plays a key role in the neurodegeneration and dementia of AD (Schneider, J. A. et al., 2007; Wharton, S. B. et al. 2011) and may be the most common form of dementia in the elderly (Gorelick, P. B. et al., 2011; Roman, G. C. et al. 2001). This distinction is important, because cerebrovascular disease may be a key factor in the pathophysiology of AD (Kalaria, R. N. & Ballard, C., 1999). Hence, early detection and treatment of cerebrovascular disease may reduce the risk of AD with aging. Early biomarkers of dementia and AD could save the current US population as much as $12.72 to $92.56 billion per year from 2025 to 2050 (Alzheimer's Disease Facts and Figures, 2018).

The exact biochemical mechanisms underpinning AD are as yet unsolved. Thus, there exists a compelling need to develop quantitative and reliable physiological biomarkers for diagnostics, prognosis, and treatment of AD and related forms of dementia.

SUMMARY

Provided herein is a method of diagnosing a likelihood of onset or progression of Alzheimer's Disease and Related Dementia (ADRD) in a subject. The method employs a measurement technique to quantify vascularity throughout the entire brain using magnetic resonance imaging (MRI) ultra-short time-to-echo (UTE) pulse sequences and an intravascular contrast agent. The technique allows measurement of blood volume per unit volume of an anatomical region of the brain. This, in turn allows a determination of whether a given anatomical region of the brain is hypervascularized or hypovascularized. According to an aspect of the technology presented herein, a greater number of hypervascularization regions relative to hypovascularization regions indicate an increased likelihood of onset of ADRD. A greater number of hypovascularization regions relative to hypervascularization regions indicate an increased likelihood of progression of ADRD. In another aspect of the present technology, a greater volume of hypervascularized regions of the brain than hypovascularized regions indicates an increased likelihood of onset of ADRD, while a greater volume of hypovascularized regions than hypervascularized regions indicates an increased likelihood of progression of ADRD. In yet other aspects, an increased ratio of hypervascularized regions to hypovascularized regions indicates increased likelihood of onset of ADRD, while a decrease in this ratio indicates an increased likelihood of progression of ADRD. Similarly, in other aspects of the technology, an increase of hypervascularization compared to a normal subject indicates an increased likelihood of onset of ADRD, while an increase of hypovascularization compared to a normal subject indicates an increased likelihood of progression of ADRD. In addition, dynamic measurements using this technique can be performed either by utilizing shorter time scales to observe normal oscillations in physiological blood volume modulation for the purposes of determining the connectivity of brain regions, or by providing a measurement of blood volume after neurofunctional modulation, e.g., hypoxia, whereby the vascular reserve can be obtained to assess cerebrovascular reactivity (CVR). Also provided herein are diagnostic markers for onset and for progression of ADRD.

Using this measurement technique, a hypervascular trend was observed in an APOE4 preclinical rat model of ADRD before disease onset (7 m of age). This trend is related primarily to the microvasculature, suggesting that an increased capillary, or small vessel, density may be a coping mechanism for metabolic dysfunction in dementia. A trend towards hypovascularization was observed (rats 2 y of age) in the APOE4 preclinical rat model of ADRD both in terms of small vessels (microvasculature or capillary density) but also mean vascularity (includes larger vessels in calculations).

These observations are consistent with evidence from human MRI studies in which human APOE4 carriers were found to have hyperperfusion before ADRD onset, while hypoperfusion was observed after ADRD onset (Kim, S. M. et al., 2013).

In $CO_2$ challenge in a respiration model, in which air is replaced with 95% air and 5% $CO_2$, the response in the rat APOE4 model of AD was substantially more significant than the wild type (WT) (about 95/174 anatomical regions vs. 25/174 regions with significant changes p<0.05).

$CO_2$ challenge produced an enduring response in the APOE4 genetically modified rat model for ADRD. WT rats recovered almost immediately from the $CO_2$ challenge, whereas APOE4+ rats almost did not recover at all. Furthermore, upon re-application of the challenge, the response was magnified in APOE4+ rats (now ~125/174 regions significantly different, p<0.05), whereas the WT response was only ~20/174 regions. Therefore, a hypersensitive response was found in APOE4+ rats at 2y of age. In the latest stages of pre-dementia, or dementia, in humans, one could expect that hypoxic challenge may result in no dilation of vasculature if the vascular reserve is already being utilized at rest, such as is found in the disease Moyamoya, which is known to include a high amount of hypervascularization but low CVR, since the reserve is utilized constantly.

Patterns of microvasculature and mean vascularity measurements, such as per anatomical region, can be used to identify statistical variance in normal aging. Vascular progression can be assessed per anatomical region of the brain in a quantitative manner. Thus, vascular pathophysiology of specific brain regions and their role in ADRD can be assessed. Regional quantification also enables network-level analysis. The mean vascularity is calculated simply as the average of the region-of-interest (ROI), and the mode of the distribution of each ROI can be used as a proxy for the microvascularity.

The hyper- to hypo-vascular trend from disease onset to progression has not been demonstrated previously. The demonstration herein of the hyper- to hypo-vascular trend is in part made possible by the use of the aforementioned measurement technique. Vascular pathogenesis of dementia is poorly understood up to now, and the trend described herein provides a novel biomarker for the early detection of ADRD, which accounts for 60-70% of dementia, or for the early detection of dementia in general.

Existing methods for detecting brain modifications that occur before dementia onset require a large group/-number of subjects for relevant statistics and thus are not appropriate for early detection. These methods typically use functional MRI (fMRI), amyloid position emission tomography (PET) or tau PET imaging. In contrast, in the method described herein, detection is possible by means of a single measurement.

Accordingly, in some aspects of the present technology, methods of detecting increased probability of onset or progression of Alzheimer's disease or ADRD in a subject are provided. In some aspects, the method includes (a) obtaining one or more quantitative ultrashort time-to-echo contrast-enhanced (QUTE-CE) MRI images of the subject's brain, whereby usually pre- and post-contrast images are acquired; (b) obtaining B1+ and B1- field measurements to be used for more accurate results; (c) producing a quantitative cerebral blood volume (qCBV) map of the subject's brain from the images incorporating B1 measurements; (d) determining regions of hypervascularization and regions of hypovascularization in the subject's brain based on comparing the qCBV map obtained in step (c) to a pre-determined qCBV map representative of a healthy subject brain; and (e) diagnosing the subject's likelihood of developing ADRD, onset of ADRD, or progression of ADRD based on an analysis of the hypervascularization and hypovascularization patterns.

Regions of hypervascularization are those having increased intravascular blood volume compared to the same region of a normal brain of similar age in a similar physiological state. Regions of hypovascularization are those having decreased intravascular blood volume compared to the same region of a normal brain of similar age in a similar physiological state. The boundaries of a region can be selected at will, or can be defined by standard anatomical definitions.

In further aspects, the method includes (a) obtaining one or more quantitative ultrashort time-to-echo contrast-enhanced (QUTE-CE) MRI images of the subject's brain, both pre- and post-contrast; (b) producing a quantitative cerebral blood volume (qCBV) map of the subject's brain from the image; (c) determining regions of hypervascularization and regions of hypovascularization in the subject's brain based on comparing the qCBV map obtained in step (b) to a pre-determined qCBV map representative of a healthy subject brain; and (d) diagnosing the subject's likelihood of developing Alzheimer's disease, onset of Alzheimer's disease, or progression of Alzheimer's disease based on an analysis of the hypervascularization and hypovascularization regions.

In other aspects, a diagnostic marker, or a set of diagnostic markers, for onset of ADRD in a subject are provided. Suitable diagnostic markers include hypervascularization of one or more regions of the subject's brain, selected from the group consisting of ventral tegmental area, raphe linear, reticulotegmental nucleus, raphe obscurus nucleus, habenula nucleus, median raphe nucleus, dorsomedial tegmental area, dorsal raphe, pontine nuclei, raphe magnus, ventral subiculum, motor trigeminal nucleus, copula of the pyramis, pontine reticular nucleus caudal, pontine reticular nucleus oral, trapezoid body, subiculum dorsal, parabrachial nucleus, reticular nucleus midbrain, retrosplenial caudal ctx, pedunculopontine tegmental area, red nucleus, sub coeruleus nucleus, PCRt, inferior colliculus, facial nucleus, 9th cerebellar lobule, gigantocellular reticular nucleus, principal sensory nucleus trigeminal, entorhinal ctx, root of trigeminal nerve, visual 1 ctx, 10th cerebellar lobule, prelimbic ctx, precuniform nucleus, infralimbic ctx, superior colliculus, solitary tract nucleus, periaqueductal gray thalamus, and combinations thereof. The hypervascularization can be determined, for example, by comparing a qCBV map of the subject's brain produced from one or more images of the subject's brain obtained using one or more QUTE-CE MRI images of the subject's brain and comparing the qCBV map to a qCBV map representative of a healthy subject's brain, preferably of a similar age and physiological state, and preferably predetermined.

In further aspects, a diagnostic marker, or a set of diagnostic markers, for progression of ADRD in a subject are provided. The diagnostic marker includes hypovascularization of one or more regions of the subject's brain selected from the group consisting of ventral tegmental area, raphe linear, reticulotegmental nucleus, raphe obscurus nucleus, habenula nucleus, median raphe nucleus, dorsomedial tegmental area, dorsal raphe, pontine nuclei, raphe magnus, ventral subiculum, motor trigeminal nucleus, copula of the pyramis, pontine reticular nucleus caudal, pontine reticular nucleus oral, trapezoid body, subiculum dorsal, parabrachial nucleus, reticular nucleus midbrain, retrosplenial caudal ctx, pedunculopontine tegmental area, red nucleus, sub coeruleus nucleus, PCRt, inferior colliculus, facial nucleus, 9th cerebellar lobule, gigantocellular reticular nucleus, principal sensory nucleus trigeminal, entorhinal ctx, root of trigeminal nerve, visual 1 ctx, 10th cerebellar lobule, prelimbic ctx, precuniform nucleus, infralimbic ctx, superior colliculus, solitary tract nucleus, and periaqueductal gray thalamus, wherein the hypovascularization is determined by comparing a qCBV map of the subject's brain produced from one or more images of the subject's brain obtained using one or more QUTE-CE MRI images of the subject's brain and comparing the qCBV map to a qCBV map representative of a healthy subject's brain, preferably in a similar age and physiological state, and preferably predetermined..

In yet other aspects, a diagnostic marker for onset of diagnostic marker in a subject is provided. The diagnostic marker comprises hypervascularization of one or more regions of the subject's brain selected from the group consisting of paraventricular nucleus, ventral subiculum, dorsal raphe, visual 2 ctx, dorsomedial tegmental area, inferior colliculus, motor trigeminal nucleus, primary somatosensory ctx trunk, triangular septal nucleus, ventral medial striatum, lateral preoptic area, wherein the hypervascularization is determined by comparing a qCBV map of the subject's brain produced from one or more images of the subject's brain using one or more QUTE-CE MRI images of the subject's brain and comparing the qCBV map to a predetermined qCBV map representative of a healthy subject's brain.

In further aspects, a diagnostic marker, or a set of diagnostic markers, for progression of diagnostic marker in a subject are provided. A suitable diagnostic marker includes hypervascularization of one or more regions of the subject's brain selected from the group consisting of paraventricular nucleus, ventral subiculum, dorsal raphe, visual 2 ctx, dorsomedial tegmental area, inferior colliculus, motor trigeminal nucleus, primary somatosensory ctx trunk, triangular septal nucleus, ventral medial striatum, lateral preoptic area, wherein the hypovascularization is det!rmined by comparing a qCBV map of the subject's brain produced from one or more images of the subject's brain using one or more QUTE-CE MRI images of the subject's brain and comparing the qCBV map to a qCBV map representative of a healthy subject's brain of similar age and physiological state, and preferably predetermined.

Dynamic measurements in response to a challenge, such as $CO_2$, can be made in clinical practice. Thus, measures of vascular reserve also can serve as a diagnostic marker. Metabolic dysfunction in the disease state can include an enduring response to challenges such as hypoxia, which may improve characterization of the disease or sensitivity of detection.

The vascular diagnostic markers described here can be used for early diagnosis, prognosis, projection of costs for caring for patients, and as a biomarker for drug development. Many clinical trials for ADRD drugs fail because of lack of endpoint characterization and treatment that begins too late (after disease onset). Early detection allows for preventative care and planning.

The present technology further has the following aspects.

1. A method of diagnosing onset or progression of Alzheimer's Disease or Related Dementias (ADRD) in a subject, the method comprising the steps of:

(a) obtaining one or more quantitative ultrashort time-to-echo contrast-enhanced (QUTE-CE) MRI images of the subject's brain;
(b) producing a quantitative cerebral blood volume (qCBV) map of the subject's brain from the image;
(c) determining regions of hypervascularization and regions of hypovascularization in the subject's brain based on comparing the qCBV map obtained in step (b) to a pre-determined qCBV map representative of a normal brain; and
(d) diagnosing the subject's likelihood of onset of ADRD or progression of ADRD based on an analysis of the hypervascularization and hypovascularization regions.

2. The method of aspect I, wherein in step (d), a greater number of hypervascularization regions relative to hypovascularization regions indicates that onset of ADRD has occurred in the subject.
3. The method of aspect 1 or 2, wherein in step (d), a greater number of hypovascularization regions relative to hypervascularization regions indicates that progression of ADRD has occurred in the subject.

4. The method of any of aspects 1-3, further comprising repeating the method at a later time, wherein a decrease in the number or extent of hypovascularization regions found in step (d) at the later time indicates progression of ADRD in the subject.

5. The method of any of aspects 1-4, further comprising repeating the method at a later time, wherein an increase in the number or extent of hypervascularization regions found in step (d) at the later timeindicates progression of ADRD in the subject.

6. The method of any of aspects 1-5, wherein hypovascularization and/or hypervascularization is determined based on a measurement of microvasculature, capillary density, or mean vascularity.

7. The method of any of aspects 1-6, wherein progression of ADRD is indicated in the subject, and wherein a degree of hypovascularization and/or hypervascularization indicates a degree of progression of ADRD in the subject.

8. The method of any of aspects 1-7, wherein said hypervascularization is in one or more regions of the subject's brain selected from the group consisting of ventral tegmental area, raphe linear, reticulotegmental nucleus, raphe obscurus nucleus, habenula nucleus, median raphe nucleus, dorsomedial tegmental area, dorsal raphe, pontine nuclei, raphe magnus, ventral subiculum, motor trigeminal nucleus, copula of the pyramis, pontine reticular nucleus caudal, pontine reticular nucleus oral, trapezoid body, subiculum dorsal, parabrachial nucleus, reticular nucleus midbrain, retrosplenial caudal ctx, pedunculopontine tegmental area, red nucleus, sub coeruleus nucleus, PCRt, inferior colliculus, facial nucleus, 9th cerebellar lobule, gigantocellular reticular nucleus, principal sensory nucleus trigeminal, entorhinal ctx, root of trigeminal nerve, visual 1 ctx, 10th cerebellar lobule, prelimbic ctx, precuniform nucleus, infralimbic etx, superior colliculus, solitary tract nucleus, and periaqueductal gray thalamus.

9. The method of any of aspects 1-8, wherein said hypovascularization is in one or more regions of the subject's brain selected from the group consisting of ventral tegmental area, raphe linear, reticulotegmental nucleus, raphe obscurus nucleus, habenula nucleus, median raphe nucleus, dorsomedial tegmental area, dorsal raphe, pontine nuclei, raphe magnus, ventral subiculum, motor trigeminal nucleus, copula of the pyramis, pontine reticular nucleus caudal, pontine reticular nucleus oral, trapezoid body, subiculum dorsal, parabrachial nucleus, reticular nucleus midbrain, retrosplenial caudal ctx, pedunculopontine tegmental area, red nucleus, sub coeruleus nucleus, PCRt, inferior colliculus, facial nucleus, 9th cerebellar lobule, gigantocellular reticular nucleus, principal sensory nucleus trigeminal, entorhinal ctx, root of trigeminal nerve, visual 1 ctx, 10th cerebellar lobule, prelimbic ctx, precuniform nucleus, infralimbic etx, superior colliculus, solitary tract nucleus, and periaqueductal gray thalamus.

10. The method of any of aspects 1-9, wherein said hypervascularization is in one or more regions of the subject's brain selected from the group consisting of paraventricular nucleus, ventral subiculum, dorsal raphe, visual 2 ctx, dorsomedial tegmental area, inferior colliculus, motor trigeminal nucleus, primary somatosensory ctx trunk, triangular septal nucleus, ventral medial striatum, lateral preoptic area.

11. The method of any of aspects 1-10, wherein said hypovascularization is in one or more regions of the subject's brain selected from the group consisting of paraventricular nucleus, ventral subiculum, dorsal raphe, visual 2 ctx, dorsomedial tegmental area, inferior colliculus, motor trigeminal nucleus, primary somatosensory ctx trunk, triangular septal nucleus, ventral medial striatum, lateral preoptic area.

12. The method of any of aspects 1-11, wherein obtaining QUTE-CE MRI images comprises introducing a paramagnetic or superparamagnetic contrast agent into the brain of the subject.

13. The method of aspect 12, wherein the paramagnetic or superparamagnetic contrast agent is selected from the group consisting of iron oxide nanoparticles, a gadolinium chelate, and a gadolinium compound.

14. The method of aspect 13, wherein the iron oxide nanoparticles comprise a material selected from the group consisting of $Fe_3O_4$ (magnetite), y-$Fe_2O_3$ (maghemite), a-$Fe_2O_3$ (hematite), ferumoxytol, ferumoxides, ferucarbotran, and ferumoxtran.

15. The method of aspect 14, wherein the iron oxide nanoparticles comprise ferumoxytol.

16. A method of treating ADRD in a human subject, the method comprising:

performing the method of any of aspects 1-15 to diagnose the onset or progression of ADRD in the human subject; and

treating the human subject for ADRD.

17. The method of aspect 16, wherein the step of treating comprises administering a cholinesterase inhibitor, such as donepezil, rivastigmine, galantamine, memantine; an antidepressantsuch as citalopram, fluoxetine, paroxeine, sertraline, or trazodone; an anxiolytic, auch as lorazepam or oxazepam; or an antipsychotic, such as aripiprazole, clozapine, haloperidol, olanzapine, quetiapine, risperidone, or ziprasidone or any combination thereof.

18. The method of aspect 16 or 17, wherein the step of treating the human subject comprising applying a behavioral therapy;such as changing an environment, redirecting attention, avoiding a confrontation, providing rest, or monitoring one or more of pain, hunger, thirst, constipation, full bladder, fatigue, infection, skin irritation, and room temperature; and any combination thereof.

DESCRIPTION OF THE DRAWINGS

Figs. 1A-1C show representative images of QUTE-CE MRI raw intensity rendered in 3DSlicer. Fig. 1A shows a pre-contrast image (before ferumoxytol contrast injection), and Fig. 1B a post-contrast image (after injection of ferumoxytol 14mg/kg). Rendering parameters are equivalent. Fig. 1C shows a segmented brain from Fig. 1B. Relevant parameters include: 3D Radial UTE; FOV 3x3x3 cm$^3$; matrix mesh size 200x200x200; TE 13 $\mu$s; TR 3.5 ms; and $\theta$ = 20°, scan time = 8m 22seconds, 2 averages.

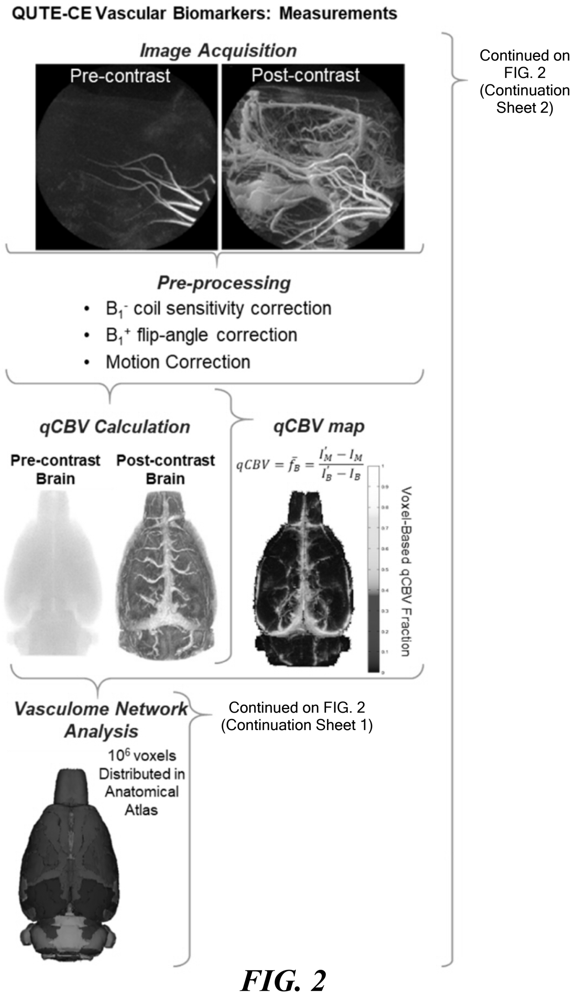

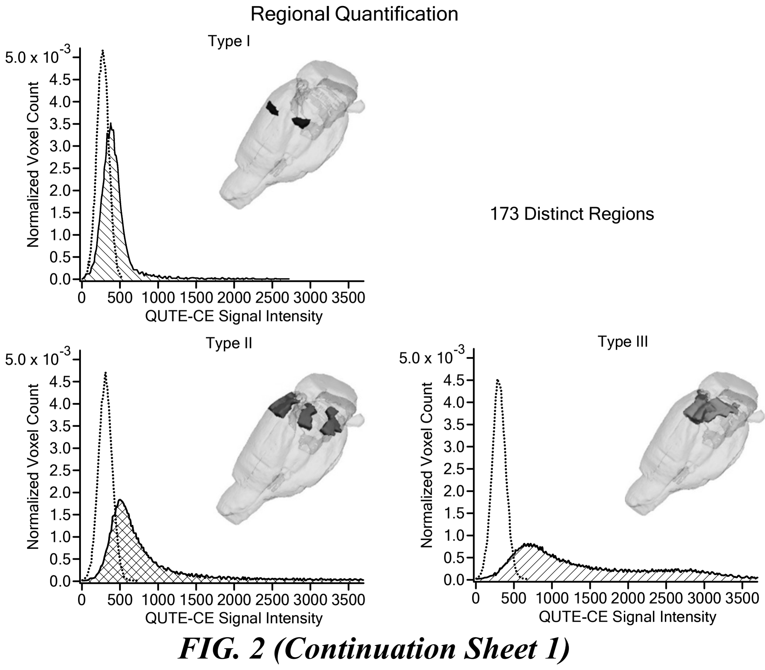

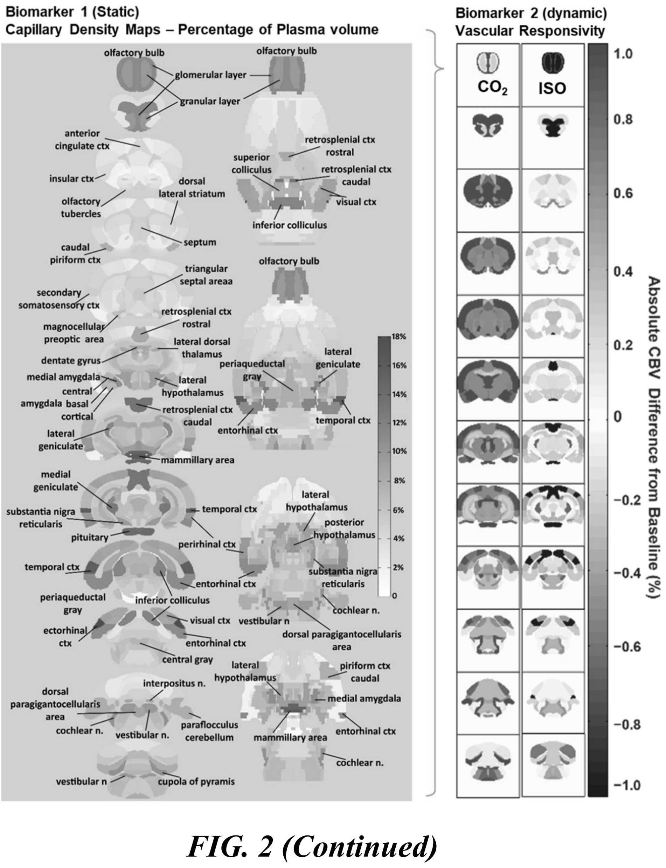

Fig. 2 illustrates a QUTE-CE MRI image analysis pipeline and biomarker measurements in Sprague Dawley rats. Regarding the method: In the top-left image: Nullcontrast and angiographic images are obtained pre- and post-ferumoxytol injection respectively. Note how the contrast in the maximum Intensity Projection (MIP) images is almost solely due to ferumoxytol presence at 7T, and blood flow effects are confined to the periphery. Field corrections for coil sensitivity (B1) and flip-angle distribution (B1+) are applied along with motion correction between the pre- and post-contrast images. In the middleleft image: The qCBV is calculated by cropping out the brain and applying the corresponding formula with the quantitative intensity values. In the bottom-left imnage: Regions are characterized by distributing the 500,000 voxels within the brain into a 173-region atlas via coregistration affine transform using EVA Software (Ekam Solutions, Boston, MA USA). In the middle image: A vascular atlas was constructed of Male Sprague Dawley rats (9-10 weeks old, n=11) and (right) $CO_2$ can was applied to measure vascular responsivity. Of note, imaging was performed on awake, yet mechanically restrained, rats to simulate neurophysiological conditions for human-brain clinical imaging experiments. Thus, the direction and magnitude of vascular changes during isoflurane anesthesia were also determined for rats (labeled "ISO").

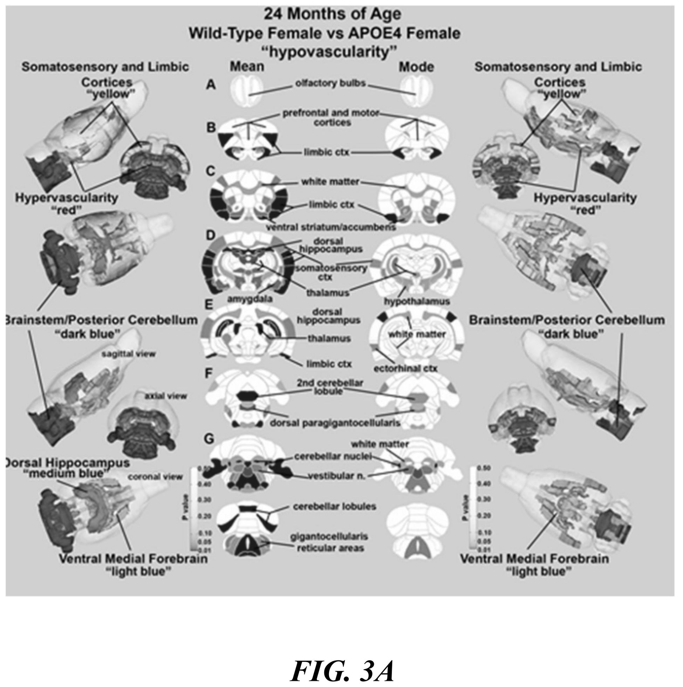

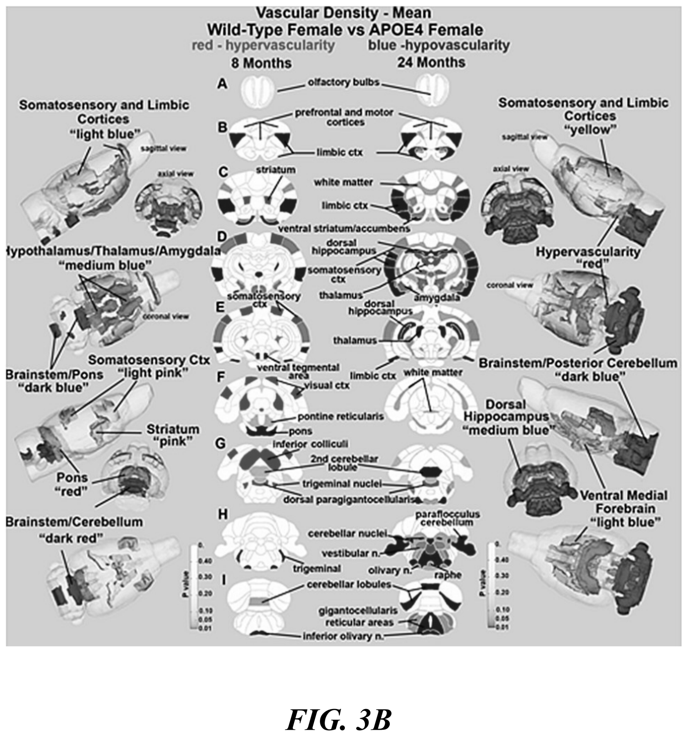

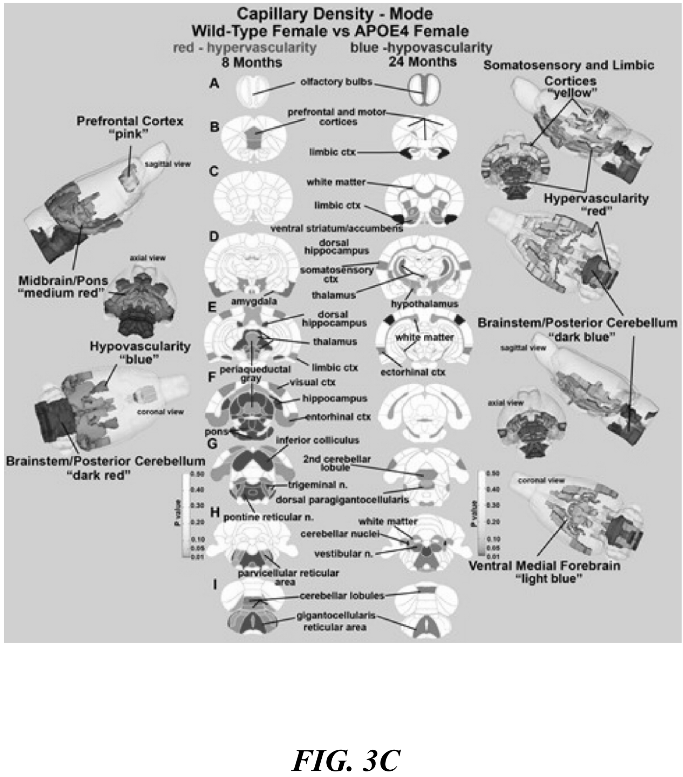

Fig. 3A illustrates hypovascularity of wild-type (WT) female vs. APOE4 female rats at 24 months of age. Fig. 3B illustrates vascular density - mean of wild-type female vs. APOE4 female rats at 8 months and 24 months of age. Fig. 3C illustrates capillary density (or small vessels) - mode of wild-type female vs. APOE4 female rats at 8 months and 24 months of age.

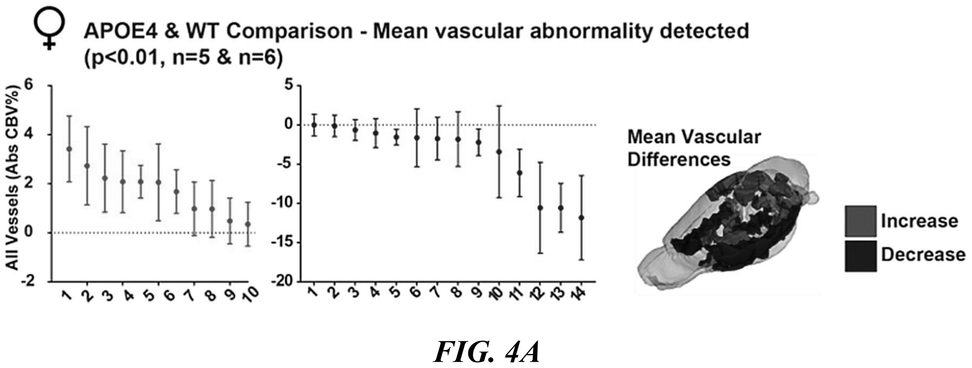

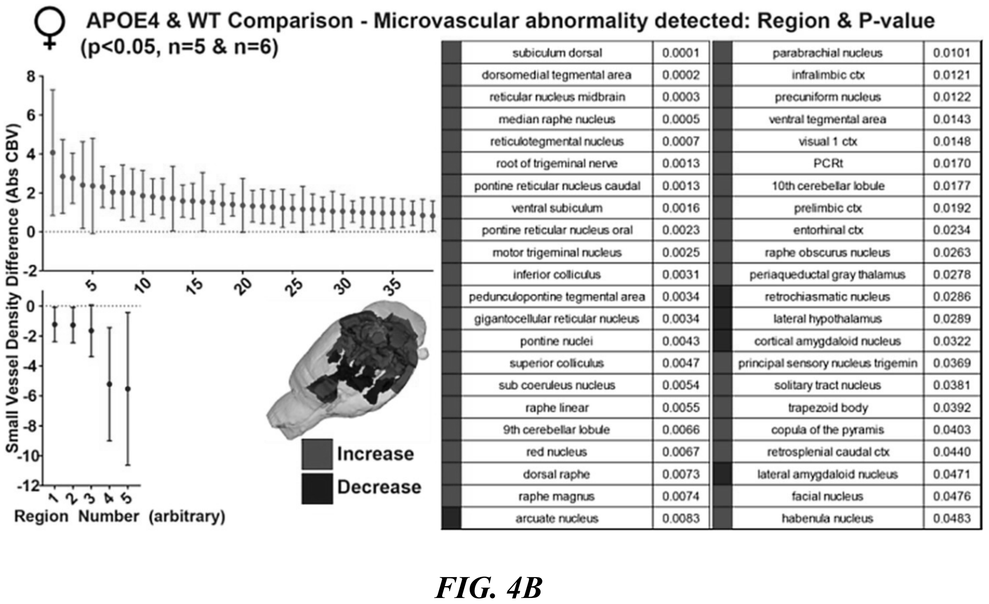

Fig. 4A illustrates an APOE4 and WT comparison of mean vascular abnormality detected at 8 months. Fig. 4B illustrates an APOE4 and WT comparison of microvascular abnormality detected by region at 8 months.

Fig. 5A is a graph of vascular abnormalities for small vessels and all vessels at 8 months and at 2 years, illustrating a hypovascular trend at 2 years. Fig. 5B is a graph of $CO_2$ challenge demonstrating metabolic dysfunction with $CO_2$ channels in APOE4 and WT.

Fig. 6 is a table of results of the mean of structural differences at 8 months.

Fig. 7 is a graphical illustration of the results of Fig. 6.

Fig. 8 is a table of results of the mode of structural differences at 8 months.

Fig. 9 is a graphical illustration of the results of Fig. 8.

Fig. 10 is a table of results of the mean of structural differences at 2 years.

Fig. 11 is a graphical illustration of the results of Fig. 10.

Fig. 12 is a table of results of the mode of structural differences at 2 years.

Fig. 13 is a graphical illustration of the results of Fig. 12.

Fig. 14 is a table of results of WT mean differences in $CO_2$ challenge states for WT rats at 2 years.

Fig. 15 is a table of results of WT mode differences in $CO_2$ challenge states for WT rats at 2 years.

Fig. 16 is a table of results of APOE mean differences in $CO_2$ challenge states for APOE rats at 2 years.

Fig. 17 is a table of results of APOE mode differences in $CO_2$ challenge states for APOE rats at 2 years.

DETAILED DESCRIPTION

The present technology utilizes an imaging modality termed quantitative ultra-short time-to-echo contrast-enhanced (QUTE-CE) MRI (Gharagouzloo et al; 2017; Gharagouzloo et al, 2015), which is able to overcome the semi-quantitative nature of the MRI signal. The preliminary data demonstrate that QUTE-CE MRI can provide a highly accurate quantitative map of the brain "vasculome" from small to large vessels, offering a firm basis for assessment of microvascular contribution to brain function and neurological disorders.

The present inventors have utilized an imaging modality, QUTE-CE MRI, to study the micro- and macro- vascular abnormalities in an APOE-ε4 knock-in rat model. It is noteworthy that the APOE-ε4 allele is the single most important genetic risk factor for AD. The study involved characterizing vascular changes in 173 regions of the brain. While the 173-region characterization revealed both hyper- and hypovascularization, the changes in microvascularity were almost entirely hypervascular early on (rats at 8 months), and hypovascular later in life (24 months).

The resolution and sensitivity are such that QUTE-CE can map the physiological CBV across the entire rat brain in 500,000 small volumes (or voxels), which can be distributed over 173 anatomically distinct 3D volumes for network-level analysis indicating capillary density, small vessel responsivity and vascular reserve. Importantly, QUTE-CE MRI can be immediately translated and there is an ongoing human clinical trial to map the healthy brain vascularity of a small group of individuals. The preliminary preclinical data in an ApoE4 human-knock in genetically modified rat model for AD reveals a trend of hyper- to hypo-vascularization early in development before cognitive decline.

QUTE-CE MRI is a method that utilizes a 3D UTE pulse sequence and an intra-vascular contrast agent (CA) to render high contrast-to-noise ratio (CNR) vascular images with quantitative signal. (See WO 2017/019812, incorporated by reference herein.) At this ultrashort TE, contrast is inverted from the typical negative-contrast obtained from superparamagnetic iron-oxide nanoparticles (SPIONs) to purely TI-enhanced positive-contrast. Quantitative measurements of the micro- and macro-vasculature can be mapped throughout the whole rat brain and functional changes of state can also be measured (Gharagouzloo et al., 2017). The absolute cerebral blood volume (qCBV) is calculated by simple partial volume calculations using a two-compartment model for signal from blood and tissue:

$$I_M = f_B I_B + (1 - f_B) I_T \qquad (1)$$

where $I_M$ is the measured signal intensity at each voxel, $I_T$ is the tissue intensity, $I_B$ is the blood intensity and $f_B$ is the fraction of the voxel occupied by blood. qCBV is calculated voxel-wise by subtracting a pre-contrast intensity from post-contrast after catheter injection of ferumoxytol (Feraheme, AMAG Pharmaceuticals, Waltham, Massachusetts, USA, 7mg/kg). Solving for the blood volume fraction,

$$qCBV = f_B = \frac{I'_M - I_M}{I'_B - I_B} \qquad (2)$$

is measured in whole brain, with blood reference signal intensity taken from the superior sagittal sinus (SSS), per image. Images are then fit to a 173 anatomic region atlas for segmented analyses using anatomical scans. The mean qCBV is reported per region, and the mode is taken as a proxy for microvessel density.

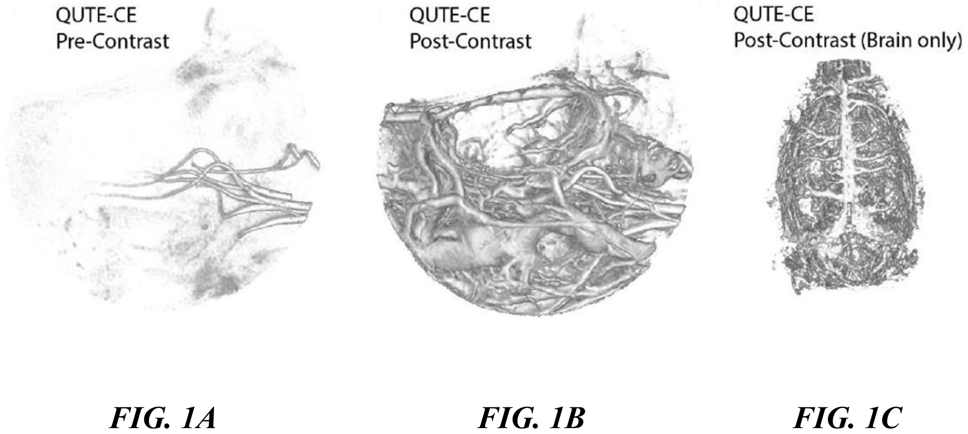

Quantitative vascular mapping of the rat brain begins with acquisition of pre- and post-ferumoxytol scans. A 3D UTE sequence with optimized parameters for blood contrast and quantification is utilized. Field corrections for coil sensitivity (B1-) and flip-angle distribution (B1+) are applied along with motion correction between the pre- and post-contrast images. A voxel-wise calculation for the quantitative CBV (qCBV) is performed to produce the qCBV map using a two-volume blood/tissue model with knowledge of blood intensity obtained from large vessels. See Figs. 1A-1C for image contrast, see Fig. 2 for image processing pipeline. For the rat models described here, voxels are distributed into an anatomically segmented atlas with 173 regions for quantitative analysis of the whole brain, both in terms of the mean vascularity and the microvessel density obtained from a regional characterization of the model. Statistically significant abnormalities are found by comparing healthy, normal vasculature to genetically modified model of disease in rats.

The procedure for producing QUTE-CE MRI images of a subject is described in detail in WO 2017/019182. As applied to a subject's brain, a magnetic field is applied to the brain followed by application of a radio frequency pulse sequence at a selected repetition time (TR) and application of a magnetic field gradient to provide a selected flip angle (FA) to excite protons in the region of interest. Generally, the repetition time is less than about 10 ms, and the FA ranges from about 10° to about 30°, around the Ernst angle of doped blood or at the angle of maximum contrast between doped- and undoped-blood. A response signal is measured during relaxation of the protons at a selected time-to-echo (TE) and a T1-weighted signal acquired. The time to echo is an ultra-short time to echo and is set to less than about 300 $\mu$s. In this manner,

an image of the brain is generated. Next, a paramagnetic or superparamagnetic CA is introduced into the brain of the subject by injecting the agent into vasculature. The acquired signal is representative of a concentration of the CA in the brain and the amount of blood in a particular region in that region of the brain.

The TE can, for example, be set at less than 180 $\mu$s, 160 $\mu$s, 140 $\mu$s, 120 $\mu$s, 100 $\mu$s, 90 $\mu$s, 80 $\mu$s, 70 $\mu$s, 60 $\mu$s, 50 $\mu$s, 40 $\mu$s, 30 $\mu$s, 20 $\mu$s, or 10 $\mu$s. Also, the time to echo can be set to less than a time in which blood volume displacement in the region of interest in the brain is about one order of magnitude smaller than a voxel size. The TR can be set to a value from about 2 to about 10 ms. The image of the ROI can have a contrast to noise ratio (CNR) of at least 4, at least 5, at least 10, at least 15, at least 20, at least 30, at least 40, at least 50, or at least 60. The CNR is determined between an ROI represented in a post-contrast image and a pre-contrast image. For example, a CNR is found by examining an ROI in the SSS and taking the difference between the two signal to noise ratios (SNRs)- which itself is defined by the mean of any given ROI divided by the standard deviation of the noise in that respective image. The response signal can be measured along trajectories in k-space in which total acquisition time can be longer than TE. The magnetic field can have a strength ranging from 0.2 T to 14.0 T.

It should be noted that the region of interest (ROI), such as a particular area of the brain, can comprises a volume fraction occupied by blood and a volume fraction occupied by tissue. Determining the volume fraction occupied by blood comprises, prior to introducing the CA to the ROI, applying the radio frequency pulse sequence at the selected TR to excite protons in the region of interest, and measuring a response signal during relaxation of the protons at the selected TE to acquire a signal from the ROI; and comparing signal intensities of the ROI prior to introducing the CA and after introducing the contrast agent.

Any paramagnetic formulation that provides contrast in MRI can serve as a CA for the QUTE-CE method. Compounds containing paramagnetic iron-oxide nanoparticles, gadolinium-based contrast agents (GBCAs), such molecular chelates or nanoparticles, or manganese nanoparticles, can serve as CAs. If the CA is ferumoxytol, for example, the CA is introduced in the blood at a concentration of 0.1 to 15 mg/kg. The nanoparticles can be delivered by a bolus intravenous or intraarterial injection, which may be optionally repeated. Paramagnetic and superparamagnetic nanoparticles can serve as the CA. Paramagnetic molecular chelates and superparamagnetic nanoparticles can serve as the CA. Example paramagnetic nanoparticles can be iron oxide, gadolinium, or manganese nanoparticles. Iron oxide nanoparticles can be $Fe_3O_4$ (magnetite), $\gamma$-$Fe_2O_3$ (maghemite), a-$Fe_2O_3$ (hematite), ferumoxytol, ferumoxides, ferucarbotran, or ferumoxtran. Iron oxide nanoparticles can be coated with a carbohydrate and have a diameter from about 1 nm and about 999 nm, or from about 2 nm and about 100 nm, or from about 10 nm and about 100 nm, measured with dynamic light scattering. Nanoparticle CAs can have other coatings that allow them to circulate in the blood. Nanoparticles CAs can have various sizes that allow them to either be excreted by the kidneys or by the liver. Some gadolinium compounds include gadofosveset trisodium, gadoterate meglumine, gadoxetic acid disodium salt, gadobutrol, gadopentetic dimeglumine, gadobenate dimeglumine, gadodiamide, gadoversetamide, or gadoteridol.

Blood volume fraction in an ROI is determined as described in the following. Initially, a magnetic field is applied. A radio frequency pulse sequence is applied next at a selected TR and at a magnetic field gradient to provide a selected flip angle to excite protons in the region of interest. The TR is less than about 10 ms and the flip angle ranges from about 10° to about 30°. A response signal is measured during relaxation of the protons at a selected TE to acquire a T1-weighted signal from the ROI. The TE is an ultra-short time to echo. It is less than about 300 $\mu$s. A first image is generated without CA using 3D UTE sequences. Next, a paramagnetic or superparamagnetic CA is introduced into the blood. Then, A second image of the ROI is generated. Determining blood volume fraction comprises comparing signal intensities of the region of interest prior to introducing the CA and after introducing the CA. Specifically, determining the blood volume fraction comprises determining a difference in total signal intensities between the first image and the second image and determining a difference in blood signal intensities between the first image and the second image, wherein the blood volume fraction comprises a ratio of the total signal intensity difference to the blood signal intensity difference.

QUTE-CE MRI is quantitative, leading to direct assay of the CA concentration for quantitative MRI, as with nuclear imaging but without radiation toxicity or the other complications associated with radio-pharmaceuticals. Because the acquired signal is quantitative, the technique can be used for partial blood volume measurements using two volume methods. To date, there are no reported techniques that can potentially make absolute measurements of cerebral blood volume (qCBV) throughout the brain. QUTE-CE MRI can be used for identifying hyper- or hypo-vascularization, small vessel density, and vascular reserve, vascular responsivity to $CO_2$ challenge, perfusion defects and standardized uptake values or organ absorbed dose, at the individual voxel and regional levels using an anatomical or functional atlas. Thus, QUTE-CE MRI provides an advantageous set of imaging biomarkers or diagnostic markers for assessing function and state.

In some aspects, the CA can be ferumoxytol, an ultra-small superparamagnetic iron oxide nanoparticles (USPION) with a dextran coating. Since the size exceeds the cutoff (~6nm) for glomerular filtration, ferumoxytol is not cleared by the kidney, and instead is an excellent blood pool contrast agent with a long intravascular half-life of ~15 h (Bremerich et al., 2007). Numerous clinical MRI studies using ferumoxytol have been conducted in children and adults, demonstrating no major adverse effects (Muehe et al., 2016); thus QUTE-CE can be readily used in the clinic to study SVD.

**Comparison to prior clinical image techniques.**

Currently, clinical imaging of cerebral SVD is indirect and mostly represents its sequelae, such as ischemic (white matter hyperintensity (WMH) (Reijmer et al., 2016), lacunar infarcts) and hemorrhagic (cerebral microbleeds (CMBs)) lesions (Shi et al., 2016; Greenberg et al., 2009). Such consequences of SVD can be detected in the clinical setting on conventional MRI sequences, including T2/FLAIR (WMH, chronic infarcts), DWI (acute infarcts), and susceptibility weighted imaging (SWI) (CMBs) (Wardlaw et al., 2013). However, these techniques are limited in specificity, accuracy, and reliability in the assessment of total, quantitative burden of microvascular disease state (Wey et al., 2013; Brunser et al., 2013). Furthermore, current MRI techniques provide no insight regarding the microarchitecture of the brain's global network of cerebral small vasculature, or vasculome (Guo et al., 2012), which may play a crucial role in understanding of the underlying SVD pathology and mechanisms of disease in patients with stroke as well as apparently healthy aging adults with SVD that could be silent but unremittingly progressive and, ultimately, disabling. Thus, novel diagnostic methods to reliably quantify the total extent of SVD are urgently needed to address the growing burden of SVD-related disability.

**Comparison to other prior art approaches for measuring CBV.**

Other MRI methods for SPION imaging utilize long-range susceptibility induced effects (Cunninham et al., 2005; Stuber et al., 2007; Seppenwoolde et al., 2003), whereas QUTE-CE MRI avoids them by making $T_1$-enhanced measurements with TEs 1000 times shorter than standard modalities. Note that regular $T_1$-weighted imaging is not quantitative and does not lead to the detailed images obtained with QUTE-CE. Dynamic susceptibility contrast (DSC), or perfusion-weighted MRI is commonly used for measuring CBV values (Barbier et al., 2001), but requires accurate determination of the arterial input function (AIF) (Rempp et al., 1994; Yankeelov et al., 2009), or gadolinium based contrast agent (GBCA) concentration versus time curve, which is typically 15-30% inaccurate (Walker-Samuel et al., 2007; Schabel et al., 2008). Other techniques for measuring the CBV, such as steady-state susceptibility contrast mapping (SSGRE), steady state CBV (SS_CBV), and $\Delta$R2 (Troprès et al., 2001; Christen et al., 2012) all utilize $T_2$ and $T_2^*$ effects, which are susceptible to intra- and extravoxular dephasing, flow artifacts and vessel size, density and orientation (Kim et al., 2012). Iron fMRI using SPIONs differs from QUTE-CE in that it is $T_2^*$ weighted, requires high CA doses and is sensitive to extra-vascular space (Stuber et al., 2007; Mandeville, 2012). QUTE-CE is the only MR imaging technique that leads to positive contrast imaging without susceptibility-induced signal dropout.

Thus, qCBV measurements derived from QUTE-CE MRI can be used as a quantitative diagnostic marker for ADRD.

**Examples**

All animal experiments were conducted in accordance with institutional IACUC approved protocols. QUTE-CE measurements were made on 5 wild-type (WT) and 6 APOE-ε4 knock-in female rats of 7 months of age showing signs of mild cognitive impairment.

The data suggests that initial hypervascularization may be a coping mechanism to compensate for metabolic dysfunction in aging and dementia.

Methods

Behavioral Testing

The measure of cognitive behavior routinely performed at the Center for Translational Neuroimaging (CTNI) are Barnes maze for spatial memory and the Novel Object Preference (NOP) for object memory. Both the Barnes and NOP are hippocampus dependent but involve different "learning" strategies (McLay et al., 1997; Assini et al., 2009; Larkin et al., 2014; Pardo et al., 2016). While one test can be enough, at least two tests preferably are performed that recruit the same function/area (e.g. memory/hippocampus). The focus on hippocampaldependent functions is desirable because of its involvement in psychiatric disorders and similarities across species (Squire et al., 1992).

**Animal Experiments**

**QUTE-CE MRI Biomarkers**

The QUTE-CE MRI measurement pipeline is illustrated in Fig. 2. QUTE-CE Vascular Biomarkers: The (1) voxel-based, physiological blood fraction (qCBV) is measured from 0-1, with 1 being an artery or a vein. Regional (2) macro- and (3) micro-vascular measurements are obtained by considering the mean or the mode of regional distributions, respectively. Regional volumes of interest (VOIs) detailed by a high-resolution, 173-region rat anatomical atlas (Ekam Solutions, Boston, MA USA) that is fit digitally to the brain using an affine transform. (4) Regions can be classified for vascular heterogeneity by considering distance between the mean and the mode. (5) Dynamic functional tests, such as a hypercapnic challenge with 5% $CO_2$, can be applied to examine the responsivity of the vascular reserve.

**Rationale for data format and analysis**

In these measurements, the qCBV was calculated by using the pre- and post-contrast UTE images. Throughout the entire rat brain, about 500,000 voxels at 150 micro-meter isotropic resolution were obtained in about 8 minutes. However, it can be noticed that while outstanding vascular images are produced, the quantification at the voxel level still has a high degree of error. Considering the voxel-based error and given the slight variation in neuroanatomy from one animal to the next, it was preferable to quantify regional vascular measurements in these small animals. In order to accomplish this, each rat's brain was fit to an anatomical atlas with 173 regions using a manually adjusted affine transform. In the atlas, the left and right halves of the brain were lumped into a single region by default. Thus, the 500,000 voxels were distributed into the 173 regions, and the mean and mode were calculated for each region.

To test for inter-group comparison, the list of means and modes for one group was compared to the means or modes of another group for all 173 regions. Statistical significance was achieved through a t-test for difference ($P<0.05$ or $P<0.01$) between the two lists.

Microvascular, or small vessel, changes were associated with the mode; the rationale for this is that most of the brain volume can be expected to be filled with mainly smaller vessels, and it was a way to remove influence from large vessels that are 100% filled with blood.

QUTE-CE MRI measurements were made on female wild-type (WT) and APOE4+ Sprague Dawley (SD) rats at 8 months of age and at 2 years of age.

Experiments at 8 months old (all female SDs)

    Structural QUTE-CE
    Group 1 (n=5): WT
    Group 2 (n=5): APOE4+

Experiments at 2y old (all female SDs)

    Structural QUTE-CE
    Group 1 (n=5): WT
    Group 2 (n=5): APOE4+

Dynamic $CO_2$ Challenge at 2y (all female (SDs)

    Dynamic QUTE-CE (Multiple CO2 challenges)
    Group 1 (n=5): WT
    Group 2 (n=5): APOE4+

**Results**

The structural and functional changes of the vasculature were measured using QUTE-CE MRI longitudinally in aging of APOE4 knock-in female rats. The method utilized an FDA approved contrast agent and was compatible with existing clinical scanners, indicated that it can be implemented in humans for routine screening of CNS diseases.

**Hypervascularization found in ApoE4 Rats at 8m of age**

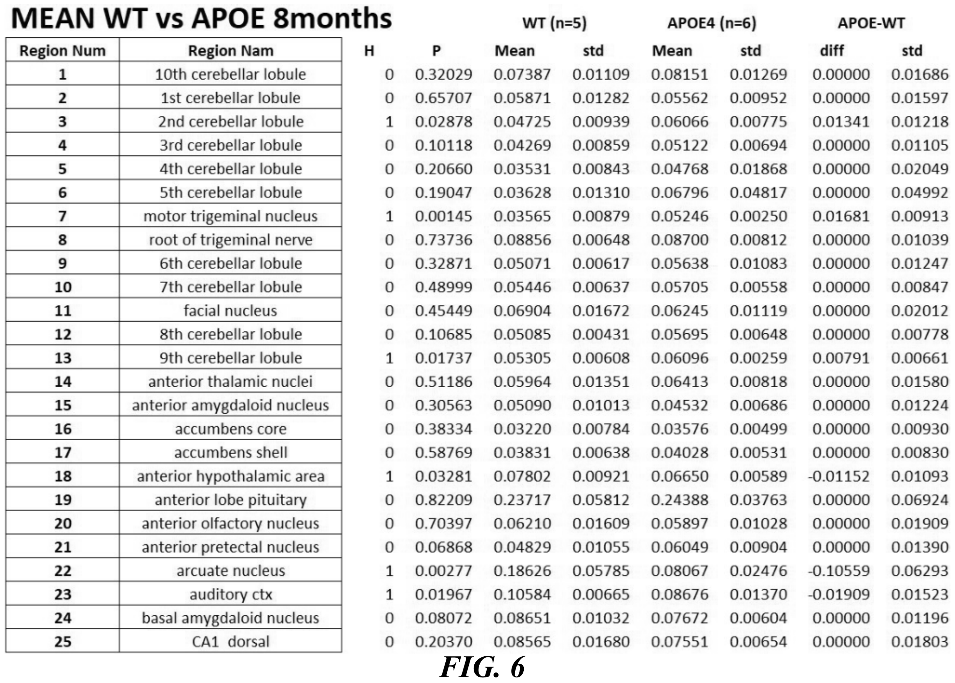

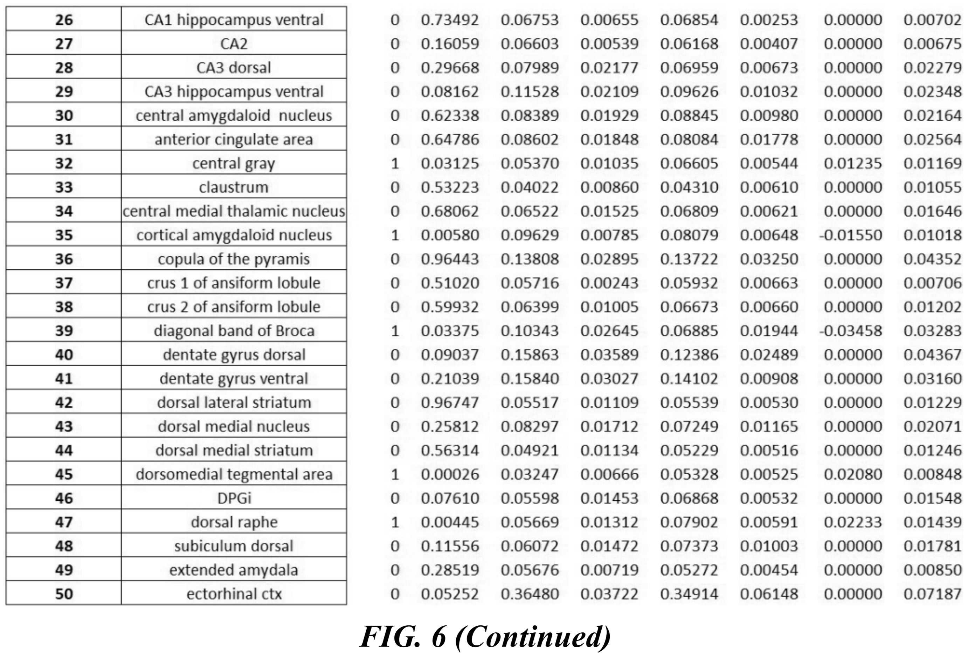

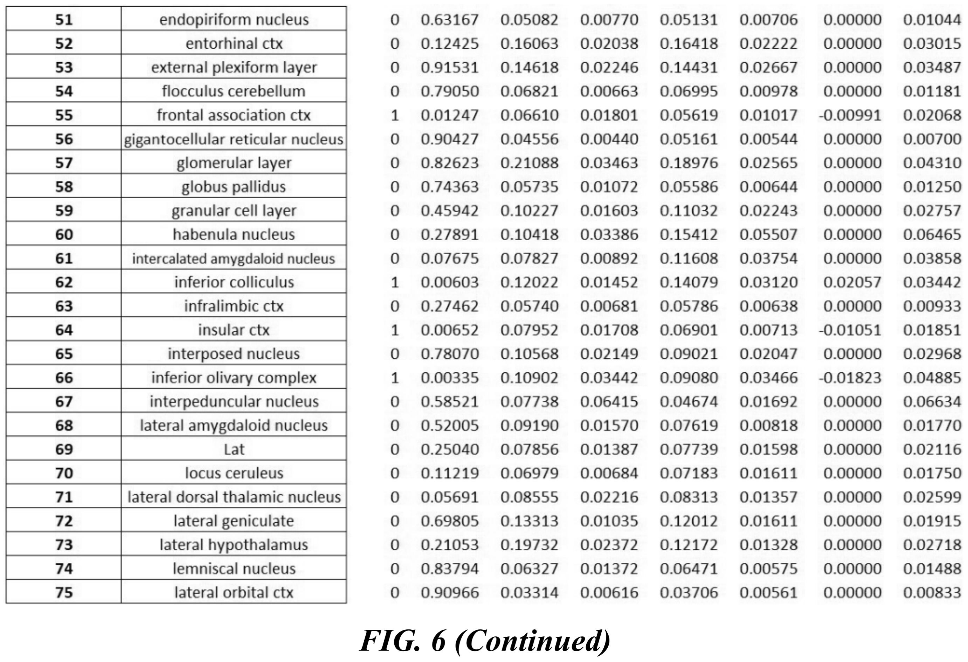

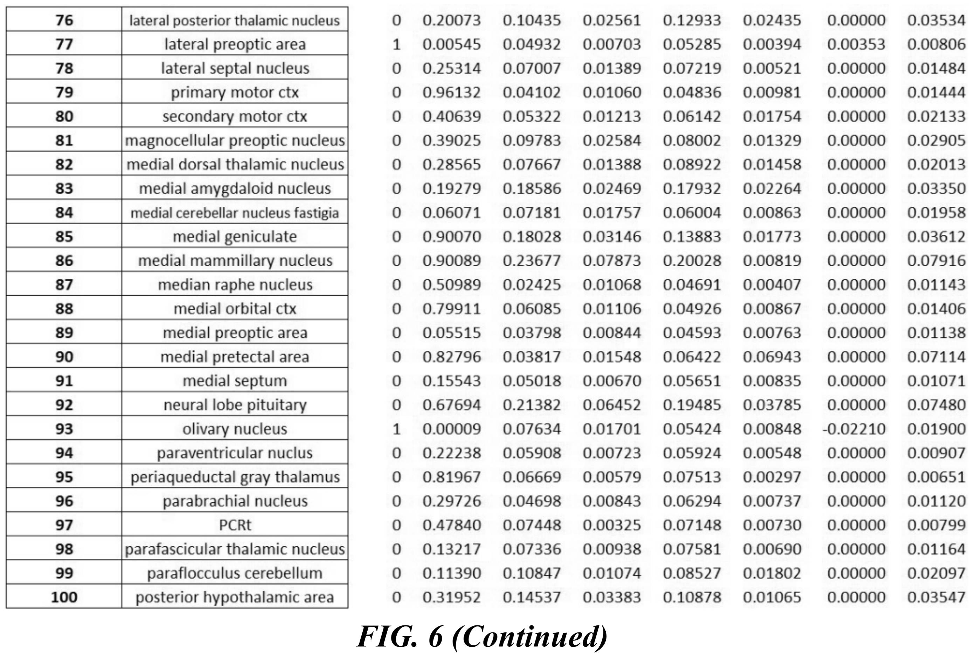

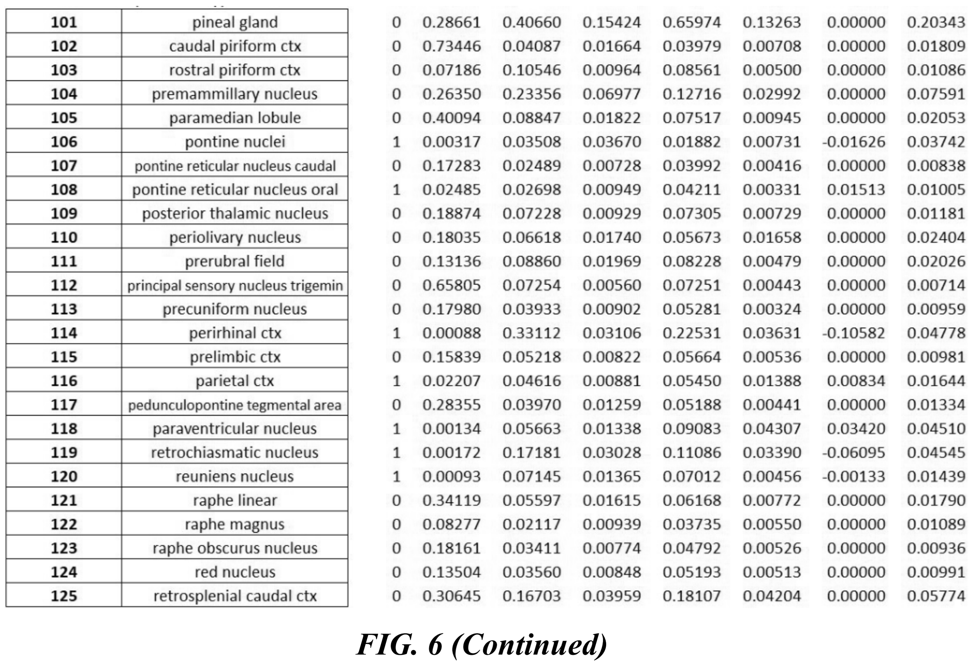

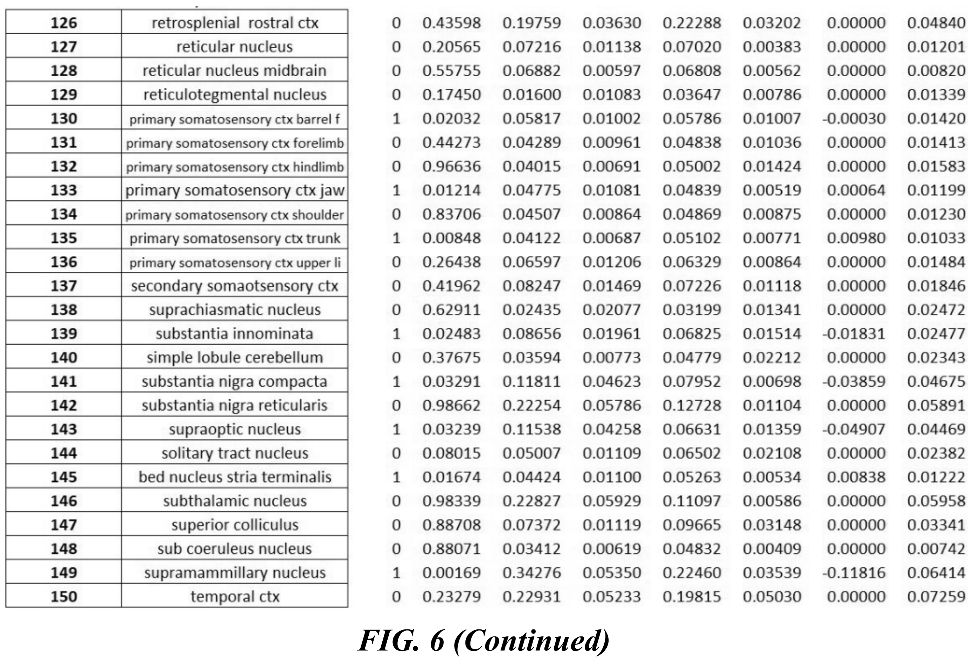

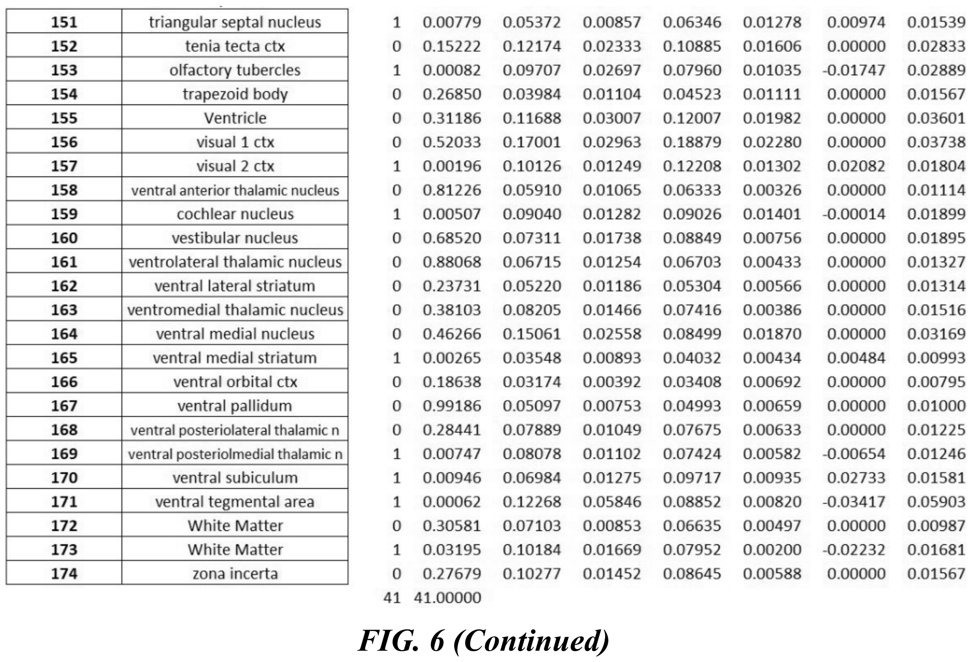

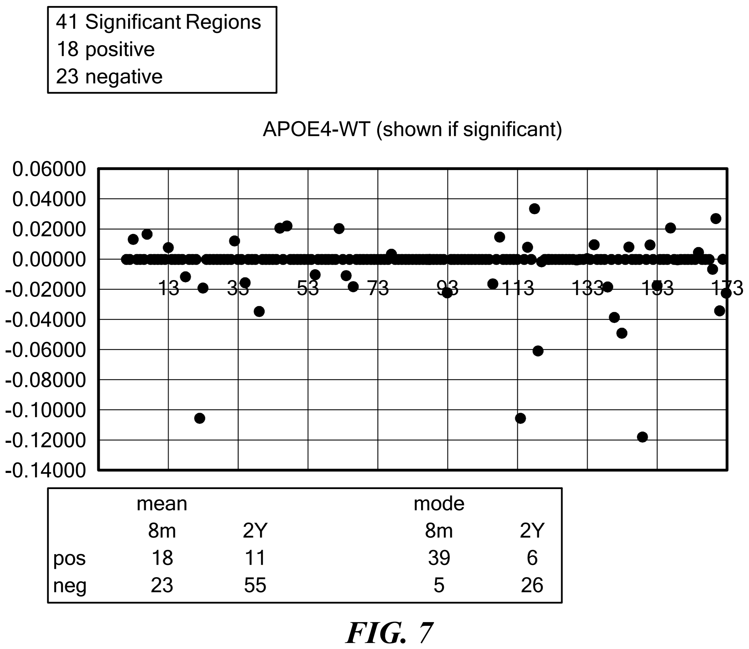

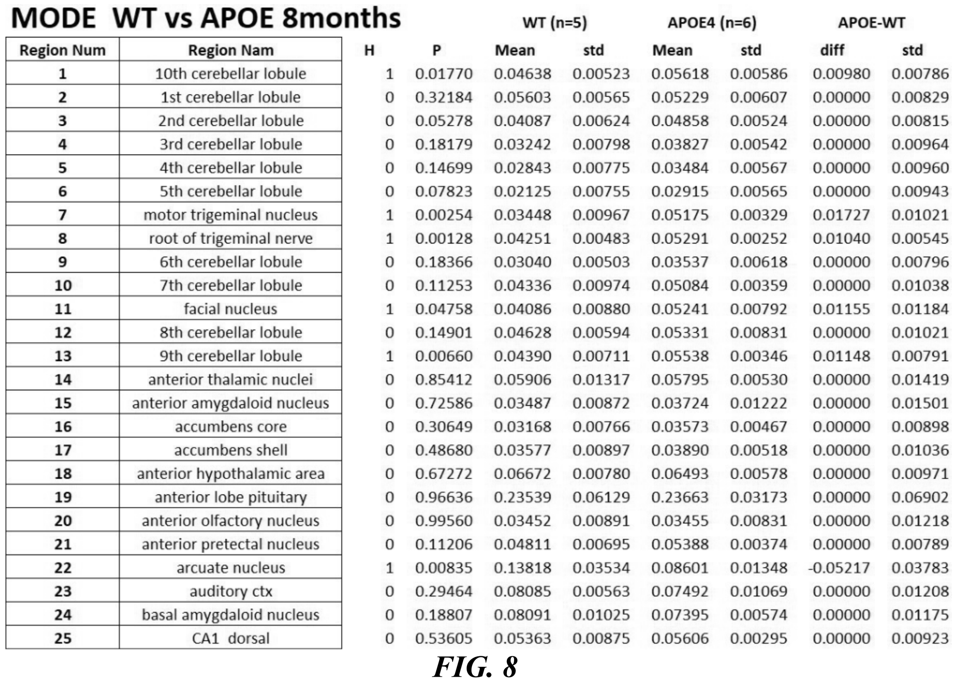

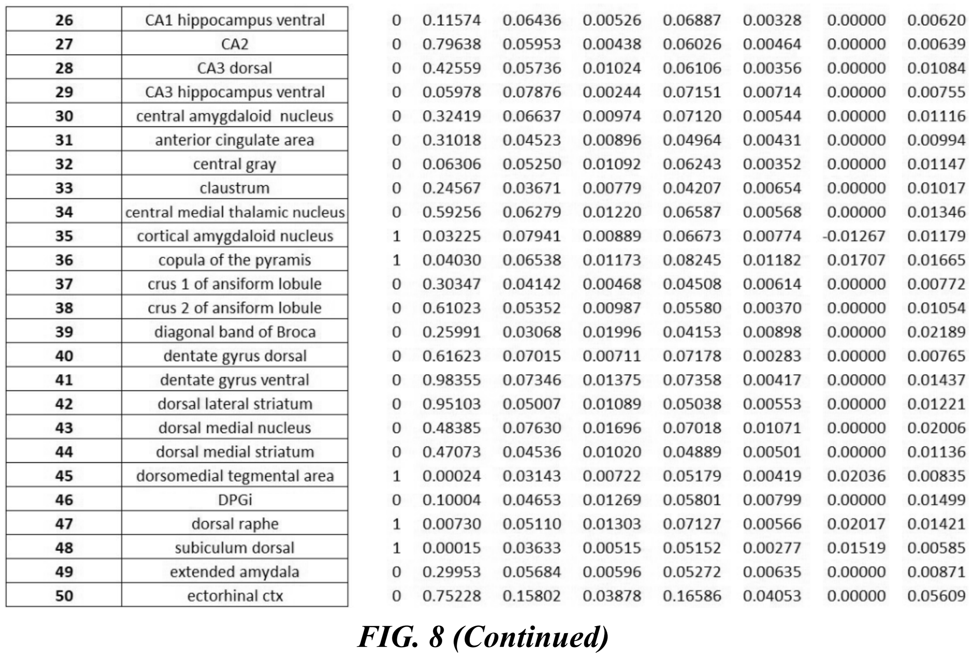

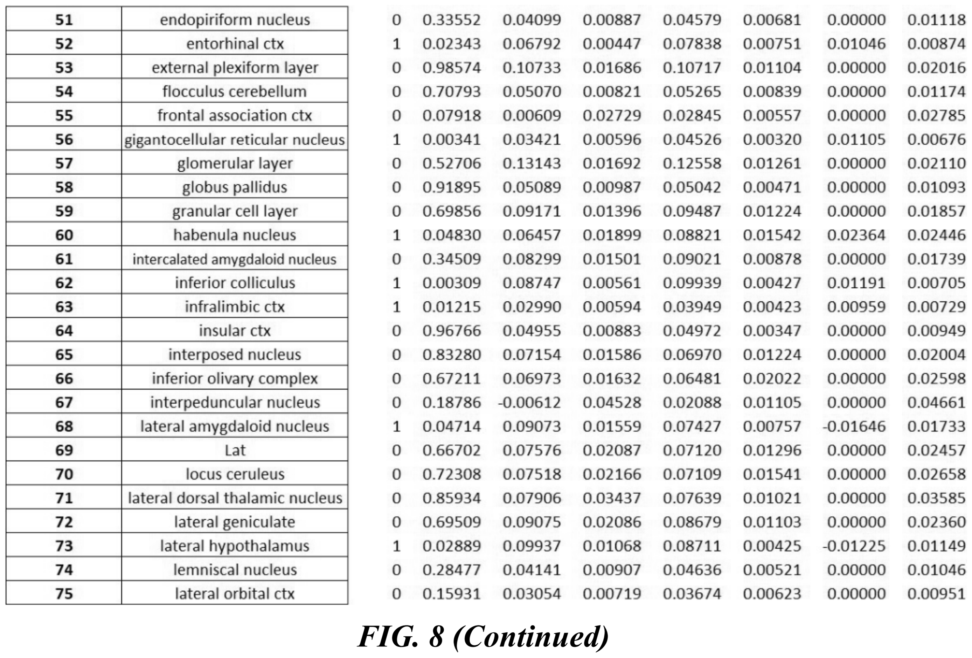

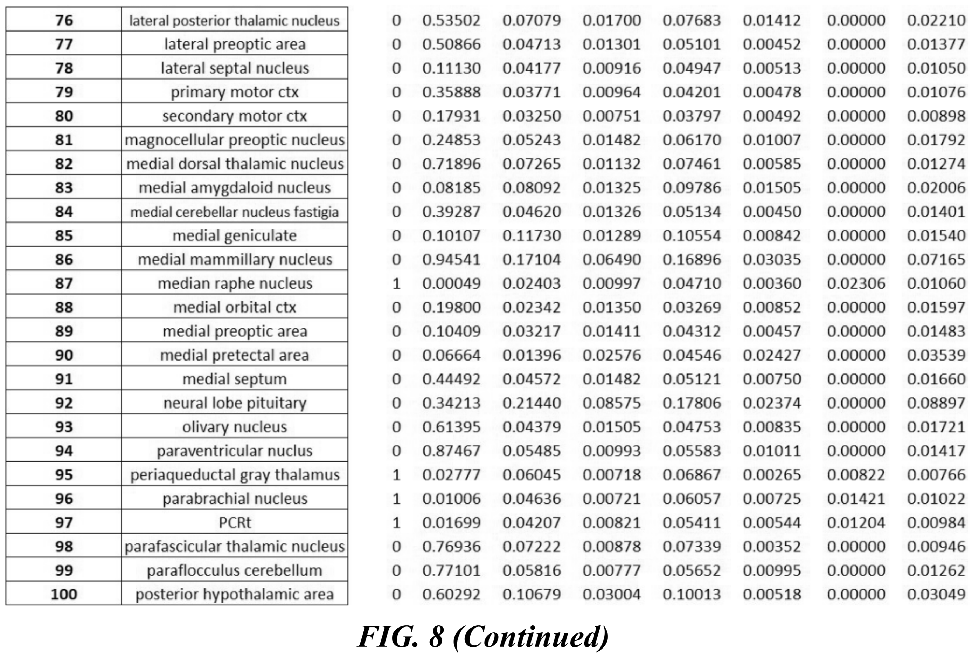

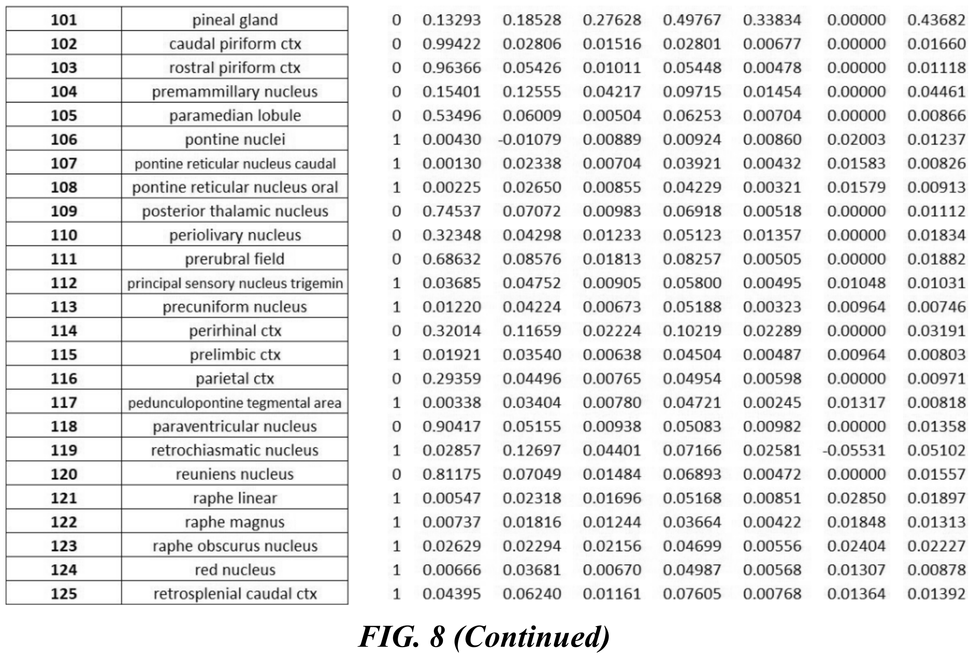

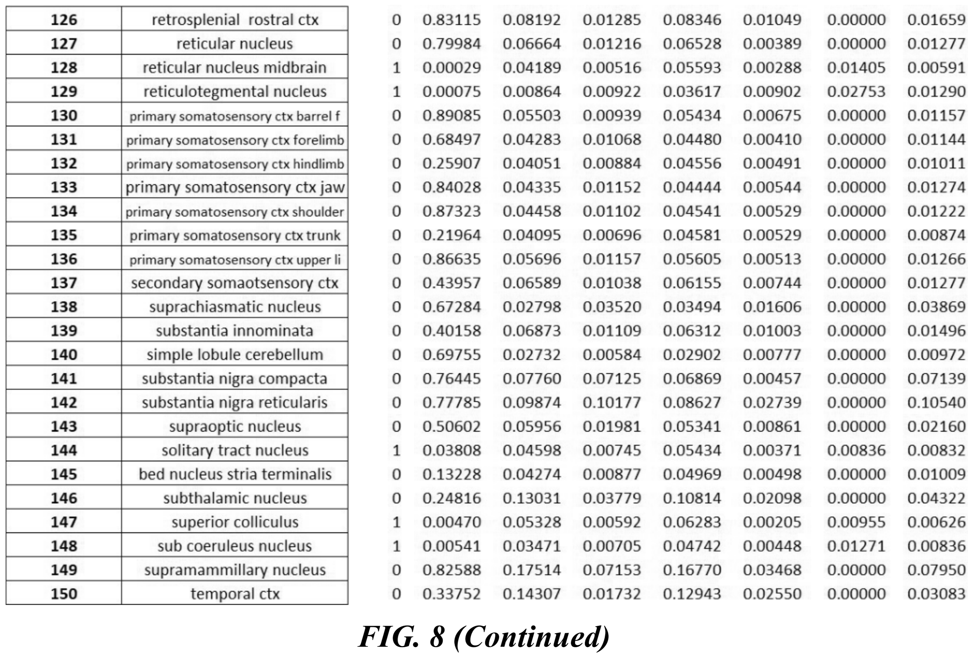

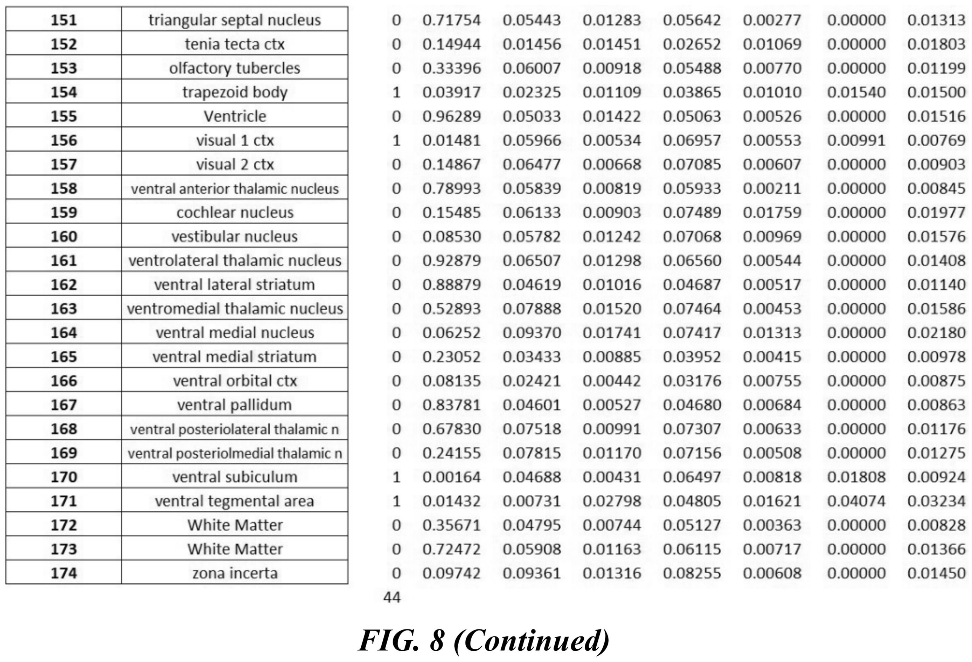

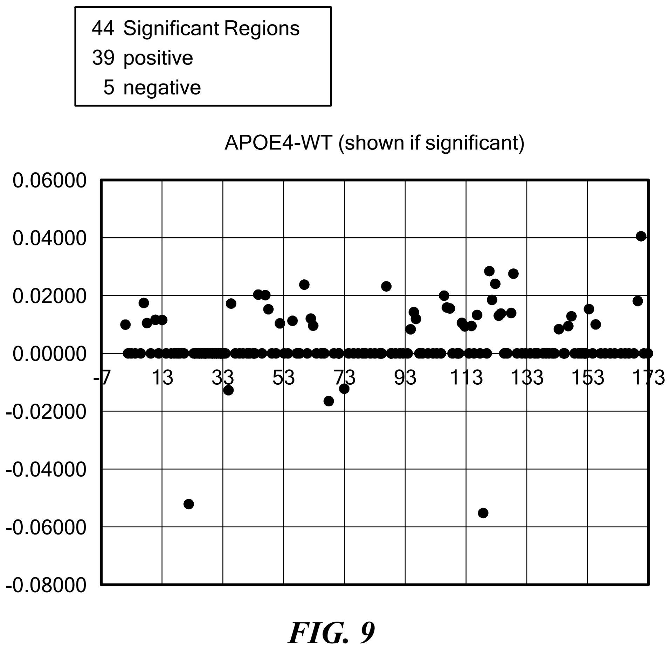

Small Vessels: 44 of 173 regions changed ($P<0.05$), of which 39 showed increases. See Figs. 6-9. A pattern of mean vascular changes was also observed consisting of 11 increasing and 25 decreasing regions (p<0.01).

**Trend towards hypovascularization at 2 years of age**

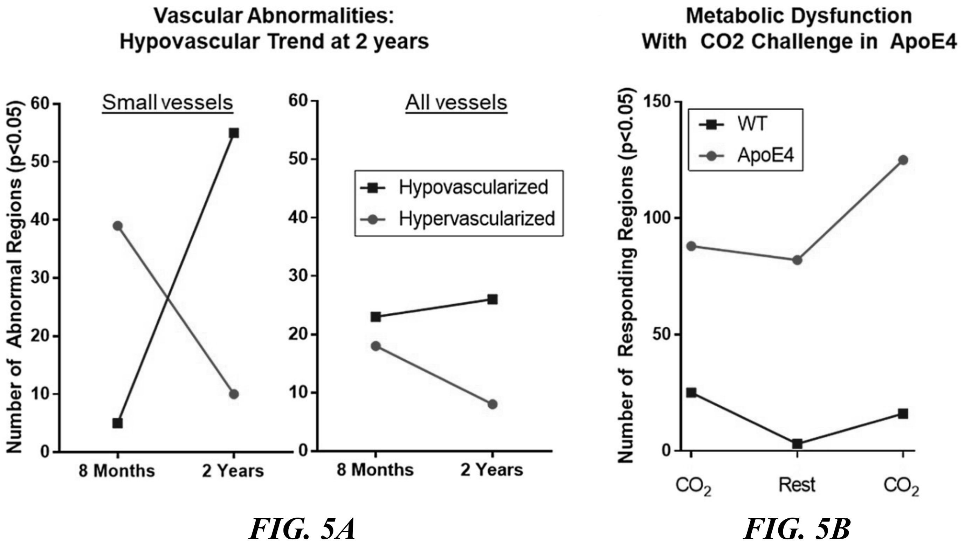

In this model, AD advanced quicker for males than females, and though females did not yet exhibit statistically significant cognitive impairment, static QUTE-CE MRI revealed an age-dependent hyper- to hypo-microvascular remodeling trend (Figs. 5A, 5B). This male/female trend in impairment due to the APOE4 gene has been validated in rodents (www.ncbi.nlm.nih.gov/pmc/articles/PMC4687024/pdf/nihms740272.pdf). The hypermicrovascular trend was found to highly correlate with brain regions exhibiting hyperconnectivity, as measured by Echo-Planar Imaging (EPI). The females were found to exhibit cognitive impairment later at 2 years of age, as measured by the Barnes Maze and Novel Object Recognition tests (p<0.05).

**ApoE4 rats: Hypersensitive and hysteretic vascular dysfunction**

Dynamic QUTE-CE MRI revealed hypersensitive recruitment of the vascular reserve. (Fig. 5B) A very significant response to breathing in 5% $CO_2$ gas was observed for the ApoE4 rats, and unlike the WT, they did not recover during the 1 min rest period. These data could suggest that initial hypervascularization may be a coping mechanism to compensate for metabolic dysfunction in aging and dementia.

Structural differences at 8 m

The tables in Figs. 6-9 list the results regarding the structural differences, both in terms of the mean (p<0.01, top) and the mode (p<0.05, bottom). The P-value is shown, and the mean qCBV along with the STD is available between all animals in the two groups (see Methods, group comparison at 8m of age). The region number is the assigned region number in the anatomical atlas, and the rows have been organized such that the largest qCBV (APOE4 mean or mode - WT mean or mode) is at the top (hypervascularized APOE4). On the right of the bottom table comparing the modes, the region names and their p-values are listed in order of most to least significant. Of the 173 regions, non-significant regions have been omitted.

Structural differences at 2 y

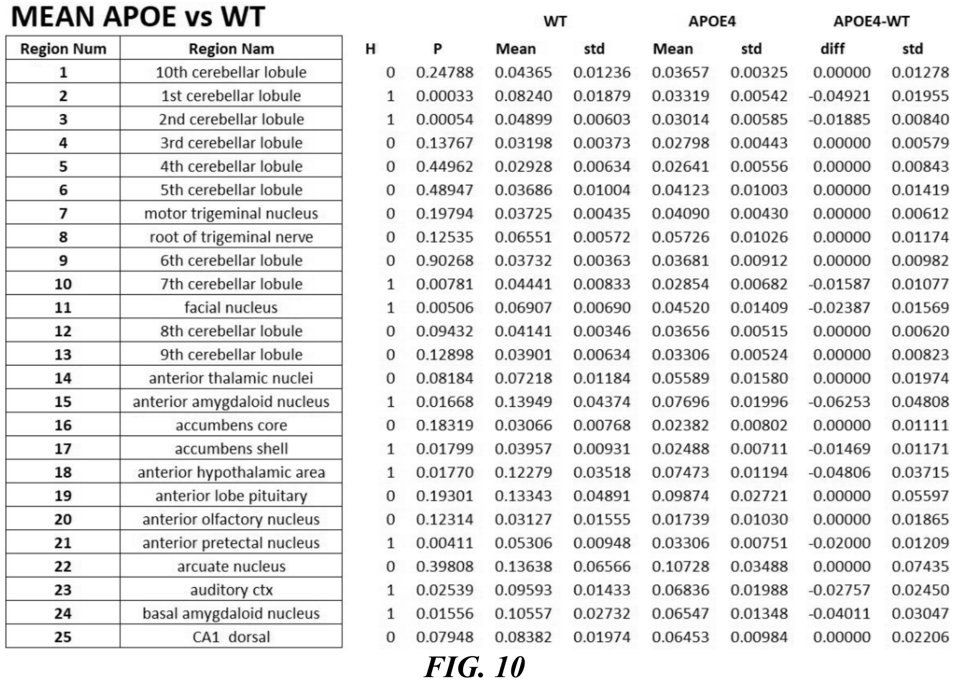

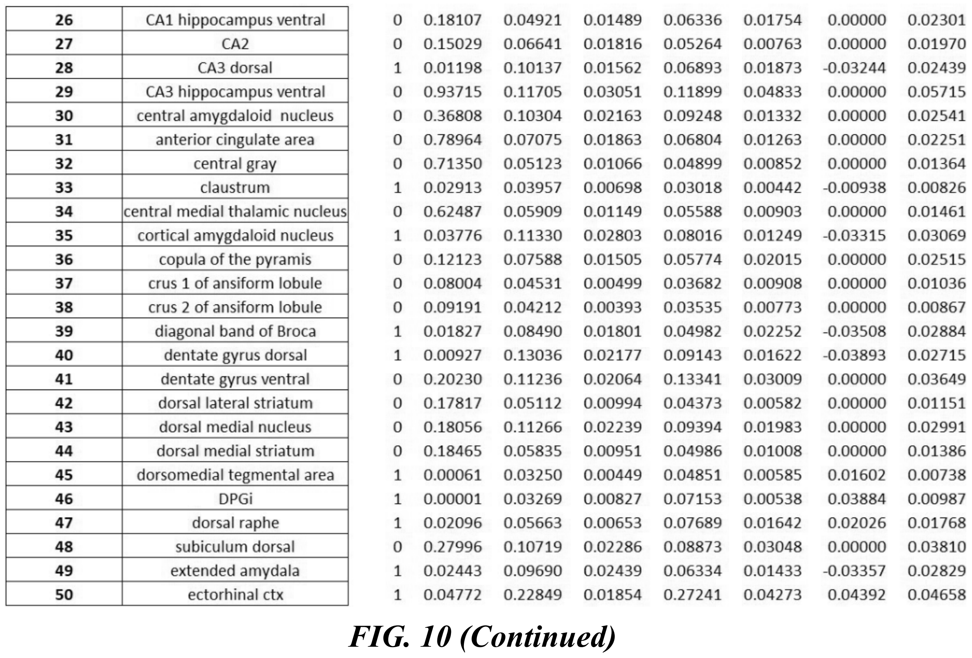

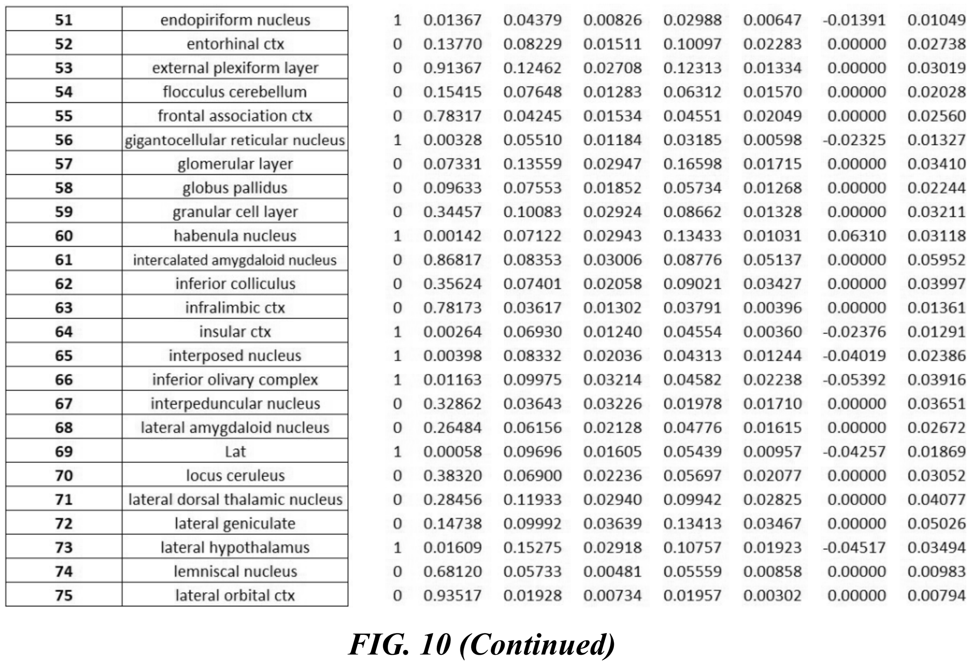

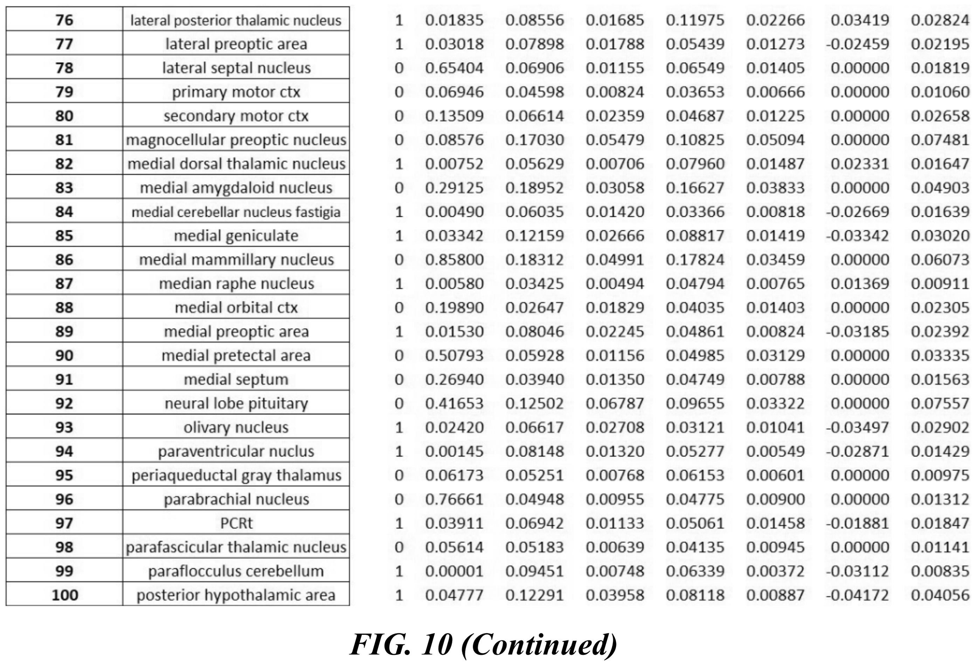

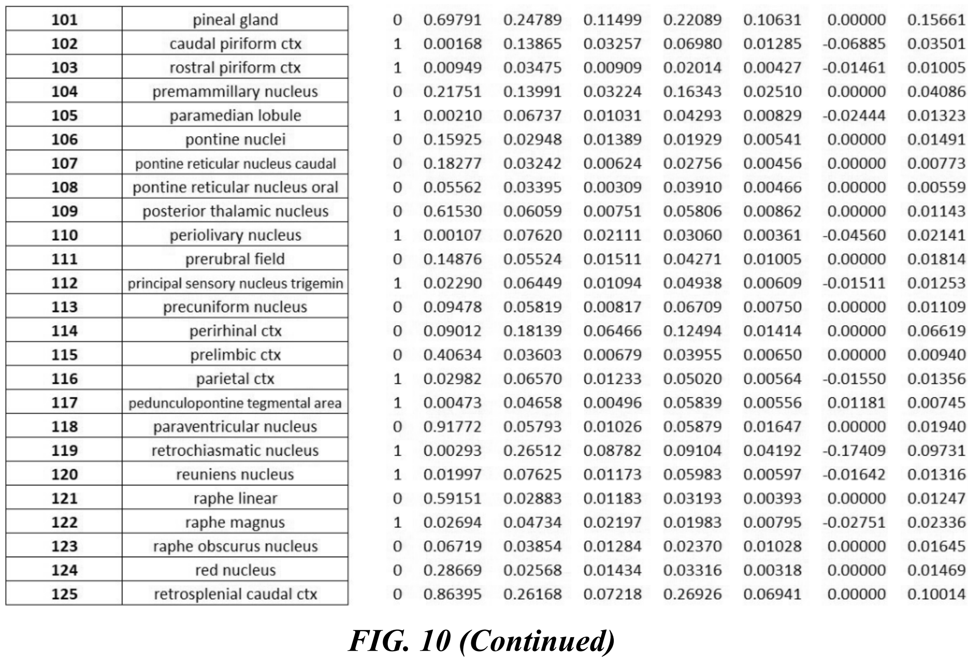

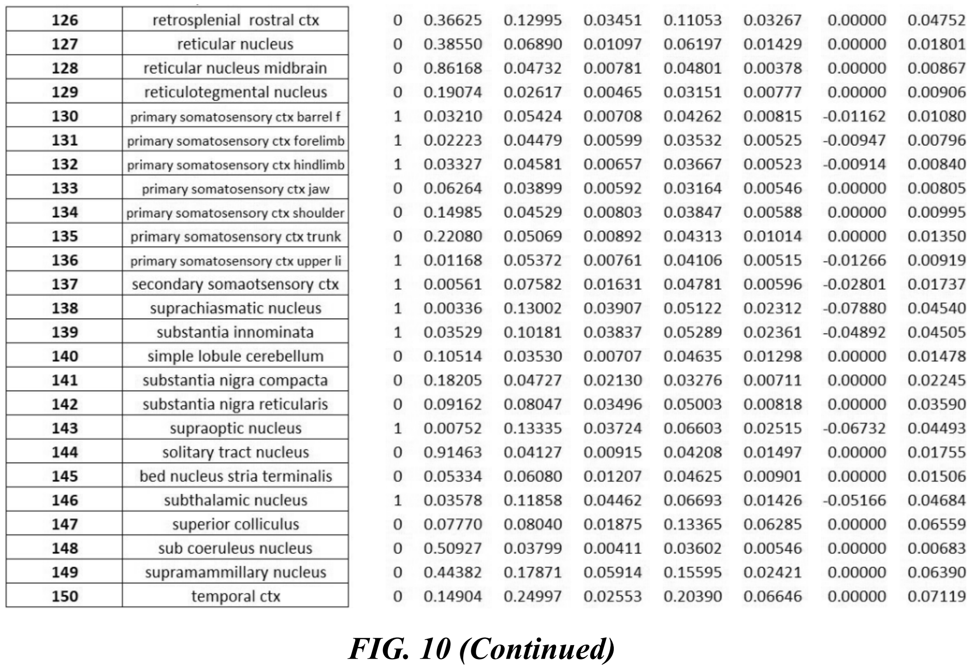

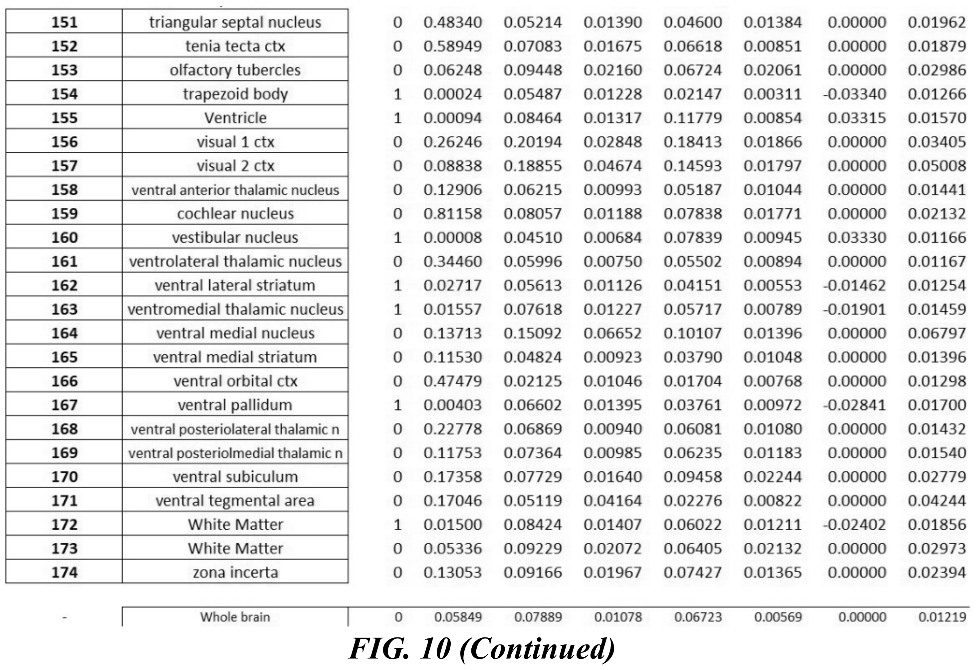

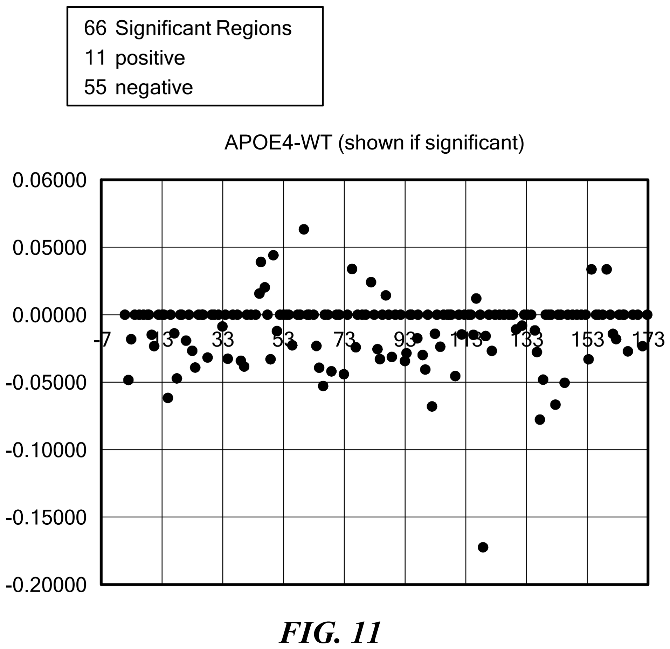

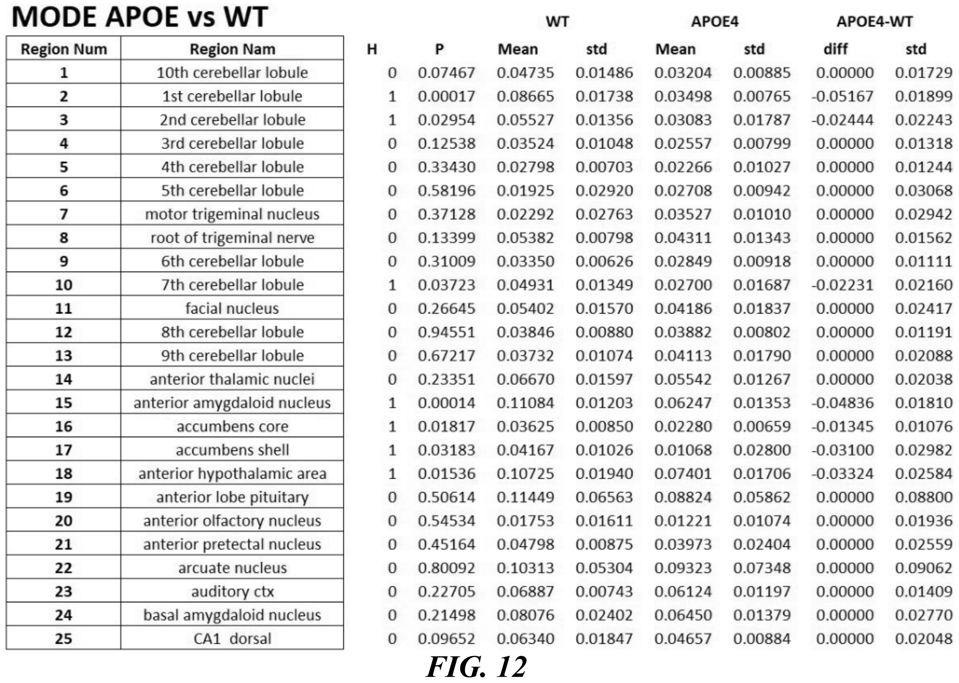

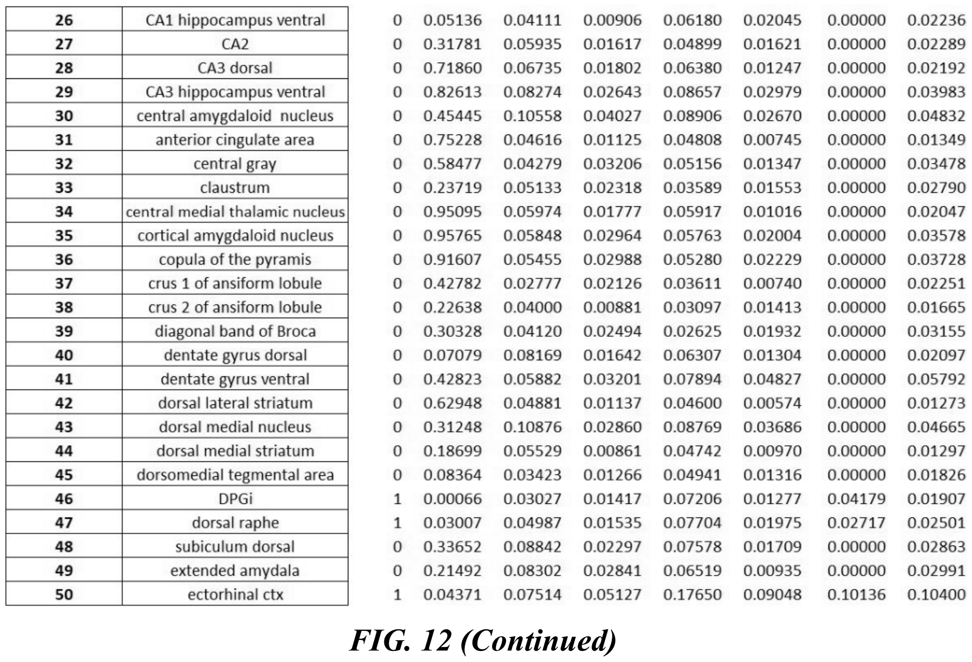

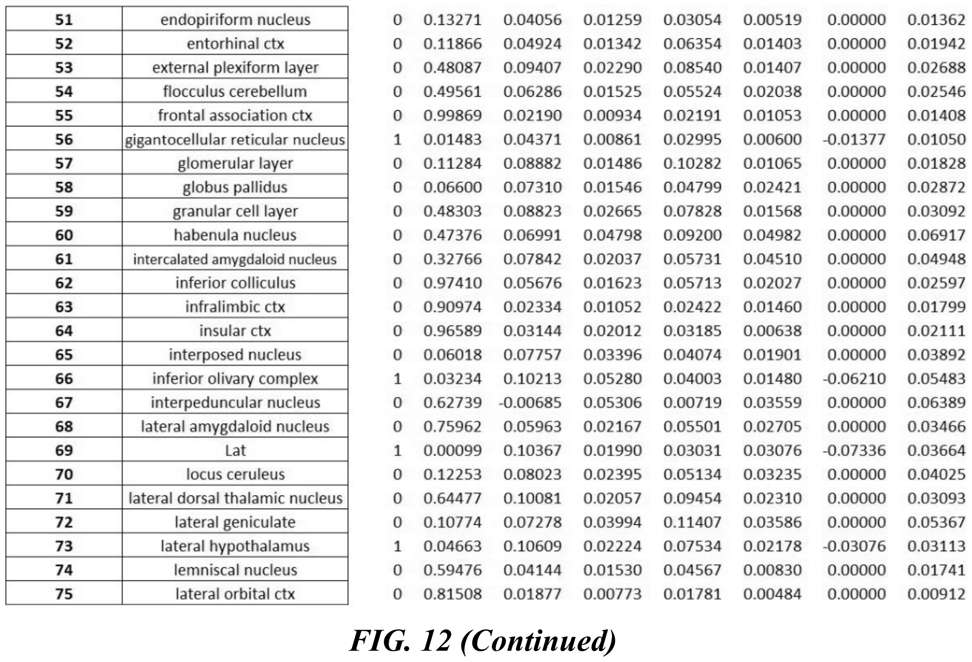

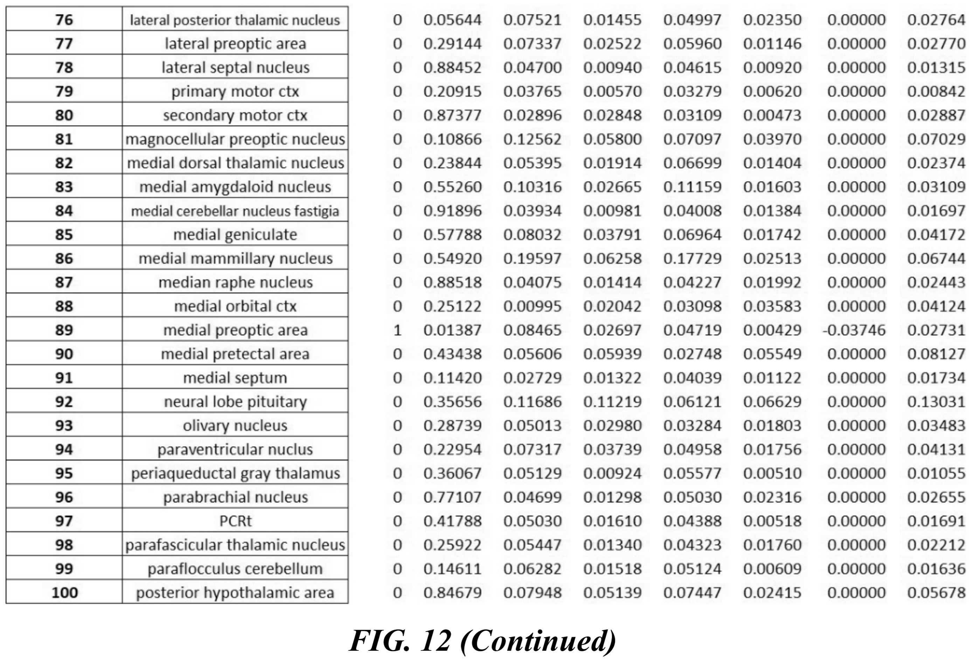

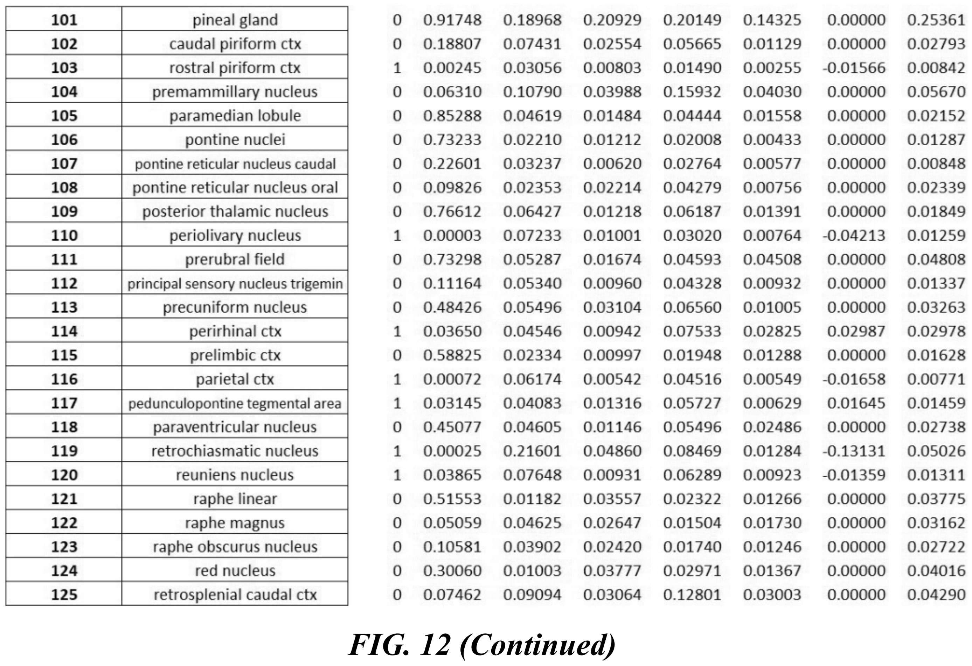

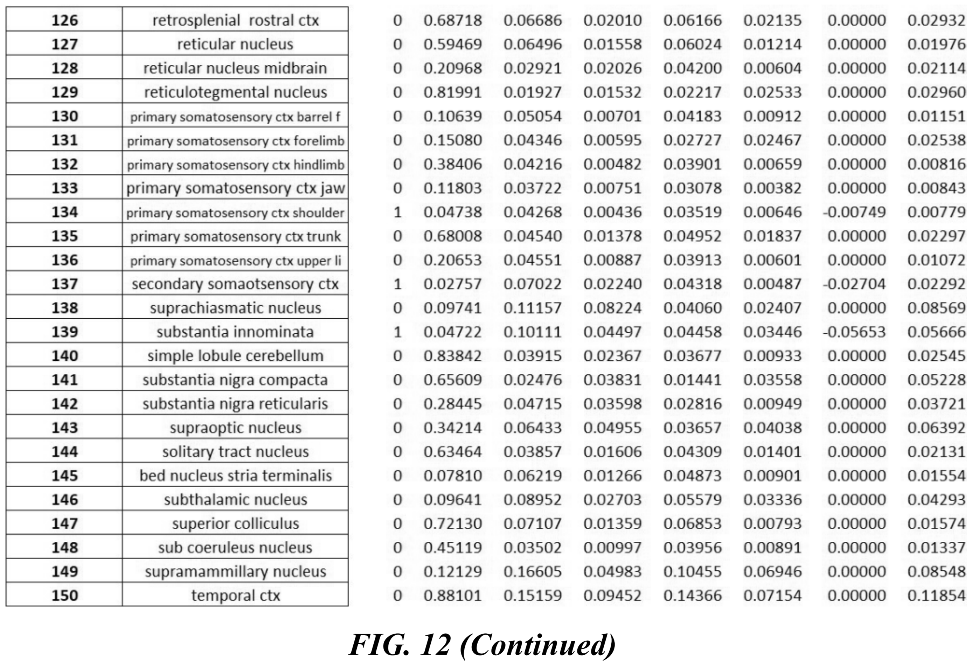

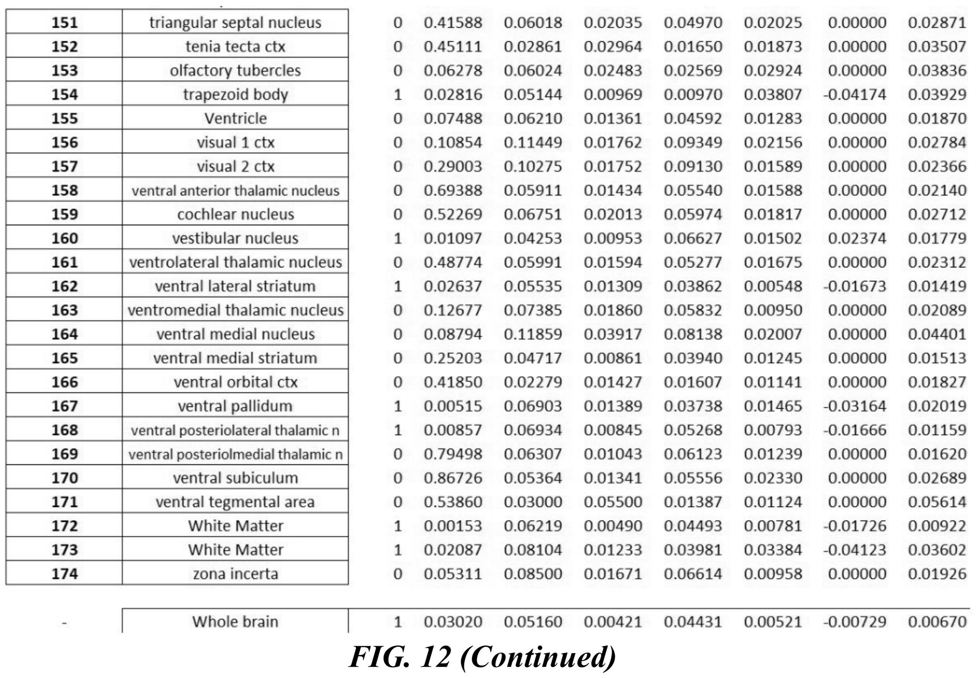

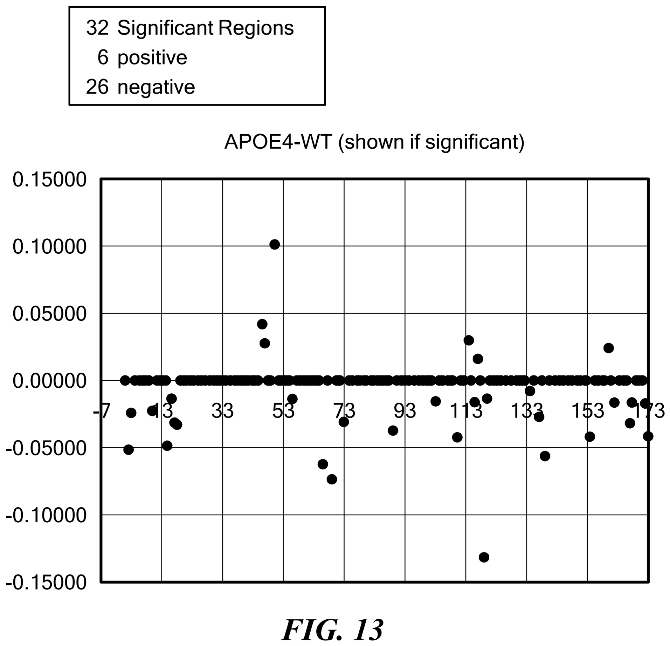

The tables in Figs. 10-13 list the results regarding the structural differences, both in terms of the mean (p<0.01, left) and the mode (p<0.05, right). The H-values and P-values are presented, and the mean qCBV for WT and APOE4 are available along with the STD. Differences were calculated between the two groups and standard deviations of the differences were calculated using error propagation for subtraction of means. The region number is the assigned region number in the anatomical atlas, and the rows have been organized such that the highest decrease in is at the top (hypovascularized APOE4) and so on. The regions have been colored dark gray and darker gray if those same regions were also statistically significant for change in the 8m-old rats. If they were hypervascularized, the color on the left representing the 8m state is dark grey (red in original); if hypovascularized it is darker gray (blue in original). The color to the right corresponds to the hypo- or hyper-vascularization of the 2y-old state.
For the mean, it can be observed that of the 66 regions that are statistically significant for change at 2y, 18 were also significant at 8m-old. Thirteen of those regions were hypovascularized and stayed hypovascularized, three were hypervascularized and stayed hypervascularized, two were hypervascularized and went hypovascularized, but none went from hypo- to hyper-vascularization.
For the mode, it can be observed that of the 32 regions that are statistically significant for change at 2y, six were also significant at 8m-old. Two of those regions were hypovascularized and stayed hypovascularized, two were hypervascularized and stayed hypervascularized, two were hypervascularized and went hypovascularized, but none went from hypo- to hyper-vascularization.

Dynamic $CO_2$ at 2Y

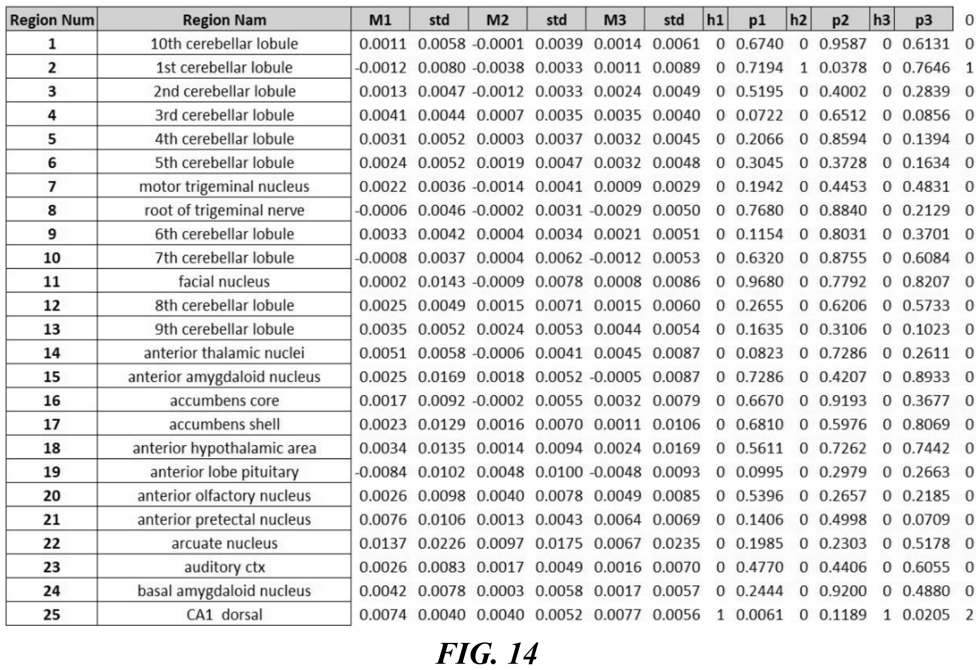

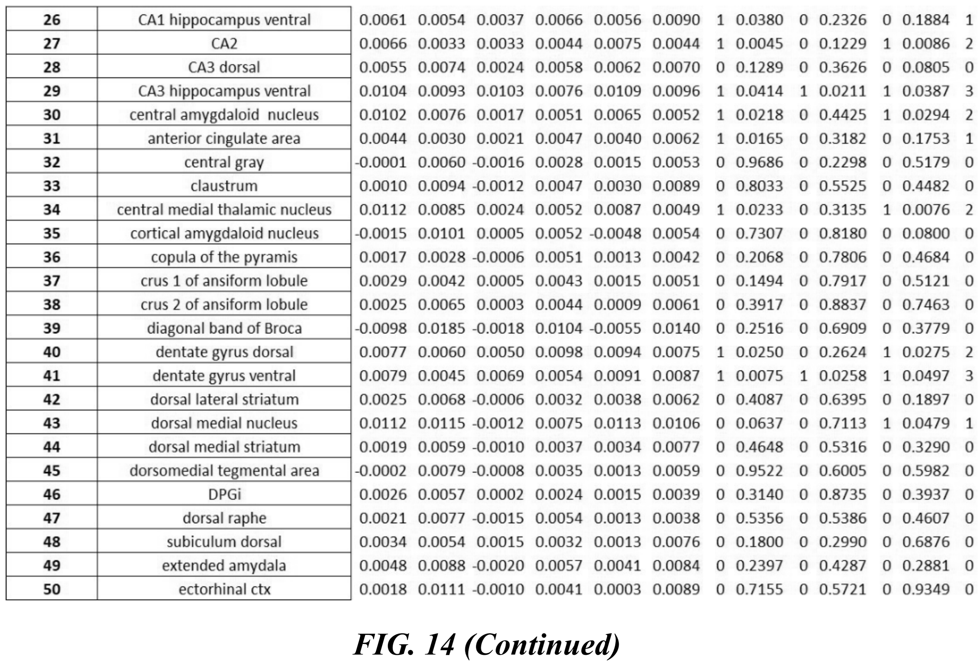

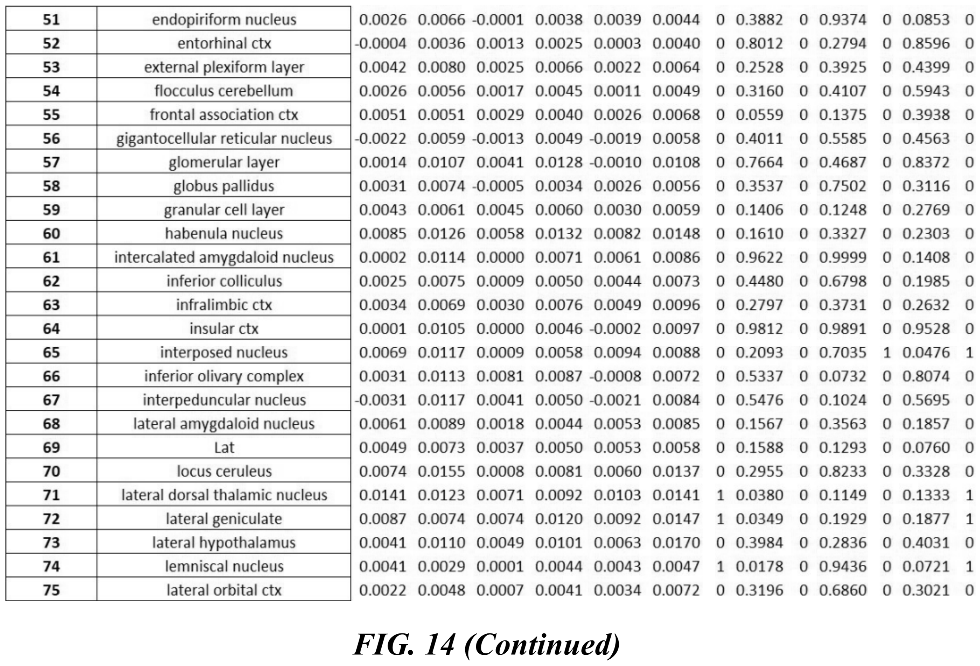

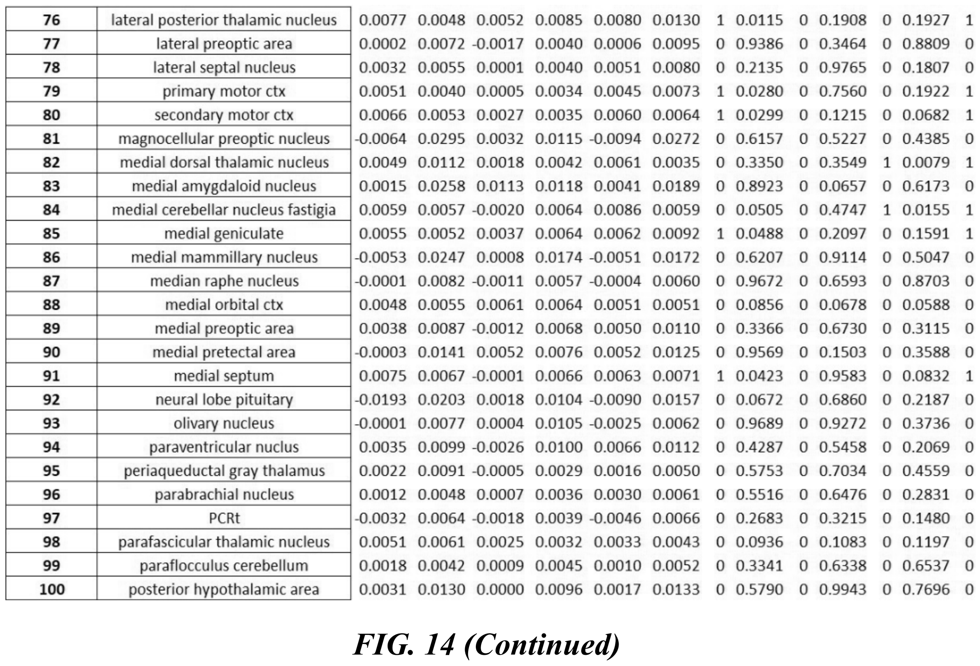

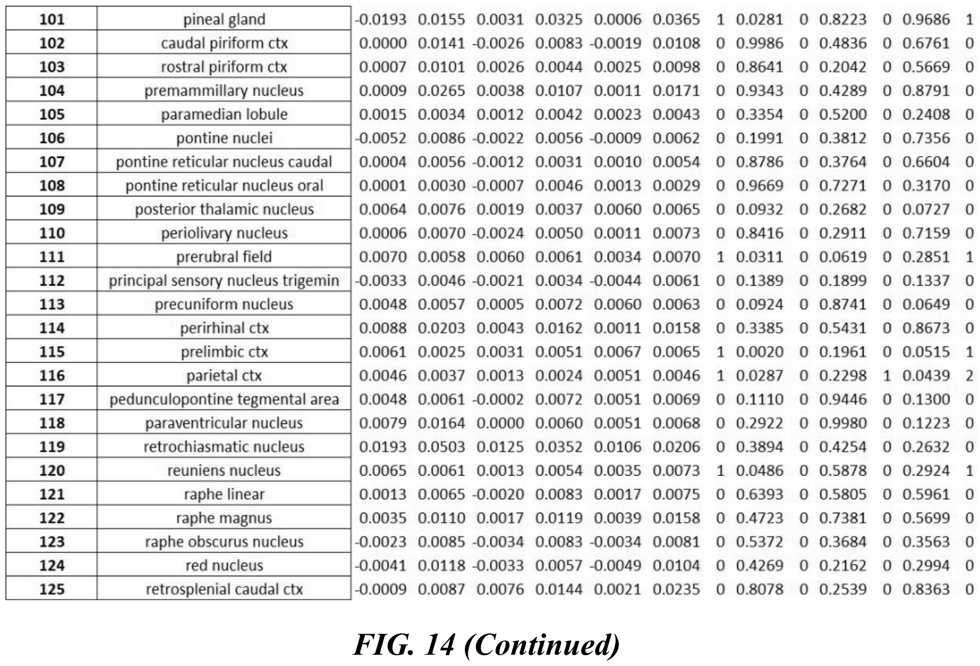

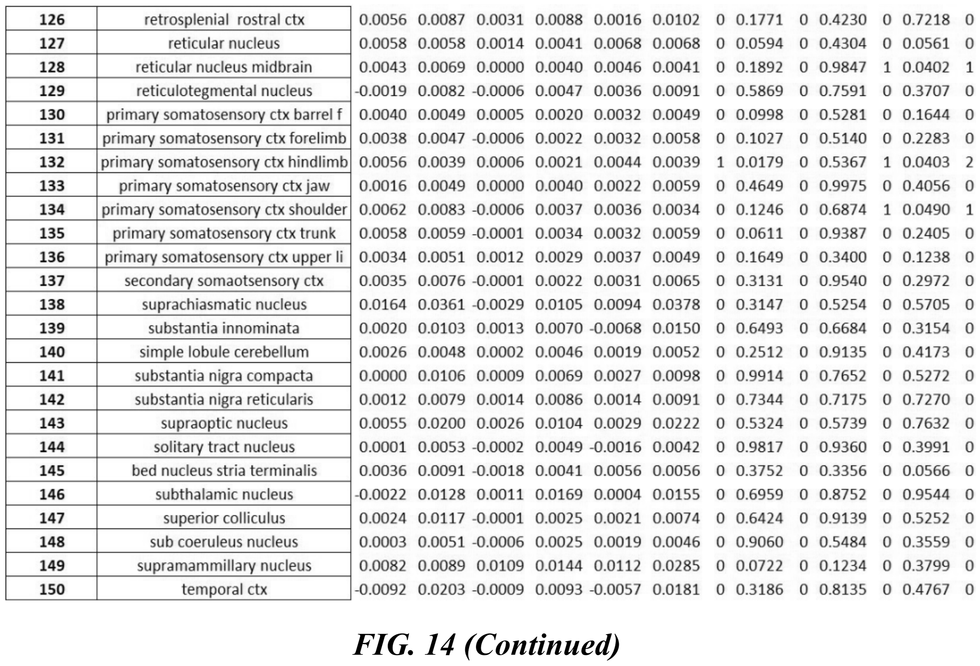

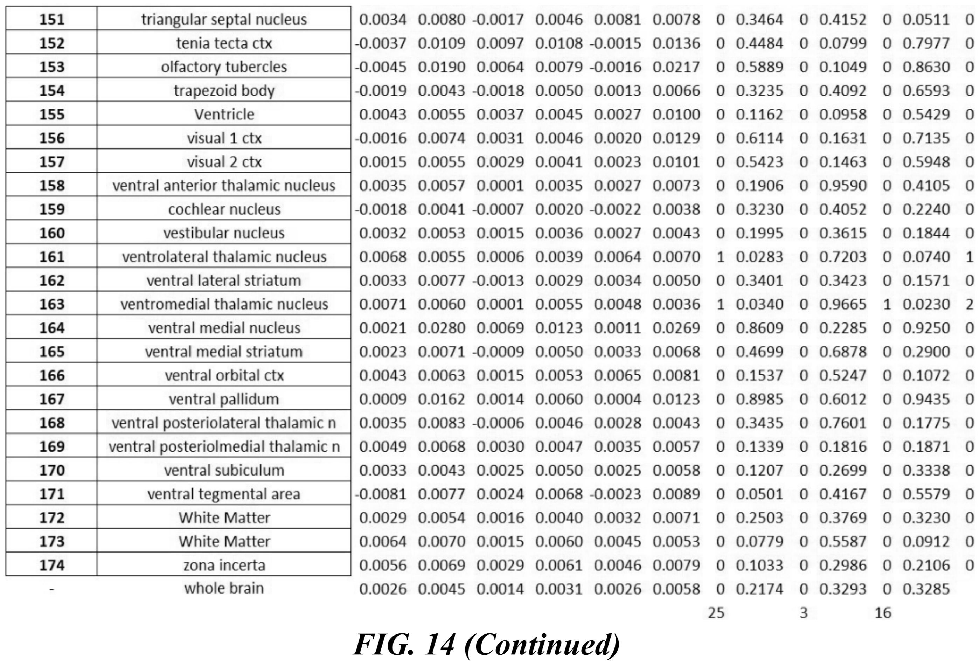

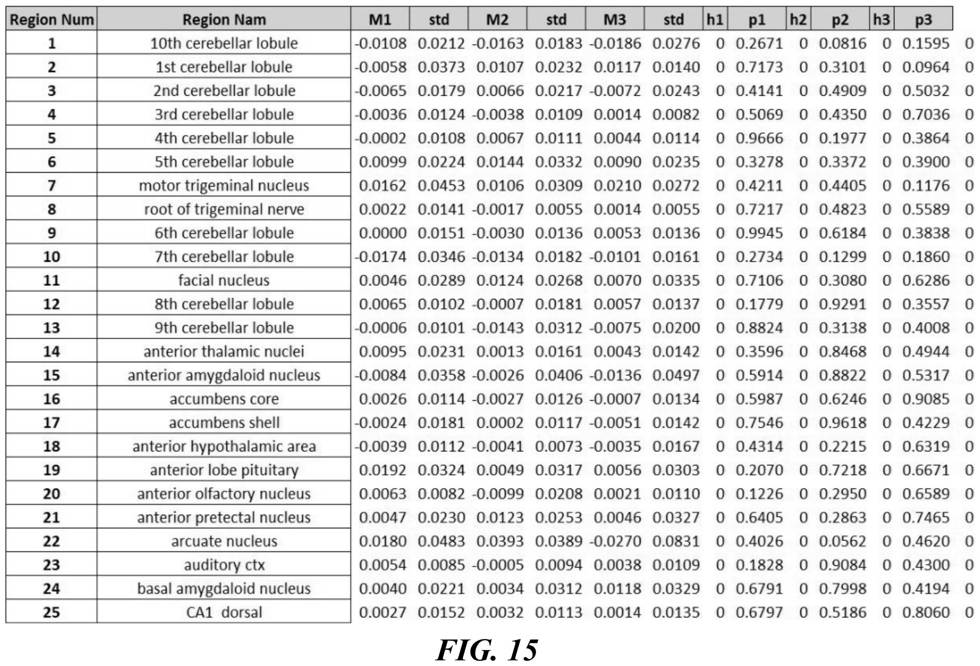

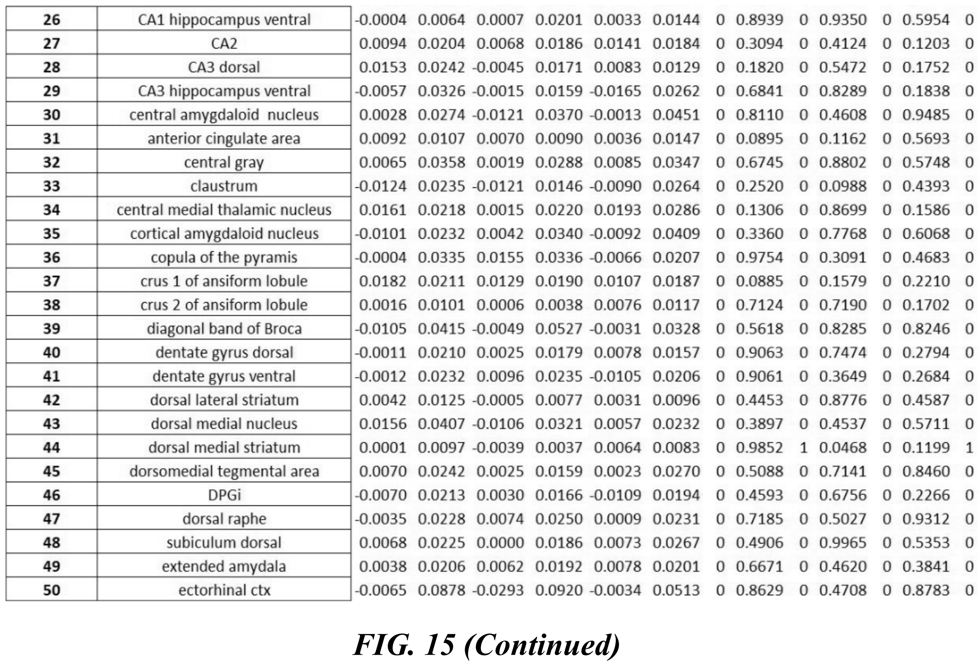

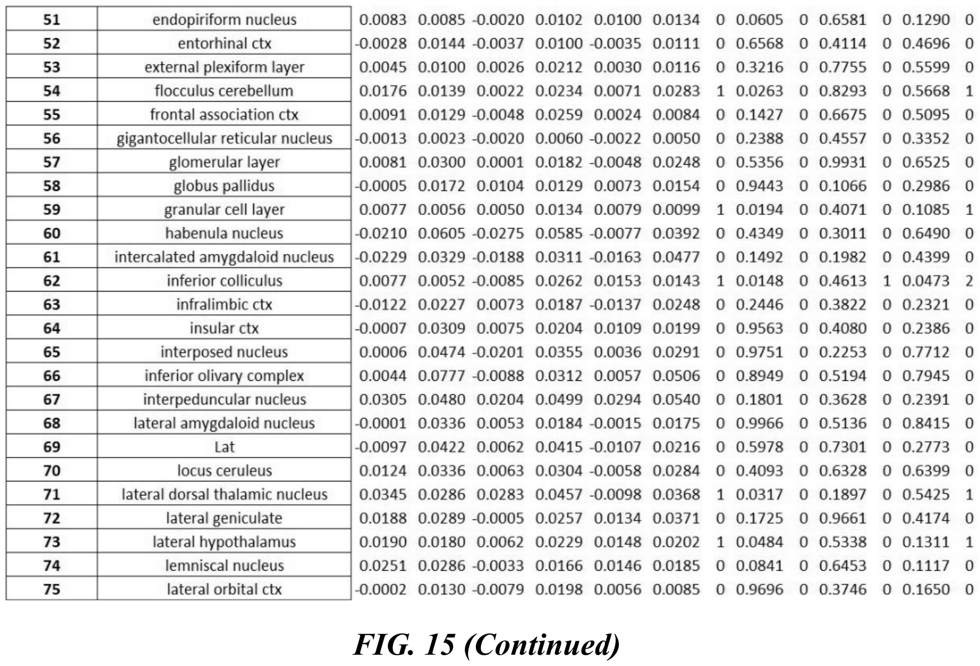

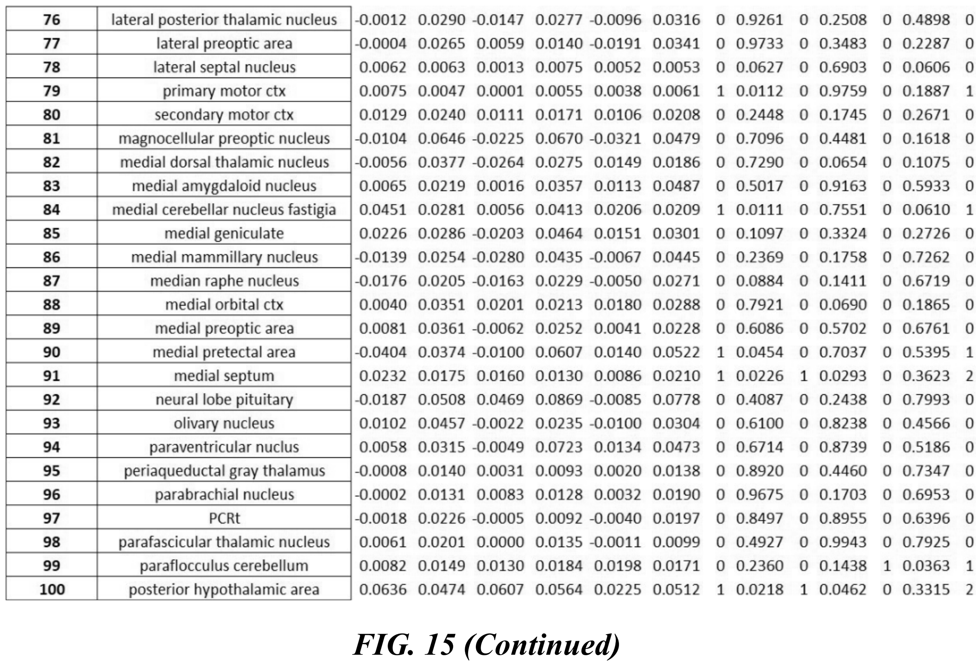

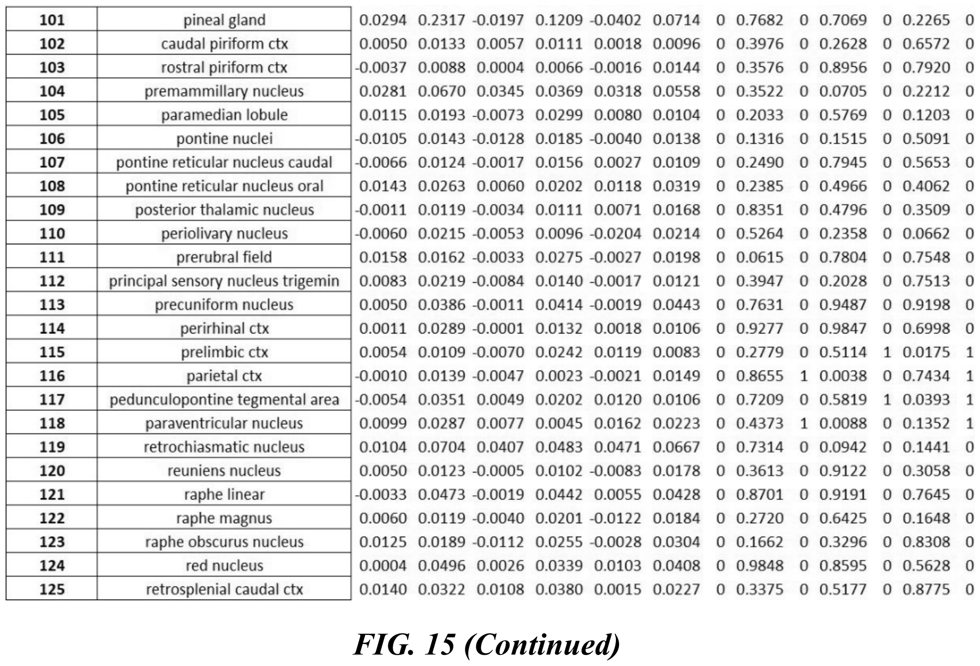

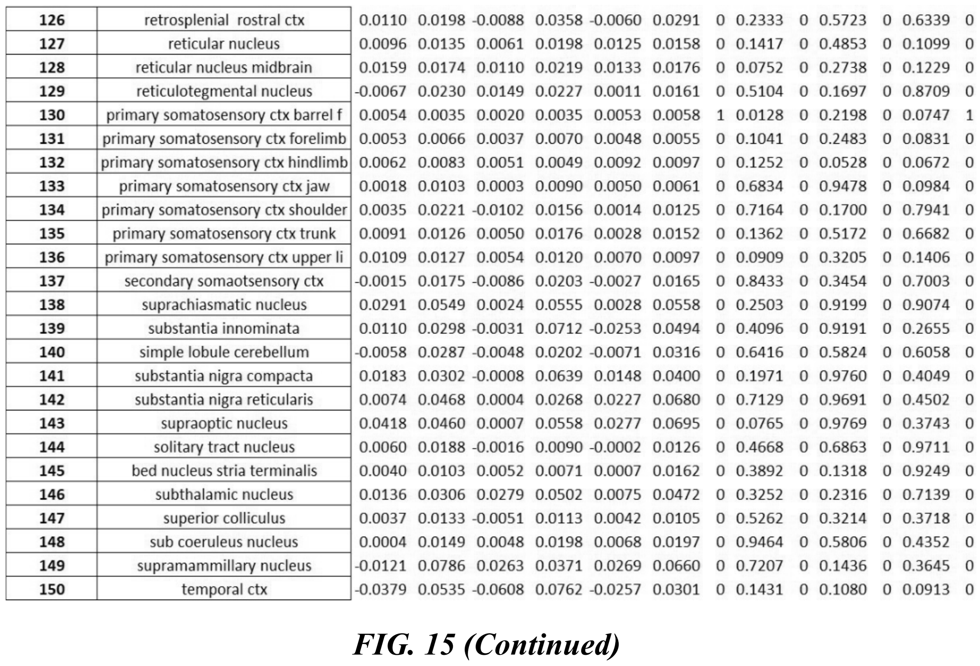

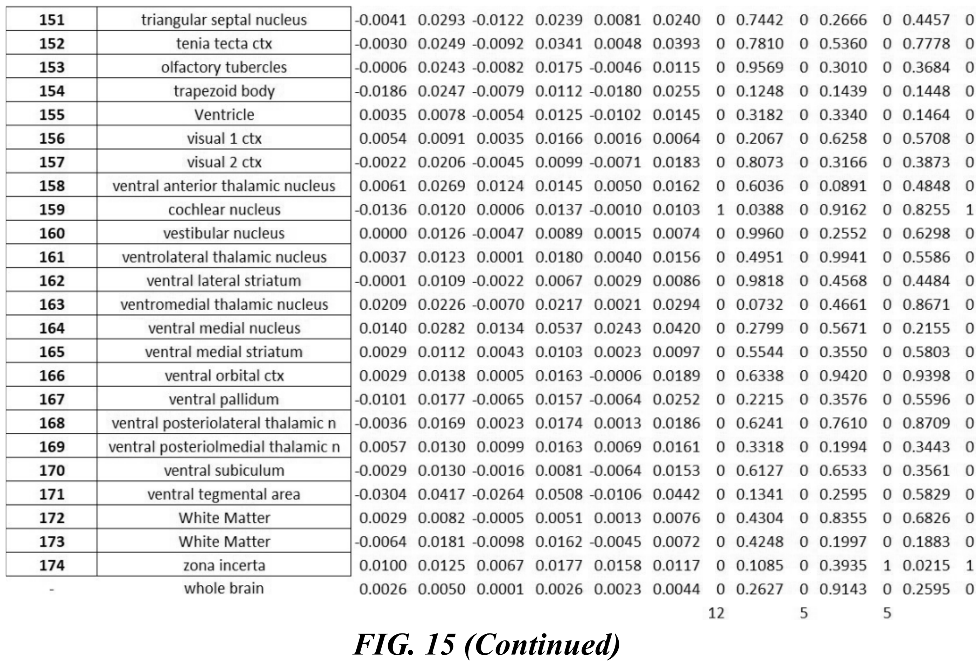

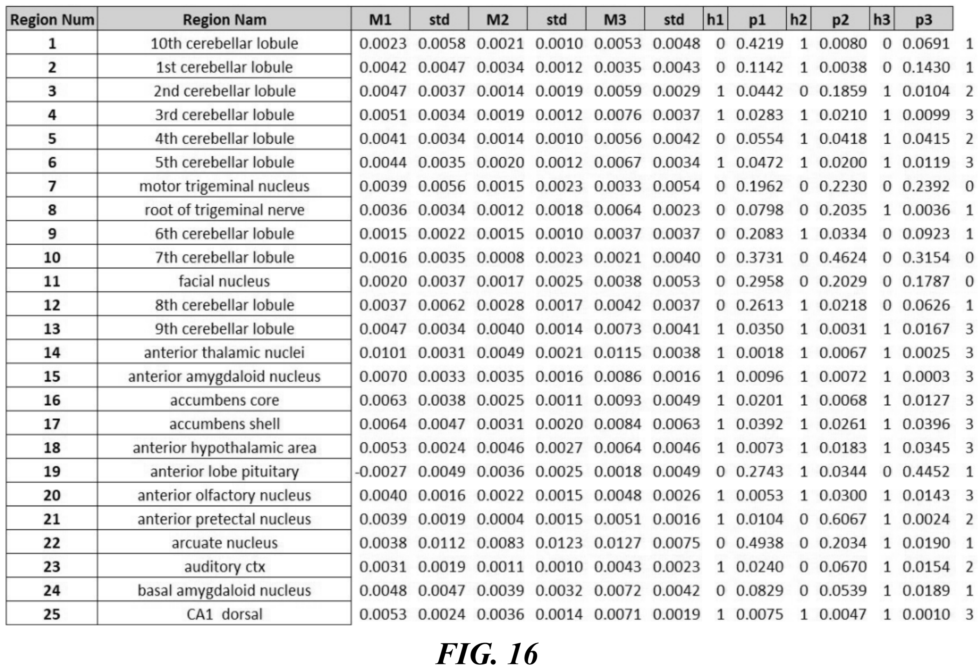

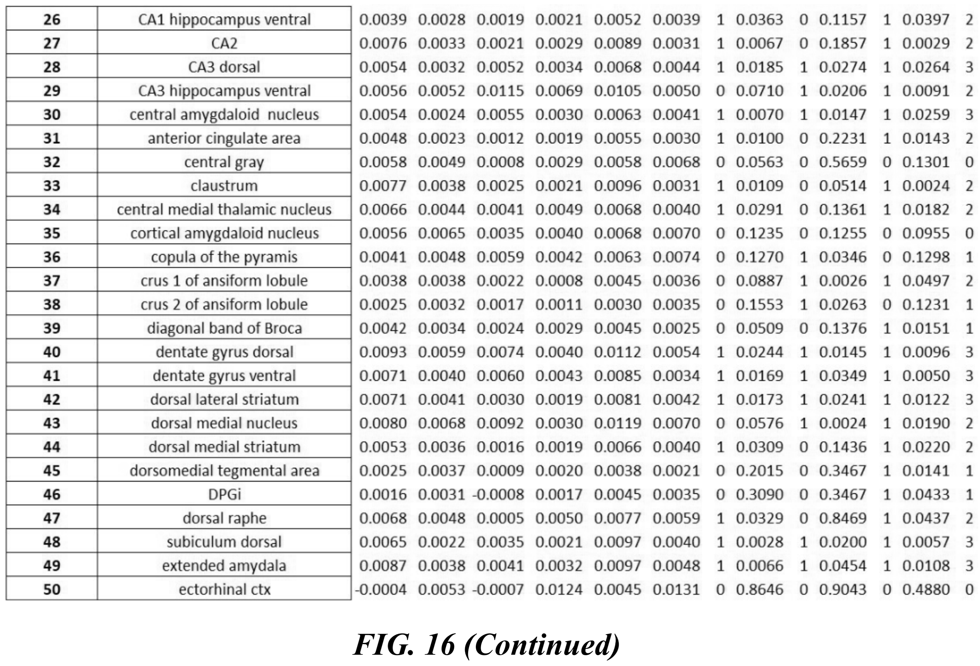

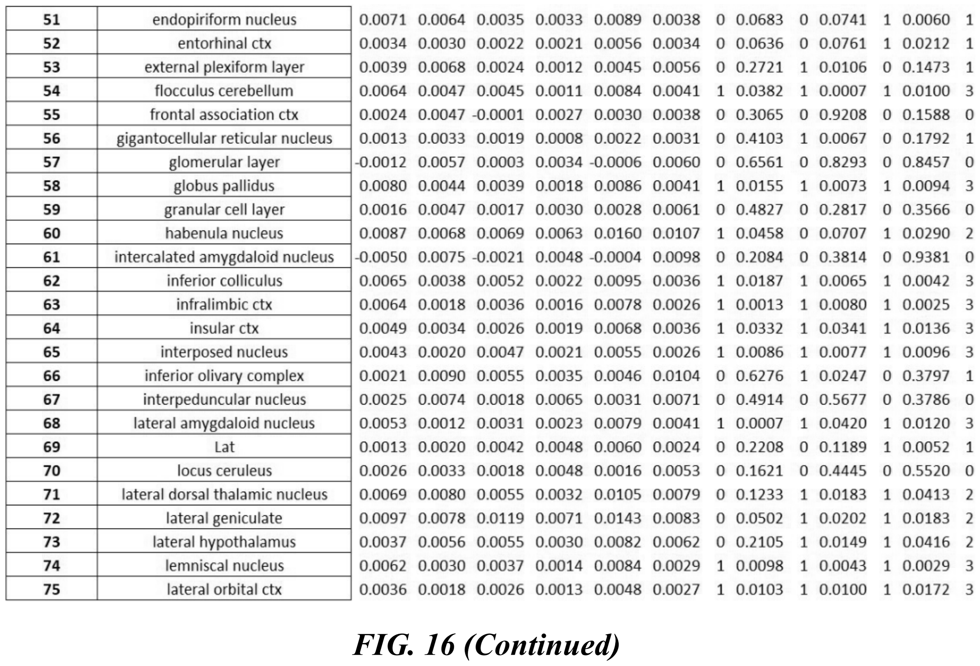

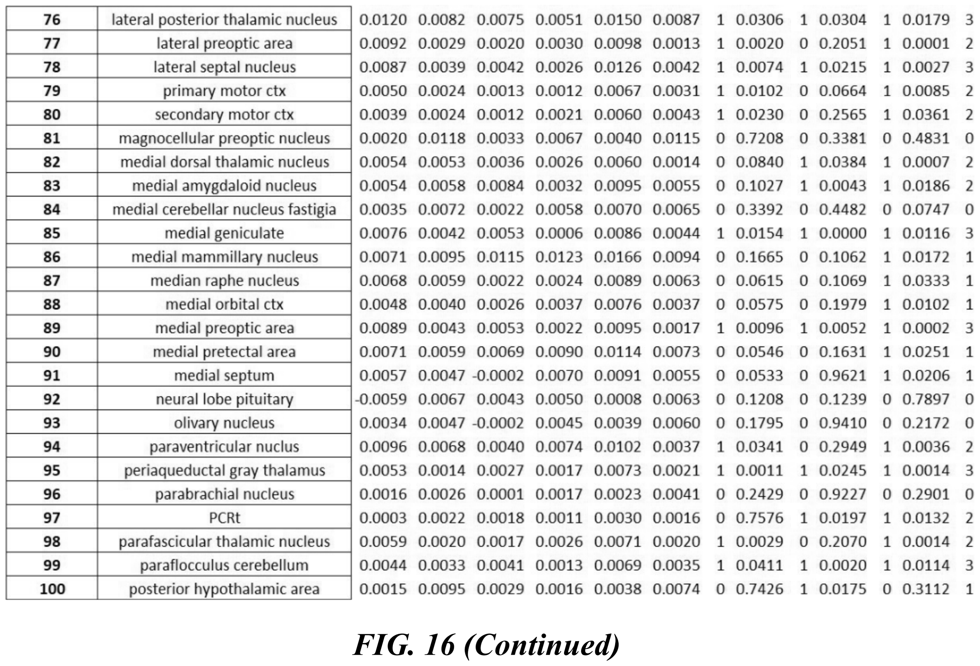

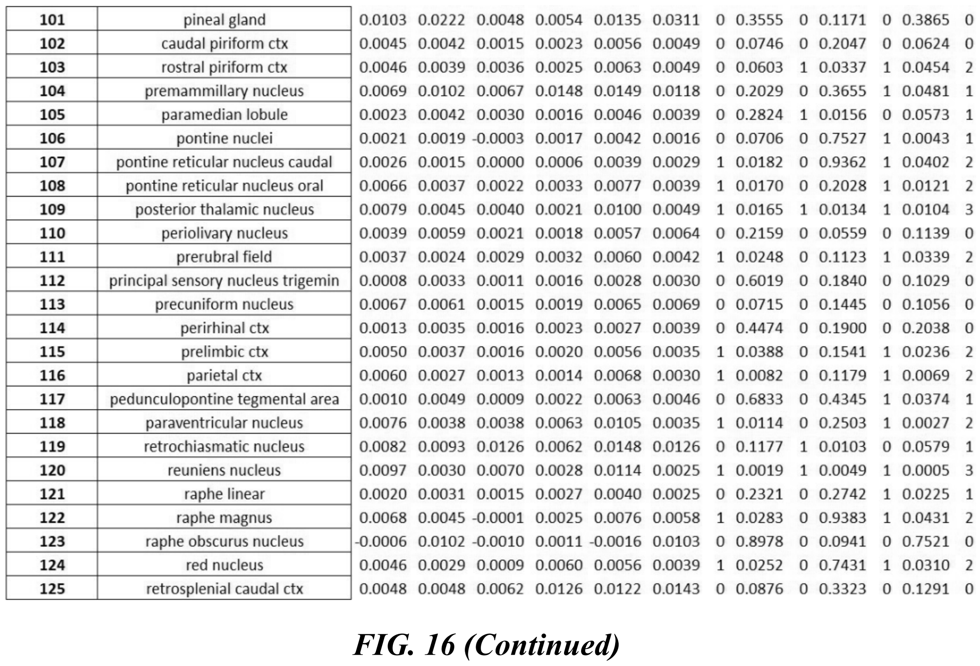

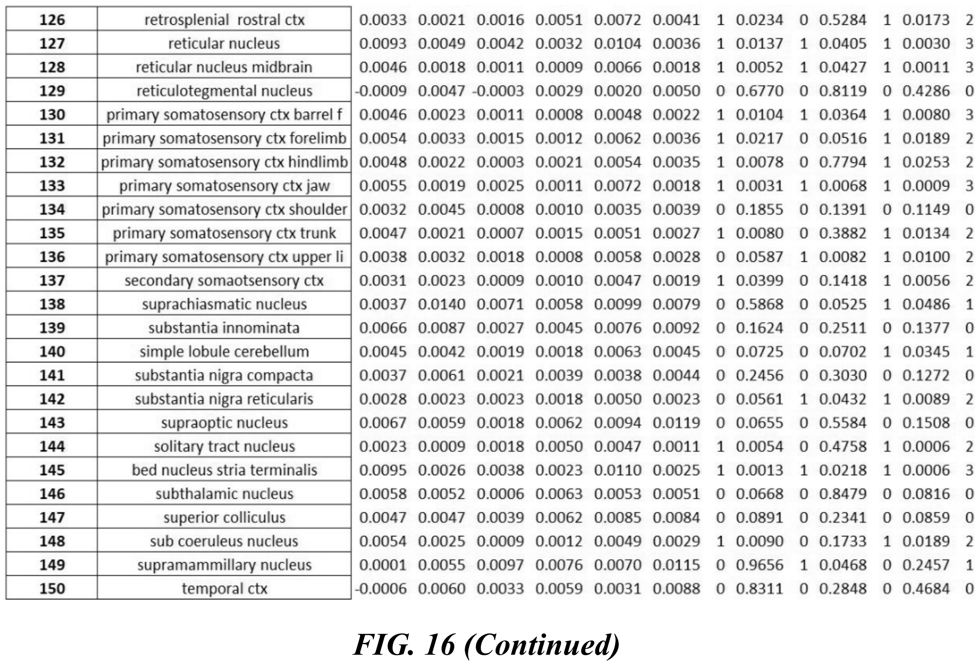

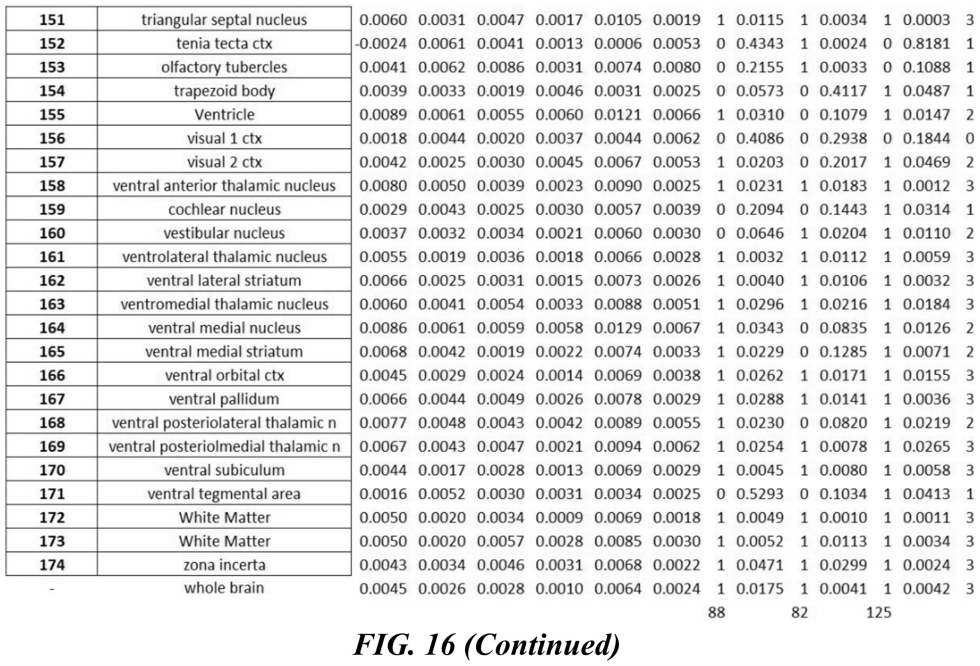

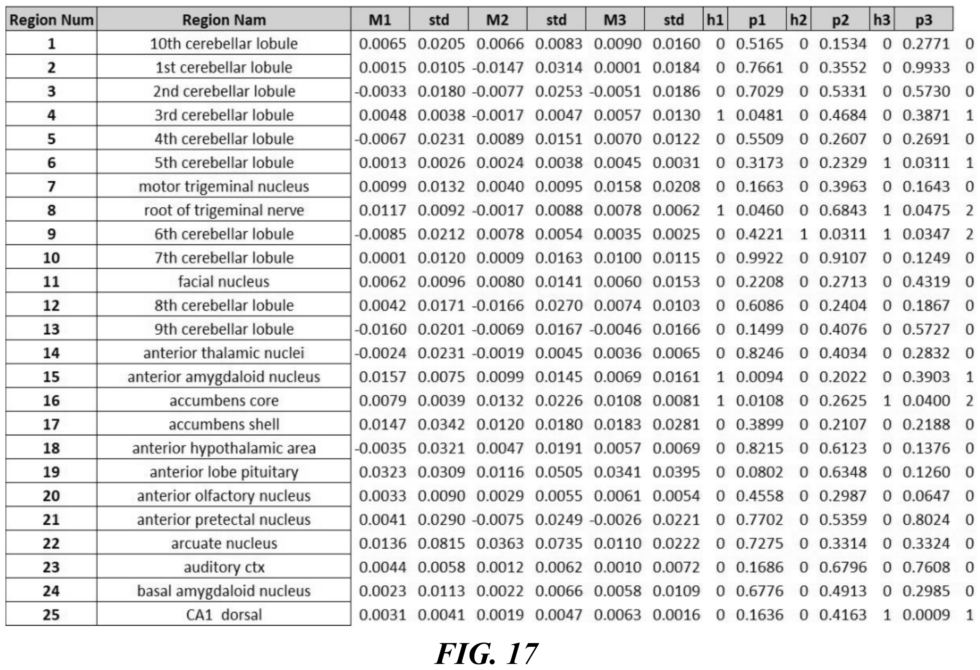

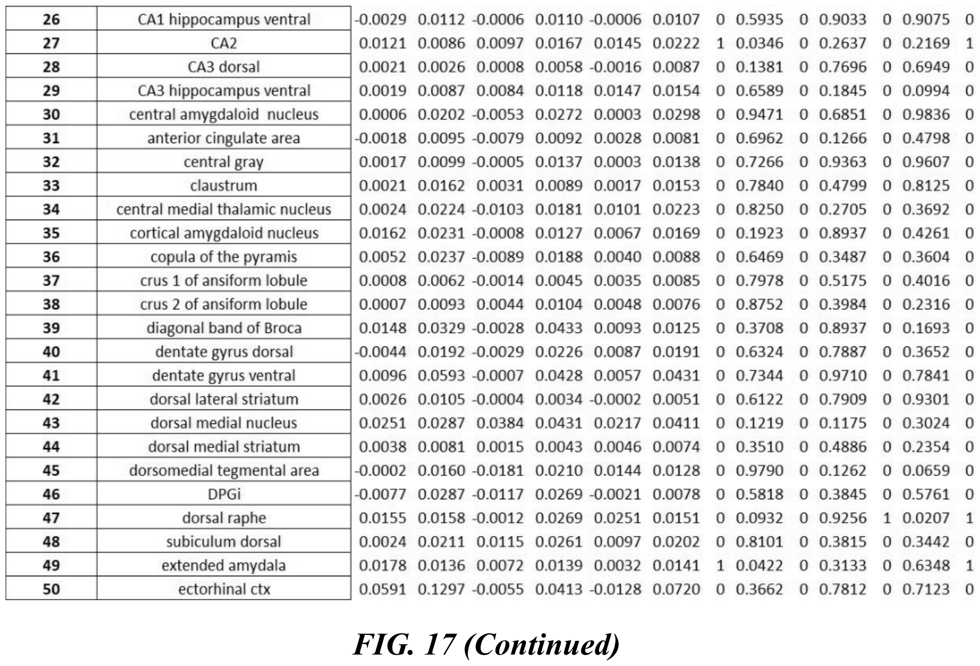

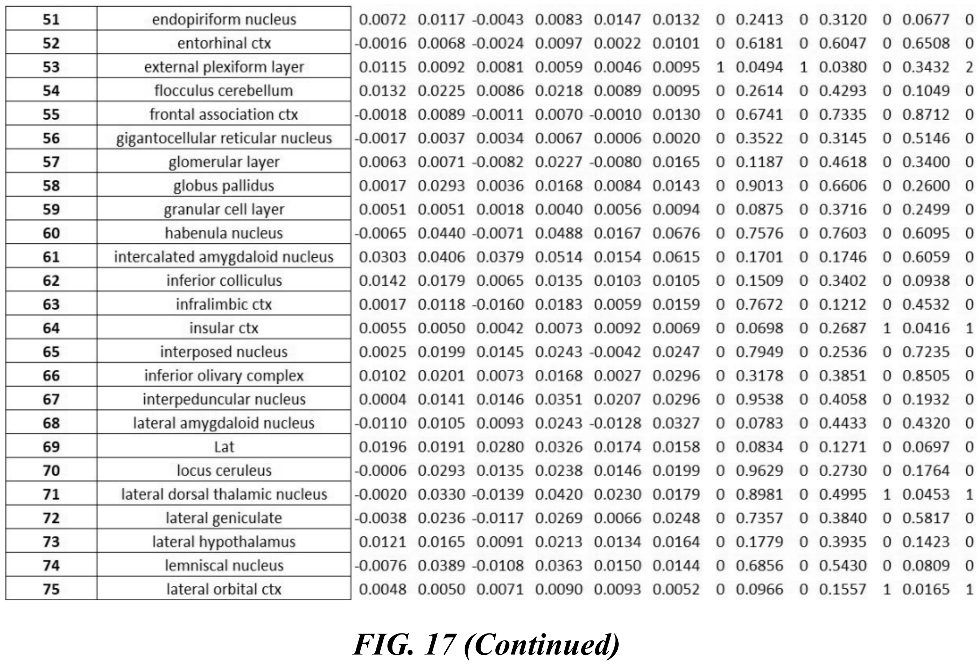

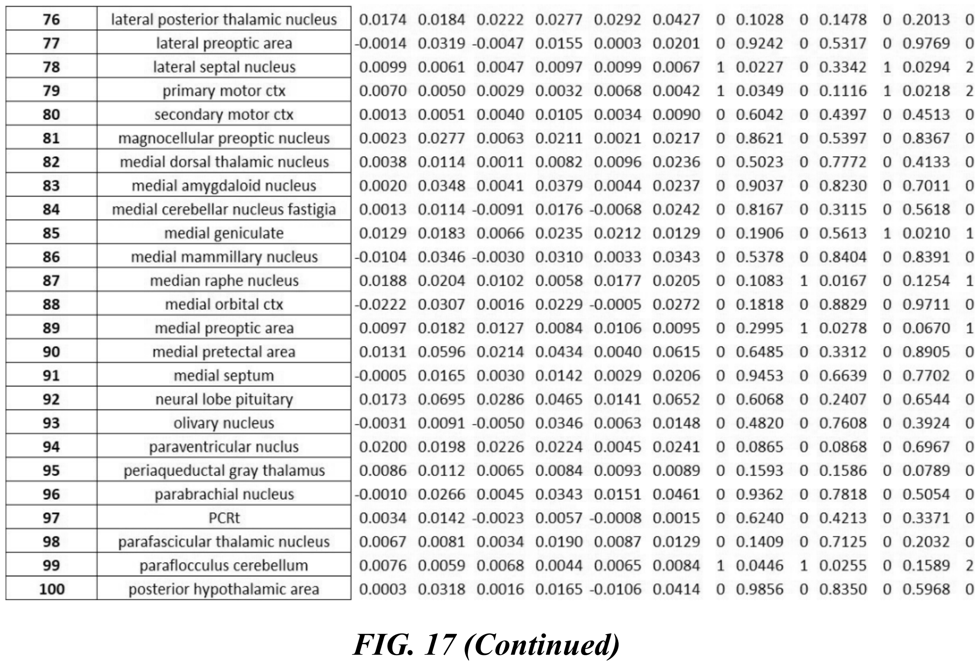

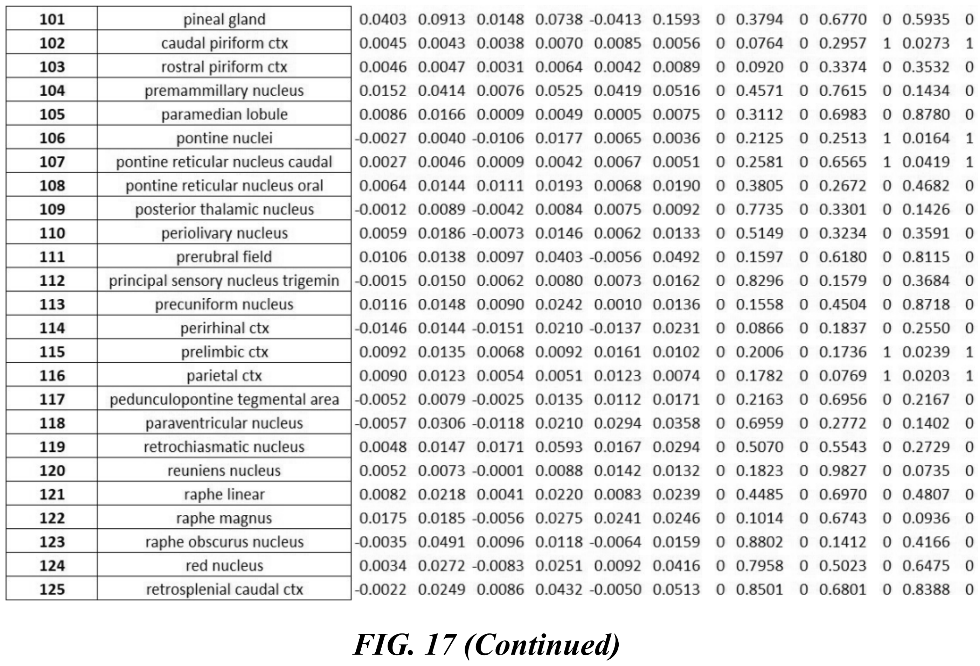

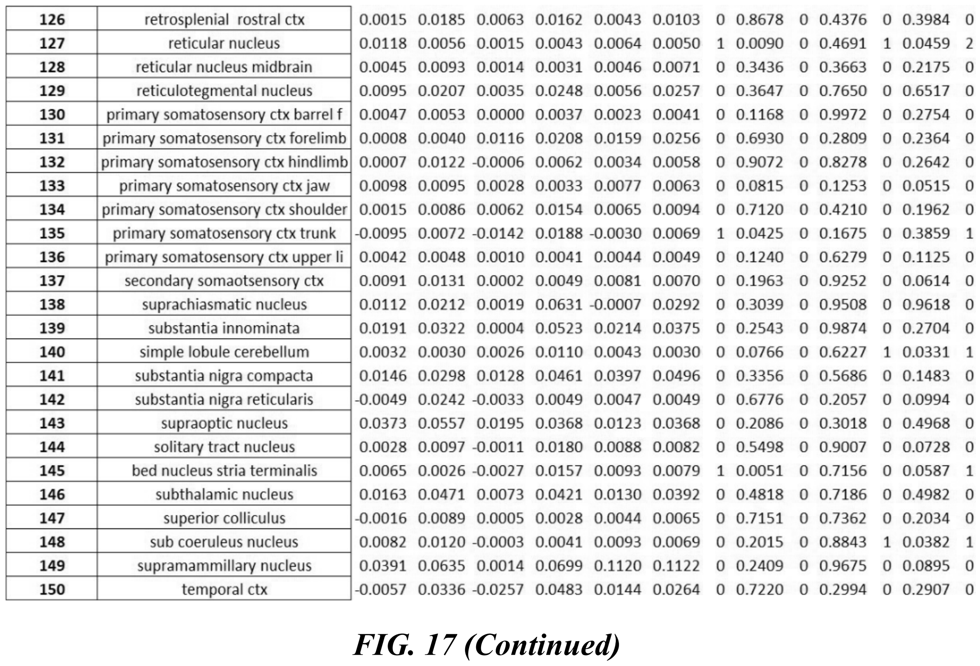

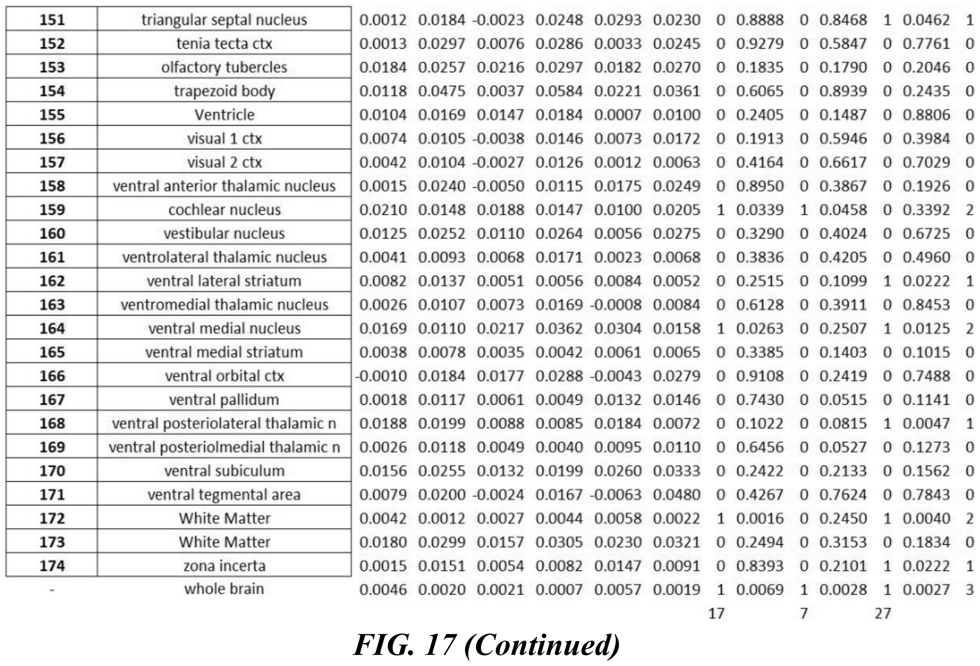

The tables in Figs. 14-17 list the results for the two groups that were tested at 2y of age. The tables list the mean and mode differences in the $CO_2$ challenge states. That means that M1 and its STD represent the mean of the differences that each animal had per region. By following the mean of the intra-animal differences rather than the absolute values, statistical significance can be tested while neglecting inter-animal comparison.
Everything was compared to the first post-contrast scan in which the qCBV is calculated (scan 1). Scans 2-4 were performed with $CO_2$ on (M1 is the mean difference), with $CO_2$ back off (M2 is the mean difference), and finally with $CO_2$ toggled back on (M3 is the mean difference). The h- and p-values are also presented. It is noted that the comparisons h1, p1 correspond to the first $CO_2$ state; h2, p2 is when the $CO_2$ is toggled back off; h3, p3 is when the $CO_2$ is toggled back on.

As used herein, "consisting essentially of" allows the inclusion of materials or steps that do not materially affect the basic and novel characteristics of the claim. Any recitation herein of the term "comprising," particularly in a description of components of a composition or in a description of elements of a device, can be exchanged with "consisting essentially of" or "consisting of".

While the present technology has been described in conjunction with certain preferred embodiments, one of ordinary skill, after reading the foregoing specification, will be able to effect various changes, substitutions of equivalents, and other alterations to the compositions and methods set forth herein.

**References**

Alzheimer's Association. 2018 Alzheimer's Disease Facts and Figures. Alzheimers Dement. 14(3):367-429 (2018).

Assini FL, Duzzioni M, Takahashi RN. Object location memory in mice: Pharmacological validation and further evidence of hippocampal CA1 participation. Behav Brain Res. 2009;204(1):206-211. doi:10.1016/j.bbr.2009.06.005

Barbier EL, Lamalle L, Décorps M. Methodology of brain perfusion imaging. J Magn Reson Imaging. 2001;13(4):496-520. doi:10.1002/jmri.1073

Bremerich J, Bilecen D, Reimer P. MR angiography with blood pool contrast agents. Eur Radiol. 2007;17(12):3017-3024. doi:10.1007/s00330-007-0712-0

Brunser AM, Hoppe A, Illanes S, et al. Accuracy of diffusion-weighted imaging in the diagnosis of stroke in patients with suspected cerebral infarct. Stroke. 2013. doi:10.1161/STROKEAHA.111.000527

Chen, C.-C. V, Chen, Y.-C., Hsiao, H.-Y., Chang, C. & Chern, Y. Neurovascular abnormalities in brain disorders: highlights with angiogenesis and magnetic resonance imaging studies. J. Biomed Sci. 20, 47 (2013).

Christen T, Ni W, Qiu D, et al. High-resolution cerebral blood volume imaging in humans using the blood pool contrast agent ferumoxytol. Magn Reson Med. 2012;Im:705-710. doi:10.1002/mrm.24500

Cunningham CH, Arai T, Yang PC, McConnell M V., Pauly JM, Conolly SM. Positive contrast magnetic resonance imaging of cells labeled with magnetic nanoparticles. Magn Reson Med. 2005;53(5):999-1005. doi:10.1002/mrm.20477

Gharagouzloo CA, McMahon PN, Sridhar S. Quantitative contrast-enhanced MRI with superparamagnetic nanoparticles using ultrashort time-to-echo pulse sequences. Magn Reson Med. 2015;74(2):431-441. doi:10.1002/mrm.25426

Gharagouzloo CA, Timms L, Qiao J, et al. Quantitative vascular neuroimaging of the rat brain using superparamagnetic nanoparticles: New insights on vascular organization and brain function. Neuroimage. 2017;163:24-33. doi:10.1016/j.neuroimage.2017.09.003

Greenberg SM, Vernooij MW, Cordonnier C, et al. Cerebral microbleeds: a guide to detection and interpretation. Lancet Neurol. 2009. doi:10.1016/S1474-4422(09)70013-4

Guo S, Zhou Y, Xing C, et al. The Vasculome of the Mouse Brain. PLoS One. 2012. doi:10.1371/journal.pone.0052665

Huang, Y. & Mucke, L. Alzheimer mechanisms and therapeutic strategies. Cell 148, 1204-1222 (2012).

Kim SM, Kim MJ, Rhee HY, et al. Regional cerebral perfusion in patients with Alzheimer's disease and mild cognitive impairment: effect of APOE Epsilon4 allele. Neuroradiology. 2013;55(1):25-34. doi:10.1007/s00234-012-1077-x

Larkin MC, Lykken C, Tye LD, Wickelgren JG, Frank LM. Hippocampal output area CA1 broadcasts a generalized novelty signal during an object-place recognition task. Hippocampus. 2014;24(7):773-783. doi:10.1002/hipo.22268

Li L, Jiang Q, Zhang L, et al. Angiogenesis and improved cerebral blood flow in the ischemic boundary area detected by MRI after administration of sildenafil to rats with embolic stroke. Brain Res. 2007;1132(1):185-192. doi:10.1016/j.brainres.2006.10.098

Mandeville JB. IRON fMRI measurements of CBV and implications for BOLD signal. Neuroimage. 2012;62(2):1000-1008. doi:10.1016/j.neuroimage.2012.01.070

McLay RN, Freeman SM, Harlan RE, Ide CF, Kastin AJ, Zadina JE. Aging in the hippocampus: Interrelated actions of neurotrophins and glucocorticoids. Neurosci Biobehav Rev. 1997;21(5):615-629. doi:10.1016/S0149-7634(96)00046-2

Muehe AM, Feng D, Von Eyben R, et al. Safety Report of Ferumoxytol for Magnetic Resonance Imaging in Children and Young Adults. Invest Radiol. 2016;51(4):221-227. doi:10.1097/RLI.0000000000000230

Pardo J, Uriarte M, Cónsole GM, et al. Insulin-like growth factor-I gene therapy increases hippocampal neurogenesis, astrocyte branching and improves spatial memory in female aging rats. Eur J Neurosci. 2016;44(4):2120-2128. doi:10.1111/ejn.13278

Reijmer YD, van Veluw SJ, Greenberg SM. Ischemic brain injury in cerebral amyloid angiopathy. J Cereb Blood Flow Metab. 2016. doi:10.1038/jcbfm.2015.88

Rempp KA, Brix G, Wenz F, Becker CR, Gückel F, Lorenz WJ. Quantification of regional cerebral blood flow and volume with dynamic susceptibility contrast-enhanced MR imaging. Radiology. 1994;193:637-641. doi:10.1148/radiology.193.3.7972800

Schabel MC, Parker DL. Uncertainty and bias in contrast concentration measurements using spoiled gradient echo pulse sequences. Phys Med Biol. 2008;53(9):2345-2373. doi:10.1088/0031-9155/53/9/010

Seppenwoolde JH, Viergever MA, Bakker CJG. Passive tracking exploiting local signal conservation: The white marker phenomenon. Magn Reson Med. 2003;50(4):784-790. doi:10.1002/mrm.10574

Shi Y, Wardlaw JM. Update on cerebral small vessel disease: a dynamic whole-brain disease. BMJ. 2016;1(3):83-92. doi:10.1136/svn-2016-000035

Squire LR. Memory and the hippocampus: A synthesis from findings with rats, monkeys, and humans. Psychol Rev. 1992;99(2):195-231. doi:10.1037/0033-295X.99.2.195

Stuber M, Gilson WD, Schär M, et al. Positive contrast visualization of iron oxide-labeled stem cells using inversion-recovery with ON-resonant water suppression (IRON). Magn Reson Med. 2007;58:1072-1077. doi:10.1002/mrm.21399

Troprès I, Grimault S, Vaeth A, et al. Vessel size imaging. Magn Reson Med. 2001;45:397-408. doi:10.1002/1522-2594(200103)45:3<397::AID-MRM1052>3.0.CO;2-3

Walker-Samuel S, Leach MO, Collins DJ. Reference tissue quantification of DCE-MRI data without a contrast agent calibration. Phys Med Biol. 2007;52:589-601. doi:10.1088/0031-9155/52/3/004

Wardlaw JM, Smith EE, Biessels GJ, et al. Neuroimaging standards for research into small vessel disease and its contribution to ageing and neurodegeneration. Lancet Neurol. 2013;12(8):822-838. doi:10.1016/S1474-4422(13)70124-8

Wey H-Y, Desai VR, Duong TQ. A review of current imaging methods used in stroke research. Neurol Res. 2013. doi:10.1179/1743132813Y.0000000250

WHO | Dementia. WHO Fact sheet Updated May 2017 (2017). doi:/entity/mediacentre/factsheets/fs297/en/index.html

Yankeelov T, Gore J. Dynamic contrast enhanced magnetic resonance imaging in oncology: theory, data acquisition, analysis, and examples. Curr Med Imaging Rev. 2009;3(2):91-107. doi:10.2174/157340507780619179.Dynamic

Zlokovic, B. V. Neurovascular pathways to neurodegeneration in Alzheimer's disease and other disorders. Nat. Rev. Neurosci. (2011). doi:10.1038/nrn31 I.

**Claims**

1. A method of determining a likelihood of onset or progression of a neurophysiological condition in a subject, the method comprising:

   generating a first representative quantitative cerebral blood volume (qCBV) for one or more regions of the brain of the subject using one or more first magnetic resonance imaging (MRI) images of the brain of the subject;
   generating a second representative qCBV for the one or more regions of the brain of the subject using one or more second MRI images of the brain of the subject generated during and/or following the subject undergoing a vascular challenge;
   determining one or more functional changes of the vasculature of the brain of the subject based on the first representative qCBV and the second representative qCBV; and
   detecting the likelihood of onset or progression of the neurophysiological condition in the subject based on the one or more functional changes of the vasculature of the brain of the subject.

2. The method of claim 1, wherein the vascular challenge of the subject is a hypercapnic challenge of the subject.

3. The method of claim 1, wherein the vascular challenge of the subject is a hypoxic challenge of the subject.

4. The method of claim 1, wherein the one or more second MRI images of the brain of the subject were generated while the subject breathed $CO_2$-enriched air.

5. The method of claim 1, wherein the one or more second MRI images of the brain of the subject were generated after cessation of the subject breathing $CO_2$-enriched air.

6. The method of claim 1, wherein the one or more functional changes of the vasculature of the brain of the subject comprises a difference between an amount of cerebral blood volume in the one or more regions of the brain indicated by the first representative qCBV and an amount of cerebral blood volume in the one or more regions of the brain indicated by the second representative qCBV.

7. A computing system comprising:

   one or more processors; and
   storage encoded with instructions that, when executed by the one or more processors, cause the one or more processors to perform a method comprising:

      generating a first representative quantitative cerebral blood volume (qCBV) for one or more regions of the brain of the subject using one or more first magnetic resonance imaging (MRI) images of the brain of the subject;
      generating a second representative qCBV for the one or more regions of the brain of the subject using one or more second MRI images of the brain of the subject generated during and/or following the subject undergoing a vascular challenge;
      determining one or more functional changes of the vasculature of the brain of the subject based on the first representative qCBV and the second representative qCBV; and
      detecting the likelihood of onset or progression of the neurophysiological condition in the subject based on the one or more functional changes of the vasculature of the brain of the subject.

8. The computing system of claim 7, wherein the vascular challenge of the subject is a hypercapnic challenge of the subject.

9. The computing system of claim 7, wherein the vascular challenge of the subject is a hypoxic challenge of the subject.

**10.** The computing system of claim 7, wherein the one or more second MRI images of the brain of the subject were generated while the subject breathed CO2-enriched air or after cessation of the subject breathing $CO_2$-enriched air.

**11.** The computing system of claim 7, wherein the one or more functional changes of the vasculature of the brain of the subject comprises a difference between an amount of cerebral blood volume in the one or more regions of the brain indicated by the first representative qCBV and an amount of cerebral blood volume in the one or more regions of the brain indicated by the second representative qCBV.

**12.** At least one non-transitory computer-readable storage medium having encoded thereon instructions that, when executed by at least one processor, cause the at least one processor to carry out a method comprising:

generating a first representative quantitative cerebral blood volume (qCBV) for one or more regions of the brain of the subject using one or more first magnetic resonance imaging (MRI) images of the brain of the subject;
generating a second representative qCBV for the one or more regions of the brain of the subject using one or more second MRI images of the brain of the subject generated during and/or following the subject undergoing a vascular challenge;
determining one or more functional changes of the vasculature of the brain of the subject based on the first representative qCBV and the second representative qCBV; and
detecting the likelihood of onset or progression of the neurophysiological condition in the subject based on the one or more functional changes of the vasculature of the brain of the subject.

**13.** The at least one non-transitory computer-readable storage medium of claim 12, wherein the vascular challenge of the subject is a hypercapnic challenge or a hypoxic challenge of the subject.

**14.** The at least one non-transitory computer-readable storage medium of claim 12, wherein the one or more second MRI images of the brain of the subject were generated while the subject breathed $CO_2$-enriched air or after cessation of the subject breathing CO2-enriched air.

**15.** The at least one non-transitory computer-readable storage medium of claim 12, wherein the one or more functional changes of the vasculature of the brain of the subject comprises a difference between an amount of cerebral blood volume in the one or more regions of the brain indicated by the first representative qCBV and an amount of cerebral blood volume in the one or more regions of the brain indicated by the second representative qCBV.

QUTE-CE
Pre-Contrast

QUTE-CE
Post-Contrast

QUTE-CE
Post-Contrast (Brain only)

*FIG. 1A*                    *FIG. 1B*                    *FIG. 1C*

**QUTE-CE Vascular Biomarkers: Measurements**

**Image Acquisition**

Pre-contrast | Post-contrast

**Pre-processing**

- $B_1^-$ coil sensitivity correction
- $B_1^+$ flip-angle correction
- Motion Correction

**qCBV Calculation**

Pre-contrast Brain | Post-contrast Brain

**qCBV map**

$$qCBV = \bar{f}_B = \frac{I'_M - I_M}{I'_B - I_B}$$

Voxel-Based qCBV Fraction

**Vasculome Network Analysis**

$10^6$ voxels Distributed in Anatomical Atlas

*FIG. 2*

*FIG. 2 (Continuation Sheet 1)*

**FIG. 2 (Continued)**

**FIG. 3A**

**FIG. 3B**

**FIG. 3C**

**FIG. 4A**

EP 4 741 014 A2

FIG. 4B

**Vascular Abnormalities:**
**Hypovascular Trend at 2 years**

Small vessels

All vessels

- ■ Hypovascularized
- ● Hypervascularized

Number of Abnormal Regions ($p<0.05$)

8 Months    2 Years

8 Months    2 Years

**FIG. 5A**

**Metabolic Dysfunction**
**With CO2 Challenge in ApoE4**

- ■ WT
- ● ApoE4

Number of Responding Regions ($p<0.05$)

$CO_2$    Rest    $CO_2$

**FIG. 5B**

EP 4 741 014 A2

# MEAN WT vs APOE 8months

| Region Num | Region Nam | H | P | WT (n=5) Mean | std | APOE4 (n=6) Mean | std | APOE-WT diff | std |
|---|---|---|---|---|---|---|---|---|---|
| 1 | 10th cerebellar lobule | 0 | 0.32029 | 0.07387 | 0.01109 | 0.08151 | 0.01269 | 0.00000 | 0.01686 |
| 2 | 1st cerebellar lobule | 0 | 0.65707 | 0.05871 | 0.01282 | 0.05562 | 0.00952 | 0.00000 | 0.01597 |
| 3 | 2nd cerebellar lobule | 1 | 0.02878 | 0.04725 | 0.00939 | 0.06066 | 0.00775 | 0.01341 | 0.01218 |
| 4 | 3rd cerebellar lobule | 0 | 0.10118 | 0.04269 | 0.00859 | 0.05122 | 0.00694 | 0.00000 | 0.01105 |
| 5 | 4th cerebellar lobule | 0 | 0.20660 | 0.03531 | 0.00843 | 0.04768 | 0.01868 | 0.00000 | 0.02049 |
| 6 | 5th cerebellar lobule | 0 | 0.19047 | 0.03628 | 0.01310 | 0.06796 | 0.04817 | 0.00000 | 0.04992 |
| 7 | motor trigeminal nucleus | 1 | 0.00145 | 0.03565 | 0.00879 | 0.05246 | 0.00250 | 0.01681 | 0.00913 |
| 8 | root of trigeminal nerve | 0 | 0.73736 | 0.08856 | 0.00648 | 0.08700 | 0.00812 | 0.00000 | 0.01039 |
| 9 | 6th cerebellar lobule | 0 | 0.32871 | 0.05071 | 0.00617 | 0.05638 | 0.01083 | 0.00000 | 0.01247 |
| 10 | 7th cerebellar lobule | 0 | 0.48999 | 0.05446 | 0.00637 | 0.05705 | 0.00558 | 0.00000 | 0.00847 |
| 11 | facial nucleus | 0 | 0.45449 | 0.06904 | 0.01672 | 0.06245 | 0.01119 | 0.00000 | 0.02012 |
| 12 | 8th cerebellar lobule | 0 | 0.10685 | 0.05085 | 0.00431 | 0.05695 | 0.00648 | 0.00000 | 0.00778 |
| 13 | 9th cerebellar lobule | 1 | 0.01737 | 0.05305 | 0.00608 | 0.06096 | 0.00259 | 0.00791 | 0.00661 |
| 14 | anterior thalamic nuclei | 0 | 0.51186 | 0.05964 | 0.01351 | 0.06413 | 0.00818 | 0.00000 | 0.01580 |
| 15 | anterior amygdaloid nucleus | 0 | 0.30563 | 0.05090 | 0.01013 | 0.04532 | 0.00686 | 0.00000 | 0.01224 |
| 16 | accumbens core | 0 | 0.38334 | 0.03220 | 0.00784 | 0.03576 | 0.00499 | 0.00000 | 0.00930 |
| 17 | accumbens shell | 0 | 0.58769 | 0.03831 | 0.00638 | 0.04028 | 0.00531 | 0.00000 | 0.00830 |
| 18 | anterior hypothalamic area | 1 | 0.03281 | 0.07802 | 0.00921 | 0.06650 | 0.00589 | -0.01152 | 0.01093 |
| 19 | anterior lobe pituitary | 0 | 0.82209 | 0.23717 | 0.05812 | 0.24388 | 0.03763 | 0.00000 | 0.06924 |
| 20 | anterior olfactory nucleus | 0 | 0.70397 | 0.06210 | 0.01609 | 0.05897 | 0.01028 | 0.00000 | 0.01909 |
| 21 | anterior pretectal nucleus | 0 | 0.06868 | 0.04829 | 0.01055 | 0.06049 | 0.00904 | 0.00000 | 0.01390 |
| 22 | arcuate nucleus | 1 | 0.00277 | 0.18626 | 0.05785 | 0.08067 | 0.02476 | -0.10559 | 0.06293 |
| 23 | auditory ctx | 1 | 0.01967 | 0.10584 | 0.00665 | 0.08676 | 0.01370 | -0.01909 | 0.01523 |
| 24 | basal amygdaloid nucleus | 0 | 0.08072 | 0.08651 | 0.01032 | 0.07672 | 0.00604 | 0.00000 | 0.01196 |
| 25 | CA1 dorsal | 0 | 0.20370 | 0.08565 | 0.01680 | 0.07551 | 0.00654 | 0.00000 | 0.01803 |

*FIG. 6*

| | | | | | | | | | |
|---|---|---|---|---|---|---|---|---|---|
| 26 | CA1 hippocampus ventral | 0 | 0.73492 | 0.06753 | 0.00655 | 0.06854 | 0.00253 | 0.00000 | 0.00702 |
| 27 | CA2 | 0 | 0.16059 | 0.06603 | 0.00539 | 0.06168 | 0.00407 | 0.00000 | 0.00675 |
| 28 | CA3 dorsal | 0 | 0.29668 | 0.07989 | 0.02177 | 0.06959 | 0.00673 | 0.00000 | 0.02279 |
| 29 | CA3 hippocampus ventral | 0 | 0.08162 | 0.11528 | 0.02109 | 0.09626 | 0.01032 | 0.00000 | 0.02348 |
| 30 | central amygdaloid nucleus | 0 | 0.62338 | 0.08389 | 0.01929 | 0.08845 | 0.00980 | 0.00000 | 0.02164 |
| 31 | anterior cingulate area | 0 | 0.64786 | 0.08602 | 0.01848 | 0.08084 | 0.01778 | 0.00000 | 0.02564 |
| 32 | central gray | 1 | 0.03125 | 0.05370 | 0.01035 | 0.06605 | 0.00544 | 0.01235 | 0.01169 |
| 33 | claustrum | 0 | 0.53223 | 0.04022 | 0.00860 | 0.04310 | 0.00610 | 0.00000 | 0.01055 |
| 34 | central medial thalamic nucleus | 0 | 0.68062 | 0.06522 | 0.01525 | 0.06809 | 0.00621 | 0.00000 | 0.01646 |
| 35 | cortical amygdaloid nucleus | 1 | 0.00580 | 0.09629 | 0.00785 | 0.08079 | 0.00648 | -0.01550 | 0.01018 |
| 36 | copula of the pyramis | 0 | 0.96443 | 0.13808 | 0.02895 | 0.13722 | 0.03250 | 0.00000 | 0.04352 |
| 37 | crus 1 of ansiform lobule | 0 | 0.51020 | 0.05716 | 0.00243 | 0.05932 | 0.00663 | 0.00000 | 0.00706 |
| 38 | crus 2 of ansiform lobule | 0 | 0.59932 | 0.06399 | 0.01005 | 0.06673 | 0.00660 | 0.00000 | 0.01202 |
| 39 | diagonal band of Broca | 1 | 0.03375 | 0.10343 | 0.02645 | 0.06885 | 0.01944 | -0.03458 | 0.03283 |
| 40 | dentate gyrus dorsal | 0 | 0.09037 | 0.15863 | 0.03589 | 0.12386 | 0.02489 | 0.00000 | 0.04367 |
| 41 | dentate gyrus ventral | 0 | 0.21039 | 0.15840 | 0.03027 | 0.14102 | 0.00908 | 0.00000 | 0.03160 |
| 42 | dorsal lateral striatum | 0 | 0.96747 | 0.05517 | 0.01109 | 0.05539 | 0.00530 | 0.00000 | 0.01229 |
| 43 | dorsal medial nucleus | 0 | 0.25812 | 0.08297 | 0.01712 | 0.07249 | 0.01165 | 0.00000 | 0.02071 |
| 44 | dorsal medial striatum | 0 | 0.56314 | 0.04921 | 0.01134 | 0.05229 | 0.00516 | 0.00000 | 0.01246 |
| 45 | dorsomedial tegmental area | 1 | 0.00026 | 0.03247 | 0.00666 | 0.05328 | 0.00525 | 0.02080 | 0.00848 |
| 46 | DPGi | 0 | 0.07610 | 0.05598 | 0.01453 | 0.06868 | 0.00532 | 0.00000 | 0.01548 |
| 47 | dorsal raphe | 1 | 0.00445 | 0.05669 | 0.01312 | 0.07902 | 0.00591 | 0.02233 | 0.01439 |
| 48 | subiculum dorsal | 0 | 0.11556 | 0.06072 | 0.01472 | 0.07373 | 0.01003 | 0.00000 | 0.01781 |
| 49 | extended amydala | 0 | 0.28519 | 0.05676 | 0.00719 | 0.05272 | 0.00454 | 0.00000 | 0.00850 |
| 50 | ectorhinal ctx | 0 | 0.05252 | 0.36480 | 0.03722 | 0.34914 | 0.06148 | 0.00000 | 0.07187 |

*FIG. 6 (Continued)*

| | | | | | | | | | |
|---|---|---|---|---|---|---|---|---|---|
| 51 | endopiriform nucleus | 0 | 0.63167 | 0.05082 | 0.00770 | 0.05131 | 0.00706 | 0.00000 | 0.01044 |
| 52 | entorhinal ctx | 0 | 0.12425 | 0.16063 | 0.02038 | 0.16418 | 0.02222 | 0.00000 | 0.03015 |
| 53 | external plexiform layer | 0 | 0.91531 | 0.14618 | 0.02246 | 0.14431 | 0.02667 | 0.00000 | 0.03487 |
| 54 | flocculus cerebellum | 0 | 0.79050 | 0.06821 | 0.00663 | 0.06995 | 0.00978 | 0.00000 | 0.01181 |
| 55 | frontal association ctx | 1 | 0.01247 | 0.06610 | 0.01801 | 0.05619 | 0.01017 | -0.00991 | 0.02068 |
| 56 | gigantocellular reticular nucleus | 0 | 0.90427 | 0.04556 | 0.00440 | 0.05161 | 0.00544 | 0.00000 | 0.00700 |
| 57 | glomerular layer | 0 | 0.82623 | 0.21088 | 0.03463 | 0.18976 | 0.02565 | 0.00000 | 0.04310 |
| 58 | globus pallidus | 0 | 0.74363 | 0.05735 | 0.01072 | 0.05586 | 0.00644 | 0.00000 | 0.01250 |
| 59 | granular cell layer | 0 | 0.45942 | 0.10227 | 0.01603 | 0.11032 | 0.02243 | 0.00000 | 0.02757 |
| 60 | habenula nucleus | 0 | 0.27891 | 0.10418 | 0.03386 | 0.15412 | 0.05507 | 0.00000 | 0.06465 |
| 61 | intercalated amygdaloid nucleus | 0 | 0.07675 | 0.07827 | 0.00892 | 0.11608 | 0.03754 | 0.00000 | 0.03858 |
| 62 | inferior colliculus | 1 | 0.00603 | 0.12022 | 0.01452 | 0.14079 | 0.03120 | 0.02057 | 0.03442 |
| 63 | infralimbic ctx | 0 | 0.27462 | 0.05740 | 0.00681 | 0.05786 | 0.00638 | 0.00000 | 0.00933 |
| 64 | insular ctx | 1 | 0.00652 | 0.07952 | 0.01708 | 0.06901 | 0.00713 | -0.01051 | 0.01851 |
| 65 | interposed nucleus | 0 | 0.78070 | 0.10568 | 0.02149 | 0.09021 | 0.02047 | 0.00000 | 0.02968 |
| 66 | inferior olivary complex | 1 | 0.00335 | 0.10902 | 0.03442 | 0.09080 | 0.03466 | -0.01823 | 0.04885 |
| 67 | interpeduncular nucleus | 0 | 0.58521 | 0.07738 | 0.06415 | 0.04674 | 0.01692 | 0.00000 | 0.06634 |
| 68 | lateral amygdaloid nucleus | 0 | 0.52005 | 0.09190 | 0.01570 | 0.07619 | 0.00818 | 0.00000 | 0.01770 |
| 69 | Lat | 0 | 0.25040 | 0.07856 | 0.01387 | 0.07739 | 0.01598 | 0.00000 | 0.02116 |
| 70 | locus ceruleus | 0 | 0.11219 | 0.06979 | 0.00684 | 0.07183 | 0.01611 | 0.00000 | 0.01750 |
| 71 | lateral dorsal thalamic nucleus | 0 | 0.05691 | 0.08555 | 0.02216 | 0.08313 | 0.01357 | 0.00000 | 0.02599 |
| 72 | lateral geniculate | 0 | 0.69805 | 0.13313 | 0.01035 | 0.12012 | 0.01611 | 0.00000 | 0.01915 |
| 73 | lateral hypothalamus | 0 | 0.21053 | 0.19732 | 0.02372 | 0.12172 | 0.01328 | 0.00000 | 0.02718 |
| 74 | lemniscal nucleus | 0 | 0.83794 | 0.06327 | 0.01372 | 0.06471 | 0.00575 | 0.00000 | 0.01488 |
| 75 | lateral orbital ctx | 0 | 0.90966 | 0.03314 | 0.00616 | 0.03706 | 0.00561 | 0.00000 | 0.00833 |

**FIG. 6 (Continued)**

| | | | | | | | | | |
|---|---|---|---|---|---|---|---|---|---|
| 76 | lateral posterior thalamic nucleus | 0 | 0.20073 | 0.10435 | 0.02561 | 0.12933 | 0.02435 | 0.00000 | 0.03534 |
| 77 | lateral preoptic area | 1 | 0.00545 | 0.04932 | 0.00703 | 0.05285 | 0.00394 | 0.00353 | 0.00806 |
| 78 | lateral septal nucleus | 0 | 0.25314 | 0.07007 | 0.01389 | 0.07219 | 0.00521 | 0.00000 | 0.01484 |
| 79 | primary motor ctx | 0 | 0.96132 | 0.04102 | 0.01060 | 0.04836 | 0.00981 | 0.00000 | 0.01444 |
| 80 | secondary motor ctx | 0 | 0.40639 | 0.05322 | 0.01213 | 0.06142 | 0.01754 | 0.00000 | 0.02133 |
| 81 | magnocellular preoptic nucleus | 0 | 0.39025 | 0.09783 | 0.02584 | 0.08002 | 0.01329 | 0.00000 | 0.02905 |
| 82 | medial dorsal thalamic nucleus | 0 | 0.28565 | 0.07667 | 0.01388 | 0.08922 | 0.01458 | 0.00000 | 0.02013 |
| 83 | medial amygdaloid nucleus | 0 | 0.19279 | 0.18586 | 0.02469 | 0.17932 | 0.02264 | 0.00000 | 0.03350 |
| 84 | medial cerebellar nucleus fastigia | 0 | 0.06071 | 0.07181 | 0.01757 | 0.06004 | 0.00863 | 0.00000 | 0.01958 |
| 85 | medial geniculate | 0 | 0.90070 | 0.18028 | 0.03146 | 0.13883 | 0.01773 | 0.00000 | 0.03612 |
| 86 | medial mammillary nucleus | 0 | 0.90089 | 0.23677 | 0.07873 | 0.20028 | 0.00819 | 0.00000 | 0.07916 |
| 87 | median raphe nucleus | 0 | 0.50989 | 0.02425 | 0.01068 | 0.04691 | 0.00407 | 0.00000 | 0.01143 |
| 88 | medial orbital ctx | 0 | 0.79911 | 0.06085 | 0.01106 | 0.04926 | 0.00867 | 0.00000 | 0.01406 |
| 89 | medial preoptic area | 0 | 0.05515 | 0.03798 | 0.00844 | 0.04593 | 0.00763 | 0.00000 | 0.01138 |
| 90 | medial pretectal area | 0 | 0.82796 | 0.03817 | 0.01548 | 0.06422 | 0.06943 | 0.00000 | 0.07114 |
| 91 | medial septum | 0 | 0.15543 | 0.05018 | 0.00670 | 0.05651 | 0.00835 | 0.00000 | 0.01071 |
| 92 | neural lobe pituitary | 0 | 0.67694 | 0.21382 | 0.06452 | 0.19485 | 0.03785 | 0.00000 | 0.07480 |
| 93 | olivary nucleus | 1 | 0.00009 | 0.07634 | 0.01701 | 0.05424 | 0.00848 | -0.02210 | 0.01900 |
| 94 | paraventricular nuclus | 0 | 0.22238 | 0.05908 | 0.00723 | 0.05924 | 0.00548 | 0.00000 | 0.00907 |
| 95 | periaqueductal gray thalamus | 0 | 0.81967 | 0.06669 | 0.00579 | 0.07513 | 0.00297 | 0.00000 | 0.00651 |
| 96 | parabrachial nucleus | 0 | 0.29726 | 0.04698 | 0.00843 | 0.06294 | 0.00737 | 0.00000 | 0.01120 |
| 97 | PCRt | 0 | 0.47840 | 0.07448 | 0.00325 | 0.07148 | 0.00730 | 0.00000 | 0.00799 |
| 98 | parafascicular thalamic nucleus | 0 | 0.13217 | 0.07336 | 0.00938 | 0.07581 | 0.00690 | 0.00000 | 0.01164 |
| 99 | paraflocculus cerebellum | 0 | 0.11390 | 0.10847 | 0.01074 | 0.08527 | 0.01802 | 0.00000 | 0.02097 |
| 100 | posterior hypothalamic area | 0 | 0.31952 | 0.14537 | 0.03383 | 0.10878 | 0.01065 | 0.00000 | 0.03547 |

**FIG. 6 (Continued)**

| | | | | | | | | | |
|---|---|---|---|---|---|---|---|---|---|
| 101 | pineal gland | 0 | 0.28661 | 0.40660 | 0.15424 | 0.65974 | 0.13263 | 0.00000 | 0.20343 |
| 102 | caudal piriform ctx | 0 | 0.73446 | 0.04087 | 0.01664 | 0.03979 | 0.00708 | 0.00000 | 0.01809 |
| 103 | rostral piriform ctx | 0 | 0.07186 | 0.10546 | 0.00964 | 0.08561 | 0.00500 | 0.00000 | 0.01086 |
| 104 | premammillary nucleus | 0 | 0.26350 | 0.23356 | 0.06977 | 0.12716 | 0.02992 | 0.00000 | 0.07591 |
| 105 | paramedian lobule | 0 | 0.40094 | 0.08847 | 0.01822 | 0.07517 | 0.00945 | 0.00000 | 0.02053 |
| 106 | pontine nuclei | 1 | 0.00317 | 0.03508 | 0.03670 | 0.01882 | 0.00731 | -0.01626 | 0.03742 |
| 107 | pontine reticular nucleus caudal | 0 | 0.17283 | 0.02489 | 0.00728 | 0.03992 | 0.00416 | 0.00000 | 0.00838 |
| 108 | pontine reticular nucleus oral | 1 | 0.02485 | 0.02698 | 0.00949 | 0.04211 | 0.00331 | 0.01513 | 0.01005 |
| 109 | posterior thalamic nucleus | 0 | 0.18874 | 0.07228 | 0.00929 | 0.07305 | 0.00729 | 0.00000 | 0.01181 |
| 110 | periolivary nucleus | 0 | 0.18035 | 0.06618 | 0.01740 | 0.05673 | 0.01658 | 0.00000 | 0.02404 |
| 111 | prerubral field | 0 | 0.13136 | 0.08860 | 0.01969 | 0.08228 | 0.00479 | 0.00000 | 0.02026 |
| 112 | principal sensory nucleus trigemin | 0 | 0.65805 | 0.07254 | 0.00560 | 0.07251 | 0.00443 | 0.00000 | 0.00714 |
| 113 | precuniform nucleus | 0 | 0.17980 | 0.03933 | 0.00902 | 0.05281 | 0.00324 | 0.00000 | 0.00959 |
| 114 | perirhinal ctx | 1 | 0.00088 | 0.33112 | 0.03106 | 0.22531 | 0.03631 | -0.10582 | 0.04778 |
| 115 | prelimbic ctx | 0 | 0.15839 | 0.05218 | 0.00822 | 0.05664 | 0.00536 | 0.00000 | 0.00981 |
| 116 | parietal ctx | 1 | 0.02207 | 0.04616 | 0.00881 | 0.05450 | 0.01388 | 0.00834 | 0.01644 |
| 117 | pedunculopontine tegmental area | 0 | 0.28355 | 0.03970 | 0.01259 | 0.05188 | 0.00441 | 0.00000 | 0.01334 |
| 118 | paraventricular nucleus | 1 | 0.00134 | 0.05663 | 0.01338 | 0.09083 | 0.04307 | 0.03420 | 0.04510 |
| 119 | retrochiasmatic nucleus | 1 | 0.00172 | 0.17181 | 0.03028 | 0.11086 | 0.03390 | -0.06095 | 0.04545 |
| 120 | reuniens nucleus | 1 | 0.00093 | 0.07145 | 0.01365 | 0.07012 | 0.00456 | -0.00133 | 0.01439 |
| 121 | raphe linear | 0 | 0.34119 | 0.05597 | 0.01615 | 0.06168 | 0.00772 | 0.00000 | 0.01790 |
| 122 | raphe magnus | 0 | 0.08277 | 0.02117 | 0.00939 | 0.03735 | 0.00550 | 0.00000 | 0.01089 |
| 123 | raphe obscurus nucleus | 0 | 0.18161 | 0.03411 | 0.00774 | 0.04792 | 0.00526 | 0.00000 | 0.00936 |
| 124 | red nucleus | 0 | 0.13504 | 0.03560 | 0.00848 | 0.05193 | 0.00513 | 0.00000 | 0.00991 |
| 125 | retrosplenial caudal ctx | 0 | 0.30645 | 0.16703 | 0.03959 | 0.18107 | 0.04204 | 0.00000 | 0.05774 |

## FIG. 6 (Continued)

| | | | | | | | | |
|---|---|---|---|---|---|---|---|---|---|
| 126 | retrosplenial rostral ctx | 0 | 0.43598 | 0.19759 | 0.03630 | 0.22288 | 0.03202 | 0.00000 | 0.04840 |
| 127 | reticular nucleus | 0 | 0.20565 | 0.07216 | 0.01138 | 0.07020 | 0.00383 | 0.00000 | 0.01201 |
| 128 | reticular nucleus midbrain | 0 | 0.55755 | 0.06882 | 0.00597 | 0.06808 | 0.00562 | 0.00000 | 0.00820 |
| 129 | reticulotegmental nucleus | 0 | 0.17450 | 0.01600 | 0.01083 | 0.03647 | 0.00786 | 0.00000 | 0.01339 |
| 130 | primary somatosensory ctx barrel f | 1 | 0.02032 | 0.05817 | 0.01002 | 0.05786 | 0.01007 | -0.00030 | 0.01420 |
| 131 | primary somatosensory ctx forelimb | 0 | 0.44273 | 0.04289 | 0.00961 | 0.04838 | 0.01036 | 0.00000 | 0.01413 |
| 132 | primary somatosensory ctx hindlimb | 0 | 0.96636 | 0.04015 | 0.00691 | 0.05002 | 0.01424 | 0.00000 | 0.01583 |
| 133 | primary somatosensory ctx jaw | 1 | 0.01214 | 0.04775 | 0.01081 | 0.04839 | 0.00519 | 0.00064 | 0.01199 |
| 134 | primary somatosensory ctx shoulder | 0 | 0.83706 | 0.04507 | 0.00864 | 0.04869 | 0.00875 | 0.00000 | 0.01230 |
| 135 | primary somatosensory ctx trunk | 1 | 0.00848 | 0.04122 | 0.00687 | 0.05102 | 0.00771 | 0.00980 | 0.01033 |
| 136 | primary somatosensory ctx upper li | 0 | 0.26438 | 0.06597 | 0.01206 | 0.06329 | 0.00864 | 0.00000 | 0.01484 |
| 137 | secondary somaotsensory ctx | 0 | 0.41962 | 0.08247 | 0.01469 | 0.07226 | 0.01118 | 0.00000 | 0.01846 |
| 138 | suprachiasmatic nucleus | 0 | 0.62911 | 0.02435 | 0.02077 | 0.03199 | 0.01341 | 0.00000 | 0.02472 |
| 139 | substantia innominata | 1 | 0.02483 | 0.08656 | 0.01961 | 0.06825 | 0.01514 | -0.01831 | 0.02477 |
| 140 | simple lobule cerebellum | 0 | 0.37675 | 0.03594 | 0.00773 | 0.04779 | 0.02212 | 0.00000 | 0.02343 |
| 141 | substantia nigra compacta | 1 | 0.03291 | 0.11811 | 0.04623 | 0.07952 | 0.00698 | -0.03859 | 0.04675 |
| 142 | substantia nigra reticularis | 0 | 0.98662 | 0.22254 | 0.05786 | 0.12728 | 0.01104 | 0.00000 | 0.05891 |
| 143 | supraoptic nucleus | 1 | 0.03239 | 0.11538 | 0.04258 | 0.06631 | 0.01359 | -0.04907 | 0.04469 |
| 144 | solitary tract nucleus | 0 | 0.08015 | 0.05007 | 0.01109 | 0.06502 | 0.02108 | 0.00000 | 0.02382 |
| 145 | bed nucleus stria terminalis | 1 | 0.01674 | 0.04424 | 0.01100 | 0.05263 | 0.00534 | 0.00838 | 0.01222 |
| 146 | subthalamic nucleus | 0 | 0.98339 | 0.22827 | 0.05929 | 0.11097 | 0.00586 | 0.00000 | 0.05958 |
| 147 | superior colliculus | 0 | 0.88708 | 0.07372 | 0.01119 | 0.09665 | 0.03148 | 0.00000 | 0.03341 |
| 148 | sub coeruleus nucleus | 0 | 0.88071 | 0.03412 | 0.00619 | 0.04832 | 0.00409 | 0.00000 | 0.00742 |
| 149 | supramammillary nucleus | 1 | 0.00169 | 0.34276 | 0.05350 | 0.22460 | 0.03539 | -0.11816 | 0.06414 |
| 150 | temporal ctx | 0 | 0.23279 | 0.22931 | 0.05233 | 0.19815 | 0.05030 | 0.00000 | 0.07259 |

*FIG. 6 (Continued)*

| | | | | | | | | | |
|---|---|---|---|---|---|---|---|---|---|---|
| 151 | triangular septal nucleus | 1 | 0.00779 | 0.05372 | 0.00857 | 0.06346 | 0.01278 | 0.00974 | 0.01539 |
| 152 | tenia tecta ctx | 0 | 0.15222 | 0.12174 | 0.02333 | 0.10885 | 0.01606 | 0.00000 | 0.02833 |
| 153 | olfactory tubercles | 1 | 0.00082 | 0.09707 | 0.02697 | 0.07960 | 0.01035 | -0.01747 | 0.02889 |
| 154 | trapezoid body | 0 | 0.26850 | 0.03984 | 0.01104 | 0.04523 | 0.01111 | 0.00000 | 0.01567 |
| 155 | Ventricle | 0 | 0.31186 | 0.11688 | 0.03007 | 0.12007 | 0.01982 | 0.00000 | 0.03601 |
| 156 | visual 1 ctx | 0 | 0.52033 | 0.17001 | 0.02963 | 0.18879 | 0.02280 | 0.00000 | 0.03738 |
| 157 | visual 2 ctx | 1 | 0.00196 | 0.10126 | 0.01249 | 0.12208 | 0.01302 | 0.02082 | 0.01804 |
| 158 | ventral anterior thalamic nucleus | 0 | 0.81226 | 0.05910 | 0.01065 | 0.06333 | 0.00326 | 0.00000 | 0.01114 |
| 159 | cochlear nucleus | 1 | 0.00507 | 0.09040 | 0.01282 | 0.09026 | 0.01401 | -0.00014 | 0.01899 |
| 160 | vestibular nucleus | 0 | 0.68520 | 0.07311 | 0.01738 | 0.08849 | 0.00756 | 0.00000 | 0.01895 |
| 161 | ventrolateral thalamic nucleus | 0 | 0.88068 | 0.06715 | 0.01254 | 0.06703 | 0.00433 | 0.00000 | 0.01327 |
| 162 | ventral lateral striatum | 0 | 0.23731 | 0.05220 | 0.01186 | 0.05304 | 0.00566 | 0.00000 | 0.01314 |
| 163 | ventromedial thalamic nucleus | 0 | 0.38103 | 0.08205 | 0.01466 | 0.07416 | 0.00386 | 0.00000 | 0.01516 |
| 164 | ventral medial nucleus | 0 | 0.46266 | 0.15061 | 0.02558 | 0.08499 | 0.01870 | 0.00000 | 0.03169 |
| 165 | ventral medial striatum | 1 | 0.00265 | 0.03548 | 0.00893 | 0.04032 | 0.00434 | 0.00484 | 0.00993 |
| 166 | ventral orbital ctx | 0 | 0.18638 | 0.03174 | 0.00392 | 0.03408 | 0.00692 | 0.00000 | 0.00795 |
| 167 | ventral pallidum | 0 | 0.99186 | 0.05097 | 0.00753 | 0.04993 | 0.00659 | 0.00000 | 0.01000 |
| 168 | ventral posteriolateral thalamic n | 0 | 0.28441 | 0.07889 | 0.01049 | 0.07675 | 0.00633 | 0.00000 | 0.01225 |
| 169 | ventral posteriolmedial thalamic n | 1 | 0.00747 | 0.08078 | 0.01102 | 0.07424 | 0.00582 | -0.00654 | 0.01246 |
| 170 | ventral subiculum | 1 | 0.00946 | 0.06984 | 0.01275 | 0.09717 | 0.00935 | 0.02733 | 0.01581 |
| 171 | ventral tegmental area | 1 | 0.00062 | 0.12268 | 0.05846 | 0.08852 | 0.00820 | -0.03417 | 0.05903 |
| 172 | White Matter | 0 | 0.30581 | 0.07103 | 0.00853 | 0.06635 | 0.00497 | 0.00000 | 0.00987 |
| 173 | White Matter | 1 | 0.03195 | 0.10184 | 0.01669 | 0.07952 | 0.00200 | -0.02232 | 0.01681 |
| 174 | zona incerta | 0 | 0.27679 | 0.10277 | 0.01452 | 0.08645 | 0.00588 | 0.00000 | 0.01567 |
| | | 41 | 41.00000 | | | | | | |

**FIG. 6 (Continued)**

FIG. 7

# MODE  WT vs APOE 8months

| Region Num | Region Nam | H | P | WT (n=5) Mean | WT (n=5) std | APOE4 (n=6) Mean | APOE4 (n=6) std | APOE-WT diff | APOE-WT std |
|---|---|---|---|---|---|---|---|---|---|
| 1 | 10th cerebellar lobule | 1 | 0.01770 | 0.04638 | 0.00523 | 0.05618 | 0.00586 | 0.00980 | 0.00786 |
| 2 | 1st cerebellar lobule | 0 | 0.32184 | 0.05603 | 0.00565 | 0.05229 | 0.00607 | 0.00000 | 0.00829 |
| 3 | 2nd cerebellar lobule | 0 | 0.05278 | 0.04087 | 0.00624 | 0.04858 | 0.00524 | 0.00000 | 0.00815 |
| 4 | 3rd cerebellar lobule | 0 | 0.18179 | 0.03242 | 0.00798 | 0.03827 | 0.00542 | 0.00000 | 0.00964 |
| 5 | 4th cerebellar lobule | 0 | 0.14699 | 0.02843 | 0.00775 | 0.03484 | 0.00567 | 0.00000 | 0.00960 |
| 6 | 5th cerebellar lobule | 0 | 0.07823 | 0.02125 | 0.00755 | 0.02915 | 0.00565 | 0.00000 | 0.00943 |
| 7 | motor trigeminal nucleus | 1 | 0.00254 | 0.03448 | 0.00967 | 0.05175 | 0.00329 | 0.01727 | 0.01021 |
| 8 | root of trigeminal nerve | 1 | 0.00128 | 0.04251 | 0.00483 | 0.05291 | 0.00252 | 0.01040 | 0.00545 |
| 9 | 6th cerebellar lobule | 0 | 0.18366 | 0.03040 | 0.00503 | 0.03537 | 0.00618 | 0.00000 | 0.00796 |
| 10 | 7th cerebellar lobule | 0 | 0.11253 | 0.04336 | 0.00974 | 0.05084 | 0.00359 | 0.00000 | 0.01038 |
| 11 | facial nucleus | 1 | 0.04758 | 0.04086 | 0.00880 | 0.05241 | 0.00792 | 0.01155 | 0.01184 |
| 12 | 8th cerebellar lobule | 0 | 0.14901 | 0.04628 | 0.00594 | 0.05331 | 0.00831 | 0.00000 | 0.01021 |
| 13 | 9th cerebellar lobule | 1 | 0.00660 | 0.04390 | 0.00711 | 0.05538 | 0.00346 | 0.01148 | 0.00791 |
| 14 | anterior thalamic nuclei | 0 | 0.85412 | 0.05906 | 0.01317 | 0.05795 | 0.00530 | 0.00000 | 0.01419 |
| 15 | anterior amygdaloid nucleus | 0 | 0.72586 | 0.03487 | 0.00872 | 0.03724 | 0.01222 | 0.00000 | 0.01501 |
| 16 | accumbens core | 0 | 0.30649 | 0.03168 | 0.00766 | 0.03573 | 0.00467 | 0.00000 | 0.00898 |
| 17 | accumbens shell | 0 | 0.48680 | 0.03577 | 0.00897 | 0.03890 | 0.00518 | 0.00000 | 0.01036 |
| 18 | anterior hypothalamic area | 0 | 0.67272 | 0.06672 | 0.00780 | 0.06493 | 0.00578 | 0.00000 | 0.00971 |
| 19 | anterior lobe pituitary | 0 | 0.96636 | 0.23539 | 0.06129 | 0.23663 | 0.03173 | 0.00000 | 0.06902 |
| 20 | anterior olfactory nucleus | 0 | 0.99560 | 0.03452 | 0.00891 | 0.03455 | 0.00831 | 0.00000 | 0.01218 |
| 21 | anterior pretectal nucleus | 0 | 0.11206 | 0.04811 | 0.00695 | 0.05388 | 0.00374 | 0.00000 | 0.00789 |
| 22 | arcuate nucleus | 1 | 0.00835 | 0.13818 | 0.03534 | 0.08601 | 0.01348 | -0.05217 | 0.03783 |
| 23 | auditory ctx | 0 | 0.29464 | 0.08085 | 0.00563 | 0.07492 | 0.01069 | 0.00000 | 0.01208 |
| 24 | basal amygdaloid nucleus | 0 | 0.18807 | 0.08091 | 0.01025 | 0.07395 | 0.00574 | 0.00000 | 0.01175 |
| 25 | CA1  dorsal | 0 | 0.53605 | 0.05363 | 0.00875 | 0.05606 | 0.00295 | 0.00000 | 0.00923 |

*FIG. 8*

| | | | | | | | | | |
|---|---|---|---|---|---|---|---|---|---|
| 26 | CA1 hippocampus ventral | 0 | 0.11574 | 0.06436 | 0.00526 | 0.06887 | 0.00328 | 0.00000 | 0.00620 |
| 27 | CA2 | 0 | 0.79638 | 0.05953 | 0.00438 | 0.06026 | 0.00464 | 0.00000 | 0.00639 |
| 28 | CA3 dorsal | 0 | 0.42559 | 0.05736 | 0.01024 | 0.06106 | 0.00356 | 0.00000 | 0.01084 |
| 29 | CA3 hippocampus ventral | 0 | 0.05978 | 0.07876 | 0.00244 | 0.07151 | 0.00714 | 0.00000 | 0.00755 |
| 30 | central amygdaloid nucleus | 0 | 0.32419 | 0.06637 | 0.00974 | 0.07120 | 0.00544 | 0.00000 | 0.01116 |
| 31 | anterior cingulate area | 0 | 0.31018 | 0.04523 | 0.00896 | 0.04964 | 0.00431 | 0.00000 | 0.00994 |
| 32 | central gray | 0 | 0.06306 | 0.05250 | 0.01092 | 0.06243 | 0.00352 | 0.00000 | 0.01147 |
| 33 | claustrum | 0 | 0.24567 | 0.03671 | 0.00779 | 0.04207 | 0.00654 | 0.00000 | 0.01017 |
| 34 | central medial thalamic nucleus | 0 | 0.59256 | 0.06279 | 0.01220 | 0.06587 | 0.00568 | 0.00000 | 0.01346 |
| 35 | cortical amygdaloid nucleus | 1 | 0.03225 | 0.07941 | 0.00889 | 0.06673 | 0.00774 | -0.01267 | 0.01179 |
| 36 | copula of the pyramis | 1 | 0.04030 | 0.06538 | 0.01173 | 0.08245 | 0.01182 | 0.01707 | 0.01665 |
| 37 | crus 1 of ansiform lobule | 0 | 0.30347 | 0.04142 | 0.00468 | 0.04508 | 0.00614 | 0.00000 | 0.00772 |
| 38 | crus 2 of ansiform lobule | 0 | 0.61023 | 0.05352 | 0.00987 | 0.05580 | 0.00370 | 0.00000 | 0.01054 |
| 39 | diagonal band of Broca | 0 | 0.25991 | 0.03068 | 0.01996 | 0.04153 | 0.00898 | 0.00000 | 0.02189 |
| 40 | dentate gyrus dorsal | 0 | 0.61623 | 0.07015 | 0.00711 | 0.07178 | 0.00283 | 0.00000 | 0.00765 |
| 41 | dentate gyrus ventral | 0 | 0.98355 | 0.07346 | 0.01375 | 0.07358 | 0.00417 | 0.00000 | 0.01437 |
| 42 | dorsal lateral striatum | 0 | 0.95103 | 0.05007 | 0.01089 | 0.05038 | 0.00553 | 0.00000 | 0.01221 |
| 43 | dorsal medial nucleus | 0 | 0.48385 | 0.07630 | 0.01696 | 0.07018 | 0.01071 | 0.00000 | 0.02006 |
| 44 | dorsal medial striatum | 0 | 0.47073 | 0.04536 | 0.01020 | 0.04889 | 0.00501 | 0.00000 | 0.01136 |
| 45 | dorsomedial tegmental area | 1 | 0.00024 | 0.03143 | 0.00722 | 0.05179 | 0.00419 | 0.02036 | 0.00835 |
| 46 | DPGi | 0 | 0.10004 | 0.04653 | 0.01269 | 0.05801 | 0.00799 | 0.00000 | 0.01499 |
| 47 | dorsal raphe | 1 | 0.00730 | 0.05110 | 0.01303 | 0.07127 | 0.00566 | 0.02017 | 0.01421 |
| 48 | subiculum dorsal | 1 | 0.00015 | 0.03633 | 0.00515 | 0.05152 | 0.00277 | 0.01519 | 0.00585 |
| 49 | extended amydala | 0 | 0.29953 | 0.05684 | 0.00596 | 0.05272 | 0.00635 | 0.00000 | 0.00871 |
| 50 | ectorhinal ctx | 0 | 0.75228 | 0.15802 | 0.03878 | 0.16586 | 0.04053 | 0.00000 | 0.05609 |

**FIG. 8 (Continued)**

| | | | | | | | | | |
|---|---|---|---|---|---|---|---|---|---|
| 51 | endopiriform nucleus | 0 | 0.33552 | 0.04099 | 0.00887 | 0.04579 | 0.00681 | 0.00000 | 0.01118 |
| 52 | entorhinal ctx | 1 | 0.02343 | 0.06792 | 0.00447 | 0.07838 | 0.00751 | 0.01046 | 0.00874 |
| 53 | external plexiform layer | 0 | 0.98574 | 0.10733 | 0.01686 | 0.10717 | 0.01104 | 0.00000 | 0.02016 |
| 54 | flocculus cerebellum | 0 | 0.70793 | 0.05070 | 0.00821 | 0.05265 | 0.00839 | 0.00000 | 0.01174 |
| 55 | frontal association ctx | 0 | 0.07918 | 0.00609 | 0.02729 | 0.02845 | 0.00557 | 0.00000 | 0.02785 |
| 56 | gigantocellular reticular nucleus | 1 | 0.00341 | 0.03421 | 0.00596 | 0.04526 | 0.00320 | 0.01105 | 0.00676 |
| 57 | glomerular layer | 0 | 0.52706 | 0.13143 | 0.01692 | 0.12558 | 0.01261 | 0.00000 | 0.02110 |
| 58 | globus pallidus | 0 | 0.91895 | 0.05089 | 0.00987 | 0.05042 | 0.00471 | 0.00000 | 0.01093 |
| 59 | granular cell layer | 0 | 0.69856 | 0.09171 | 0.01396 | 0.09487 | 0.01224 | 0.00000 | 0.01857 |
| 60 | habenula nucleus | 1 | 0.04830 | 0.06457 | 0.01899 | 0.08821 | 0.01542 | 0.02364 | 0.02446 |
| 61 | intercalated amygdaloid nucleus | 0 | 0.34509 | 0.08299 | 0.01501 | 0.09021 | 0.00878 | 0.00000 | 0.01739 |
| 62 | inferior colliculus | 1 | 0.00309 | 0.08747 | 0.00561 | 0.09939 | 0.00427 | 0.01191 | 0.00705 |
| 63 | infralimbic ctx | 1 | 0.01215 | 0.02990 | 0.00594 | 0.03949 | 0.00423 | 0.00959 | 0.00729 |
| 64 | insular ctx | 0 | 0.96766 | 0.04955 | 0.00883 | 0.04972 | 0.00347 | 0.00000 | 0.00949 |
| 65 | interposed nucleus | 0 | 0.83280 | 0.07154 | 0.01586 | 0.06970 | 0.01224 | 0.00000 | 0.02004 |
| 66 | inferior olivary complex | 0 | 0.67211 | 0.06973 | 0.01632 | 0.06481 | 0.02022 | 0.00000 | 0.02598 |
| 67 | interpeduncular nucleus | 0 | 0.18786 | -0.00612 | 0.04528 | 0.02088 | 0.01105 | 0.00000 | 0.04661 |
| 68 | lateral amygdaloid nucleus | 1 | 0.04714 | 0.09073 | 0.01559 | 0.07427 | 0.00757 | -0.01646 | 0.01733 |
| 69 | Lat | 0 | 0.66702 | 0.07576 | 0.02087 | 0.07120 | 0.01296 | 0.00000 | 0.02457 |
| 70 | locus ceruleus | 0 | 0.72308 | 0.07518 | 0.02166 | 0.07109 | 0.01541 | 0.00000 | 0.02658 |
| 71 | lateral dorsal thalamic nucleus | 0 | 0.85934 | 0.07906 | 0.03437 | 0.07639 | 0.01021 | 0.00000 | 0.03585 |
| 72 | lateral geniculate | 0 | 0.69509 | 0.09075 | 0.02086 | 0.08679 | 0.01103 | 0.00000 | 0.02360 |
| 73 | lateral hypothalamus | 1 | 0.02889 | 0.09937 | 0.01068 | 0.08711 | 0.00425 | -0.01225 | 0.01149 |
| 74 | lemniscal nucleus | 0 | 0.28477 | 0.04141 | 0.00907 | 0.04636 | 0.00521 | 0.00000 | 0.01046 |
| 75 | lateral orbital ctx | 0 | 0.15931 | 0.03054 | 0.00719 | 0.03674 | 0.00623 | 0.00000 | 0.00951 |

*FIG. 8 (Continued)*

EP 4 741 014 A2

| | | | | | | | | | |
|---|---|---|---|---|---|---|---|---|---|---|
| 76 | lateral posterior thalamic nucleus | 0 | 0.53502 | 0.07079 | 0.01700 | 0.07683 | 0.01412 | 0.00000 | 0.02210 |
| 77 | lateral preoptic area | 0 | 0.50866 | 0.04713 | 0.01301 | 0.05101 | 0.00452 | 0.00000 | 0.01377 |
| 78 | lateral septal nucleus | 0 | 0.11130 | 0.04177 | 0.00916 | 0.04947 | 0.00513 | 0.00000 | 0.01050 |
| 79 | primary motor ctx | 0 | 0.35888 | 0.03771 | 0.00964 | 0.04201 | 0.00478 | 0.00000 | 0.01076 |
| 80 | secondary motor ctx | 0 | 0.17931 | 0.03250 | 0.00751 | 0.03797 | 0.00492 | 0.00000 | 0.00898 |
| 81 | magnocellular preoptic nucleus | 0 | 0.24853 | 0.05243 | 0.01482 | 0.06170 | 0.01007 | 0.00000 | 0.01792 |
| 82 | medial dorsal thalamic nucleus | 0 | 0.71896 | 0.07265 | 0.01132 | 0.07461 | 0.00585 | 0.00000 | 0.01274 |
| 83 | medial amygdaloid nucleus | 0 | 0.08185 | 0.08092 | 0.01325 | 0.09786 | 0.01505 | 0.00000 | 0.02006 |
| 84 | medial cerebellar nucleus fastigia | 0 | 0.39287 | 0.04620 | 0.01326 | 0.05134 | 0.00450 | 0.00000 | 0.01401 |
| 85 | medial geniculate | 0 | 0.10107 | 0.11730 | 0.01289 | 0.10554 | 0.00842 | 0.00000 | 0.01540 |
| 86 | medial mammillary nucleus | 0 | 0.94541 | 0.17104 | 0.06490 | 0.16896 | 0.03035 | 0.00000 | 0.07165 |
| 87 | median raphe nucleus | 1 | 0.00049 | 0.02403 | 0.00997 | 0.04710 | 0.00360 | 0.02306 | 0.01060 |
| 88 | medial orbital ctx | 0 | 0.19800 | 0.02342 | 0.01350 | 0.03269 | 0.00852 | 0.00000 | 0.01597 |
| 89 | medial preoptic area | 0 | 0.10409 | 0.03217 | 0.01411 | 0.04312 | 0.00457 | 0.00000 | 0.01483 |
| 90 | medial pretectal area | 0 | 0.06664 | 0.01396 | 0.02576 | 0.04546 | 0.02427 | 0.00000 | 0.03539 |
| 91 | medial septum | 0 | 0.44492 | 0.04572 | 0.01482 | 0.05121 | 0.00750 | 0.00000 | 0.01660 |
| 92 | neural lobe pituitary | 0 | 0.34213 | 0.21440 | 0.08575 | 0.17806 | 0.02374 | 0.00000 | 0.08897 |
| 93 | olivary nucleus | 0 | 0.61395 | 0.04379 | 0.01505 | 0.04753 | 0.00835 | 0.00000 | 0.01721 |
| 94 | paraventricular nuclus | 0 | 0.87467 | 0.05485 | 0.00993 | 0.05583 | 0.01011 | 0.00000 | 0.01417 |
| 95 | periaqueductal gray thalamus | 1 | 0.02777 | 0.06045 | 0.00718 | 0.06867 | 0.00265 | 0.00822 | 0.00766 |
| 96 | parabrachial nucleus | 1 | 0.01006 | 0.04636 | 0.00721 | 0.06057 | 0.00725 | 0.01421 | 0.01022 |
| 97 | PCRt | 1 | 0.01699 | 0.04207 | 0.00821 | 0.05411 | 0.00544 | 0.01204 | 0.00984 |
| 98 | parafascicular thalamic nucleus | 0 | 0.76936 | 0.07222 | 0.00878 | 0.07339 | 0.00352 | 0.00000 | 0.00946 |
| 99 | paraflocculus cerebellum | 0 | 0.77101 | 0.05816 | 0.00777 | 0.05652 | 0.00995 | 0.00000 | 0.01262 |
| 100 | posterior hypothalamic area | 0 | 0.60292 | 0.10679 | 0.03004 | 0.10013 | 0.00518 | 0.00000 | 0.03049 |

### FIG. 8 (Continued)

| | | | | | | | | |
|---|---|---|---|---|---|---|---|---|---|
| 101 | pineal gland | 0 | 0.13293 | 0.18528 | 0.27628 | 0.49767 | 0.33834 | 0.00000 | 0.43682 |
| 102 | caudal piriform ctx | 0 | 0.99422 | 0.02806 | 0.01516 | 0.02801 | 0.00677 | 0.00000 | 0.01660 |
| 103 | rostral piriform ctx | 0 | 0.96366 | 0.05426 | 0.01011 | 0.05448 | 0.00478 | 0.00000 | 0.01118 |
| 104 | premammillary nucleus | 0 | 0.15401 | 0.12555 | 0.04217 | 0.09715 | 0.01454 | 0.00000 | 0.04461 |
| 105 | paramedian lobule | 0 | 0.53496 | 0.06009 | 0.00504 | 0.06253 | 0.00704 | 0.00000 | 0.00866 |
| 106 | pontine nuclei | 1 | 0.00430 | -0.01079 | 0.00889 | 0.00924 | 0.00860 | 0.02003 | 0.01237 |
| 107 | pontine reticular nucleus caudal | 1 | 0.00130 | 0.02338 | 0.00704 | 0.03921 | 0.00432 | 0.01583 | 0.00826 |
| 108 | pontine reticular nucleus oral | 1 | 0.00225 | 0.02650 | 0.00855 | 0.04229 | 0.00321 | 0.01579 | 0.00913 |
| 109 | posterior thalamic nucleus | 0 | 0.74537 | 0.07072 | 0.00983 | 0.06918 | 0.00518 | 0.00000 | 0.01112 |
| 110 | periolivary nucleus | 0 | 0.32348 | 0.04298 | 0.01233 | 0.05123 | 0.01357 | 0.00000 | 0.01834 |
| 111 | prerubral field | 0 | 0.68632 | 0.08576 | 0.01813 | 0.08257 | 0.00505 | 0.00000 | 0.01882 |
| 112 | principal sensory nucleus trigemin | 1 | 0.03685 | 0.04752 | 0.00905 | 0.05800 | 0.00495 | 0.01048 | 0.01031 |
| 113 | precuniform nucleus | 1 | 0.01220 | 0.04224 | 0.00673 | 0.05188 | 0.00323 | 0.00964 | 0.00746 |
| 114 | perirhinal ctx | 0 | 0.32014 | 0.11659 | 0.02224 | 0.10219 | 0.02289 | 0.00000 | 0.03191 |
| 115 | prelimbic ctx | 1 | 0.01921 | 0.03540 | 0.00638 | 0.04504 | 0.00487 | 0.00964 | 0.00803 |
| 116 | parietal ctx | 0 | 0.29359 | 0.04496 | 0.00765 | 0.04954 | 0.00598 | 0.00000 | 0.00971 |
| 117 | pedunculopontine tegmental area | 1 | 0.00338 | 0.03404 | 0.00780 | 0.04721 | 0.00245 | 0.01317 | 0.00818 |
| 118 | paraventricular nucleus | 0 | 0.90417 | 0.05155 | 0.00938 | 0.05083 | 0.00982 | 0.00000 | 0.01358 |
| 119 | retrochiasmatic nucleus | 1 | 0.02857 | 0.12697 | 0.04401 | 0.07166 | 0.02581 | -0.05531 | 0.05102 |
| 120 | reuniens nucleus | 0 | 0.81175 | 0.07049 | 0.01484 | 0.06893 | 0.00472 | 0.00000 | 0.01557 |
| 121 | raphe linear | 1 | 0.00547 | 0.02318 | 0.01696 | 0.05168 | 0.00851 | 0.02850 | 0.01897 |
| 122 | raphe magnus | 1 | 0.00737 | 0.01816 | 0.01244 | 0.03664 | 0.00422 | 0.01848 | 0.01313 |
| 123 | raphe obscurus nucleus | 1 | 0.02629 | 0.02294 | 0.02156 | 0.04699 | 0.00556 | 0.02404 | 0.02227 |
| 124 | red nucleus | 1 | 0.00666 | 0.03681 | 0.00670 | 0.04987 | 0.00568 | 0.01307 | 0.00878 |
| 125 | retrosplenial caudal ctx | 1 | 0.04395 | 0.06240 | 0.01161 | 0.07605 | 0.00768 | 0.01364 | 0.01392 |

## FIG. 8 (Continued)

| | | | | | | | | | |
|---|---|---|---|---|---|---|---|---|---|---|
| 126 | retrosplenial rostral ctx | 0 | 0.83115 | 0.08192 | 0.01285 | 0.08346 | 0.01049 | 0.00000 | 0.01659 |
| 127 | reticular nucleus | 0 | 0.79984 | 0.06664 | 0.01216 | 0.06528 | 0.00389 | 0.00000 | 0.01277 |
| 128 | reticular nucleus midbrain | 1 | 0.00029 | 0.04189 | 0.00516 | 0.05593 | 0.00288 | 0.01405 | 0.00591 |
| 129 | reticulotegmental nucleus | 1 | 0.00075 | 0.00864 | 0.00922 | 0.03617 | 0.00902 | 0.02753 | 0.01290 |
| 130 | primary somatosensory ctx barrel f | 0 | 0.89085 | 0.05503 | 0.00939 | 0.05434 | 0.00675 | 0.00000 | 0.01157 |
| 131 | primary somatosensory ctx forelimb | 0 | 0.68497 | 0.04283 | 0.01068 | 0.04480 | 0.00410 | 0.00000 | 0.01144 |
| 132 | primary somatosensory ctx hindlimb | 0 | 0.25907 | 0.04051 | 0.00884 | 0.04556 | 0.00491 | 0.00000 | 0.01011 |
| 133 | primary somatosensory ctx jaw | 0 | 0.84028 | 0.04335 | 0.01152 | 0.04444 | 0.00544 | 0.00000 | 0.01274 |
| 134 | primary somatosensory ctx shoulder | 0 | 0.87323 | 0.04458 | 0.01102 | 0.04541 | 0.00529 | 0.00000 | 0.01222 |
| 135 | primary somatosensory ctx trunk | 0 | 0.21964 | 0.04095 | 0.00696 | 0.04581 | 0.00529 | 0.00000 | 0.00874 |
| 136 | primary somatosensory ctx upper li | 0 | 0.86635 | 0.05696 | 0.01157 | 0.05605 | 0.00513 | 0.00000 | 0.01266 |
| 137 | secondary somaotsensory ctx | 0 | 0.43957 | 0.06589 | 0.01038 | 0.06155 | 0.00744 | 0.00000 | 0.01277 |
| 138 | suprachiasmatic nucleus | 0 | 0.67284 | 0.02798 | 0.03520 | 0.03494 | 0.01606 | 0.00000 | 0.03869 |
| 139 | substantia innominata | 0 | 0.40158 | 0.06873 | 0.01109 | 0.06312 | 0.01003 | 0.00000 | 0.01496 |
| 140 | simple lobule cerebellum | 0 | 0.69755 | 0.02732 | 0.00584 | 0.02902 | 0.00777 | 0.00000 | 0.00972 |
| 141 | substantia nigra compacta | 0 | 0.76445 | 0.07760 | 0.07125 | 0.06869 | 0.00457 | 0.00000 | 0.07139 |
| 142 | substantia nigra reticularis | 0 | 0.77785 | 0.09874 | 0.10177 | 0.08627 | 0.02739 | 0.00000 | 0.10540 |
| 143 | supraoptic nucleus | 0 | 0.50602 | 0.05956 | 0.01981 | 0.05341 | 0.00861 | 0.00000 | 0.02160 |
| 144 | solitary tract nucleus | 1 | 0.03808 | 0.04598 | 0.00745 | 0.05434 | 0.00371 | 0.00836 | 0.00832 |
| 145 | bed nucleus stria terminalis | 0 | 0.13228 | 0.04274 | 0.00877 | 0.04969 | 0.00498 | 0.00000 | 0.01009 |
| 146 | subthalamic nucleus | 0 | 0.24816 | 0.13031 | 0.03779 | 0.10814 | 0.02098 | 0.00000 | 0.04322 |
| 147 | superior colliculus | 1 | 0.00470 | 0.05328 | 0.00592 | 0.06283 | 0.00205 | 0.00955 | 0.00626 |
| 148 | sub coeruleus nucleus | 1 | 0.00541 | 0.03471 | 0.00705 | 0.04742 | 0.00448 | 0.01271 | 0.00836 |
| 149 | supramammillary nucleus | 0 | 0.82588 | 0.17514 | 0.07153 | 0.16770 | 0.03468 | 0.00000 | 0.07950 |
| 150 | temporal ctx | 0 | 0.33752 | 0.14307 | 0.01732 | 0.12943 | 0.02550 | 0.00000 | 0.03083 |

*FIG. 8 (Continued)*

| | | | | | | | | |
|---|---|---|---|---|---|---|---|---|---|
| 151 | triangular septal nucleus | 0 | 0.71754 | 0.05443 | 0.01283 | 0.05642 | 0.00277 | 0.00000 | 0.01313 |
| 152 | tenia tecta ctx | 0 | 0.14944 | 0.01456 | 0.01451 | 0.02652 | 0.01069 | 0.00000 | 0.01803 |
| 153 | olfactory tubercles | 0 | 0.33396 | 0.06007 | 0.00918 | 0.05488 | 0.00770 | 0.00000 | 0.01199 |
| 154 | trapezoid body | 1 | 0.03917 | 0.02325 | 0.01109 | 0.03865 | 0.01010 | 0.01540 | 0.01500 |
| 155 | Ventricle | 0 | 0.96289 | 0.05033 | 0.01422 | 0.05063 | 0.00526 | 0.00000 | 0.01516 |
| 156 | visual 1 ctx | 1 | 0.01481 | 0.05966 | 0.00534 | 0.06957 | 0.00553 | 0.00991 | 0.00769 |
| 157 | visual 2 ctx | 0 | 0.14867 | 0.06477 | 0.00668 | 0.07085 | 0.00607 | 0.00000 | 0.00903 |
| 158 | ventral anterior thalamic nucleus | 0 | 0.78993 | 0.05839 | 0.00819 | 0.05933 | 0.00211 | 0.00000 | 0.00845 |
| 159 | cochlear nucleus | 0 | 0.15485 | 0.06133 | 0.00903 | 0.07489 | 0.01759 | 0.00000 | 0.01977 |
| 160 | vestibular nucleus | 0 | 0.08530 | 0.05782 | 0.01242 | 0.07068 | 0.00969 | 0.00000 | 0.01576 |
| 161 | ventrolateral thalamic nucleus | 0 | 0.92879 | 0.06507 | 0.01298 | 0.06560 | 0.00544 | 0.00000 | 0.01408 |
| 162 | ventral lateral striatum | 0 | 0.88879 | 0.04619 | 0.01016 | 0.04687 | 0.00517 | 0.00000 | 0.01140 |
| 163 | ventromedial thalamic nucleus | 0 | 0.52893 | 0.07888 | 0.01520 | 0.07464 | 0.00453 | 0.00000 | 0.01586 |
| 164 | ventral medial nucleus | 0 | 0.06252 | 0.09370 | 0.01741 | 0.07417 | 0.01313 | 0.00000 | 0.02180 |
| 165 | ventral medial striatum | 0 | 0.23052 | 0.03433 | 0.00885 | 0.03952 | 0.00415 | 0.00000 | 0.00978 |
| 166 | ventral orbital ctx | 0 | 0.08135 | 0.02421 | 0.00442 | 0.03176 | 0.00755 | 0.00000 | 0.00875 |
| 167 | ventral pallidum | 0 | 0.83781 | 0.04601 | 0.00527 | 0.04680 | 0.00684 | 0.00000 | 0.00863 |
| 168 | ventral posteriolateral thalamic n | 0 | 0.67830 | 0.07518 | 0.00991 | 0.07307 | 0.00633 | 0.00000 | 0.01176 |
| 169 | ventral posteriolmedial thalamic n | 0 | 0.24155 | 0.07815 | 0.01170 | 0.07156 | 0.00508 | 0.00000 | 0.01275 |
| 170 | ventral subiculum | 1 | 0.00164 | 0.04688 | 0.00431 | 0.06497 | 0.00818 | 0.01808 | 0.00924 |
| 171 | ventral tegmental area | 1 | 0.01432 | 0.00731 | 0.02798 | 0.04805 | 0.01621 | 0.04074 | 0.03234 |
| 172 | White Matter | 0 | 0.35671 | 0.04795 | 0.00744 | 0.05127 | 0.00363 | 0.00000 | 0.00828 |
| 173 | White Matter | 0 | 0.72472 | 0.05908 | 0.01163 | 0.06115 | 0.00717 | 0.00000 | 0.01366 |
| 174 | zona incerta | 0 | 0.09742 | 0.09361 | 0.01316 | 0.08255 | 0.00608 | 0.00000 | 0.01450 |

44

*FIG. 8 (Continued)*

EP 4 741 014 A2

44 Significant Regions
39 positive
 5 negative

APOE4-WT (shown if significant)

*FIG. 9*

# MEAN APOE vs WT

| Region Num | Region Nam | H | P | WT Mean | WT std | APOE4 Mean | APOE4 std | APOE4-WT diff | APOE4-WT std |
|---|---|---|---|---|---|---|---|---|---|
| 1 | 10th cerebellar lobule | 0 | 0.24788 | 0.04365 | 0.01236 | 0.03657 | 0.00325 | 0.00000 | 0.01278 |
| 2 | 1st cerebellar lobule | 1 | 0.00033 | 0.08240 | 0.01879 | 0.03319 | 0.00542 | -0.04921 | 0.01955 |
| 3 | 2nd cerebellar lobule | 1 | 0.00054 | 0.04899 | 0.00603 | 0.03014 | 0.00585 | -0.01885 | 0.00840 |
| 4 | 3rd cerebellar lobule | 0 | 0.13767 | 0.03198 | 0.00373 | 0.02798 | 0.00443 | 0.00000 | 0.00579 |
| 5 | 4th cerebellar lobule | 0 | 0.44962 | 0.02928 | 0.00634 | 0.02641 | 0.00556 | 0.00000 | 0.00843 |
| 6 | 5th cerebellar lobule | 0 | 0.48947 | 0.03686 | 0.01004 | 0.04123 | 0.01003 | 0.00000 | 0.01419 |
| 7 | motor trigeminal nucleus | 0 | 0.19794 | 0.03725 | 0.00435 | 0.04090 | 0.00430 | 0.00000 | 0.00612 |
| 8 | root of trigeminal nerve | 0 | 0.12535 | 0.06551 | 0.00572 | 0.05726 | 0.01026 | 0.00000 | 0.01174 |
| 9 | 6th cerebellar lobule | 0 | 0.90268 | 0.03732 | 0.00363 | 0.03681 | 0.00912 | 0.00000 | 0.00982 |
| 10 | 7th cerebellar lobule | 1 | 0.00781 | 0.04441 | 0.00833 | 0.02854 | 0.00682 | -0.01587 | 0.01077 |
| 11 | facial nucleus | 1 | 0.00506 | 0.06907 | 0.00690 | 0.04520 | 0.01409 | -0.02387 | 0.01569 |
| 12 | 8th cerebellar lobule | 0 | 0.09432 | 0.04141 | 0.00346 | 0.03656 | 0.00515 | 0.00000 | 0.00620 |
| 13 | 9th cerebellar lobule | 0 | 0.12898 | 0.03901 | 0.00634 | 0.03306 | 0.00524 | 0.00000 | 0.00823 |
| 14 | anterior thalamic nuclei | 0 | 0.08184 | 0.07218 | 0.01184 | 0.05589 | 0.01580 | 0.00000 | 0.01974 |
| 15 | anterior amygdaloid nucleus | 1 | 0.01668 | 0.13949 | 0.04374 | 0.07696 | 0.01996 | -0.06253 | 0.04808 |
| 16 | accumbens core | 0 | 0.18319 | 0.03066 | 0.00768 | 0.02382 | 0.00802 | 0.00000 | 0.01111 |
| 17 | accumbens shell | 1 | 0.01799 | 0.03957 | 0.00931 | 0.02488 | 0.00711 | -0.01469 | 0.01171 |
| 18 | anterior hypothalamic area | 1 | 0.01770 | 0.12279 | 0.03518 | 0.07473 | 0.01194 | -0.04806 | 0.03715 |
| 19 | anterior lobe pituitary | 0 | 0.19301 | 0.13343 | 0.04891 | 0.09874 | 0.02721 | 0.00000 | 0.05597 |
| 20 | anterior olfactory nucleus | 0 | 0.12314 | 0.03127 | 0.01555 | 0.01739 | 0.01030 | 0.00000 | 0.01865 |
| 21 | anterior pretectal nucleus | 1 | 0.00411 | 0.05306 | 0.00948 | 0.03306 | 0.00751 | -0.02000 | 0.01209 |
| 22 | arcuate nucleus | 0 | 0.39808 | 0.13638 | 0.06566 | 0.10728 | 0.03488 | 0.00000 | 0.07435 |
| 23 | auditory ctx | 1 | 0.02539 | 0.09593 | 0.01433 | 0.06836 | 0.01988 | -0.02757 | 0.02450 |
| 24 | basal amygdaloid nucleus | 1 | 0.01556 | 0.10557 | 0.02732 | 0.06547 | 0.01348 | -0.04011 | 0.03047 |
| 25 | CA1 dorsal | 0 | 0.07948 | 0.08382 | 0.01974 | 0.06453 | 0.00984 | 0.00000 | 0.02206 |

*FIG. 10*

| | | | | | | | | |
|---|---|---|---|---|---|---|---|---|---|
| 26 | CA1 hippocampus ventral | 0 | 0.18107 | 0.04921 | 0.01489 | 0.06336 | 0.01754 | 0.00000 | 0.02301 |
| 27 | CA2 | 0 | 0.15029 | 0.06641 | 0.01816 | 0.05264 | 0.00763 | 0.00000 | 0.01970 |
| 28 | CA3 dorsal | 1 | 0.01198 | 0.10137 | 0.01562 | 0.06893 | 0.01873 | -0.03244 | 0.02439 |
| 29 | CA3 hippocampus ventral | 0 | 0.93715 | 0.11705 | 0.03051 | 0.11899 | 0.04833 | 0.00000 | 0.05715 |
| 30 | central amygdaloid  nucleus | 0 | 0.36808 | 0.10304 | 0.02163 | 0.09248 | 0.01332 | 0.00000 | 0.02541 |
| 31 | anterior cingulate area | 0 | 0.78964 | 0.07075 | 0.01863 | 0.06804 | 0.01263 | 0.00000 | 0.02251 |
| 32 | central gray | 0 | 0.71350 | 0.05123 | 0.01066 | 0.04899 | 0.00852 | 0.00000 | 0.01364 |
| 33 | claustrum | 1 | 0.02913 | 0.03957 | 0.00698 | 0.03018 | 0.00442 | -0.00938 | 0.00826 |
| 34 | central medial thalamic nucleus | 0 | 0.62487 | 0.05909 | 0.01149 | 0.05588 | 0.00903 | 0.00000 | 0.01461 |
| 35 | cortical amygdaloid nucleus | 1 | 0.03776 | 0.11330 | 0.02803 | 0.08016 | 0.01249 | -0.03315 | 0.03069 |
| 36 | copula of the pyramis | 0 | 0.12123 | 0.07588 | 0.01505 | 0.05774 | 0.02015 | 0.00000 | 0.02515 |
| 37 | crus 1 of ansiform lobule | 0 | 0.08004 | 0.04531 | 0.00499 | 0.03682 | 0.00908 | 0.00000 | 0.01036 |
| 38 | crus 2 of ansiform lobule | 0 | 0.09191 | 0.04212 | 0.00393 | 0.03535 | 0.00773 | 0.00000 | 0.00867 |
| 39 | diagonal band of Broca | 1 | 0.01827 | 0.08490 | 0.01801 | 0.04982 | 0.02252 | -0.03508 | 0.02884 |
| 40 | dentate gyrus dorsal | 1 | 0.00927 | 0.13036 | 0.02177 | 0.09143 | 0.01622 | -0.03893 | 0.02715 |
| 41 | dentate gyrus ventral | 0 | 0.20230 | 0.11236 | 0.02064 | 0.13341 | 0.03009 | 0.00000 | 0.03649 |
| 42 | dorsal lateral striatum | 0 | 0.17817 | 0.05112 | 0.00994 | 0.04373 | 0.00582 | 0.00000 | 0.01151 |
| 43 | dorsal medial nucleus | 0 | 0.18056 | 0.11266 | 0.02239 | 0.09394 | 0.01983 | 0.00000 | 0.02991 |
| 44 | dorsal medial striatum | 0 | 0.18465 | 0.05835 | 0.00951 | 0.04986 | 0.01008 | 0.00000 | 0.01386 |
| 45 | dorsomedial tegmental area | 1 | 0.00061 | 0.03250 | 0.00449 | 0.04851 | 0.00585 | 0.01602 | 0.00738 |
| 46 | DPGi | 1 | 0.00001 | 0.03269 | 0.00827 | 0.07153 | 0.00538 | 0.03884 | 0.00987 |
| 47 | dorsal raphe | 1 | 0.02096 | 0.05663 | 0.00653 | 0.07689 | 0.01642 | 0.02026 | 0.01768 |
| 48 | subiculum dorsal | 0 | 0.27996 | 0.10719 | 0.02286 | 0.08873 | 0.03048 | 0.00000 | 0.03810 |
| 49 | extended amydala | 1 | 0.02443 | 0.09690 | 0.02439 | 0.06334 | 0.01433 | -0.03357 | 0.02829 |
| 50 | ectorhinal ctx | 1 | 0.04772 | 0.22849 | 0.01854 | 0.27241 | 0.04273 | 0.04392 | 0.04658 |

**FIG. 10 (Continued)**

| | | | | | | | | | |
|---|---|---|---|---|---|---|---|---|---|---|
| 51 | endopiriform nucleus | 1 | 0.01367 | 0.04379 | 0.00826 | 0.02988 | 0.00647 | -0.01391 | 0.01049 |
| 52 | entorhinal ctx | 0 | 0.13770 | 0.08229 | 0.01511 | 0.10097 | 0.02283 | 0.00000 | 0.02738 |
| 53 | external plexiform layer | 0 | 0.91367 | 0.12462 | 0.02708 | 0.12313 | 0.01334 | 0.00000 | 0.03019 |
| 54 | flocculus cerebellum | 0 | 0.15415 | 0.07648 | 0.01283 | 0.06312 | 0.01570 | 0.00000 | 0.02028 |
| 55 | frontal association ctx | 0 | 0.78317 | 0.04245 | 0.01534 | 0.04551 | 0.02049 | 0.00000 | 0.02560 |
| 56 | gigantocellular reticular nucleus | 1 | 0.00328 | 0.05510 | 0.01184 | 0.03185 | 0.00598 | -0.02325 | 0.01327 |
| 57 | glomerular layer | 0 | 0.07331 | 0.13559 | 0.02947 | 0.16598 | 0.01715 | 0.00000 | 0.03410 |
| 58 | globus pallidus | 0 | 0.09633 | 0.07553 | 0.01852 | 0.05734 | 0.01268 | 0.00000 | 0.02244 |
| 59 | granular cell layer | 0 | 0.34457 | 0.10083 | 0.02924 | 0.08662 | 0.01328 | 0.00000 | 0.03211 |
| 60 | habenula nucleus | 1 | 0.00142 | 0.07122 | 0.02943 | 0.13433 | 0.01031 | 0.06310 | 0.03118 |
| 61 | intercalated amygdaloid nucleus | 0 | 0.86817 | 0.08353 | 0.03006 | 0.08776 | 0.05137 | 0.00000 | 0.05952 |
| 62 | inferior colliculus | 0 | 0.35624 | 0.07401 | 0.02058 | 0.09021 | 0.03427 | 0.00000 | 0.03997 |
| 63 | infralimbic ctx | 0 | 0.78173 | 0.03617 | 0.01302 | 0.03791 | 0.00396 | 0.00000 | 0.01361 |
| 64 | insular ctx | 1 | 0.00264 | 0.06930 | 0.01240 | 0.04554 | 0.00360 | -0.02376 | 0.01291 |
| 65 | interposed nucleus | 1 | 0.00398 | 0.08332 | 0.02036 | 0.04313 | 0.01244 | -0.04019 | 0.02386 |
| 66 | inferior olivary complex | 1 | 0.01163 | 0.09975 | 0.03214 | 0.04582 | 0.02238 | -0.05392 | 0.03916 |
| 67 | interpeduncular nucleus | 0 | 0.32862 | 0.03643 | 0.03226 | 0.01978 | 0.01710 | 0.00000 | 0.03651 |
| 68 | lateral amygdaloid nucleus | 0 | 0.26484 | 0.06156 | 0.02128 | 0.04776 | 0.01615 | 0.00000 | 0.02672 |
| 69 | Lat | 1 | 0.00058 | 0.09696 | 0.01605 | 0.05439 | 0.00957 | -0.04257 | 0.01869 |
| 70 | locus ceruleus | 0 | 0.38320 | 0.06900 | 0.02236 | 0.05697 | 0.02077 | 0.00000 | 0.03052 |
| 71 | lateral dorsal thalamic nucleus | 0 | 0.28456 | 0.11933 | 0.02940 | 0.09942 | 0.02825 | 0.00000 | 0.04077 |
| 72 | lateral geniculate | 0 | 0.14738 | 0.09992 | 0.03639 | 0.13413 | 0.03467 | 0.00000 | 0.05026 |
| 73 | lateral hypothalamus | 1 | 0.01609 | 0.15275 | 0.02918 | 0.10757 | 0.01923 | -0.04517 | 0.03494 |
| 74 | lemniscal nucleus | 0 | 0.68120 | 0.05733 | 0.00481 | 0.05559 | 0.00858 | 0.00000 | 0.00983 |
| 75 | lateral orbital ctx | 0 | 0.93517 | 0.01928 | 0.00734 | 0.01957 | 0.00302 | 0.00000 | 0.00794 |

**FIG. 10 (Continued)**

EP 4 741 014 A2

| | | | | | | | | |
|---|---|---|---|---|---|---|---|---|---|
| 76 | lateral posterior thalamic nucleus | 1 | 0.01835 | 0.08556 | 0.01685 | 0.11975 | 0.02266 | 0.03419 | 0.02824 |
| 77 | lateral preoptic area | 1 | 0.03018 | 0.07898 | 0.01788 | 0.05439 | 0.01273 | -0.02459 | 0.02195 |
| 78 | lateral septal nucleus | 0 | 0.65404 | 0.06906 | 0.01155 | 0.06549 | 0.01405 | 0.00000 | 0.01819 |
| 79 | primary motor ctx | 0 | 0.06946 | 0.04598 | 0.00824 | 0.03653 | 0.00666 | 0.00000 | 0.01060 |
| 80 | secondary motor ctx | 0 | 0.13509 | 0.06614 | 0.02359 | 0.04687 | 0.01225 | 0.00000 | 0.02658 |
| 81 | magnocellular preoptic nucleus | 0 | 0.08576 | 0.17030 | 0.05479 | 0.10825 | 0.05094 | 0.00000 | 0.07481 |
| 82 | medial dorsal thalamic nucleus | 1 | 0.00752 | 0.05629 | 0.00706 | 0.07960 | 0.01487 | 0.02331 | 0.01647 |
| 83 | medial amygdaloid nucleus | 0 | 0.29125 | 0.18952 | 0.03058 | 0.16627 | 0.03833 | 0.00000 | 0.04903 |
| 84 | medial cerebellar nucleus fastigia | 1 | 0.00490 | 0.06035 | 0.01420 | 0.03366 | 0.00818 | -0.02669 | 0.01639 |
| 85 | medial geniculate | 1 | 0.03342 | 0.12159 | 0.02666 | 0.08817 | 0.01419 | -0.03342 | 0.03020 |
| 86 | medial mammillary nucleus | 0 | 0.85800 | 0.18312 | 0.04991 | 0.17824 | 0.03459 | 0.00000 | 0.06073 |
| 87 | median raphe nucleus | 1 | 0.00580 | 0.03425 | 0.00494 | 0.04794 | 0.00765 | 0.01369 | 0.00911 |
| 88 | medial orbital ctx | 0 | 0.19890 | 0.02647 | 0.01829 | 0.04035 | 0.01403 | 0.00000 | 0.02305 |
| 89 | medial preoptic area | 1 | 0.01530 | 0.08046 | 0.02245 | 0.04861 | 0.00824 | -0.03185 | 0.02392 |
| 90 | medial pretectal area | 0 | 0.50793 | 0.05928 | 0.01156 | 0.04985 | 0.03129 | 0.00000 | 0.03335 |
| 91 | medial septum | 0 | 0.26940 | 0.03940 | 0.01350 | 0.04749 | 0.00788 | 0.00000 | 0.01563 |
| 92 | neural lobe pituitary | 0 | 0.41653 | 0.12502 | 0.06787 | 0.09655 | 0.03322 | 0.00000 | 0.07557 |
| 93 | olivary nucleus | 1 | 0.02420 | 0.06617 | 0.02708 | 0.03121 | 0.01041 | -0.03497 | 0.02902 |
| 94 | paraventricular nuclus | 1 | 0.00145 | 0.08148 | 0.01320 | 0.05277 | 0.00549 | -0.02871 | 0.01429 |
| 95 | periaqueductal gray thalamus | 0 | 0.06173 | 0.05251 | 0.00768 | 0.06153 | 0.00601 | 0.00000 | 0.00975 |
| 96 | parabrachial nucleus | 0 | 0.76661 | 0.04948 | 0.00955 | 0.04775 | 0.00900 | 0.00000 | 0.01312 |
| 97 | PCRt | 1 | 0.03911 | 0.06942 | 0.01133 | 0.05061 | 0.01458 | -0.01881 | 0.01847 |
| 98 | parafascicular thalamic nucleus | 0 | 0.05614 | 0.05183 | 0.00639 | 0.04135 | 0.00945 | 0.00000 | 0.01141 |
| 99 | paraflocculus cerebellum | 1 | 0.00001 | 0.09451 | 0.00748 | 0.06339 | 0.00372 | -0.03112 | 0.00835 |
| 100 | posterior hypothalamic area | 1 | 0.04777 | 0.12291 | 0.03958 | 0.08118 | 0.00887 | -0.04172 | 0.04056 |

**FIG. 10 (Continued)**

| | | | | | | | | | |
|---|---|---|---|---|---|---|---|---|---|
| 101 | pineal gland | 0 | 0.69791 | 0.24789 | 0.11499 | 0.22089 | 0.10631 | 0.00000 | 0.15661 |
| 102 | caudal piriform ctx | 1 | 0.00168 | 0.13865 | 0.03257 | 0.06980 | 0.01285 | -0.06885 | 0.03501 |
| 103 | rostral piriform ctx | 1 | 0.00949 | 0.03475 | 0.00909 | 0.02014 | 0.00427 | -0.01461 | 0.01005 |
| 104 | premammillary nucleus | 0 | 0.21751 | 0.13991 | 0.03224 | 0.16343 | 0.02510 | 0.00000 | 0.04086 |
| 105 | paramedian lobule | 1 | 0.00210 | 0.06737 | 0.01031 | 0.04293 | 0.00829 | -0.02444 | 0.01323 |
| 106 | pontine nuclei | 0 | 0.15925 | 0.02948 | 0.01389 | 0.01929 | 0.00541 | 0.00000 | 0.01491 |
| 107 | pontine reticular nucleus caudal | 0 | 0.18277 | 0.03242 | 0.00624 | 0.02756 | 0.00456 | 0.00000 | 0.00773 |
| 108 | pontine reticular nucleus oral | 0 | 0.05562 | 0.03395 | 0.00309 | 0.03910 | 0.00466 | 0.00000 | 0.00559 |
| 109 | posterior thalamic nucleus | 0 | 0.61530 | 0.06059 | 0.00751 | 0.05806 | 0.00862 | 0.00000 | 0.01143 |
| 110 | periolivary nucleus | 1 | 0.00107 | 0.07620 | 0.02111 | 0.03060 | 0.00361 | -0.04560 | 0.02141 |
| 111 | prerubral field | 0 | 0.14876 | 0.05524 | 0.01511 | 0.04271 | 0.01005 | 0.00000 | 0.01814 |
| 112 | principal sensory nucleus trigemin | 1 | 0.02290 | 0.06449 | 0.01094 | 0.04938 | 0.00609 | -0.01511 | 0.01253 |
| 113 | precuniform nucleus | 0 | 0.09478 | 0.05819 | 0.00817 | 0.06709 | 0.00750 | 0.00000 | 0.01109 |
| 114 | perirhinal ctx | 0 | 0.09012 | 0.18139 | 0.06466 | 0.12494 | 0.01414 | 0.00000 | 0.06619 |
| 115 | prelimbic ctx | 0 | 0.40634 | 0.03603 | 0.00679 | 0.03955 | 0.00650 | 0.00000 | 0.00940 |
| 116 | parietal ctx | 1 | 0.02982 | 0.06570 | 0.01233 | 0.05020 | 0.00564 | -0.01550 | 0.01356 |
| 117 | pedunculopontine tegmental area | 1 | 0.00473 | 0.04658 | 0.00496 | 0.05839 | 0.00556 | 0.01181 | 0.00745 |
| 118 | paraventricular nucleus | 0 | 0.91772 | 0.05793 | 0.01026 | 0.05879 | 0.01647 | 0.00000 | 0.01940 |
| 119 | retrochiasmatic nucleus | 1 | 0.00293 | 0.26512 | 0.08782 | 0.09104 | 0.04192 | -0.17409 | 0.09731 |
| 120 | reuniens nucleus | 1 | 0.01997 | 0.07625 | 0.01173 | 0.05983 | 0.00597 | -0.01642 | 0.01316 |
| 121 | raphe linear | 0 | 0.59151 | 0.02883 | 0.01183 | 0.03193 | 0.00393 | 0.00000 | 0.01247 |
| 122 | raphe magnus | 1 | 0.02694 | 0.04734 | 0.02197 | 0.01983 | 0.00795 | -0.02751 | 0.02336 |
| 123 | raphe obscurus nucleus | 0 | 0.06719 | 0.03854 | 0.01284 | 0.02370 | 0.01028 | 0.00000 | 0.01645 |
| 124 | red nucleus | 0 | 0.28669 | 0.02568 | 0.01434 | 0.03316 | 0.00318 | 0.00000 | 0.01469 |
| 125 | retrosplenial caudal ctx | 0 | 0.86395 | 0.26168 | 0.07218 | 0.26926 | 0.06941 | 0.00000 | 0.10014 |

*FIG. 10 (Continued)*

| | | | | | | | | | |
|---|---|---|---|---|---|---|---|---|---|
| 126 | retrosplenial  rostral ctx | 0 | 0.36625 | 0.12995 | 0.03451 | 0.11053 | 0.03267 | 0.00000 | 0.04752 |
| 127 | reticular nucleus | 0 | 0.38550 | 0.06890 | 0.01097 | 0.06197 | 0.01429 | 0.00000 | 0.01801 |
| 128 | reticular nucleus midbrain | 0 | 0.86168 | 0.04732 | 0.00781 | 0.04801 | 0.00378 | 0.00000 | 0.00867 |
| 129 | reticulotegmental nucleus | 0 | 0.19074 | 0.02617 | 0.00465 | 0.03151 | 0.00777 | 0.00000 | 0.00906 |
| 130 | primary somatosensory ctx barrel f | 1 | 0.03210 | 0.05424 | 0.00708 | 0.04262 | 0.00815 | -0.01162 | 0.01080 |
| 131 | primary somatosensory ctx forelimb | 1 | 0.02223 | 0.04479 | 0.00599 | 0.03532 | 0.00525 | -0.00947 | 0.00796 |
| 132 | primary somatosensory ctx hindlimb | 1 | 0.03327 | 0.04581 | 0.00657 | 0.03667 | 0.00523 | -0.00914 | 0.00840 |
| 133 | primary somatosensory ctx jaw | 0 | 0.06264 | 0.03899 | 0.00592 | 0.03164 | 0.00546 | 0.00000 | 0.00805 |
| 134 | primary somatosensory ctx shoulder | 0 | 0.14985 | 0.04529 | 0.00803 | 0.03847 | 0.00588 | 0.00000 | 0.00995 |
| 135 | primary somatosensory ctx trunk | 0 | 0.22080 | 0.05069 | 0.00892 | 0.04313 | 0.01014 | 0.00000 | 0.01350 |
| 136 | primary somatosensory ctx upper li | 1 | 0.01168 | 0.05372 | 0.00761 | 0.04106 | 0.00515 | -0.01266 | 0.00919 |
| 137 | secondary somaotsensory ctx | 1 | 0.00561 | 0.07582 | 0.01631 | 0.04781 | 0.00596 | -0.02801 | 0.01737 |
| 138 | suprachiasmatic nucleus | 1 | 0.00336 | 0.13002 | 0.03907 | 0.05122 | 0.02312 | -0.07880 | 0.04540 |
| 139 | substantia innominata | 1 | 0.03529 | 0.10181 | 0.03837 | 0.05289 | 0.02361 | -0.04892 | 0.04505 |
| 140 | simple lobule cerebellum | 0 | 0.10514 | 0.03530 | 0.00707 | 0.04635 | 0.01298 | 0.00000 | 0.01478 |
| 141 | substantia nigra compacta | 0 | 0.18205 | 0.04727 | 0.02130 | 0.03276 | 0.00711 | 0.00000 | 0.02245 |
| 142 | substantia nigra reticularis | 0 | 0.09162 | 0.08047 | 0.03496 | 0.05003 | 0.00818 | 0.00000 | 0.03590 |
| 143 | supraoptic nucleus | 1 | 0.00752 | 0.13335 | 0.03724 | 0.06603 | 0.02515 | -0.06732 | 0.04493 |
| 144 | solitary tract nucleus | 0 | 0.91463 | 0.04127 | 0.00915 | 0.04208 | 0.01497 | 0.00000 | 0.01755 |
| 145 | bed nucleus stria terminalis | 0 | 0.05334 | 0.06080 | 0.01207 | 0.04625 | 0.00901 | 0.00000 | 0.01506 |
| 146 | subthalamic nucleus | 1 | 0.03578 | 0.11858 | 0.04462 | 0.06693 | 0.01426 | -0.05166 | 0.04684 |
| 147 | superior colliculus | 0 | 0.07770 | 0.08040 | 0.01875 | 0.13365 | 0.06285 | 0.00000 | 0.06559 |
| 148 | sub coeruleus nucleus | 0 | 0.50927 | 0.03799 | 0.00411 | 0.03602 | 0.00546 | 0.00000 | 0.00683 |
| 149 | supramammillary nucleus | 0 | 0.44382 | 0.17871 | 0.05914 | 0.15595 | 0.02421 | 0.00000 | 0.06390 |
| 150 | temporal ctx | 0 | 0.14904 | 0.24997 | 0.02553 | 0.20390 | 0.06646 | 0.00000 | 0.07119 |

**FIG. 10 (Continued)**

| | | | | | | | | | |
|---|---|---|---|---|---|---|---|---|---|
| 151 | triangular septal nucleus | 0 | 0.48340 | 0.05214 | 0.01390 | 0.04600 | 0.01384 | 0.00000 | 0.01962 |
| 152 | tenia tecta ctx | 0 | 0.58949 | 0.07083 | 0.01675 | 0.06618 | 0.00851 | 0.00000 | 0.01879 |
| 153 | olfactory tubercles | 0 | 0.06248 | 0.09448 | 0.02160 | 0.06724 | 0.02061 | 0.00000 | 0.02986 |
| 154 | trapezoid body | 1 | 0.00024 | 0.05487 | 0.01228 | 0.02147 | 0.00311 | -0.03340 | 0.01266 |
| 155 | Ventricle | 1 | 0.00094 | 0.08464 | 0.01317 | 0.11779 | 0.00854 | 0.03315 | 0.01570 |
| 156 | visual 1 ctx | 0 | 0.26246 | 0.20194 | 0.02848 | 0.18413 | 0.01866 | 0.00000 | 0.03405 |
| 157 | visual 2 ctx | 0 | 0.08838 | 0.18855 | 0.04674 | 0.14593 | 0.01797 | 0.00000 | 0.05008 |
| 158 | ventral anterior thalamic nucleus | 0 | 0.12906 | 0.06215 | 0.00993 | 0.05187 | 0.01044 | 0.00000 | 0.01441 |
| 159 | cochlear nucleus | 0 | 0.81158 | 0.08057 | 0.01188 | 0.07838 | 0.01771 | 0.00000 | 0.02132 |
| 160 | vestibular nucleus | 1 | 0.00008 | 0.04510 | 0.00684 | 0.07839 | 0.00945 | 0.03330 | 0.01166 |
| 161 | ventrolateral thalamic nucleus | 0 | 0.34460 | 0.05996 | 0.00750 | 0.05502 | 0.00894 | 0.00000 | 0.01167 |
| 162 | ventral lateral striatum | 1 | 0.02717 | 0.05613 | 0.01126 | 0.04151 | 0.00553 | -0.01462 | 0.01254 |
| 163 | ventromedial thalamic nucleus | 1 | 0.01557 | 0.07618 | 0.01227 | 0.05717 | 0.00789 | -0.01901 | 0.01459 |
| 164 | ventral medial nucleus | 0 | 0.13713 | 0.15092 | 0.06652 | 0.10107 | 0.01396 | 0.00000 | 0.06797 |
| 165 | ventral medial striatum | 0 | 0.11530 | 0.04824 | 0.00923 | 0.03790 | 0.01048 | 0.00000 | 0.01396 |
| 166 | ventral orbital ctx | 0 | 0.47479 | 0.02125 | 0.01046 | 0.01704 | 0.00768 | 0.00000 | 0.01298 |
| 167 | ventral pallidum | 1 | 0.00403 | 0.06602 | 0.01395 | 0.03761 | 0.00972 | -0.02841 | 0.01700 |
| 168 | ventral posteriolateral thalamic n | 0 | 0.22778 | 0.06869 | 0.00940 | 0.06081 | 0.01080 | 0.00000 | 0.01432 |
| 169 | ventral posteriolmedial thalamic n | 0 | 0.11753 | 0.07364 | 0.00985 | 0.06235 | 0.01183 | 0.00000 | 0.01540 |
| 170 | ventral subiculum | 0 | 0.17358 | 0.07729 | 0.01640 | 0.09458 | 0.02244 | 0.00000 | 0.02779 |
| 171 | ventral tegmental area | 0 | 0.17046 | 0.05119 | 0.04164 | 0.02276 | 0.00822 | 0.00000 | 0.04244 |
| 172 | White Matter | 1 | 0.01500 | 0.08424 | 0.01407 | 0.06022 | 0.01211 | -0.02402 | 0.01856 |
| 173 | White Matter | 0 | 0.05336 | 0.09229 | 0.02072 | 0.06405 | 0.02132 | 0.00000 | 0.02973 |
| 174 | zona incerta | 0 | 0.13053 | 0.09166 | 0.01967 | 0.07427 | 0.01365 | 0.00000 | 0.02394 |
| - | Whole brain | 0 | 0.05849 | 0.07889 | 0.01078 | 0.06723 | 0.00569 | 0.00000 | 0.01219 |

*FIG. 10 (Continued)*

EP 4 741 014 A2

FIG. 11

# MODE APOE vs WT

| Region Num | Region Nam | H | P | WT Mean | WT std | APOE4 Mean | APOE4 std | APOE4-WT diff | APOE4-WT std |
|---|---|---|---|---|---|---|---|---|---|
| 1 | 10th cerebellar lobule | 0 | 0.07467 | 0.04735 | 0.01486 | 0.03204 | 0.00885 | 0.00000 | 0.01729 |
| 2 | 1st cerebellar lobule | 1 | 0.00017 | 0.08665 | 0.01738 | 0.03498 | 0.00765 | -0.05167 | 0.01899 |
| 3 | 2nd cerebellar lobule | 1 | 0.02954 | 0.05527 | 0.01356 | 0.03083 | 0.01787 | -0.02444 | 0.02243 |
| 4 | 3rd cerebellar lobule | 0 | 0.12538 | 0.03524 | 0.01048 | 0.02557 | 0.00799 | 0.00000 | 0.01318 |
| 5 | 4th cerebellar lobule | 0 | 0.33430 | 0.02798 | 0.00703 | 0.02266 | 0.01027 | 0.00000 | 0.01244 |
| 6 | 5th cerebellar lobule | 0 | 0.58196 | 0.01925 | 0.02920 | 0.02708 | 0.00942 | 0.00000 | 0.03068 |
| 7 | motor trigeminal nucleus | 0 | 0.37128 | 0.02292 | 0.02763 | 0.03527 | 0.01010 | 0.00000 | 0.02942 |
| 8 | root of trigeminal nerve | 0 | 0.13399 | 0.05382 | 0.00798 | 0.04311 | 0.01343 | 0.00000 | 0.01562 |
| 9 | 6th cerebellar lobule | 0 | 0.31009 | 0.03350 | 0.00626 | 0.02849 | 0.00918 | 0.00000 | 0.01111 |
| 10 | 7th cerebellar lobule | 1 | 0.03723 | 0.04931 | 0.01349 | 0.02700 | 0.01687 | -0.02231 | 0.02160 |
| 11 | facial nucleus | 0 | 0.26645 | 0.05402 | 0.01570 | 0.04186 | 0.01837 | 0.00000 | 0.02417 |
| 12 | 8th cerebellar lobule | 0 | 0.94551 | 0.03846 | 0.00880 | 0.03882 | 0.00802 | 0.00000 | 0.01191 |
| 13 | 9th cerebellar lobule | 0 | 0.67217 | 0.03732 | 0.01074 | 0.04113 | 0.01790 | 0.00000 | 0.02088 |
| 14 | anterior thalamic nuclei | 0 | 0.23351 | 0.06670 | 0.01597 | 0.05542 | 0.01267 | 0.00000 | 0.02038 |
| 15 | anterior amygdaloid nucleus | 1 | 0.00014 | 0.11084 | 0.01203 | 0.06247 | 0.01353 | -0.04836 | 0.01810 |
| 16 | accumbens core | 1 | 0.01817 | 0.03625 | 0.00850 | 0.02280 | 0.00659 | -0.01345 | 0.01076 |
| 17 | accumbens shell | 1 | 0.03183 | 0.04167 | 0.01026 | 0.01068 | 0.02800 | -0.03100 | 0.02982 |
| 18 | anterior hypothalamic area | 1 | 0.01536 | 0.10725 | 0.01940 | 0.07401 | 0.01706 | -0.03324 | 0.02584 |
| 19 | anterior lobe pituitary | 0 | 0.50614 | 0.11449 | 0.06563 | 0.08824 | 0.05862 | 0.00000 | 0.08800 |
| 20 | anterior olfactory nucleus | 0 | 0.54534 | 0.01753 | 0.01611 | 0.01221 | 0.01074 | 0.00000 | 0.01936 |
| 21 | anterior pretectal nucleus | 0 | 0.45164 | 0.04798 | 0.00875 | 0.03973 | 0.02404 | 0.00000 | 0.02559 |
| 22 | arcuate nucleus | 0 | 0.80092 | 0.10313 | 0.05304 | 0.09323 | 0.07348 | 0.00000 | 0.09062 |
| 23 | auditory ctx | 0 | 0.22705 | 0.06887 | 0.00743 | 0.06124 | 0.01197 | 0.00000 | 0.01409 |
| 24 | basal amygdaloid nucleus | 0 | 0.21498 | 0.08076 | 0.02402 | 0.06450 | 0.01379 | 0.00000 | 0.02770 |
| 25 | CA1  dorsal | 0 | 0.09652 | 0.06340 | 0.01847 | 0.04657 | 0.00884 | 0.00000 | 0.02048 |

*FIG. 12*

| | | | | | | | | | |
|---|---|---|---|---|---|---|---|---|---|
| 26 | CA1 hippocampus ventral | 0 | 0.05136 | 0.04111 | 0.00906 | 0.06180 | 0.02045 | 0.00000 | 0.02236 |
| 27 | CA2 | 0 | 0.31781 | 0.05935 | 0.01617 | 0.04899 | 0.01621 | 0.00000 | 0.02289 |
| 28 | CA3 dorsal | 0 | 0.71860 | 0.06735 | 0.01802 | 0.06380 | 0.01247 | 0.00000 | 0.02192 |
| 29 | CA3 hippocampus ventral | 0 | 0.82613 | 0.08274 | 0.02643 | 0.08657 | 0.02979 | 0.00000 | 0.03983 |
| 30 | central amygdaloid nucleus | 0 | 0.45445 | 0.10558 | 0.04027 | 0.08906 | 0.02670 | 0.00000 | 0.04832 |
| 31 | anterior cingulate area | 0 | 0.75228 | 0.04616 | 0.01125 | 0.04808 | 0.00745 | 0.00000 | 0.01349 |
| 32 | central gray | 0 | 0.58477 | 0.04279 | 0.03206 | 0.05156 | 0.01347 | 0.00000 | 0.03478 |
| 33 | claustrum | 0 | 0.23719 | 0.05133 | 0.02318 | 0.03589 | 0.01553 | 0.00000 | 0.02790 |
| 34 | central medial thalamic nucleus | 0 | 0.95095 | 0.05974 | 0.01777 | 0.05917 | 0.01016 | 0.00000 | 0.02047 |
| 35 | cortical amygdaloid nucleus | 0 | 0.95765 | 0.05848 | 0.02964 | 0.05763 | 0.02004 | 0.00000 | 0.03578 |
| 36 | copula of the pyramis | 0 | 0.91607 | 0.05455 | 0.02988 | 0.05280 | 0.02229 | 0.00000 | 0.03728 |
| 37 | crus 1 of ansiform lobule | 0 | 0.42782 | 0.02777 | 0.02126 | 0.03611 | 0.00740 | 0.00000 | 0.02251 |
| 38 | crus 2 of ansiform lobule | 0 | 0.22638 | 0.04000 | 0.00881 | 0.03097 | 0.01413 | 0.00000 | 0.01665 |
| 39 | diagonal band of Broca | 0 | 0.30328 | 0.04120 | 0.02494 | 0.02625 | 0.01932 | 0.00000 | 0.03155 |
| 40 | dentate gyrus dorsal | 0 | 0.07079 | 0.08169 | 0.01642 | 0.06307 | 0.01304 | 0.00000 | 0.02097 |
| 41 | dentate gyrus ventral | 0 | 0.42823 | 0.05882 | 0.03201 | 0.07894 | 0.04827 | 0.00000 | 0.05792 |
| 42 | dorsal lateral striatum | 0 | 0.62948 | 0.04881 | 0.01137 | 0.04600 | 0.00574 | 0.00000 | 0.01273 |
| 43 | dorsal medial nucleus | 0 | 0.31248 | 0.10876 | 0.02860 | 0.08769 | 0.03686 | 0.00000 | 0.04665 |
| 44 | dorsal medial striatum | 0 | 0.18699 | 0.05529 | 0.00861 | 0.04742 | 0.00970 | 0.00000 | 0.01297 |
| 45 | dorsomedial tegmental area | 0 | 0.08364 | 0.03423 | 0.01266 | 0.04941 | 0.01316 | 0.00000 | 0.01826 |
| 46 | DPGi | 1 | 0.00066 | 0.03027 | 0.01417 | 0.07206 | 0.01277 | 0.04179 | 0.01907 |
| 47 | dorsal raphe | 1 | 0.03007 | 0.04987 | 0.01535 | 0.07704 | 0.01975 | 0.02717 | 0.02501 |
| 48 | subiculum dorsal | 0 | 0.33652 | 0.08842 | 0.02297 | 0.07578 | 0.01709 | 0.00000 | 0.02863 |
| 49 | extended amydala | 0 | 0.21492 | 0.08302 | 0.02841 | 0.06519 | 0.00935 | 0.00000 | 0.02991 |
| 50 | ectorhinal ctx | 1 | 0.04371 | 0.07514 | 0.05127 | 0.17650 | 0.09048 | 0.10136 | 0.10400 |

*FIG. 12 (Continued)*

EP 4 741 014 A2

| | | | | | | | | | |
|---|---|---|---|---|---|---|---|---|---|
| 51 | endopiriform nucleus | 0 | 0.13271 | 0.04056 | 0.01259 | 0.03054 | 0.00519 | 0.00000 | 0.01362 |
| 52 | entorhinal ctx | 0 | 0.11866 | 0.04924 | 0.01342 | 0.06354 | 0.01403 | 0.00000 | 0.01942 |
| 53 | external plexiform layer | 0 | 0.48087 | 0.09407 | 0.02290 | 0.08540 | 0.01407 | 0.00000 | 0.02688 |
| 54 | flocculus cerebellum | 0 | 0.49561 | 0.06286 | 0.01525 | 0.05524 | 0.02038 | 0.00000 | 0.02546 |
| 55 | frontal association ctx | 0 | 0.99869 | 0.02190 | 0.00934 | 0.02191 | 0.01053 | 0.00000 | 0.01408 |
| 56 | gigantocellular reticular nucleus | 1 | 0.01483 | 0.04371 | 0.00861 | 0.02995 | 0.00600 | -0.01377 | 0.01050 |
| 57 | glomerular layer | 0 | 0.11284 | 0.08882 | 0.01486 | 0.10282 | 0.01065 | 0.00000 | 0.01828 |
| 58 | globus pallidus | 0 | 0.06600 | 0.07310 | 0.01546 | 0.04799 | 0.02421 | 0.00000 | 0.02872 |
| 59 | granular cell layer | 0 | 0.48303 | 0.08823 | 0.02665 | 0.07828 | 0.01568 | 0.00000 | 0.03092 |
| 60 | habenula nucleus | 0 | 0.47376 | 0.06991 | 0.04798 | 0.09200 | 0.04982 | 0.00000 | 0.06917 |
| 61 | intercalated amygdaloid nucleus | 0 | 0.32766 | 0.07842 | 0.02037 | 0.05731 | 0.04510 | 0.00000 | 0.04948 |
| 62 | inferior colliculus | 0 | 0.97410 | 0.05676 | 0.01623 | 0.05713 | 0.02027 | 0.00000 | 0.02597 |
| 63 | infralimbic ctx | 0 | 0.90974 | 0.02334 | 0.01052 | 0.02422 | 0.01460 | 0.00000 | 0.01799 |
| 64 | insular ctx | 0 | 0.96589 | 0.03144 | 0.02012 | 0.03185 | 0.00638 | 0.00000 | 0.02111 |
| 65 | interposed nucleus | 0 | 0.06018 | 0.07757 | 0.03396 | 0.04074 | 0.01901 | 0.00000 | 0.03892 |
| 66 | inferior olivary complex | 1 | 0.03234 | 0.10213 | 0.05280 | 0.04003 | 0.01480 | -0.06210 | 0.05483 |
| 67 | interpeduncular nucleus | 0 | 0.62739 | -0.00685 | 0.05306 | 0.00719 | 0.03559 | 0.00000 | 0.06389 |
| 68 | lateral amygdaloid nucleus | 0 | 0.75962 | 0.05963 | 0.02167 | 0.05501 | 0.02705 | 0.00000 | 0.03466 |
| 69 | Lat | 1 | 0.00099 | 0.10367 | 0.01990 | 0.03031 | 0.03076 | -0.07336 | 0.03664 |
| 70 | locus ceruleus | 0 | 0.12253 | 0.08023 | 0.02395 | 0.05134 | 0.03235 | 0.00000 | 0.04025 |
| 71 | lateral dorsal thalamic nucleus | 0 | 0.64477 | 0.10081 | 0.02057 | 0.09454 | 0.02310 | 0.00000 | 0.03093 |
| 72 | lateral geniculate | 0 | 0.10774 | 0.07278 | 0.03994 | 0.11407 | 0.03586 | 0.00000 | 0.05367 |
| 73 | lateral hypothalamus | 1 | 0.04663 | 0.10609 | 0.02224 | 0.07534 | 0.02178 | -0.03076 | 0.03113 |
| 74 | lemniscal nucleus | 0 | 0.59476 | 0.04144 | 0.01530 | 0.04567 | 0.00830 | 0.00000 | 0.01741 |
| 75 | lateral orbital ctx | 0 | 0.81508 | 0.01877 | 0.00773 | 0.01781 | 0.00484 | 0.00000 | 0.00912 |

**FIG. 12 (Continued)**

| | | | | | | | | |
|---|---|---|---|---|---|---|---|---|---|
| 76 | lateral posterior thalamic nucleus | 0 | 0.05644 | 0.07521 | 0.01455 | 0.04997 | 0.02350 | 0.00000 | 0.02764 |
| 77 | lateral preoptic area | 0 | 0.29144 | 0.07337 | 0.02522 | 0.05960 | 0.01146 | 0.00000 | 0.02770 |
| 78 | lateral septal nucleus | 0 | 0.88452 | 0.04700 | 0.00940 | 0.04615 | 0.00920 | 0.00000 | 0.01315 |
| 79 | primary motor ctx | 0 | 0.20915 | 0.03765 | 0.00570 | 0.03279 | 0.00620 | 0.00000 | 0.00842 |
| 80 | secondary motor ctx | 0 | 0.87377 | 0.02896 | 0.02848 | 0.03109 | 0.00473 | 0.00000 | 0.02887 |
| 81 | magnocellular preoptic nucleus | 0 | 0.10866 | 0.12562 | 0.05800 | 0.07097 | 0.03970 | 0.00000 | 0.07029 |
| 82 | medial dorsal thalamic nucleus | 0 | 0.23844 | 0.05395 | 0.01914 | 0.06699 | 0.01404 | 0.00000 | 0.02374 |
| 83 | medial amygdaloid nucleus | 0 | 0.55260 | 0.10316 | 0.02665 | 0.11159 | 0.01603 | 0.00000 | 0.03109 |
| 84 | medial cerebellar nucleus fastigia | 0 | 0.91896 | 0.03934 | 0.00981 | 0.04008 | 0.01384 | 0.00000 | 0.01697 |
| 85 | medial geniculate | 0 | 0.57788 | 0.08032 | 0.03791 | 0.06964 | 0.01742 | 0.00000 | 0.04172 |
| 86 | medial mammillary nucleus | 0 | 0.54920 | 0.19597 | 0.06258 | 0.17729 | 0.02513 | 0.00000 | 0.06744 |
| 87 | median raphe nucleus | 0 | 0.88518 | 0.04075 | 0.01414 | 0.04227 | 0.01992 | 0.00000 | 0.02443 |
| 88 | medial orbital ctx | 0 | 0.25122 | 0.00995 | 0.02042 | 0.03098 | 0.03583 | 0.00000 | 0.04124 |
| 89 | medial preoptic area | 1 | 0.01387 | 0.08465 | 0.02697 | 0.04719 | 0.00429 | -0.03746 | 0.02731 |
| 90 | medial pretectal area | 0 | 0.43438 | 0.05606 | 0.05939 | 0.02748 | 0.05549 | 0.00000 | 0.08127 |
| 91 | medial septum | 0 | 0.11420 | 0.02729 | 0.01322 | 0.04039 | 0.01122 | 0.00000 | 0.01734 |
| 92 | neural lobe pituitary | 0 | 0.35656 | 0.11686 | 0.11219 | 0.06121 | 0.06629 | 0.00000 | 0.13031 |
| 93 | olivary nucleus | 0 | 0.28739 | 0.05013 | 0.02980 | 0.03284 | 0.01803 | 0.00000 | 0.03483 |
| 94 | paraventricular nucleus | 0 | 0.22954 | 0.07317 | 0.03739 | 0.04958 | 0.01756 | 0.00000 | 0.04131 |
| 95 | periaqueductal gray thalamus | 0 | 0.36067 | 0.05129 | 0.00924 | 0.05577 | 0.00510 | 0.00000 | 0.01055 |
| 96 | parabrachial nucleus | 0 | 0.77107 | 0.04699 | 0.01298 | 0.05030 | 0.02316 | 0.00000 | 0.02655 |
| 97 | PCRt | 0 | 0.41788 | 0.05030 | 0.01610 | 0.04388 | 0.00518 | 0.00000 | 0.01691 |
| 98 | parafascicular thalamic nucleus | 0 | 0.25922 | 0.05447 | 0.01340 | 0.04323 | 0.01760 | 0.00000 | 0.02212 |
| 99 | paraflocculus cerebellum | 0 | 0.14611 | 0.06282 | 0.01518 | 0.05124 | 0.00609 | 0.00000 | 0.01636 |
| 100 | posterior hypothalamic area | 0 | 0.84679 | 0.07948 | 0.05139 | 0.07447 | 0.02415 | 0.00000 | 0.05678 |

**FIG. 12 (Continued)**

EP 4 741 014 A2

| | | | | | | | | |
|---|---|---|---|---|---|---|---|---|---|
| **101** | pineal gland | 0 | 0.91748 | 0.18968 | 0.20929 | 0.20149 | 0.14325 | 0.00000 | 0.25361 |
| **102** | caudal piriform ctx | 0 | 0.18807 | 0.07431 | 0.02554 | 0.05665 | 0.01129 | 0.00000 | 0.02793 |
| **103** | rostral piriform ctx | 1 | 0.00245 | 0.03056 | 0.00803 | 0.01490 | 0.00255 | -0.01566 | 0.00842 |
| **104** | premammillary nucleus | 0 | 0.06310 | 0.10790 | 0.03988 | 0.15932 | 0.04030 | 0.00000 | 0.05670 |
| **105** | paramedian lobule | 0 | 0.85288 | 0.04619 | 0.01484 | 0.04444 | 0.01558 | 0.00000 | 0.02152 |
| **106** | pontine nuclei | 0 | 0.73233 | 0.02210 | 0.01212 | 0.02008 | 0.00433 | 0.00000 | 0.01287 |
| **107** | pontine reticular nucleus caudal | 0 | 0.22601 | 0.03237 | 0.00620 | 0.02764 | 0.00577 | 0.00000 | 0.00848 |
| **108** | pontine reticular nucleus oral | 0 | 0.09826 | 0.02353 | 0.02214 | 0.04279 | 0.00756 | 0.00000 | 0.02339 |
| **109** | posterior thalamic nucleus | 0 | 0.76612 | 0.06427 | 0.01218 | 0.06187 | 0.01391 | 0.00000 | 0.01849 |
| **110** | periolivary nucleus | 1 | 0.00003 | 0.07233 | 0.01001 | 0.03020 | 0.00764 | -0.04213 | 0.01259 |
| **111** | prerubral field | 0 | 0.73298 | 0.05287 | 0.01674 | 0.04593 | 0.04508 | 0.00000 | 0.04808 |
| **112** | principal sensory nucleus trigemin | 0 | 0.11164 | 0.05340 | 0.00960 | 0.04328 | 0.00932 | 0.00000 | 0.01337 |
| **113** | precuniform nucleus | 0 | 0.48426 | 0.05496 | 0.03104 | 0.06560 | 0.01005 | 0.00000 | 0.03263 |
| **114** | perirhinal ctx | 1 | 0.03650 | 0.04546 | 0.00942 | 0.07533 | 0.02825 | 0.02987 | 0.02978 |
| **115** | prelimbic ctx | 0 | 0.58825 | 0.02334 | 0.00997 | 0.01948 | 0.01288 | 0.00000 | 0.01628 |
| **116** | parietal ctx | 1 | 0.00072 | 0.06174 | 0.00542 | 0.04516 | 0.00549 | -0.01658 | 0.00771 |
| **117** | pedunculopontine tegmental area | 1 | 0.03145 | 0.04083 | 0.01316 | 0.05727 | 0.00629 | 0.01645 | 0.01459 |
| **118** | paraventricular nucleus | 0 | 0.45077 | 0.04605 | 0.01146 | 0.05496 | 0.02486 | 0.00000 | 0.02738 |
| **119** | retrochiasmatic nucleus | 1 | 0.00025 | 0.21601 | 0.04860 | 0.08469 | 0.01284 | -0.13131 | 0.05026 |
| **120** | reuniens nucleus | 1 | 0.03865 | 0.07648 | 0.00931 | 0.06289 | 0.00923 | -0.01359 | 0.01311 |
| **121** | raphe linear | 0 | 0.51553 | 0.01182 | 0.03557 | 0.02322 | 0.01266 | 0.00000 | 0.03775 |
| **122** | raphe magnus | 0 | 0.05059 | 0.04625 | 0.02647 | 0.01504 | 0.01730 | 0.00000 | 0.03162 |
| **123** | raphe obscurus nucleus | 0 | 0.10581 | 0.03902 | 0.02420 | 0.01740 | 0.01246 | 0.00000 | 0.02722 |
| **124** | red nucleus | 0 | 0.30060 | 0.01003 | 0.03777 | 0.02971 | 0.01367 | 0.00000 | 0.04016 |
| **125** | retrosplenial caudal ctx | 0 | 0.07462 | 0.09094 | 0.03064 | 0.12801 | 0.03003 | 0.00000 | 0.04290 |

**FIG. 12 (Continued)**

EP 4 741 014 A2

| | | | | | | | | | |
|---|---|---|---|---|---|---|---|---|---|
| 126 | retrosplenial  rostral ctx | 0 | 0.68718 | 0.06686 | 0.02010 | 0.06166 | 0.02135 | 0.00000 | 0.02932 |
| 127 | reticular nucleus | 0 | 0.59469 | 0.06496 | 0.01558 | 0.06024 | 0.01214 | 0.00000 | 0.01976 |
| 128 | reticular nucleus midbrain | 0 | 0.20968 | 0.02921 | 0.02026 | 0.04200 | 0.00604 | 0.00000 | 0.02114 |
| 129 | reticulotegmental nucleus | 0 | 0.81991 | 0.01927 | 0.01532 | 0.02217 | 0.02533 | 0.00000 | 0.02960 |
| 130 | primary somatosensory ctx barrel f | 0 | 0.10639 | 0.05054 | 0.00701 | 0.04183 | 0.00912 | 0.00000 | 0.01151 |
| 131 | primary somatosensory ctx forelimb | 0 | 0.15080 | 0.04346 | 0.00595 | 0.02727 | 0.02467 | 0.00000 | 0.02538 |
| 132 | primary somatosensory ctx hindlimb | 0 | 0.38406 | 0.04216 | 0.00482 | 0.03901 | 0.00659 | 0.00000 | 0.00816 |
| 133 | primary somatosensory ctx jaw | 0 | 0.11803 | 0.03722 | 0.00751 | 0.03078 | 0.00382 | 0.00000 | 0.00843 |
| 134 | primary somatosensory ctx shoulder | 1 | 0.04738 | 0.04268 | 0.00436 | 0.03519 | 0.00646 | -0.00749 | 0.00779 |
| 135 | primary somatosensory ctx trunk | 0 | 0.68008 | 0.04540 | 0.01378 | 0.04952 | 0.01837 | 0.00000 | 0.02297 |
| 136 | primary somatosensory ctx upper li | 0 | 0.20653 | 0.04551 | 0.00887 | 0.03913 | 0.00601 | 0.00000 | 0.01072 |
| 137 | secondary somaotsensory ctx | 1 | 0.02757 | 0.07022 | 0.02240 | 0.04318 | 0.00487 | -0.02704 | 0.02292 |
| 138 | suprachiasmatic nucleus | 0 | 0.09741 | 0.11157 | 0.08224 | 0.04060 | 0.02407 | 0.00000 | 0.08569 |
| 139 | substantia innominata | 1 | 0.04722 | 0.10111 | 0.04497 | 0.04458 | 0.03446 | -0.05653 | 0.05666 |
| 140 | simple lobule cerebellum | 0 | 0.83842 | 0.03915 | 0.02367 | 0.03677 | 0.00933 | 0.00000 | 0.02545 |
| 141 | substantia nigra compacta | 0 | 0.65609 | 0.02476 | 0.03831 | 0.01441 | 0.03558 | 0.00000 | 0.05228 |
| 142 | substantia nigra reticularis | 0 | 0.28445 | 0.04715 | 0.03598 | 0.02816 | 0.00949 | 0.00000 | 0.03721 |
| 143 | supraoptic nucleus | 0 | 0.34214 | 0.06433 | 0.04955 | 0.03657 | 0.04038 | 0.00000 | 0.06392 |
| 144 | solitary tract nucleus | 0 | 0.63464 | 0.03857 | 0.01606 | 0.04309 | 0.01401 | 0.00000 | 0.02131 |
| 145 | bed nucleus stria terminalis | 0 | 0.07810 | 0.06219 | 0.01266 | 0.04873 | 0.00901 | 0.00000 | 0.01554 |
| 146 | subthalamic nucleus | 0 | 0.09641 | 0.08952 | 0.02703 | 0.05579 | 0.03336 | 0.00000 | 0.04293 |
| 147 | superior colliculus | 0 | 0.72130 | 0.07107 | 0.01359 | 0.06853 | 0.00793 | 0.00000 | 0.01574 |
| 148 | sub coeruleus nucleus | 0 | 0.45119 | 0.03502 | 0.00997 | 0.03956 | 0.00891 | 0.00000 | 0.01337 |
| 149 | supramammillary nucleus | 0 | 0.12129 | 0.16605 | 0.04983 | 0.10455 | 0.06946 | 0.00000 | 0.08548 |
| 150 | temporal ctx | 0 | 0.88101 | 0.15159 | 0.09452 | 0.14366 | 0.07154 | 0.00000 | 0.11854 |

**FIG. 12 (Continued)**

| | | | | | | | | | |
|---|---|---|---|---|---|---|---|---|---|
| 151 | triangular septal nucleus | 0 | 0.41588 | 0.06018 | 0.02035 | 0.04970 | 0.02025 | 0.00000 | 0.02871 |
| 152 | tenia tecta ctx | 0 | 0.45111 | 0.02861 | 0.02964 | 0.01650 | 0.01873 | 0.00000 | 0.03507 |
| 153 | olfactory tubercles | 0 | 0.06278 | 0.06024 | 0.02483 | 0.02569 | 0.02924 | 0.00000 | 0.03836 |
| 154 | trapezoid body | 1 | 0.02816 | 0.05144 | 0.00969 | 0.00970 | 0.03807 | -0.04174 | 0.03929 |
| 155 | Ventricle | 0 | 0.07488 | 0.06210 | 0.01361 | 0.04592 | 0.01283 | 0.00000 | 0.01870 |
| 156 | visual 1 ctx | 0 | 0.10854 | 0.11449 | 0.01762 | 0.09349 | 0.02156 | 0.00000 | 0.02784 |
| 157 | visual 2 ctx | 0 | 0.29003 | 0.10275 | 0.01752 | 0.09130 | 0.01589 | 0.00000 | 0.02366 |
| 158 | ventral anterior thalamic nucleus | 0 | 0.69388 | 0.05911 | 0.01434 | 0.05540 | 0.01588 | 0.00000 | 0.02140 |
| 159 | cochlear nucleus | 0 | 0.52269 | 0.06751 | 0.02013 | 0.05974 | 0.01817 | 0.00000 | 0.02712 |
| 160 | vestibular nucleus | 1 | 0.01097 | 0.04253 | 0.00953 | 0.06627 | 0.01502 | 0.02374 | 0.01779 |
| 161 | ventrolateral thalamic nucleus | 0 | 0.48774 | 0.05991 | 0.01594 | 0.05277 | 0.01675 | 0.00000 | 0.02312 |
| 162 | ventral lateral striatum | 1 | 0.02637 | 0.05535 | 0.01309 | 0.03862 | 0.00548 | -0.01673 | 0.01419 |
| 163 | ventromedial thalamic nucleus | 0 | 0.12677 | 0.07385 | 0.01860 | 0.05832 | 0.00950 | 0.00000 | 0.02089 |
| 164 | ventral medial nucleus | 0 | 0.08794 | 0.11859 | 0.03917 | 0.08138 | 0.02007 | 0.00000 | 0.04401 |
| 165 | ventral medial striatum | 0 | 0.25203 | 0.04717 | 0.00861 | 0.03940 | 0.01245 | 0.00000 | 0.01513 |
| 166 | ventral orbital ctx | 0 | 0.41850 | 0.02279 | 0.01427 | 0.01607 | 0.01141 | 0.00000 | 0.01827 |
| 167 | ventral pallidum | 1 | 0.00515 | 0.06903 | 0.01389 | 0.03738 | 0.01465 | -0.03164 | 0.02019 |
| 168 | ventral posteriolateral thalamic n | 1 | 0.00857 | 0.06934 | 0.00845 | 0.05268 | 0.00793 | -0.01666 | 0.01159 |
| 169 | ventral posteriolmedial thalamic n | 0 | 0.79498 | 0.06307 | 0.01043 | 0.06123 | 0.01239 | 0.00000 | 0.01620 |
| 170 | ventral subiculum | 0 | 0.86726 | 0.05364 | 0.01341 | 0.05556 | 0.02330 | 0.00000 | 0.02689 |
| 171 | ventral tegmental area | 0 | 0.53860 | 0.03000 | 0.05500 | 0.01387 | 0.01124 | 0.00000 | 0.05614 |
| 172 | White Matter | 1 | 0.00153 | 0.06219 | 0.00490 | 0.04493 | 0.00781 | -0.01726 | 0.00922 |
| 173 | White Matter | 1 | 0.02087 | 0.08104 | 0.01233 | 0.03981 | 0.03384 | -0.04123 | 0.03602 |
| 174 | zona incerta | 0 | 0.05311 | 0.08500 | 0.01671 | 0.06614 | 0.00958 | 0.00000 | 0.01926 |
| - | Whole brain | 1 | 0.03020 | 0.05160 | 0.00421 | 0.04431 | 0.00521 | -0.00729 | 0.00670 |

*FIG. 12 (Continued)*

EP 4 741 014 A2

FIG. 13

| Region Num | Region Nam | M1 | std | M2 | std | M3 | std | h1 | p1 | h2 | p2 | h3 | p3 | 0 |
|---|---|---|---|---|---|---|---|---|---|---|---|---|---|---|
| 1 | 10th cerebellar lobule | 0.0011 | 0.0058 | -0.0001 | 0.0039 | 0.0014 | 0.0061 | 0 | 0.6740 | 0 | 0.9587 | 0 | 0.6131 | 0 |
| 2 | 1st cerebellar lobule | -0.0012 | 0.0080 | -0.0038 | 0.0033 | 0.0011 | 0.0089 | 0 | 0.7194 | 1 | 0.0378 | 0 | 0.7646 | 1 |
| 3 | 2nd cerebellar lobule | 0.0013 | 0.0047 | -0.0012 | 0.0033 | 0.0024 | 0.0049 | 0 | 0.5195 | 0 | 0.4002 | 0 | 0.2839 | 0 |
| 4 | 3rd cerebellar lobule | 0.0041 | 0.0044 | 0.0007 | 0.0035 | 0.0035 | 0.0040 | 0 | 0.0722 | 0 | 0.6512 | 0 | 0.0856 | 0 |
| 5 | 4th cerebellar lobule | 0.0031 | 0.0052 | 0.0003 | 0.0037 | 0.0032 | 0.0045 | 0 | 0.2066 | 0 | 0.8594 | 0 | 0.1394 | 0 |
| 6 | 5th cerebellar lobule | 0.0024 | 0.0052 | 0.0019 | 0.0047 | 0.0032 | 0.0048 | 0 | 0.3045 | 0 | 0.3728 | 0 | 0.1634 | 0 |
| 7 | motor trigeminal nucleus | 0.0022 | 0.0036 | -0.0014 | 0.0041 | 0.0009 | 0.0029 | 0 | 0.1942 | 0 | 0.4453 | 0 | 0.4831 | 0 |
| 8 | root of trigeminal nerve | -0.0006 | 0.0046 | -0.0002 | 0.0031 | -0.0029 | 0.0050 | 0 | 0.7680 | 0 | 0.8840 | 0 | 0.2129 | 0 |
| 9 | 6th cerebellar lobule | 0.0033 | 0.0042 | 0.0004 | 0.0034 | 0.0021 | 0.0051 | 0 | 0.1154 | 0 | 0.8031 | 0 | 0.3701 | 0 |
| 10 | 7th cerebellar lobule | -0.0008 | 0.0037 | 0.0004 | 0.0062 | -0.0012 | 0.0053 | 0 | 0.6320 | 0 | 0.8755 | 0 | 0.6084 | 0 |
| 11 | facial nucleus | 0.0002 | 0.0143 | -0.0009 | 0.0078 | 0.0008 | 0.0086 | 0 | 0.9680 | 0 | 0.7792 | 0 | 0.8207 | 0 |
| 12 | 8th cerebellar lobule | 0.0025 | 0.0049 | 0.0015 | 0.0071 | 0.0015 | 0.0060 | 0 | 0.2655 | 0 | 0.6206 | 0 | 0.5733 | 0 |
| 13 | 9th cerebellar lobule | 0.0035 | 0.0052 | 0.0024 | 0.0053 | 0.0044 | 0.0054 | 0 | 0.1635 | 0 | 0.3106 | 0 | 0.1023 | 0 |
| 14 | anterior thalamic nuclei | 0.0051 | 0.0058 | -0.0006 | 0.0041 | 0.0045 | 0.0087 | 0 | 0.0823 | 0 | 0.7286 | 0 | 0.2611 | 0 |
| 15 | anterior amygdaloid nucleus | 0.0025 | 0.0169 | 0.0018 | 0.0052 | -0.0005 | 0.0087 | 0 | 0.7286 | 0 | 0.4207 | 0 | 0.8933 | 0 |
| 16 | accumbens core | 0.0017 | 0.0092 | -0.0002 | 0.0055 | 0.0032 | 0.0079 | 0 | 0.6670 | 0 | 0.9193 | 0 | 0.3677 | 0 |
| 17 | accumbens shell | 0.0023 | 0.0129 | 0.0016 | 0.0070 | 0.0011 | 0.0106 | 0 | 0.6810 | 0 | 0.5976 | 0 | 0.8069 | 0 |
| 18 | anterior hypothalamic area | 0.0034 | 0.0135 | 0.0014 | 0.0094 | 0.0024 | 0.0169 | 0 | 0.5611 | 0 | 0.7262 | 0 | 0.7442 | 0 |
| 19 | anterior lobe pituitary | -0.0084 | 0.0102 | 0.0048 | 0.0100 | -0.0048 | 0.0093 | 0 | 0.0995 | 0 | 0.2979 | 0 | 0.2663 | 0 |
| 20 | anterior olfactory nucleus | 0.0026 | 0.0098 | 0.0040 | 0.0078 | 0.0049 | 0.0085 | 0 | 0.5396 | 0 | 0.2657 | 0 | 0.2185 | 0 |
| 21 | anterior pretectal nucleus | 0.0076 | 0.0106 | 0.0013 | 0.0043 | 0.0064 | 0.0069 | 0 | 0.1406 | 0 | 0.4998 | 0 | 0.0709 | 0 |
| 22 | arcuate nucleus | 0.0137 | 0.0226 | 0.0097 | 0.0175 | 0.0067 | 0.0235 | 0 | 0.1985 | 0 | 0.2303 | 0 | 0.5178 | 0 |
| 23 | auditory ctx | 0.0026 | 0.0083 | 0.0017 | 0.0049 | 0.0016 | 0.0070 | 0 | 0.4770 | 0 | 0.4406 | 0 | 0.6055 | 0 |
| 24 | basal amygdaloid nucleus | 0.0042 | 0.0078 | 0.0003 | 0.0058 | 0.0017 | 0.0057 | 0 | 0.2444 | 0 | 0.9200 | 0 | 0.4880 | 0 |
| 25 | CA1 dorsal | 0.0074 | 0.0040 | 0.0040 | 0.0052 | 0.0077 | 0.0056 | 1 | 0.0061 | 0 | 0.1189 | 1 | 0.0205 | 2 |

*FIG. 14*

EP 4 741 014 A2

| | | | | | | | | | | | | | |
|---|---|---|---|---|---|---|---|---|---|---|---|---|---|
| 26 | CA1 hippocampus ventral | 0.0061 | 0.0054 | 0.0037 | 0.0066 | 0.0056 | 0.0090 | 1 | 0.0380 | 0 | 0.2326 | 0 | 0.1884 | 1 |
| 27 | CA2 | 0.0066 | 0.0033 | 0.0033 | 0.0044 | 0.0075 | 0.0044 | 1 | 0.0045 | 0 | 0.1229 | 1 | 0.0086 | 2 |
| 28 | CA3 dorsal | 0.0055 | 0.0074 | 0.0024 | 0.0058 | 0.0062 | 0.0070 | 0 | 0.1289 | 0 | 0.3626 | 0 | 0.0805 | 0 |
| 29 | CA3 hippocampus ventral | 0.0104 | 0.0093 | 0.0103 | 0.0076 | 0.0109 | 0.0096 | 1 | 0.0414 | 1 | 0.0211 | 1 | 0.0387 | 3 |
| 30 | central amygdaloid nucleus | 0.0102 | 0.0076 | 0.0017 | 0.0051 | 0.0065 | 0.0052 | 1 | 0.0218 | 0 | 0.4425 | 1 | 0.0294 | 2 |
| 31 | anterior cingulate area | 0.0044 | 0.0030 | 0.0021 | 0.0047 | 0.0040 | 0.0062 | 1 | 0.0165 | 0 | 0.3182 | 0 | 0.1753 | 1 |
| 32 | central gray | -0.0001 | 0.0060 | -0.0016 | 0.0028 | 0.0015 | 0.0053 | 0 | 0.9686 | 0 | 0.2298 | 0 | 0.5179 | 0 |
| 33 | claustrum | 0.0010 | 0.0094 | -0.0012 | 0.0047 | 0.0030 | 0.0089 | 0 | 0.8033 | 0 | 0.5525 | 0 | 0.4482 | 0 |
| 34 | central medial thalamic nucleus | 0.0112 | 0.0085 | 0.0024 | 0.0052 | 0.0087 | 0.0049 | 1 | 0.0233 | 0 | 0.3135 | 1 | 0.0076 | 2 |
| 35 | cortical amygdaloid nucleus | -0.0015 | 0.0101 | 0.0005 | 0.0052 | -0.0048 | 0.0054 | 0 | 0.7307 | 0 | 0.8180 | 0 | 0.0800 | 0 |
| 36 | copula of the pyramis | 0.0017 | 0.0028 | -0.0006 | 0.0051 | 0.0013 | 0.0042 | 0 | 0.2068 | 0 | 0.7806 | 0 | 0.4684 | 0 |
| 37 | crus 1 of ansiform lobule | 0.0029 | 0.0042 | 0.0005 | 0.0043 | 0.0015 | 0.0051 | 0 | 0.1494 | 0 | 0.7917 | 0 | 0.5121 | 0 |
| 38 | crus 2 of ansiform lobule | 0.0025 | 0.0065 | 0.0003 | 0.0044 | 0.0009 | 0.0061 | 0 | 0.3917 | 0 | 0.8837 | 0 | 0.7463 | 0 |
| 39 | diagonal band of Broca | -0.0098 | 0.0185 | -0.0018 | 0.0104 | -0.0055 | 0.0140 | 0 | 0.2516 | 0 | 0.6909 | 0 | 0.3779 | 0 |
| 40 | dentate gyrus dorsal | 0.0077 | 0.0060 | 0.0050 | 0.0098 | 0.0094 | 0.0075 | 1 | 0.0250 | 0 | 0.2624 | 1 | 0.0275 | 2 |
| 41 | dentate gyrus ventral | 0.0079 | 0.0045 | 0.0069 | 0.0054 | 0.0091 | 0.0087 | 1 | 0.0075 | 1 | 0.0258 | 1 | 0.0497 | 3 |
| 42 | dorsal lateral striatum | 0.0025 | 0.0068 | -0.0006 | 0.0032 | 0.0038 | 0.0062 | 0 | 0.4087 | 0 | 0.6395 | 0 | 0.1897 | 0 |
| 43 | dorsal medial nucleus | 0.0112 | 0.0115 | -0.0012 | 0.0075 | 0.0113 | 0.0106 | 0 | 0.0637 | 0 | 0.7113 | 1 | 0.0479 | 1 |
| 44 | dorsal medial striatum | 0.0019 | 0.0059 | -0.0010 | 0.0037 | 0.0034 | 0.0077 | 0 | 0.4648 | 0 | 0.5316 | 0 | 0.3290 | 0 |
| 45 | dorsomedial tegmental area | -0.0002 | 0.0079 | -0.0008 | 0.0035 | 0.0013 | 0.0059 | 0 | 0.9522 | 0 | 0.6005 | 0 | 0.5982 | 0 |
| 46 | DPGi | 0.0026 | 0.0057 | 0.0002 | 0.0024 | 0.0015 | 0.0039 | 0 | 0.3140 | 0 | 0.8735 | 0 | 0.3937 | 0 |
| 47 | dorsal raphe | 0.0021 | 0.0077 | -0.0015 | 0.0054 | 0.0013 | 0.0038 | 0 | 0.5356 | 0 | 0.5386 | 0 | 0.4607 | 0 |
| 48 | subiculum dorsal | 0.0034 | 0.0054 | 0.0015 | 0.0032 | 0.0013 | 0.0076 | 0 | 0.1800 | 0 | 0.2990 | 0 | 0.6876 | 0 |
| 49 | extended amydala | 0.0048 | 0.0088 | -0.0020 | 0.0057 | 0.0041 | 0.0084 | 0 | 0.2397 | 0 | 0.4287 | 0 | 0.2881 | 0 |
| 50 | ectorhinal ctx | 0.0018 | 0.0111 | -0.0010 | 0.0041 | 0.0003 | 0.0089 | 0 | 0.7155 | 0 | 0.5721 | 0 | 0.9349 | 0 |

*FIG. 14 (Continued)*

EP 4 741 014 A2

| | | | | | | | | | | | | | |
|---|---|---|---|---|---|---|---|---|---|---|---|---|---|
| 51 | endopiriform nucleus | 0.0026 | 0.0066 | -0.0001 | 0.0038 | 0.0039 | 0.0044 | 0 | 0.3882 | 0 | 0.9374 | 0 | 0.0853 | 0 |
| 52 | entorhinal ctx | -0.0004 | 0.0036 | 0.0013 | 0.0025 | 0.0003 | 0.0040 | 0 | 0.8012 | 0 | 0.2794 | 0 | 0.8596 | 0 |
| 53 | external plexiform layer | 0.0042 | 0.0080 | 0.0025 | 0.0066 | 0.0022 | 0.0064 | 0 | 0.2528 | 0 | 0.3925 | 0 | 0.4399 | 0 |
| 54 | flocculus cerebellum | 0.0026 | 0.0056 | 0.0017 | 0.0045 | 0.0011 | 0.0049 | 0 | 0.3160 | 0 | 0.4107 | 0 | 0.5943 | 0 |
| 55 | frontal association ctx | 0.0051 | 0.0051 | 0.0029 | 0.0040 | 0.0026 | 0.0068 | 0 | 0.0559 | 0 | 0.1375 | 0 | 0.3938 | 0 |
| 56 | gigantocellular reticular nucleus | -0.0022 | 0.0059 | -0.0013 | 0.0049 | -0.0019 | 0.0058 | 0 | 0.4011 | 0 | 0.5585 | 0 | 0.4563 | 0 |
| 57 | glomerular layer | 0.0014 | 0.0107 | 0.0041 | 0.0128 | -0.0010 | 0.0108 | 0 | 0.7664 | 0 | 0.4687 | 0 | 0.8372 | 0 |
| 58 | globus pallidus | 0.0031 | 0.0074 | -0.0005 | 0.0034 | 0.0026 | 0.0056 | 0 | 0.3537 | 0 | 0.7502 | 0 | 0.3116 | 0 |
| 59 | granular cell layer | 0.0043 | 0.0061 | 0.0045 | 0.0060 | 0.0030 | 0.0059 | 0 | 0.1406 | 0 | 0.1248 | 0 | 0.2769 | 0 |
| 60 | habenula nucleus | 0.0085 | 0.0126 | 0.0058 | 0.0132 | 0.0082 | 0.0148 | 0 | 0.1610 | 0 | 0.3327 | 0 | 0.2303 | 0 |
| 61 | intercalated amygdaloid nucleus | 0.0002 | 0.0114 | 0.0000 | 0.0071 | 0.0061 | 0.0086 | 0 | 0.9622 | 0 | 0.9999 | 0 | 0.1408 | 0 |
| 62 | inferior colliculus | 0.0025 | 0.0075 | 0.0009 | 0.0050 | 0.0044 | 0.0073 | 0 | 0.4480 | 0 | 0.6798 | 0 | 0.1985 | 0 |
| 63 | infralimbic ctx | 0.0034 | 0.0069 | 0.0030 | 0.0076 | 0.0049 | 0.0096 | 0 | 0.2797 | 0 | 0.3731 | 0 | 0.2632 | 0 |
| 64 | insular ctx | 0.0001 | 0.0105 | 0.0000 | 0.0046 | -0.0002 | 0.0097 | 0 | 0.9812 | 0 | 0.9891 | 0 | 0.9528 | 0 |
| 65 | interposed nucleus | 0.0069 | 0.0117 | 0.0009 | 0.0058 | 0.0094 | 0.0088 | 0 | 0.2093 | 0 | 0.7035 | 1 | 0.0476 | 1 |
| 66 | inferior olivary complex | 0.0031 | 0.0113 | 0.0081 | 0.0087 | -0.0008 | 0.0072 | 0 | 0.5337 | 0 | 0.0732 | 0 | 0.8074 | 0 |
| 67 | interpeduncular nucleus | -0.0031 | 0.0117 | 0.0041 | 0.0050 | -0.0021 | 0.0084 | 0 | 0.5476 | 0 | 0.1024 | 0 | 0.5695 | 0 |
| 68 | lateral amygdaloid nucleus | 0.0061 | 0.0089 | 0.0018 | 0.0044 | 0.0053 | 0.0085 | 0 | 0.1567 | 0 | 0.3563 | 0 | 0.1857 | 0 |
| 69 | Lat | 0.0049 | 0.0073 | 0.0037 | 0.0050 | 0.0053 | 0.0058 | 0 | 0.1588 | 0 | 0.1293 | 0 | 0.0760 | 0 |
| 70 | locus ceruleus | 0.0074 | 0.0155 | 0.0008 | 0.0081 | 0.0060 | 0.0137 | 0 | 0.2955 | 0 | 0.8233 | 0 | 0.3328 | 0 |
| 71 | lateral dorsal thalamic nucleus | 0.0141 | 0.0123 | 0.0071 | 0.0092 | 0.0103 | 0.0141 | 1 | 0.0380 | 0 | 0.1149 | 0 | 0.1333 | 1 |
| 72 | lateral geniculate | 0.0087 | 0.0074 | 0.0074 | 0.0120 | 0.0092 | 0.0147 | 1 | 0.0349 | 0 | 0.1929 | 0 | 0.1877 | 1 |
| 73 | lateral hypothalamus | 0.0041 | 0.0110 | 0.0049 | 0.0101 | 0.0063 | 0.0170 | 0 | 0.3984 | 0 | 0.2836 | 0 | 0.4031 | 0 |
| 74 | lemniscal nucleus | 0.0041 | 0.0029 | 0.0001 | 0.0044 | 0.0043 | 0.0047 | 1 | 0.0178 | 0 | 0.9436 | 0 | 0.0721 | 1 |
| 75 | lateral orbital ctx | 0.0022 | 0.0048 | 0.0007 | 0.0041 | 0.0034 | 0.0072 | 0 | 0.3196 | 0 | 0.6860 | 0 | 0.3021 | 0 |

*FIG. 14 (Continued)*

| # | Region | | | | | | | | | | | | | |
|---|---|---|---|---|---|---|---|---|---|---|---|---|---|---|
| 76 | lateral posterior thalamic nucleus | 0.0077 | 0.0048 | 0.0052 | 0.0085 | 0.0080 | 0.0130 | 1 | 0.0115 | 0 | 0.1908 | 0 | 0.1927 | 1 |
| 77 | lateral preoptic area | 0.0002 | 0.0072 | -0.0017 | 0.0040 | 0.0006 | 0.0095 | 0 | 0.9386 | 0 | 0.3464 | 0 | 0.8809 | 0 |
| 78 | lateral septal nucleus | 0.0032 | 0.0055 | 0.0001 | 0.0040 | 0.0051 | 0.0080 | 0 | 0.2135 | 0 | 0.9765 | 0 | 0.1807 | 0 |
| 79 | primary motor ctx | 0.0051 | 0.0040 | 0.0005 | 0.0034 | 0.0045 | 0.0073 | 1 | 0.0280 | 0 | 0.7560 | 0 | 0.1922 | 1 |
| 80 | secondary motor ctx | 0.0066 | 0.0053 | 0.0027 | 0.0035 | 0.0060 | 0.0064 | 1 | 0.0299 | 0 | 0.1215 | 0 | 0.0682 | 1 |
| 81 | magnocellular preoptic nucleus | -0.0064 | 0.0295 | 0.0032 | 0.0115 | -0.0094 | 0.0272 | 0 | 0.6157 | 0 | 0.5227 | 0 | 0.4385 | 0 |
| 82 | medial dorsal thalamic nucleus | 0.0049 | 0.0112 | 0.0018 | 0.0042 | 0.0061 | 0.0035 | 0 | 0.3350 | 0 | 0.3549 | 1 | 0.0079 | 1 |
| 83 | medial amygdaloid nucleus | 0.0015 | 0.0258 | 0.0113 | 0.0118 | 0.0041 | 0.0189 | 0 | 0.8923 | 0 | 0.0657 | 0 | 0.6173 | 0 |
| 84 | medial cerebellar nucleus fastigia | 0.0059 | 0.0057 | -0.0020 | 0.0064 | 0.0086 | 0.0059 | 0 | 0.0505 | 0 | 0.4747 | 1 | 0.0155 | 1 |
| 85 | medial geniculate | 0.0055 | 0.0052 | 0.0037 | 0.0064 | 0.0062 | 0.0092 | 1 | 0.0488 | 0 | 0.2097 | 0 | 0.1591 | 1 |
| 86 | medial mammillary nucleus | -0.0053 | 0.0247 | 0.0008 | 0.0174 | -0.0051 | 0.0172 | 0 | 0.6207 | 0 | 0.9114 | 0 | 0.5047 | 0 |
| 87 | median raphe nucleus | -0.0001 | 0.0082 | -0.0011 | 0.0057 | -0.0004 | 0.0060 | 0 | 0.9672 | 0 | 0.6593 | 0 | 0.8703 | 0 |
| 88 | medial orbital ctx | 0.0048 | 0.0055 | 0.0061 | 0.0064 | 0.0051 | 0.0051 | 0 | 0.0856 | 0 | 0.0678 | 0 | 0.0588 | 0 |
| 89 | medial preoptic area | 0.0038 | 0.0087 | -0.0012 | 0.0068 | 0.0050 | 0.0110 | 0 | 0.3366 | 0 | 0.6730 | 0 | 0.3115 | 0 |
| 90 | medial pretectal area | -0.0003 | 0.0141 | 0.0052 | 0.0076 | 0.0052 | 0.0125 | 0 | 0.9569 | 0 | 0.1503 | 0 | 0.3588 | 0 |
| 91 | medial septum | 0.0075 | 0.0067 | -0.0001 | 0.0066 | 0.0063 | 0.0071 | 1 | 0.0423 | 0 | 0.9583 | 0 | 0.0832 | 1 |
| 92 | neural lobe pituitary | -0.0193 | 0.0203 | 0.0018 | 0.0104 | -0.0090 | 0.0157 | 0 | 0.0672 | 0 | 0.6860 | 0 | 0.2187 | 0 |
| 93 | olivary nucleus | -0.0001 | 0.0077 | 0.0004 | 0.0105 | -0.0025 | 0.0062 | 0 | 0.9689 | 0 | 0.9272 | 0 | 0.3736 | 0 |
| 94 | paraventricular nuclus | 0.0035 | 0.0099 | -0.0026 | 0.0100 | 0.0066 | 0.0112 | 0 | 0.4287 | 0 | 0.5458 | 0 | 0.2069 | 0 |
| 95 | periaqueductal gray thalamus | 0.0022 | 0.0091 | -0.0005 | 0.0029 | 0.0016 | 0.0050 | 0 | 0.5753 | 0 | 0.7034 | 0 | 0.4559 | 0 |
| 96 | parabrachial nucleus | 0.0012 | 0.0048 | 0.0007 | 0.0036 | 0.0030 | 0.0061 | 0 | 0.5516 | 0 | 0.6476 | 0 | 0.2831 | 0 |
| 97 | PCRt | -0.0032 | 0.0064 | -0.0018 | 0.0039 | -0.0046 | 0.0066 | 0 | 0.2683 | 0 | 0.3215 | 0 | 0.1480 | 0 |
| 98 | parafascicular thalamic nucleus | 0.0051 | 0.0061 | 0.0025 | 0.0032 | 0.0033 | 0.0043 | 0 | 0.0936 | 0 | 0.1083 | 0 | 0.1197 | 0 |
| 99 | paraflocculus cerebellum | 0.0018 | 0.0042 | 0.0009 | 0.0045 | 0.0010 | 0.0052 | 0 | 0.3341 | 0 | 0.6338 | 0 | 0.6537 | 0 |
| 100 | posterior hypothalamic area | 0.0031 | 0.0130 | 0.0000 | 0.0096 | 0.0017 | 0.0133 | 0 | 0.5790 | 0 | 0.9943 | 0 | 0.7696 | 0 |

*FIG. 14 (Continued)*

EP 4 741 014 A2

| 101 | pineal gland | -0.0193 | 0.0155 | 0.0031 | 0.0325 | 0.0006 | 0.0365 | 1 | 0.0281 | 0 | 0.8223 | 0 | 0.9686 | 1 |
|-----|--------------|---------|--------|--------|--------|--------|--------|---|--------|---|--------|---|--------|---|
| 102 | caudal piriform ctx | 0.0000 | 0.0141 | -0.0026 | 0.0083 | -0.0019 | 0.0108 | 0 | 0.9986 | 0 | 0.4836 | 0 | 0.6761 | 0 |
| 103 | rostral piriform ctx | 0.0007 | 0.0101 | 0.0026 | 0.0044 | 0.0025 | 0.0098 | 0 | 0.8641 | 0 | 0.2042 | 0 | 0.5669 | 0 |
| 104 | premammillary nucleus | 0.0009 | 0.0265 | 0.0038 | 0.0107 | 0.0011 | 0.0171 | 0 | 0.9343 | 0 | 0.4289 | 0 | 0.8791 | 0 |
| 105 | paramedian lobule | 0.0015 | 0.0034 | 0.0012 | 0.0042 | 0.0023 | 0.0043 | 0 | 0.3354 | 0 | 0.5200 | 0 | 0.2408 | 0 |
| 106 | pontine nuclei | -0.0052 | 0.0086 | -0.0022 | 0.0056 | -0.0009 | 0.0062 | 0 | 0.1991 | 0 | 0.3812 | 0 | 0.7356 | 0 |
| 107 | pontine reticular nucleus caudal | 0.0004 | 0.0056 | -0.0012 | 0.0031 | 0.0010 | 0.0054 | 0 | 0.8786 | 0 | 0.3764 | 0 | 0.6604 | 0 |
| 108 | pontine reticular nucleus oral | 0.0001 | 0.0030 | -0.0007 | 0.0046 | 0.0013 | 0.0029 | 0 | 0.9669 | 0 | 0.7271 | 0 | 0.3170 | 0 |
| 109 | posterior thalamic nucleus | 0.0064 | 0.0076 | 0.0019 | 0.0037 | 0.0060 | 0.0065 | 0 | 0.0932 | 0 | 0.2682 | 0 | 0.0727 | 0 |
| 110 | periolivary nucleus | 0.0006 | 0.0070 | -0.0024 | 0.0050 | 0.0011 | 0.0073 | 0 | 0.8416 | 0 | 0.2911 | 0 | 0.7159 | 0 |
| 111 | prerubral field | 0.0070 | 0.0058 | 0.0060 | 0.0061 | 0.0034 | 0.0070 | 1 | 0.0311 | 0 | 0.0619 | 0 | 0.2851 | 1 |
| 112 | principal sensory nucleus trigemin | -0.0033 | 0.0046 | -0.0021 | 0.0034 | -0.0044 | 0.0061 | 0 | 0.1389 | 0 | 0.1899 | 0 | 0.1337 | 0 |
| 113 | precuniform nucleus | 0.0048 | 0.0057 | 0.0005 | 0.0072 | 0.0060 | 0.0063 | 0 | 0.0924 | 0 | 0.8741 | 0 | 0.0649 | 0 |
| 114 | perirhinal ctx | 0.0088 | 0.0203 | 0.0043 | 0.0162 | 0.0011 | 0.0158 | 0 | 0.3385 | 0 | 0.5431 | 0 | 0.8673 | 0 |
| 115 | prelimbic ctx | 0.0061 | 0.0025 | 0.0031 | 0.0051 | 0.0067 | 0.0065 | 1 | 0.0020 | 0 | 0.1961 | 0 | 0.0515 | 1 |
| 116 | parietal ctx | 0.0046 | 0.0037 | 0.0013 | 0.0024 | 0.0051 | 0.0046 | 1 | 0.0287 | 0 | 0.2298 | 1 | 0.0439 | 2 |
| 117 | pedunculopontine tegmental area | 0.0048 | 0.0061 | -0.0002 | 0.0072 | 0.0051 | 0.0069 | 0 | 0.1110 | 0 | 0.9446 | 0 | 0.1300 | 0 |
| 118 | paraventricular nucleus | 0.0079 | 0.0164 | 0.0000 | 0.0060 | 0.0051 | 0.0068 | 0 | 0.2922 | 0 | 0.9980 | 0 | 0.1223 | 0 |
| 119 | retrochiasmatic nucleus | 0.0193 | 0.0503 | 0.0125 | 0.0352 | 0.0106 | 0.0206 | 0 | 0.3894 | 0 | 0.4254 | 0 | 0.2632 | 0 |
| 120 | reuniens nucleus | 0.0065 | 0.0061 | 0.0013 | 0.0054 | 0.0035 | 0.0073 | 1 | 0.0486 | 0 | 0.5878 | 0 | 0.2924 | 1 |
| 121 | raphe linear | 0.0013 | 0.0065 | -0.0020 | 0.0083 | 0.0017 | 0.0075 | 0 | 0.6393 | 0 | 0.5805 | 0 | 0.5961 | 0 |
| 122 | raphe magnus | 0.0035 | 0.0110 | 0.0017 | 0.0119 | 0.0039 | 0.0158 | 0 | 0.4723 | 0 | 0.7381 | 0 | 0.5699 | 0 |
| 123 | raphe obscurus nucleus | -0.0023 | 0.0085 | -0.0034 | 0.0083 | -0.0034 | 0.0081 | 0 | 0.5372 | 0 | 0.3684 | 0 | 0.3563 | 0 |
| 124 | red nucleus | -0.0041 | 0.0118 | -0.0033 | 0.0057 | -0.0049 | 0.0104 | 0 | 0.4269 | 0 | 0.2162 | 0 | 0.2994 | 0 |
| 125 | retrosplenial caudal ctx | -0.0009 | 0.0087 | 0.0076 | 0.0144 | 0.0021 | 0.0235 | 0 | 0.8078 | 0 | 0.2539 | 0 | 0.8363 | 0 |

**FIG. 14 (Continued)**

EP 4 741 014 A2

| | | | | | | | | | | | | | | |
|---|---|---|---|---|---|---|---|---|---|---|---|---|---|---|
| 126 | retrosplenial rostral ctx | 0.0056 | 0.0087 | 0.0031 | 0.0088 | 0.0016 | 0.0102 | 0 | 0.1771 | 0 | 0.4230 | 0 | 0.7218 | 0 |
| 127 | reticular nucleus | 0.0058 | 0.0058 | 0.0014 | 0.0041 | 0.0068 | 0.0068 | 0 | 0.0594 | 0 | 0.4304 | 0 | 0.0561 | 0 |
| 128 | reticular nucleus midbrain | 0.0043 | 0.0069 | 0.0000 | 0.0040 | 0.0046 | 0.0041 | 0 | 0.1892 | 0 | 0.9847 | 1 | 0.0402 | 1 |
| 129 | reticulotegmental nucleus | -0.0019 | 0.0082 | -0.0006 | 0.0047 | 0.0036 | 0.0091 | 0 | 0.5869 | 0 | 0.7591 | 0 | 0.3707 | 0 |
| 130 | primary somatosensory ctx barrel f | 0.0040 | 0.0049 | 0.0005 | 0.0020 | 0.0032 | 0.0049 | 0 | 0.0998 | 0 | 0.5281 | 0 | 0.1644 | 0 |
| 131 | primary somatosensory ctx forelimb | 0.0038 | 0.0047 | -0.0006 | 0.0022 | 0.0032 | 0.0058 | 0 | 0.1027 | 0 | 0.5140 | 0 | 0.2283 | 0 |
| 132 | primary somatosensory ctx hindlimb | 0.0056 | 0.0039 | 0.0006 | 0.0021 | 0.0044 | 0.0039 | 1 | 0.0179 | 0 | 0.5367 | 1 | 0.0403 | 2 |
| 133 | primary somatosensory ctx jaw | 0.0016 | 0.0049 | 0.0000 | 0.0040 | 0.0022 | 0.0059 | 0 | 0.4649 | 0 | 0.9975 | 0 | 0.4056 | 0 |
| 134 | primary somatosensory ctx shoulder | 0.0062 | 0.0083 | -0.0006 | 0.0037 | 0.0036 | 0.0034 | 0 | 0.1246 | 0 | 0.6874 | 1 | 0.0490 | 1 |
| 135 | primary somatosensory ctx trunk | 0.0058 | 0.0059 | -0.0001 | 0.0034 | 0.0032 | 0.0059 | 0 | 0.0611 | 0 | 0.9387 | 0 | 0.2405 | 0 |
| 136 | primary somatosensory ctx upper li | 0.0034 | 0.0051 | 0.0012 | 0.0029 | 0.0037 | 0.0049 | 0 | 0.1649 | 0 | 0.3400 | 0 | 0.1238 | 0 |
| 137 | secondary somaotsensory ctx | 0.0035 | 0.0076 | -0.0001 | 0.0022 | 0.0031 | 0.0065 | 0 | 0.3131 | 0 | 0.9540 | 0 | 0.2972 | 0 |
| 138 | suprachiasmatic nucleus | 0.0164 | 0.0361 | -0.0029 | 0.0105 | 0.0094 | 0.0378 | 0 | 0.3147 | 0 | 0.5254 | 0 | 0.5705 | 0 |
| 139 | substantia innominata | 0.0020 | 0.0103 | 0.0013 | 0.0070 | -0.0068 | 0.0150 | 0 | 0.6493 | 0 | 0.6684 | 0 | 0.3154 | 0 |
| 140 | simple lobule cerebellum | 0.0026 | 0.0048 | 0.0002 | 0.0046 | 0.0019 | 0.0052 | 0 | 0.2512 | 0 | 0.9135 | 0 | 0.4173 | 0 |
| 141 | substantia nigra compacta | 0.0000 | 0.0106 | 0.0009 | 0.0069 | 0.0027 | 0.0098 | 0 | 0.9914 | 0 | 0.7652 | 0 | 0.5272 | 0 |
| 142 | substantia nigra reticularis | 0.0012 | 0.0079 | 0.0014 | 0.0086 | 0.0014 | 0.0091 | 0 | 0.7344 | 0 | 0.7175 | 0 | 0.7270 | 0 |
| 143 | supraoptic nucleus | 0.0055 | 0.0200 | 0.0026 | 0.0104 | 0.0029 | 0.0222 | 0 | 0.5324 | 0 | 0.5739 | 0 | 0.7632 | 0 |
| 144 | solitary tract nucleus | 0.0001 | 0.0053 | -0.0002 | 0.0049 | -0.0016 | 0.0042 | 0 | 0.9817 | 0 | 0.9360 | 0 | 0.3991 | 0 |
| 145 | bed nucleus stria terminalis | 0.0036 | 0.0091 | -0.0018 | 0.0041 | 0.0056 | 0.0056 | 0 | 0.3752 | 0 | 0.3356 | 0 | 0.0566 | 0 |
| 146 | subthalamic nucleus | -0.0022 | 0.0128 | 0.0011 | 0.0169 | 0.0004 | 0.0155 | 0 | 0.6959 | 0 | 0.8752 | 0 | 0.9544 | 0 |
| 147 | superior colliculus | 0.0024 | 0.0117 | -0.0001 | 0.0025 | 0.0021 | 0.0074 | 0 | 0.6424 | 0 | 0.9139 | 0 | 0.5252 | 0 |
| 148 | sub coeruleus nucleus | 0.0003 | 0.0051 | -0.0006 | 0.0025 | 0.0019 | 0.0046 | 0 | 0.9060 | 0 | 0.5484 | 0 | 0.3559 | 0 |
| 149 | supramammillary nucleus | 0.0082 | 0.0089 | 0.0109 | 0.0144 | 0.0112 | 0.0285 | 0 | 0.0722 | 0 | 0.1234 | 0 | 0.3799 | 0 |
| 150 | temporal ctx | -0.0092 | 0.0203 | -0.0009 | 0.0093 | -0.0057 | 0.0181 | 0 | 0.3186 | 0 | 0.8135 | 0 | 0.4767 | 0 |

*FIG. 14 (Continued)*

EP 4 741 014 A2

| | | | | | | | | | | | | | |
|---|---|---|---|---|---|---|---|---|---|---|---|---|---|
| 151 | triangular septal nucleus | 0.0034 | 0.0080 | -0.0017 | 0.0046 | 0.0081 | 0.0078 | 0 | 0.3464 | 0 | 0.4152 | 0 | 0.0511 | 0 |
| 152 | tenia tecta ctx | -0.0037 | 0.0109 | 0.0097 | 0.0108 | -0.0015 | 0.0136 | 0 | 0.4484 | 0 | 0.0799 | 0 | 0.7977 | 0 |
| 153 | olfactory tubercles | -0.0045 | 0.0190 | 0.0064 | 0.0079 | -0.0016 | 0.0217 | 0 | 0.5889 | 0 | 0.1049 | 0 | 0.8630 | 0 |
| 154 | trapezoid body | -0.0019 | 0.0043 | -0.0018 | 0.0050 | 0.0013 | 0.0066 | 0 | 0.3235 | 0 | 0.4092 | 0 | 0.6593 | 0 |
| 155 | Ventricle | 0.0043 | 0.0055 | 0.0037 | 0.0045 | 0.0027 | 0.0100 | 0 | 0.1162 | 0 | 0.0958 | 0 | 0.5429 | 0 |
| 156 | visual 1 ctx | -0.0016 | 0.0074 | 0.0031 | 0.0046 | 0.0020 | 0.0129 | 0 | 0.6114 | 0 | 0.1631 | 0 | 0.7135 | 0 |
| 157 | visual 2 ctx | 0.0015 | 0.0055 | 0.0029 | 0.0041 | 0.0023 | 0.0101 | 0 | 0.5423 | 0 | 0.1463 | 0 | 0.5948 | 0 |
| 158 | ventral anterior thalamic nucleus | 0.0035 | 0.0057 | 0.0001 | 0.0035 | 0.0027 | 0.0073 | 0 | 0.1906 | 0 | 0.9590 | 0 | 0.4105 | 0 |
| 159 | cochlear nucleus | -0.0018 | 0.0041 | -0.0007 | 0.0020 | -0.0022 | 0.0038 | 0 | 0.3230 | 0 | 0.4052 | 0 | 0.2240 | 0 |
| 160 | vestibular nucleus | 0.0032 | 0.0053 | 0.0015 | 0.0036 | 0.0027 | 0.0043 | 0 | 0.1995 | 0 | 0.3615 | 0 | 0.1844 | 0 |
| 161 | ventrolateral thalamic nucleus | 0.0068 | 0.0055 | 0.0006 | 0.0039 | 0.0064 | 0.0070 | 1 | 0.0283 | 0 | 0.7203 | 0 | 0.0740 | 1 |
| 162 | ventral lateral striatum | 0.0033 | 0.0077 | -0.0013 | 0.0029 | 0.0034 | 0.0050 | 0 | 0.3401 | 0 | 0.3423 | 0 | 0.1571 | 0 |
| 163 | ventromedial thalamic nucleus | 0.0071 | 0.0060 | 0.0001 | 0.0055 | 0.0048 | 0.0036 | 1 | 0.0340 | 0 | 0.9665 | 1 | 0.0230 | 2 |
| 164 | ventral medial nucleus | 0.0021 | 0.0280 | 0.0069 | 0.0123 | 0.0011 | 0.0269 | 0 | 0.8609 | 0 | 0.2285 | 0 | 0.9250 | 0 |
| 165 | ventral medial striatum | 0.0023 | 0.0071 | -0.0009 | 0.0050 | 0.0033 | 0.0068 | 0 | 0.4699 | 0 | 0.6878 | 0 | 0.2900 | 0 |
| 166 | ventral orbital ctx | 0.0043 | 0.0063 | 0.0015 | 0.0053 | 0.0065 | 0.0081 | 0 | 0.1537 | 0 | 0.5247 | 0 | 0.1072 | 0 |
| 167 | ventral pallidum | 0.0009 | 0.0162 | 0.0014 | 0.0060 | 0.0004 | 0.0123 | 0 | 0.8985 | 0 | 0.6012 | 0 | 0.9435 | 0 |
| 168 | ventral posteriolateral thalamic n | 0.0035 | 0.0083 | -0.0006 | 0.0046 | 0.0028 | 0.0043 | 0 | 0.3435 | 0 | 0.7601 | 0 | 0.1775 | 0 |
| 169 | ventral posteriolmedial thalamic n | 0.0049 | 0.0068 | 0.0030 | 0.0047 | 0.0035 | 0.0057 | 0 | 0.1339 | 0 | 0.1816 | 0 | 0.1871 | 0 |
| 170 | ventral subiculum | 0.0033 | 0.0043 | 0.0025 | 0.0050 | 0.0025 | 0.0058 | 0 | 0.1207 | 0 | 0.2699 | 0 | 0.3338 | 0 |
| 171 | ventral tegmental area | -0.0081 | 0.0077 | 0.0024 | 0.0068 | -0.0023 | 0.0089 | 0 | 0.0501 | 0 | 0.4167 | 0 | 0.5579 | 0 |
| 172 | White Matter | 0.0029 | 0.0054 | 0.0016 | 0.0040 | 0.0032 | 0.0071 | 0 | 0.2503 | 0 | 0.3769 | 0 | 0.3230 | 0 |
| 173 | White Matter | 0.0064 | 0.0070 | 0.0015 | 0.0060 | 0.0045 | 0.0053 | 0 | 0.0779 | 0 | 0.5587 | 0 | 0.0912 | 0 |
| 174 | zona incerta | 0.0056 | 0.0069 | 0.0029 | 0.0061 | 0.0046 | 0.0079 | 0 | 0.1033 | 0 | 0.2986 | 0 | 0.2106 | 0 |
| - | whole brain | 0.0026 | 0.0045 | 0.0014 | 0.0031 | 0.0026 | 0.0058 | 0 | 0.2174 | 0 | 0.3293 | 0 | 0.3285 | |
| | | | | | | | | 25 | | 3 | | 16 | | |

**FIG. 14 (Continued)**

| Region Num | Region Nam | M1 | std | M2 | std | M3 | std | h1 | p1 | h2 | p2 | h3 | p3 | |
|---|---|---|---|---|---|---|---|---|---|---|---|---|---|---|
| 1 | 10th cerebellar lobule | -0.0108 | 0.0212 | -0.0163 | 0.0183 | -0.0186 | 0.0276 | 0 | 0.2671 | 0 | 0.0816 | 0 | 0.1595 | 0 |
| 2 | 1st cerebellar lobule | -0.0058 | 0.0373 | 0.0107 | 0.0232 | 0.0117 | 0.0140 | 0 | 0.7173 | 0 | 0.3101 | 0 | 0.0964 | 0 |
| 3 | 2nd cerebellar lobule | -0.0065 | 0.0179 | 0.0066 | 0.0217 | -0.0072 | 0.0243 | 0 | 0.4141 | 0 | 0.4909 | 0 | 0.5032 | 0 |
| 4 | 3rd cerebellar lobule | -0.0036 | 0.0124 | -0.0038 | 0.0109 | 0.0014 | 0.0082 | 0 | 0.5069 | 0 | 0.4350 | 0 | 0.7036 | 0 |
| 5 | 4th cerebellar lobule | -0.0002 | 0.0108 | 0.0067 | 0.0111 | 0.0044 | 0.0114 | 0 | 0.9666 | 0 | 0.1977 | 0 | 0.3864 | 0 |
| 6 | 5th cerebellar lobule | 0.0099 | 0.0224 | 0.0144 | 0.0332 | 0.0090 | 0.0235 | 0 | 0.3278 | 0 | 0.3372 | 0 | 0.3900 | 0 |
| 7 | motor trigeminal nucleus | 0.0162 | 0.0453 | 0.0106 | 0.0309 | 0.0210 | 0.0272 | 0 | 0.4211 | 0 | 0.4405 | 0 | 0.1176 | 0 |
| 8 | root of trigeminal nerve | 0.0022 | 0.0141 | -0.0017 | 0.0055 | 0.0014 | 0.0055 | 0 | 0.7217 | 0 | 0.4823 | 0 | 0.5589 | 0 |
| 9 | 6th cerebellar lobule | 0.0000 | 0.0151 | -0.0030 | 0.0136 | 0.0053 | 0.0136 | 0 | 0.9945 | 0 | 0.6184 | 0 | 0.3838 | 0 |
| 10 | 7th cerebellar lobule | -0.0174 | 0.0346 | -0.0134 | 0.0182 | -0.0101 | 0.0161 | 0 | 0.2734 | 0 | 0.1299 | 0 | 0.1860 | 0 |
| 11 | facial nucleus | 0.0046 | 0.0289 | 0.0124 | 0.0268 | 0.0070 | 0.0335 | 0 | 0.7106 | 0 | 0.3080 | 0 | 0.6286 | 0 |
| 12 | 8th cerebellar lobule | 0.0065 | 0.0102 | -0.0007 | 0.0181 | 0.0057 | 0.0137 | 0 | 0.1779 | 0 | 0.9291 | 0 | 0.3557 | 0 |
| 13 | 9th cerebellar lobule | -0.0006 | 0.0101 | -0.0143 | 0.0312 | -0.0075 | 0.0200 | 0 | 0.8824 | 0 | 0.3138 | 0 | 0.4008 | 0 |
| 14 | anterior thalamic nuclei | 0.0095 | 0.0231 | 0.0013 | 0.0161 | 0.0043 | 0.0142 | 0 | 0.3596 | 0 | 0.8468 | 0 | 0.4944 | 0 |
| 15 | anterior amygdaloid nucleus | -0.0084 | 0.0358 | -0.0026 | 0.0406 | -0.0136 | 0.0497 | 0 | 0.5914 | 0 | 0.8822 | 0 | 0.5317 | 0 |
| 16 | accumbens core | 0.0026 | 0.0114 | -0.0027 | 0.0126 | -0.0007 | 0.0134 | 0 | 0.5987 | 0 | 0.6246 | 0 | 0.9085 | 0 |
| 17 | accumbens shell | -0.0024 | 0.0181 | 0.0002 | 0.0117 | -0.0051 | 0.0142 | 0 | 0.7546 | 0 | 0.9618 | 0 | 0.4229 | 0 |
| 18 | anterior hypothalamic area | -0.0039 | 0.0112 | -0.0041 | 0.0073 | -0.0035 | 0.0167 | 0 | 0.4314 | 0 | 0.2215 | 0 | 0.6319 | 0 |
| 19 | anterior lobe pituitary | 0.0192 | 0.0324 | 0.0049 | 0.0317 | 0.0056 | 0.0303 | 0 | 0.2070 | 0 | 0.7218 | 0 | 0.6671 | 0 |
| 20 | anterior olfactory nucleus | 0.0063 | 0.0082 | -0.0099 | 0.0208 | 0.0021 | 0.0110 | 0 | 0.1226 | 0 | 0.2950 | 0 | 0.6589 | 0 |
| 21 | anterior pretectal nucleus | 0.0047 | 0.0230 | 0.0123 | 0.0253 | 0.0046 | 0.0327 | 0 | 0.6405 | 0 | 0.2863 | 0 | 0.7465 | 0 |
| 22 | arcuate nucleus | 0.0180 | 0.0483 | 0.0393 | 0.0389 | -0.0270 | 0.0831 | 0 | 0.4026 | 0 | 0.0562 | 0 | 0.4620 | 0 |
| 23 | auditory ctx | 0.0054 | 0.0085 | -0.0005 | 0.0094 | 0.0038 | 0.0109 | 0 | 0.1828 | 0 | 0.9084 | 0 | 0.4300 | 0 |
| 24 | basal amygdaloid nucleus | 0.0040 | 0.0221 | 0.0034 | 0.0312 | 0.0118 | 0.0329 | 0 | 0.6791 | 0 | 0.7998 | 0 | 0.4194 | 0 |
| 25 | CA1 dorsal | 0.0027 | 0.0152 | 0.0032 | 0.0113 | 0.0014 | 0.0135 | 0 | 0.6797 | 0 | 0.5186 | 0 | 0.8060 | 0 |

*FIG. 15*

| # | Region | | | | | | | | | | | | | |
|---|---|---|---|---|---|---|---|---|---|---|---|---|---|---|
| 26 | CA1 hippocampus ventral | -0.0004 | 0.0064 | 0.0007 | 0.0201 | 0.0033 | 0.0144 | 0 | 0.8939 | 0 | 0.9350 | 0 | 0.5954 | 0 |
| 27 | CA2 | 0.0094 | 0.0204 | 0.0068 | 0.0186 | 0.0141 | 0.0184 | 0 | 0.3094 | 0 | 0.4124 | 0 | 0.1203 | 0 |
| 28 | CA3 dorsal | 0.0153 | 0.0242 | -0.0045 | 0.0171 | 0.0083 | 0.0129 | 0 | 0.1820 | 0 | 0.5472 | 0 | 0.1752 | 0 |
| 29 | CA3 hippocampus ventral | -0.0057 | 0.0326 | -0.0015 | 0.0159 | -0.0165 | 0.0262 | 0 | 0.6841 | 0 | 0.8289 | 0 | 0.1838 | 0 |
| 30 | central amygdaloid nucleus | 0.0028 | 0.0274 | -0.0121 | 0.0370 | -0.0013 | 0.0451 | 0 | 0.8110 | 0 | 0.4608 | 0 | 0.9485 | 0 |
| 31 | anterior cingulate area | 0.0092 | 0.0107 | 0.0070 | 0.0090 | 0.0036 | 0.0147 | 0 | 0.0895 | 0 | 0.1162 | 0 | 0.5693 | 0 |
| 32 | central gray | 0.0065 | 0.0358 | 0.0019 | 0.0288 | 0.0085 | 0.0347 | 0 | 0.6745 | 0 | 0.8802 | 0 | 0.5748 | 0 |
| 33 | claustrum | -0.0124 | 0.0235 | -0.0121 | 0.0146 | -0.0090 | 0.0264 | 0 | 0.2520 | 0 | 0.0988 | 0 | 0.4393 | 0 |
| 34 | central medial thalamic nucleus | 0.0161 | 0.0218 | 0.0015 | 0.0220 | 0.0193 | 0.0286 | 0 | 0.1306 | 0 | 0.8699 | 0 | 0.1586 | 0 |
| 35 | cortical amygdaloid nucleus | -0.0101 | 0.0232 | 0.0042 | 0.0340 | -0.0092 | 0.0409 | 0 | 0.3360 | 0 | 0.7768 | 0 | 0.6068 | 0 |
| 36 | copula of the pyramis | -0.0004 | 0.0335 | 0.0155 | 0.0336 | -0.0066 | 0.0207 | 0 | 0.9754 | 0 | 0.3091 | 0 | 0.4683 | 0 |
| 37 | crus 1 of ansiform lobule | 0.0182 | 0.0211 | 0.0129 | 0.0190 | 0.0107 | 0.0187 | 0 | 0.0885 | 0 | 0.1579 | 0 | 0.2210 | 0 |
| 38 | crus 2 of ansiform lobule | 0.0016 | 0.0101 | 0.0006 | 0.0038 | 0.0076 | 0.0117 | 0 | 0.7124 | 0 | 0.7190 | 0 | 0.1702 | 0 |
| 39 | diagonal band of Broca | -0.0105 | 0.0415 | -0.0049 | 0.0527 | -0.0031 | 0.0328 | 0 | 0.5618 | 0 | 0.8285 | 0 | 0.8246 | 0 |
| 40 | dentate gyrus dorsal | -0.0011 | 0.0210 | 0.0025 | 0.0179 | 0.0078 | 0.0157 | 0 | 0.9063 | 0 | 0.7474 | 0 | 0.2794 | 0 |
| 41 | dentate gyrus ventral | -0.0012 | 0.0232 | 0.0096 | 0.0235 | -0.0105 | 0.0206 | 0 | 0.9061 | 0 | 0.3649 | 0 | 0.2684 | 0 |
| 42 | dorsal lateral striatum | 0.0042 | 0.0125 | -0.0005 | 0.0077 | 0.0031 | 0.0096 | 0 | 0.4453 | 0 | 0.8776 | 0 | 0.4587 | 0 |
| 43 | dorsal medial nucleus | 0.0156 | 0.0407 | -0.0106 | 0.0321 | 0.0057 | 0.0232 | 0 | 0.3897 | 0 | 0.4537 | 0 | 0.5711 | 0 |
| 44 | dorsal medial striatum | 0.0001 | 0.0097 | -0.0039 | 0.0037 | 0.0064 | 0.0083 | 0 | 0.9852 | 1 | 0.0468 | 0 | 0.1199 | 1 |
| 45 | dorsomedial tegmental area | 0.0070 | 0.0242 | 0.0025 | 0.0159 | 0.0023 | 0.0270 | 0 | 0.5088 | 0 | 0.7141 | 0 | 0.8460 | 0 |
| 46 | DPGi | -0.0070 | 0.0213 | 0.0030 | 0.0166 | -0.0109 | 0.0194 | 0 | 0.4593 | 0 | 0.6756 | 0 | 0.2266 | 0 |
| 47 | dorsal raphe | -0.0035 | 0.0228 | 0.0074 | 0.0250 | 0.0009 | 0.0231 | 0 | 0.7185 | 0 | 0.5027 | 0 | 0.9312 | 0 |
| 48 | subiculum dorsal | 0.0068 | 0.0225 | 0.0000 | 0.0186 | 0.0073 | 0.0267 | 0 | 0.4906 | 0 | 0.9965 | 0 | 0.5353 | 0 |
| 49 | extended amydala | 0.0038 | 0.0206 | 0.0062 | 0.0192 | 0.0078 | 0.0201 | 0 | 0.6671 | 0 | 0.4620 | 0 | 0.3841 | 0 |
| 50 | ectorhinal ctx | -0.0065 | 0.0878 | -0.0293 | 0.0920 | -0.0034 | 0.0513 | 0 | 0.8629 | 0 | 0.4708 | 0 | 0.8783 | 0 |

**FIG. 15 (Continued)**

EP 4 741 014 A2

| | | | | | | | | | | | | | | |
|---|---|---|---|---|---|---|---|---|---|---|---|---|---|---|
| 51 | endopiriform nucleus | 0.0083 | 0.0085 | -0.0020 | 0.0102 | 0.0100 | 0.0134 | 0 | 0.0605 | 0 | 0.6581 | 0 | 0.1290 | 0 |
| 52 | entorhinal ctx | -0.0028 | 0.0144 | -0.0037 | 0.0100 | -0.0035 | 0.0111 | 0 | 0.6568 | 0 | 0.4114 | 0 | 0.4696 | 0 |
| 53 | external plexiform layer | 0.0045 | 0.0100 | 0.0026 | 0.0212 | 0.0030 | 0.0116 | 0 | 0.3216 | 0 | 0.7755 | 0 | 0.5599 | 0 |
| 54 | flocculus cerebellum | 0.0176 | 0.0139 | 0.0022 | 0.0234 | 0.0071 | 0.0283 | 1 | 0.0263 | 0 | 0.8293 | 0 | 0.5668 | 1 |
| 55 | frontal association ctx | 0.0091 | 0.0129 | -0.0048 | 0.0259 | 0.0024 | 0.0084 | 0 | 0.1427 | 0 | 0.6675 | 0 | 0.5095 | 0 |
| 56 | gigantocellular reticular nucleus | -0.0013 | 0.0023 | -0.0020 | 0.0060 | -0.0022 | 0.0050 | 0 | 0.2388 | 0 | 0.4557 | 0 | 0.3352 | 0 |
| 57 | glomerular layer | 0.0081 | 0.0300 | 0.0001 | 0.0182 | -0.0048 | 0.0248 | 0 | 0.5356 | 0 | 0.9931 | 0 | 0.6525 | 0 |
| 58 | globus pallidus | -0.0005 | 0.0172 | 0.0104 | 0.0129 | 0.0073 | 0.0154 | 0 | 0.9443 | 0 | 0.1066 | 0 | 0.2986 | 0 |
| 59 | granular cell layer | 0.0077 | 0.0056 | 0.0050 | 0.0134 | 0.0079 | 0.0099 | 1 | 0.0194 | 0 | 0.4071 | 0 | 0.1085 | 1 |
| 60 | habenula nucleus | -0.0210 | 0.0605 | -0.0275 | 0.0585 | -0.0077 | 0.0392 | 0 | 0.4349 | 0 | 0.3011 | 0 | 0.6490 | 0 |
| 61 | intercalated amygdaloid nucleus | -0.0229 | 0.0329 | -0.0188 | 0.0311 | -0.0163 | 0.0477 | 0 | 0.1492 | 0 | 0.1982 | 0 | 0.4399 | 0 |
| 62 | inferior colliculus | 0.0077 | 0.0052 | -0.0085 | 0.0262 | 0.0153 | 0.0143 | 1 | 0.0148 | 0 | 0.4613 | 1 | 0.0473 | 2 |
| 63 | infralimbic ctx | -0.0122 | 0.0227 | 0.0073 | 0.0187 | -0.0137 | 0.0248 | 0 | 0.2446 | 0 | 0.3822 | 0 | 0.2321 | 0 |
| 64 | insular ctx | -0.0007 | 0.0309 | 0.0075 | 0.0204 | 0.0109 | 0.0199 | 0 | 0.9563 | 0 | 0.4080 | 0 | 0.2386 | 0 |
| 65 | interposed nucleus | 0.0006 | 0.0474 | -0.0201 | 0.0355 | 0.0036 | 0.0291 | 0 | 0.9751 | 0 | 0.2253 | 0 | 0.7712 | 0 |
| 66 | inferior olivary complex | 0.0044 | 0.0777 | -0.0088 | 0.0312 | 0.0057 | 0.0506 | 0 | 0.8949 | 0 | 0.5194 | 0 | 0.7945 | 0 |
| 67 | interpeduncular nucleus | 0.0305 | 0.0480 | 0.0204 | 0.0499 | 0.0294 | 0.0540 | 0 | 0.1801 | 0 | 0.3628 | 0 | 0.2391 | 0 |
| 68 | lateral amygdaloid nucleus | -0.0001 | 0.0336 | 0.0053 | 0.0184 | -0.0015 | 0.0175 | 0 | 0.9966 | 0 | 0.5136 | 0 | 0.8415 | 0 |
| 69 | Lat | -0.0097 | 0.0422 | 0.0062 | 0.0415 | -0.0107 | 0.0216 | 0 | 0.5978 | 0 | 0.7301 | 0 | 0.2773 | 0 |
| 70 | locus ceruleus | 0.0124 | 0.0336 | 0.0063 | 0.0304 | -0.0058 | 0.0284 | 0 | 0.4093 | 0 | 0.6328 | 0 | 0.6399 | 0 |
| 71 | lateral dorsal thalamic nucleus | 0.0345 | 0.0286 | 0.0283 | 0.0457 | -0.0098 | 0.0368 | 1 | 0.0317 | 0 | 0.1897 | 0 | 0.5425 | 1 |
| 72 | lateral geniculate | 0.0188 | 0.0289 | -0.0005 | 0.0257 | 0.0134 | 0.0371 | 0 | 0.1725 | 0 | 0.9661 | 0 | 0.4174 | 0 |
| 73 | lateral hypothalamus | 0.0190 | 0.0180 | 0.0062 | 0.0229 | 0.0148 | 0.0202 | 1 | 0.0484 | 0 | 0.5338 | 0 | 0.1311 | 1 |
| 74 | lemniscal nucleus | 0.0251 | 0.0286 | -0.0033 | 0.0166 | 0.0146 | 0.0185 | 0 | 0.0841 | 0 | 0.6453 | 0 | 0.1117 | 0 |
| 75 | lateral orbital ctx | -0.0002 | 0.0130 | -0.0079 | 0.0198 | 0.0056 | 0.0085 | 0 | 0.9696 | 0 | 0.3746 | 0 | 0.1650 | 0 |

*FIG. 15 (Continued)*

EP 4 741 014 A2

| # | Region | | | | | | | | | | | | | |
|---|---|---|---|---|---|---|---|---|---|---|---|---|---|
| 76 | lateral posterior thalamic nucleus | -0.0012 | 0.0290 | -0.0147 | 0.0277 | -0.0096 | 0.0316 | 0 | 0.9261 | 0 | 0.2508 | 0 | 0.4898 | 0 |
| 77 | lateral preoptic area | -0.0004 | 0.0265 | 0.0059 | 0.0140 | -0.0191 | 0.0341 | 0 | 0.9733 | 0 | 0.3483 | 0 | 0.2287 | 0 |
| 78 | lateral septal nucleus | 0.0062 | 0.0063 | 0.0013 | 0.0075 | 0.0052 | 0.0053 | 0 | 0.0627 | 0 | 0.6903 | 0 | 0.0606 | 0 |
| 79 | primary motor ctx | 0.0075 | 0.0047 | 0.0001 | 0.0055 | 0.0038 | 0.0061 | 1 | 0.0112 | 0 | 0.9759 | 0 | 0.1887 | 1 |
| 80 | secondary motor ctx | 0.0129 | 0.0240 | 0.0111 | 0.0171 | 0.0106 | 0.0208 | 0 | 0.2448 | 0 | 0.1745 | 0 | 0.2671 | 0 |
| 81 | magnocellular preoptic nucleus | -0.0104 | 0.0646 | -0.0225 | 0.0670 | -0.0321 | 0.0479 | 0 | 0.7096 | 0 | 0.4481 | 0 | 0.1618 | 0 |
| 82 | medial dorsal thalamic nucleus | -0.0056 | 0.0377 | -0.0264 | 0.0275 | 0.0149 | 0.0186 | 0 | 0.7290 | 0 | 0.0654 | 0 | 0.1075 | 0 |
| 83 | medial amygdaloid nucleus | 0.0065 | 0.0219 | 0.0016 | 0.0357 | 0.0113 | 0.0487 | 0 | 0.5017 | 0 | 0.9163 | 0 | 0.5933 | 0 |
| 84 | medial cerebellar nucleus fastigia | 0.0451 | 0.0281 | 0.0056 | 0.0413 | 0.0206 | 0.0209 | 1 | 0.0111 | 0 | 0.7551 | 0 | 0.0610 | 1 |
| 85 | medial geniculate | 0.0226 | 0.0286 | -0.0203 | 0.0464 | 0.0151 | 0.0301 | 0 | 0.1097 | 0 | 0.3324 | 0 | 0.2726 | 0 |
| 86 | medial mammillary nucleus | -0.0139 | 0.0254 | -0.0280 | 0.0435 | -0.0067 | 0.0445 | 0 | 0.2369 | 0 | 0.1758 | 0 | 0.7262 | 0 |
| 87 | median raphe nucleus | -0.0176 | 0.0205 | -0.0163 | 0.0229 | -0.0050 | 0.0271 | 0 | 0.0884 | 0 | 0.1411 | 0 | 0.6719 | 0 |
| 88 | medial orbital ctx | 0.0040 | 0.0351 | 0.0201 | 0.0213 | 0.0180 | 0.0288 | 0 | 0.7921 | 0 | 0.0690 | 0 | 0.1865 | 0 |
| 89 | medial preoptic area | 0.0081 | 0.0361 | -0.0062 | 0.0252 | 0.0041 | 0.0228 | 0 | 0.6086 | 0 | 0.5702 | 0 | 0.6761 | 0 |
| 90 | medial pretectal area | -0.0404 | 0.0374 | -0.0100 | 0.0607 | 0.0140 | 0.0522 | 1 | 0.0454 | 0 | 0.7037 | 0 | 0.5395 | 1 |
| 91 | medial septum | 0.0232 | 0.0175 | 0.0160 | 0.0130 | 0.0086 | 0.0210 | 1 | 0.0226 | 1 | 0.0293 | 0 | 0.3623 | 2 |
| 92 | neural lobe pituitary | -0.0187 | 0.0508 | 0.0469 | 0.0869 | -0.0085 | 0.0778 | 0 | 0.4087 | 0 | 0.2438 | 0 | 0.7993 | 0 |
| 93 | olivary nucleus | 0.0102 | 0.0457 | -0.0022 | 0.0235 | -0.0100 | 0.0304 | 0 | 0.6100 | 0 | 0.8238 | 0 | 0.4566 | 0 |
| 94 | paraventricular nuclus | 0.0058 | 0.0315 | -0.0049 | 0.0723 | 0.0134 | 0.0473 | 0 | 0.6714 | 0 | 0.8739 | 0 | 0.5186 | 0 |
| 95 | periaqueductal gray thalamus | -0.0008 | 0.0140 | 0.0031 | 0.0093 | 0.0020 | 0.0138 | 0 | 0.8920 | 0 | 0.4460 | 0 | 0.7347 | 0 |
| 96 | parabrachial nucleus | -0.0002 | 0.0131 | 0.0083 | 0.0128 | 0.0032 | 0.0190 | 0 | 0.9675 | 0 | 0.1703 | 0 | 0.6953 | 0 |
| 97 | PCRt | -0.0018 | 0.0226 | -0.0005 | 0.0092 | -0.0040 | 0.0197 | 0 | 0.8497 | 0 | 0.8955 | 0 | 0.6396 | 0 |
| 98 | parafascicular thalamic nucleus | 0.0061 | 0.0201 | 0.0000 | 0.0135 | -0.0011 | 0.0099 | 0 | 0.4927 | 0 | 0.9943 | 0 | 0.7925 | 0 |
| 99 | paraflocculus cerebellum | 0.0082 | 0.0149 | 0.0130 | 0.0184 | 0.0198 | 0.0171 | 0 | 0.2360 | 0 | 0.1438 | 1 | 0.0363 | 1 |
| 100 | posterior hypothalamic area | 0.0636 | 0.0474 | 0.0607 | 0.0564 | 0.0225 | 0.0512 | 1 | 0.0218 | 1 | 0.0462 | 0 | 0.3315 | 2 |

**FIG. 15 (Continued)**

EP 4 741 014 A2

| | | | | | | | | | | | | | |
|---|---|---|---|---|---|---|---|---|---|---|---|---|---|
| 101 | pineal gland | 0.0294 | 0.2317 | -0.0197 | 0.1209 | -0.0402 | 0.0714 | 0 | 0.7682 | 0 | 0.7069 | 0 | 0.2265 | 0 |
| 102 | caudal piriform ctx | 0.0050 | 0.0133 | 0.0057 | 0.0111 | 0.0018 | 0.0096 | 0 | 0.3976 | 0 | 0.2628 | 0 | 0.6572 | 0 |
| 103 | rostral piriform ctx | -0.0037 | 0.0088 | 0.0004 | 0.0066 | -0.0016 | 0.0144 | 0 | 0.3576 | 0 | 0.8956 | 0 | 0.7920 | 0 |
| 104 | premammillary nucleus | 0.0281 | 0.0670 | 0.0345 | 0.0369 | 0.0318 | 0.0558 | 0 | 0.3522 | 0 | 0.0705 | 0 | 0.2212 | 0 |
| 105 | paramedian lobule | 0.0115 | 0.0193 | -0.0073 | 0.0299 | 0.0080 | 0.0104 | 0 | 0.2033 | 0 | 0.5769 | 0 | 0.1203 | 0 |
| 106 | pontine nuclei | -0.0105 | 0.0143 | -0.0128 | 0.0185 | -0.0040 | 0.0138 | 0 | 0.1316 | 0 | 0.1515 | 0 | 0.5091 | 0 |
| 107 | pontine reticular nucleus caudal | -0.0066 | 0.0124 | -0.0017 | 0.0156 | 0.0027 | 0.0109 | 0 | 0.2490 | 0 | 0.7945 | 0 | 0.5653 | 0 |
| 108 | pontine reticular nucleus oral | 0.0143 | 0.0263 | 0.0060 | 0.0202 | 0.0118 | 0.0319 | 0 | 0.2385 | 0 | 0.4966 | 0 | 0.4062 | 0 |
| 109 | posterior thalamic nucleus | -0.0011 | 0.0119 | -0.0034 | 0.0111 | 0.0071 | 0.0168 | 0 | 0.8351 | 0 | 0.4796 | 0 | 0.3509 | 0 |
| 110 | periolivary nucleus | -0.0060 | 0.0215 | -0.0053 | 0.0096 | -0.0204 | 0.0214 | 0 | 0.5264 | 0 | 0.2358 | 0 | 0.0662 | 0 |
| 111 | prerubral field | 0.0158 | 0.0162 | -0.0033 | 0.0275 | -0.0027 | 0.0198 | 0 | 0.0615 | 0 | 0.7804 | 0 | 0.7548 | 0 |
| 112 | principal sensory nucleus trigemin | 0.0083 | 0.0219 | -0.0084 | 0.0140 | -0.0017 | 0.0121 | 0 | 0.3947 | 0 | 0.2028 | 0 | 0.7513 | 0 |
| 113 | precuniform nucleus | 0.0050 | 0.0386 | -0.0011 | 0.0414 | -0.0019 | 0.0443 | 0 | 0.7631 | 0 | 0.9487 | 0 | 0.9198 | 0 |
| 114 | perirhinal ctx | 0.0011 | 0.0289 | -0.0001 | 0.0132 | 0.0018 | 0.0106 | 0 | 0.9277 | 0 | 0.9847 | 0 | 0.6998 | 0 |
| 115 | prelimbic ctx | 0.0054 | 0.0109 | -0.0070 | 0.0242 | 0.0119 | 0.0083 | 0 | 0.2779 | 0 | 0.5114 | 1 | 0.0175 | 1 |
| 116 | parietal ctx | -0.0010 | 0.0139 | -0.0047 | 0.0023 | -0.0021 | 0.0149 | 0 | 0.8655 | 1 | 0.0038 | 0 | 0.7434 | 1 |
| 117 | pedunculopontine tegmental area | -0.0054 | 0.0351 | 0.0049 | 0.0202 | 0.0120 | 0.0106 | 0 | 0.7209 | 0 | 0.5819 | 1 | 0.0393 | 1 |
| 118 | paraventricular nucleus | 0.0099 | 0.0287 | 0.0077 | 0.0045 | 0.0162 | 0.0223 | 0 | 0.4373 | 1 | 0.0088 | 0 | 0.1352 | 1 |
| 119 | retrochiasmatic nucleus | 0.0104 | 0.0704 | 0.0407 | 0.0483 | 0.0471 | 0.0667 | 0 | 0.7314 | 0 | 0.0942 | 0 | 0.1441 | 0 |
| 120 | reuniens nucleus | 0.0050 | 0.0123 | -0.0005 | 0.0102 | -0.0083 | 0.0178 | 0 | 0.3613 | 0 | 0.9122 | 0 | 0.3058 | 0 |
| 121 | raphe linear | -0.0033 | 0.0473 | -0.0019 | 0.0442 | 0.0055 | 0.0428 | 0 | 0.8701 | 0 | 0.9191 | 0 | 0.7645 | 0 |
| 122 | raphe magnus | 0.0060 | 0.0119 | -0.0040 | 0.0201 | -0.0122 | 0.0184 | 0 | 0.2720 | 0 | 0.6425 | 0 | 0.1648 | 0 |
| 123 | raphe obscurus nucleus | 0.0125 | 0.0189 | -0.0112 | 0.0255 | -0.0028 | 0.0304 | 0 | 0.1662 | 0 | 0.3296 | 0 | 0.8308 | 0 |
| 124 | red nucleus | 0.0004 | 0.0496 | 0.0026 | 0.0339 | 0.0103 | 0.0408 | 0 | 0.9848 | 0 | 0.8595 | 0 | 0.5628 | 0 |
| 125 | retrosplenial caudal ctx | 0.0140 | 0.0322 | 0.0108 | 0.0380 | 0.0015 | 0.0227 | 0 | 0.3375 | 0 | 0.5177 | 0 | 0.8775 | 0 |

**FIG. 15 (Continued)**

| | | | | | | | | | | | | | | |
|---|---|---|---|---|---|---|---|---|---|---|---|---|---|---|
| 126 | retrosplenial rostral ctx | 0.0110 | 0.0198 | -0.0088 | 0.0358 | -0.0060 | 0.0291 | 0 | 0.2333 | 0 | 0.5723 | 0 | 0.6339 | 0 |
| 127 | reticular nucleus | 0.0096 | 0.0135 | 0.0061 | 0.0198 | 0.0125 | 0.0158 | 0 | 0.1417 | 0 | 0.4853 | 0 | 0.1099 | 0 |
| 128 | reticular nucleus midbrain | 0.0159 | 0.0174 | 0.0110 | 0.0219 | 0.0133 | 0.0176 | 0 | 0.0752 | 0 | 0.2738 | 0 | 0.1229 | 0 |
| 129 | reticulotegmental nucleus | -0.0067 | 0.0230 | 0.0149 | 0.0227 | 0.0011 | 0.0161 | 0 | 0.5104 | 0 | 0.1697 | 0 | 0.8709 | 0 |
| 130 | primary somatosensory ctx barrel f | 0.0054 | 0.0035 | 0.0020 | 0.0035 | 0.0053 | 0.0058 | 1 | 0.0128 | 0 | 0.2198 | 0 | 0.0747 | 1 |
| 131 | primary somatosensory ctx forelimb | 0.0053 | 0.0066 | 0.0037 | 0.0070 | 0.0048 | 0.0055 | 0 | 0.1041 | 0 | 0.2483 | 0 | 0.0831 | 0 |
| 132 | primary somatosensory ctx hindlimb | 0.0062 | 0.0083 | 0.0051 | 0.0049 | 0.0092 | 0.0097 | 0 | 0.1252 | 0 | 0.0528 | 0 | 0.0672 | 0 |
| 133 | primary somatosensory ctx jaw | 0.0018 | 0.0103 | 0.0003 | 0.0090 | 0.0050 | 0.0061 | 0 | 0.6834 | 0 | 0.9478 | 0 | 0.0984 | 0 |
| 134 | primary somatosensory ctx shoulder | 0.0035 | 0.0221 | -0.0102 | 0.0156 | 0.0014 | 0.0125 | 0 | 0.7164 | 0 | 0.1700 | 0 | 0.7941 | 0 |
| 135 | primary somatosensory ctx trunk | 0.0091 | 0.0126 | 0.0050 | 0.0176 | 0.0028 | 0.0152 | 0 | 0.1362 | 0 | 0.5172 | 0 | 0.6682 | 0 |
| 136 | primary somatosensory ctx upper li | 0.0109 | 0.0127 | 0.0054 | 0.0120 | 0.0070 | 0.0097 | 0 | 0.0909 | 0 | 0.3205 | 0 | 0.1406 | 0 |
| 137 | secondary somaotsensory ctx | -0.0015 | 0.0175 | -0.0086 | 0.0203 | -0.0027 | 0.0165 | 0 | 0.8433 | 0 | 0.3454 | 0 | 0.7003 | 0 |
| 138 | suprachiasmatic nucleus | 0.0291 | 0.0549 | 0.0024 | 0.0555 | 0.0028 | 0.0558 | 0 | 0.2503 | 0 | 0.9199 | 0 | 0.9074 | 0 |
| 139 | substantia innominata | 0.0110 | 0.0298 | -0.0031 | 0.0712 | -0.0253 | 0.0494 | 0 | 0.4096 | 0 | 0.9191 | 0 | 0.2655 | 0 |
| 140 | simple lobule cerebellum | -0.0058 | 0.0287 | -0.0048 | 0.0202 | -0.0071 | 0.0316 | 0 | 0.6416 | 0 | 0.5824 | 0 | 0.6058 | 0 |
| 141 | substantia nigra compacta | 0.0183 | 0.0302 | -0.0008 | 0.0639 | 0.0148 | 0.0400 | 0 | 0.1971 | 0 | 0.9760 | 0 | 0.4049 | 0 |
| 142 | substantia nigra reticularis | 0.0074 | 0.0468 | 0.0004 | 0.0268 | 0.0227 | 0.0680 | 0 | 0.7129 | 0 | 0.9691 | 0 | 0.4502 | 0 |
| 143 | supraoptic nucleus | 0.0418 | 0.0460 | 0.0007 | 0.0558 | 0.0277 | 0.0695 | 0 | 0.0765 | 0 | 0.9769 | 0 | 0.3743 | 0 |
| 144 | solitary tract nucleus | 0.0060 | 0.0188 | -0.0016 | 0.0090 | -0.0002 | 0.0126 | 0 | 0.4668 | 0 | 0.6863 | 0 | 0.9711 | 0 |
| 145 | bed nucleus stria terminalis | 0.0040 | 0.0103 | 0.0052 | 0.0071 | 0.0007 | 0.0162 | 0 | 0.3892 | 0 | 0.1318 | 0 | 0.9249 | 0 |
| 146 | subthalamic nucleus | 0.0136 | 0.0306 | 0.0279 | 0.0502 | 0.0075 | 0.0472 | 0 | 0.3252 | 0 | 0.2316 | 0 | 0.7139 | 0 |
| 147 | superior colliculus | 0.0037 | 0.0133 | -0.0051 | 0.0113 | 0.0042 | 0.0105 | 0 | 0.5262 | 0 | 0.3214 | 0 | 0.3718 | 0 |
| 148 | sub coeruleus nucleus | 0.0004 | 0.0149 | 0.0048 | 0.0198 | 0.0068 | 0.0197 | 0 | 0.9464 | 0 | 0.5806 | 0 | 0.4352 | 0 |
| 149 | supramammillary nucleus | -0.0121 | 0.0786 | 0.0263 | 0.0371 | 0.0269 | 0.0660 | 0 | 0.7207 | 0 | 0.1436 | 0 | 0.3645 | 0 |
| 150 | temporal ctx | -0.0379 | 0.0535 | -0.0608 | 0.0762 | -0.0257 | 0.0301 | 0 | 0.1431 | 0 | 0.1080 | 0 | 0.0913 | 0 |

**FIG. 15 (Continued)**

| # | Region | | | | | | | | | | | | | |
|---|--------|---|---|---|---|---|---|---|---|---|---|---|---|---|
| 151 | triangular septal nucleus | -0.0041 | 0.0293 | -0.0122 | 0.0239 | 0.0081 | 0.0240 | 0 | 0.7442 | 0 | 0.2666 | 0 | 0.4457 | 0 |
| 152 | tenia tecta ctx | -0.0030 | 0.0249 | -0.0092 | 0.0341 | 0.0048 | 0.0393 | 0 | 0.7810 | 0 | 0.5360 | 0 | 0.7778 | 0 |
| 153 | olfactory tubercles | -0.0006 | 0.0243 | -0.0082 | 0.0175 | -0.0046 | 0.0115 | 0 | 0.9569 | 0 | 0.3010 | 0 | 0.3684 | 0 |
| 154 | trapezoid body | -0.0186 | 0.0247 | -0.0079 | 0.0112 | -0.0180 | 0.0255 | 0 | 0.1248 | 0 | 0.1439 | 0 | 0.1448 | 0 |
| 155 | Ventricle | 0.0035 | 0.0078 | -0.0054 | 0.0125 | -0.0102 | 0.0145 | 0 | 0.3182 | 0 | 0.3340 | 0 | 0.1464 | 0 |
| 156 | visual 1 ctx | 0.0054 | 0.0091 | 0.0035 | 0.0166 | 0.0016 | 0.0064 | 0 | 0.2067 | 0 | 0.6258 | 0 | 0.5708 | 0 |
| 157 | visual 2 ctx | -0.0022 | 0.0206 | -0.0045 | 0.0099 | -0.0071 | 0.0183 | 0 | 0.8073 | 0 | 0.3166 | 0 | 0.3873 | 0 |
| 158 | ventral anterior thalamic nucleus | 0.0061 | 0.0269 | 0.0124 | 0.0145 | 0.0050 | 0.0162 | 0 | 0.6036 | 0 | 0.0891 | 0 | 0.4848 | 0 |
| 159 | cochlear nucleus | -0.0136 | 0.0120 | 0.0006 | 0.0137 | -0.0010 | 0.0103 | 1 | 0.0388 | 0 | 0.9162 | 0 | 0.8255 | 1 |
| 160 | vestibular nucleus | 0.0000 | 0.0126 | -0.0047 | 0.0089 | 0.0015 | 0.0074 | 0 | 0.9960 | 0 | 0.2552 | 0 | 0.6298 | 0 |
| 161 | ventrolateral thalamic nucleus | 0.0037 | 0.0123 | 0.0001 | 0.0180 | 0.0040 | 0.0156 | 0 | 0.4951 | 0 | 0.9941 | 0 | 0.5586 | 0 |
| 162 | ventral lateral striatum | -0.0001 | 0.0109 | -0.0022 | 0.0067 | 0.0029 | 0.0086 | 0 | 0.9818 | 0 | 0.4568 | 0 | 0.4484 | 0 |
| 163 | ventromedial thalamic nucleus | 0.0209 | 0.0226 | -0.0070 | 0.0217 | 0.0021 | 0.0294 | 0 | 0.0732 | 0 | 0.4661 | 0 | 0.8671 | 0 |
| 164 | ventral medial nucleus | 0.0140 | 0.0282 | 0.0134 | 0.0537 | 0.0243 | 0.0420 | 0 | 0.2799 | 0 | 0.5671 | 0 | 0.2155 | 0 |
| 165 | ventral medial striatum | 0.0029 | 0.0112 | 0.0043 | 0.0103 | 0.0023 | 0.0097 | 0 | 0.5544 | 0 | 0.3550 | 0 | 0.5803 | 0 |
| 166 | ventral orbital ctx | 0.0029 | 0.0138 | 0.0005 | 0.0163 | -0.0006 | 0.0189 | 0 | 0.6338 | 0 | 0.9420 | 0 | 0.9398 | 0 |
| 167 | ventral pallidum | -0.0101 | 0.0177 | -0.0065 | 0.0157 | -0.0064 | 0.0252 | 0 | 0.2215 | 0 | 0.3576 | 0 | 0.5596 | 0 |
| 168 | ventral posteriolateral thalamic n | -0.0036 | 0.0169 | 0.0023 | 0.0174 | 0.0013 | 0.0186 | 0 | 0.6241 | 0 | 0.7610 | 0 | 0.8709 | 0 |
| 169 | ventral posteriolmedial thalamic n | 0.0057 | 0.0130 | 0.0099 | 0.0163 | 0.0069 | 0.0161 | 0 | 0.3318 | 0 | 0.1994 | 0 | 0.3443 | 0 |
| 170 | ventral subiculum | -0.0029 | 0.0130 | -0.0016 | 0.0081 | -0.0064 | 0.0153 | 0 | 0.6127 | 0 | 0.6533 | 0 | 0.3561 | 0 |
| 171 | ventral tegmental area | -0.0304 | 0.0417 | -0.0264 | 0.0508 | -0.0106 | 0.0442 | 0 | 0.1341 | 0 | 0.2595 | 0 | 0.5829 | 0 |
| 172 | White Matter | 0.0029 | 0.0082 | -0.0005 | 0.0051 | 0.0013 | 0.0076 | 0 | 0.4304 | 0 | 0.8355 | 0 | 0.6826 | 0 |
| 173 | White Matter | -0.0064 | 0.0181 | -0.0098 | 0.0162 | -0.0045 | 0.0072 | 0 | 0.4248 | 0 | 0.1997 | 0 | 0.1883 | 0 |
| 174 | zona incerta | 0.0100 | 0.0125 | 0.0067 | 0.0177 | 0.0158 | 0.0117 | 0 | 0.1085 | 0 | 0.3935 | 1 | 0.0215 | 1 |
| - | whole brain | 0.0026 | 0.0050 | 0.0001 | 0.0026 | 0.0023 | 0.0044 | 0 | 0.2627 | 0 | 0.9143 | 0 | 0.2595 | 0 |
| | | | | | | | | 12 | | 5 | | 5 | | |

**FIG. 15 (Continued)**

| Region Num | Region Nam | M1 | std | M2 | std | M3 | std | h1 | p1 | h2 | p2 | h3 | p3 | |
|---|---|---|---|---|---|---|---|---|---|---|---|---|---|---|
| 1 | 10th cerebellar lobule | 0.0023 | 0.0058 | 0.0021 | 0.0010 | 0.0053 | 0.0048 | 0 | 0.4219 | 1 | 0.0080 | 0 | 0.0691 | 1 |
| 2 | 1st cerebellar lobule | 0.0042 | 0.0047 | 0.0034 | 0.0012 | 0.0035 | 0.0043 | 0 | 0.1142 | 1 | 0.0038 | 0 | 0.1430 | 1 |
| 3 | 2nd cerebellar lobule | 0.0047 | 0.0037 | 0.0014 | 0.0019 | 0.0059 | 0.0029 | 1 | 0.0442 | 0 | 0.1859 | 1 | 0.0104 | 2 |
| 4 | 3rd cerebellar lobule | 0.0051 | 0.0034 | 0.0019 | 0.0012 | 0.0076 | 0.0037 | 1 | 0.0283 | 1 | 0.0210 | 1 | 0.0099 | 3 |
| 5 | 4th cerebellar lobule | 0.0041 | 0.0034 | 0.0014 | 0.0010 | 0.0056 | 0.0042 | 0 | 0.0554 | 1 | 0.0418 | 1 | 0.0415 | 2 |
| 6 | 5th cerebellar lobule | 0.0044 | 0.0035 | 0.0020 | 0.0012 | 0.0067 | 0.0034 | 1 | 0.0472 | 1 | 0.0200 | 1 | 0.0119 | 3 |
| 7 | motor trigeminal nucleus | 0.0039 | 0.0056 | 0.0015 | 0.0023 | 0.0033 | 0.0054 | 0 | 0.1962 | 0 | 0.2230 | 0 | 0.2392 | 0 |
| 8 | root of trigeminal nerve | 0.0036 | 0.0034 | 0.0012 | 0.0018 | 0.0064 | 0.0023 | 0 | 0.0798 | 0 | 0.2035 | 1 | 0.0036 | 1 |
| 9 | 6th cerebellar lobule | 0.0015 | 0.0022 | 0.0015 | 0.0010 | 0.0037 | 0.0037 | 0 | 0.2083 | 1 | 0.0334 | 0 | 0.0923 | 1 |
| 10 | 7th cerebellar lobule | 0.0016 | 0.0035 | 0.0008 | 0.0023 | 0.0021 | 0.0040 | 0 | 0.3731 | 0 | 0.4624 | 0 | 0.3154 | 0 |
| 11 | facial nucleus | 0.0020 | 0.0037 | 0.0017 | 0.0025 | 0.0038 | 0.0053 | 0 | 0.2958 | 0 | 0.2029 | 0 | 0.1787 | 0 |
| 12 | 8th cerebellar lobule | 0.0037 | 0.0062 | 0.0028 | 0.0017 | 0.0042 | 0.0037 | 0 | 0.2613 | 1 | 0.0218 | 0 | 0.0626 | 1 |
| 13 | 9th cerebellar lobule | 0.0047 | 0.0034 | 0.0040 | 0.0014 | 0.0073 | 0.0041 | 1 | 0.0350 | 1 | 0.0031 | 1 | 0.0167 | 3 |
| 14 | anterior thalamic nuclei | 0.0101 | 0.0031 | 0.0049 | 0.0021 | 0.0115 | 0.0038 | 1 | 0.0018 | 1 | 0.0067 | 1 | 0.0025 | 3 |
| 15 | anterior amygdaloid nucleus | 0.0070 | 0.0033 | 0.0035 | 0.0016 | 0.0086 | 0.0016 | 1 | 0.0096 | 1 | 0.0072 | 1 | 0.0003 | 3 |
| 16 | accumbens core | 0.0063 | 0.0038 | 0.0025 | 0.0011 | 0.0093 | 0.0049 | 1 | 0.0201 | 1 | 0.0068 | 1 | 0.0127 | 3 |
| 17 | accumbens shell | 0.0064 | 0.0047 | 0.0031 | 0.0020 | 0.0084 | 0.0063 | 1 | 0.0392 | 1 | 0.0261 | 1 | 0.0396 | 3 |
| 18 | anterior hypothalamic area | 0.0053 | 0.0024 | 0.0046 | 0.0027 | 0.0064 | 0.0046 | 1 | 0.0073 | 1 | 0.0183 | 1 | 0.0345 | 3 |
| 19 | anterior lobe pituitary | -0.0027 | 0.0049 | 0.0036 | 0.0025 | 0.0018 | 0.0049 | 0 | 0.2743 | 1 | 0.0344 | 0 | 0.4452 | 1 |
| 20 | anterior olfactory nucleus | 0.0040 | 0.0016 | 0.0022 | 0.0015 | 0.0048 | 0.0026 | 1 | 0.0053 | 1 | 0.0300 | 1 | 0.0143 | 3 |
| 21 | anterior pretectal nucleus | 0.0039 | 0.0019 | 0.0004 | 0.0015 | 0.0051 | 0.0016 | 1 | 0.0104 | 0 | 0.6067 | 1 | 0.0024 | 2 |
| 22 | arcuate nucleus | 0.0038 | 0.0112 | 0.0083 | 0.0123 | 0.0127 | 0.0075 | 0 | 0.4938 | 0 | 0.2034 | 1 | 0.0190 | 1 |
| 23 | auditory ctx | 0.0031 | 0.0019 | 0.0011 | 0.0010 | 0.0043 | 0.0023 | 1 | 0.0240 | 0 | 0.0670 | 1 | 0.0154 | 2 |
| 24 | basal amygdaloid nucleus | 0.0048 | 0.0047 | 0.0039 | 0.0032 | 0.0072 | 0.0042 | 0 | 0.0829 | 0 | 0.0539 | 1 | 0.0189 | 1 |
| 25 | CA1  dorsal | 0.0053 | 0.0024 | 0.0036 | 0.0014 | 0.0071 | 0.0019 | 1 | 0.0075 | 1 | 0.0047 | 1 | 0.0010 | 3 |

*FIG. 16*

| | | | | | | | | | | | | | |
|---|---|---|---|---|---|---|---|---|---|---|---|---|---|
| 26 | CA1 hippocampus ventral | 0.0039 | 0.0028 | 0.0019 | 0.0021 | 0.0052 | 0.0039 | 1 | 0.0363 | 0 | 0.1157 | 1 | 0.0397 | 2 |
| 27 | CA2 | 0.0076 | 0.0033 | 0.0021 | 0.0029 | 0.0089 | 0.0031 | 1 | 0.0067 | 0 | 0.1857 | 1 | 0.0029 | 2 |
| 28 | CA3 dorsal | 0.0054 | 0.0032 | 0.0052 | 0.0034 | 0.0068 | 0.0044 | 1 | 0.0185 | 1 | 0.0274 | 1 | 0.0264 | 3 |
| 29 | CA3 hippocampus ventral | 0.0056 | 0.0052 | 0.0115 | 0.0069 | 0.0105 | 0.0050 | 0 | 0.0710 | 1 | 0.0206 | 1 | 0.0091 | 2 |
| 30 | central amygdaloid nucleus | 0.0054 | 0.0024 | 0.0055 | 0.0030 | 0.0063 | 0.0041 | 1 | 0.0070 | 1 | 0.0147 | 1 | 0.0259 | 3 |
| 31 | anterior cingulate area | 0.0048 | 0.0023 | 0.0012 | 0.0019 | 0.0055 | 0.0030 | 1 | 0.0100 | 0 | 0.2231 | 1 | 0.0143 | 2 |
| 32 | central gray | 0.0058 | 0.0049 | 0.0008 | 0.0029 | 0.0058 | 0.0068 | 0 | 0.0563 | 0 | 0.5659 | 0 | 0.1301 | 0 |
| 33 | claustrum | 0.0077 | 0.0038 | 0.0025 | 0.0021 | 0.0096 | 0.0031 | 1 | 0.0109 | 0 | 0.0514 | 1 | 0.0024 | 2 |
| 34 | central medial thalamic nucleus | 0.0066 | 0.0044 | 0.0041 | 0.0049 | 0.0068 | 0.0040 | 1 | 0.0291 | 0 | 0.1361 | 1 | 0.0182 | 2 |
| 35 | cortical amygdaloid nucleus | 0.0056 | 0.0065 | 0.0035 | 0.0040 | 0.0068 | 0.0070 | 0 | 0.1235 | 0 | 0.1255 | 0 | 0.0955 | 0 |
| 36 | copula of the pyramis | 0.0041 | 0.0048 | 0.0059 | 0.0042 | 0.0063 | 0.0074 | 0 | 0.1270 | 1 | 0.0346 | 0 | 0.1298 | 1 |
| 37 | crus 1 of ansiform lobule | 0.0038 | 0.0038 | 0.0022 | 0.0008 | 0.0045 | 0.0036 | 0 | 0.0887 | 1 | 0.0026 | 1 | 0.0497 | 2 |
| 38 | crus 2 of ansiform lobule | 0.0025 | 0.0032 | 0.0017 | 0.0011 | 0.0030 | 0.0035 | 0 | 0.1553 | 1 | 0.0263 | 0 | 0.1231 | 1 |
| 39 | diagonal band of Broca | 0.0042 | 0.0034 | 0.0024 | 0.0029 | 0.0045 | 0.0025 | 0 | 0.0509 | 0 | 0.1376 | 1 | 0.0151 | 1 |
| 40 | dentate gyrus dorsal | 0.0093 | 0.0059 | 0.0074 | 0.0040 | 0.0112 | 0.0054 | 1 | 0.0244 | 1 | 0.0145 | 1 | 0.0096 | 3 |
| 41 | dentate gyrus ventral | 0.0071 | 0.0040 | 0.0060 | 0.0043 | 0.0085 | 0.0034 | 1 | 0.0169 | 1 | 0.0349 | 1 | 0.0050 | 3 |
| 42 | dorsal lateral striatum | 0.0071 | 0.0041 | 0.0030 | 0.0019 | 0.0081 | 0.0042 | 1 | 0.0173 | 1 | 0.0241 | 1 | 0.0122 | 3 |
| 43 | dorsal medial nucleus | 0.0080 | 0.0068 | 0.0092 | 0.0030 | 0.0119 | 0.0070 | 0 | 0.0576 | 1 | 0.0024 | 1 | 0.0190 | 2 |
| 44 | dorsal medial striatum | 0.0053 | 0.0036 | 0.0016 | 0.0019 | 0.0066 | 0.0040 | 1 | 0.0309 | 0 | 0.1436 | 1 | 0.0220 | 2 |
| 45 | dorsomedial tegmental area | 0.0025 | 0.0037 | 0.0009 | 0.0020 | 0.0038 | 0.0021 | 0 | 0.2015 | 0 | 0.3467 | 1 | 0.0141 | 1 |
| 46 | DPGi | 0.0016 | 0.0031 | -0.0008 | 0.0017 | 0.0045 | 0.0035 | 0 | 0.3090 | 0 | 0.3467 | 1 | 0.0433 | 1 |
| 47 | dorsal raphe | 0.0068 | 0.0048 | 0.0005 | 0.0050 | 0.0077 | 0.0059 | 1 | 0.0329 | 0 | 0.8469 | 1 | 0.0437 | 2 |
| 48 | subiculum dorsal | 0.0065 | 0.0022 | 0.0035 | 0.0021 | 0.0097 | 0.0040 | 1 | 0.0028 | 1 | 0.0200 | 1 | 0.0057 | 3 |
| 49 | extended amydala | 0.0087 | 0.0038 | 0.0041 | 0.0032 | 0.0097 | 0.0048 | 1 | 0.0066 | 1 | 0.0454 | 1 | 0.0108 | 3 |
| 50 | ectorhinal ctx | -0.0004 | 0.0053 | -0.0007 | 0.0124 | 0.0045 | 0.0131 | 0 | 0.8646 | 0 | 0.9043 | 0 | 0.4880 | 0 |

*FIG. 16 (Continued)*

| | | | | | | | | | | | | | | |
|---|---|---|---|---|---|---|---|---|---|---|---|---|---|---|
| 51 | endopiriform nucleus | 0.0071 | 0.0064 | 0.0035 | 0.0033 | 0.0089 | 0.0038 | 0 | 0.0683 | 0 | 0.0741 | 1 | 0.0060 | 1 |
| 52 | entorhinal ctx | 0.0034 | 0.0030 | 0.0022 | 0.0021 | 0.0056 | 0.0034 | 0 | 0.0636 | 0 | 0.0761 | 1 | 0.0212 | 1 |
| 53 | external plexiform layer | 0.0039 | 0.0068 | 0.0024 | 0.0012 | 0.0045 | 0.0056 | 0 | 0.2721 | 1 | 0.0106 | 0 | 0.1473 | 1 |
| 54 | flocculus cerebellum | 0.0064 | 0.0047 | 0.0045 | 0.0011 | 0.0084 | 0.0041 | 1 | 0.0382 | 1 | 0.0007 | 1 | 0.0100 | 3 |
| 55 | frontal association ctx | 0.0024 | 0.0047 | -0.0001 | 0.0027 | 0.0030 | 0.0038 | 0 | 0.3065 | 0 | 0.9208 | 0 | 0.1588 | 0 |
| 56 | gigantocellular reticular nucleus | 0.0013 | 0.0033 | 0.0019 | 0.0008 | 0.0022 | 0.0031 | 0 | 0.4103 | 1 | 0.0067 | 0 | 0.1792 | 1 |
| 57 | glomerular layer | -0.0012 | 0.0057 | 0.0003 | 0.0034 | -0.0006 | 0.0060 | 0 | 0.6561 | 0 | 0.8293 | 0 | 0.8457 | 0 |
| 58 | globus pallidus | 0.0080 | 0.0044 | 0.0039 | 0.0018 | 0.0086 | 0.0041 | 1 | 0.0155 | 1 | 0.0073 | 1 | 0.0094 | 3 |
| 59 | granular cell layer | 0.0016 | 0.0047 | 0.0017 | 0.0030 | 0.0028 | 0.0061 | 0 | 0.4827 | 0 | 0.2817 | 0 | 0.3566 | 0 |
| 60 | habenula nucleus | 0.0087 | 0.0068 | 0.0069 | 0.0063 | 0.0160 | 0.0107 | 1 | 0.0458 | 0 | 0.0707 | 1 | 0.0290 | 2 |
| 61 | intercalated amygdaloid nucleus | -0.0050 | 0.0075 | -0.0021 | 0.0048 | -0.0004 | 0.0098 | 0 | 0.2084 | 0 | 0.3814 | 0 | 0.9381 | 0 |
| 62 | inferior colliculus | 0.0065 | 0.0038 | 0.0052 | 0.0022 | 0.0095 | 0.0036 | 1 | 0.0187 | 1 | 0.0065 | 1 | 0.0042 | 3 |
| 63 | infralimbic ctx | 0.0064 | 0.0018 | 0.0036 | 0.0016 | 0.0078 | 0.0026 | 1 | 0.0013 | 1 | 0.0080 | 1 | 0.0025 | 3 |
| 64 | insular ctx | 0.0049 | 0.0034 | 0.0026 | 0.0019 | 0.0068 | 0.0036 | 1 | 0.0332 | 1 | 0.0341 | 1 | 0.0136 | 3 |
| 65 | interposed nucleus | 0.0043 | 0.0020 | 0.0047 | 0.0021 | 0.0055 | 0.0026 | 1 | 0.0086 | 1 | 0.0077 | 1 | 0.0096 | 3 |
| 66 | inferior olivary complex | 0.0021 | 0.0090 | 0.0055 | 0.0035 | 0.0046 | 0.0104 | 0 | 0.6276 | 1 | 0.0247 | 0 | 0.3797 | 1 |
| 67 | interpeduncular nucleus | 0.0025 | 0.0074 | 0.0018 | 0.0065 | 0.0031 | 0.0071 | 0 | 0.4914 | 0 | 0.5677 | 0 | 0.3786 | 0 |
| 68 | lateral amygdaloid nucleus | 0.0053 | 0.0012 | 0.0031 | 0.0023 | 0.0079 | 0.0041 | 1 | 0.0007 | 1 | 0.0420 | 1 | 0.0120 | 3 |
| 69 | Lat | 0.0013 | 0.0020 | 0.0042 | 0.0048 | 0.0060 | 0.0024 | 0 | 0.2208 | 0 | 0.1189 | 1 | 0.0052 | 1 |
| 70 | locus ceruleus | 0.0026 | 0.0033 | 0.0018 | 0.0048 | 0.0016 | 0.0053 | 0 | 0.1621 | 0 | 0.4445 | 0 | 0.5520 | 0 |
| 71 | lateral dorsal thalamic nucleus | 0.0069 | 0.0080 | 0.0055 | 0.0032 | 0.0105 | 0.0079 | 0 | 0.1233 | 1 | 0.0183 | 1 | 0.0413 | 2 |
| 72 | lateral geniculate | 0.0097 | 0.0078 | 0.0119 | 0.0071 | 0.0143 | 0.0083 | 0 | 0.0502 | 1 | 0.0202 | 1 | 0.0183 | 2 |
| 73 | lateral hypothalamus | 0.0037 | 0.0056 | 0.0055 | 0.0030 | 0.0082 | 0.0062 | 0 | 0.2105 | 1 | 0.0149 | 1 | 0.0416 | 2 |
| 74 | lemniscal nucleus | 0.0062 | 0.0030 | 0.0037 | 0.0014 | 0.0084 | 0.0029 | 1 | 0.0098 | 1 | 0.0043 | 1 | 0.0029 | 3 |
| 75 | lateral orbital ctx | 0.0036 | 0.0018 | 0.0026 | 0.0013 | 0.0048 | 0.0027 | 1 | 0.0103 | 1 | 0.0100 | 1 | 0.0172 | 3 |

*FIG. 16 (Continued)*

| | | | | | | | | | | | | | |
|---|---|---|---|---|---|---|---|---|---|---|---|---|---|
| 76 | lateral posterior thalamic nucleus | 0.0120 | 0.0082 | 0.0075 | 0.0051 | 0.0150 | 0.0087 | 1 | 0.0306 | 1 | 0.0304 | 1 | 0.0179 | 3 |
| 77 | lateral preoptic area | 0.0092 | 0.0029 | 0.0020 | 0.0030 | 0.0098 | 0.0013 | 1 | 0.0020 | 0 | 0.2051 | 1 | 0.0001 | 2 |
| 78 | lateral septal nucleus | 0.0087 | 0.0039 | 0.0042 | 0.0026 | 0.0126 | 0.0042 | 1 | 0.0074 | 1 | 0.0215 | 1 | 0.0027 | 3 |
| 79 | primary motor ctx | 0.0050 | 0.0024 | 0.0013 | 0.0012 | 0.0067 | 0.0031 | 1 | 0.0102 | 0 | 0.0664 | 1 | 0.0085 | 2 |
| 80 | secondary motor ctx | 0.0039 | 0.0024 | 0.0012 | 0.0021 | 0.0060 | 0.0043 | 1 | 0.0230 | 0 | 0.2565 | 1 | 0.0361 | 2 |
| 81 | magnocellular preoptic nucleus | 0.0020 | 0.0118 | 0.0033 | 0.0067 | 0.0040 | 0.0115 | 0 | 0.7208 | 0 | 0.3381 | 0 | 0.4831 | 0 |
| 82 | medial dorsal thalamic nucleus | 0.0054 | 0.0053 | 0.0036 | 0.0026 | 0.0060 | 0.0014 | 0 | 0.0840 | 1 | 0.0384 | 1 | 0.0007 | 2 |
| 83 | medial amygdaloid nucleus | 0.0054 | 0.0058 | 0.0084 | 0.0032 | 0.0095 | 0.0055 | 0 | 0.1027 | 1 | 0.0043 | 1 | 0.0186 | 2 |
| 84 | medial cerebellar nucleus fastigia | 0.0035 | 0.0072 | 0.0022 | 0.0058 | 0.0070 | 0.0065 | 0 | 0.3392 | 0 | 0.4482 | 0 | 0.0747 | 0 |
| 85 | medial geniculate | 0.0076 | 0.0042 | 0.0053 | 0.0006 | 0.0086 | 0.0044 | 1 | 0.0154 | 1 | 0.0000 | 1 | 0.0116 | 3 |
| 86 | medial mammillary nucleus | 0.0071 | 0.0095 | 0.0115 | 0.0123 | 0.0166 | 0.0094 | 0 | 0.1665 | 0 | 0.1062 | 1 | 0.0172 | 1 |
| 87 | median raphe nucleus | 0.0068 | 0.0059 | 0.0022 | 0.0024 | 0.0089 | 0.0063 | 0 | 0.0615 | 0 | 0.1069 | 1 | 0.0333 | 1 |
| 88 | medial orbital ctx | 0.0048 | 0.0040 | 0.0026 | 0.0037 | 0.0076 | 0.0037 | 0 | 0.0575 | 0 | 0.1979 | 1 | 0.0102 | 1 |
| 89 | medial preoptic area | 0.0089 | 0.0043 | 0.0053 | 0.0022 | 0.0095 | 0.0017 | 1 | 0.0096 | 1 | 0.0052 | 1 | 0.0002 | 3 |
| 90 | medial pretectal area | 0.0071 | 0.0059 | 0.0069 | 0.0090 | 0.0114 | 0.0073 | 0 | 0.0546 | 0 | 0.1631 | 1 | 0.0251 | 1 |
| 91 | medial septum | 0.0057 | 0.0047 | -0.0002 | 0.0070 | 0.0091 | 0.0055 | 0 | 0.0533 | 0 | 0.9621 | 1 | 0.0206 | 1 |
| 92 | neural lobe pituitary | -0.0059 | 0.0067 | 0.0043 | 0.0050 | 0.0008 | 0.0063 | 0 | 0.1208 | 0 | 0.1239 | 0 | 0.7897 | 0 |
| 93 | olivary nucleus | 0.0034 | 0.0047 | -0.0002 | 0.0045 | 0.0039 | 0.0060 | 0 | 0.1795 | 0 | 0.9410 | 0 | 0.2172 | 0 |
| 94 | paraventricular nuclus | 0.0096 | 0.0068 | 0.0040 | 0.0074 | 0.0102 | 0.0037 | 1 | 0.0341 | 0 | 0.2949 | 1 | 0.0036 | 2 |
| 95 | periaqueductal gray thalamus | 0.0053 | 0.0014 | 0.0027 | 0.0017 | 0.0073 | 0.0021 | 1 | 0.0011 | 1 | 0.0245 | 1 | 0.0014 | 3 |
| 96 | parabrachial nucleus | 0.0016 | 0.0026 | 0.0001 | 0.0017 | 0.0023 | 0.0041 | 0 | 0.2429 | 0 | 0.9227 | 0 | 0.2901 | 0 |
| 97 | PCRt | 0.0003 | 0.0022 | 0.0018 | 0.0011 | 0.0030 | 0.0016 | 0 | 0.7576 | 1 | 0.0197 | 1 | 0.0132 | 2 |
| 98 | parafascicular thalamic nucleus | 0.0059 | 0.0020 | 0.0017 | 0.0026 | 0.0071 | 0.0020 | 1 | 0.0029 | 0 | 0.2070 | 1 | 0.0014 | 2 |
| 99 | paraflocculus cerebellum | 0.0044 | 0.0033 | 0.0041 | 0.0013 | 0.0069 | 0.0035 | 1 | 0.0411 | 1 | 0.0020 | 1 | 0.0114 | 3 |
| 100 | posterior hypothalamic area | 0.0015 | 0.0095 | 0.0029 | 0.0016 | 0.0038 | 0.0074 | 0 | 0.7426 | 1 | 0.0175 | 0 | 0.3112 | 1 |

*FIG. 16 (Continued)*

| | | | | | | | | | | | | | |
|---|---|---|---|---|---|---|---|---|---|---|---|---|---|
| 101 | pineal gland | 0.0103 | 0.0222 | 0.0048 | 0.0054 | 0.0135 | 0.0311 | 0 | 0.3555 | 0 | 0.1171 | 0 | 0.3865 | 0 |
| 102 | caudal piriform ctx | 0.0045 | 0.0042 | 0.0015 | 0.0023 | 0.0056 | 0.0049 | 0 | 0.0746 | 0 | 0.2047 | 0 | 0.0624 | 0 |
| 103 | rostral piriform ctx | 0.0046 | 0.0039 | 0.0036 | 0.0025 | 0.0063 | 0.0049 | 0 | 0.0603 | 1 | 0.0337 | 1 | 0.0454 | 2 |
| 104 | premammillary nucleus | 0.0069 | 0.0102 | 0.0067 | 0.0148 | 0.0149 | 0.0118 | 0 | 0.2029 | 0 | 0.3655 | 1 | 0.0481 | 1 |
| 105 | paramedian lobule | 0.0023 | 0.0042 | 0.0030 | 0.0016 | 0.0046 | 0.0039 | 0 | 0.2824 | 1 | 0.0156 | 0 | 0.0573 | 1 |
| 106 | pontine nuclei | 0.0021 | 0.0019 | -0.0003 | 0.0017 | 0.0042 | 0.0016 | 0 | 0.0706 | 0 | 0.7527 | 1 | 0.0043 | 1 |
| 107 | pontine reticular nucleus caudal | 0.0026 | 0.0015 | 0.0000 | 0.0006 | 0.0039 | 0.0029 | 1 | 0.0182 | 0 | 0.9362 | 1 | 0.0402 | 2 |
| 108 | pontine reticular nucleus oral | 0.0066 | 0.0037 | 0.0022 | 0.0033 | 0.0077 | 0.0039 | 1 | 0.0170 | 0 | 0.2028 | 1 | 0.0121 | 2 |
| 109 | posterior thalamic nucleus | 0.0079 | 0.0045 | 0.0040 | 0.0021 | 0.0100 | 0.0049 | 1 | 0.0165 | 1 | 0.0134 | 1 | 0.0104 | 3 |
| 110 | periolivary nucleus | 0.0039 | 0.0059 | 0.0021 | 0.0018 | 0.0057 | 0.0064 | 0 | 0.2159 | 0 | 0.0559 | 0 | 0.1139 | 0 |
| 111 | prerubral field | 0.0037 | 0.0024 | 0.0029 | 0.0032 | 0.0060 | 0.0042 | 1 | 0.0248 | 0 | 0.1123 | 1 | 0.0339 | 2 |
| 112 | principal sensory nucleus trigemin | 0.0008 | 0.0033 | 0.0011 | 0.0016 | 0.0028 | 0.0030 | 0 | 0.6019 | 0 | 0.1840 | 0 | 0.1029 | 0 |
| 113 | precuniform nucleus | 0.0067 | 0.0061 | 0.0015 | 0.0019 | 0.0065 | 0.0069 | 0 | 0.0715 | 0 | 0.1445 | 0 | 0.1056 | 0 |
| 114 | perirhinal ctx | 0.0013 | 0.0035 | 0.0016 | 0.0023 | 0.0027 | 0.0039 | 0 | 0.4474 | 0 | 0.1900 | 0 | 0.2038 | 0 |
| 115 | prelimbic ctx | 0.0050 | 0.0037 | 0.0016 | 0.0020 | 0.0056 | 0.0035 | 1 | 0.0388 | 0 | 0.1541 | 1 | 0.0236 | 2 |
| 116 | parietal ctx | 0.0060 | 0.0027 | 0.0013 | 0.0014 | 0.0068 | 0.0030 | 1 | 0.0082 | 0 | 0.1179 | 1 | 0.0069 | 2 |
| 117 | pedunculopontine tegmental area | 0.0010 | 0.0049 | 0.0009 | 0.0022 | 0.0063 | 0.0046 | 0 | 0.6833 | 0 | 0.4345 | 1 | 0.0374 | 1 |
| 118 | paraventricular nucleus | 0.0076 | 0.0038 | 0.0038 | 0.0063 | 0.0105 | 0.0035 | 1 | 0.0114 | 0 | 0.2503 | 1 | 0.0027 | 2 |
| 119 | retrochiasmatic nucleus | 0.0082 | 0.0093 | 0.0126 | 0.0062 | 0.0148 | 0.0126 | 0 | 0.1177 | 1 | 0.0103 | 0 | 0.0579 | 1 |
| 120 | reuniens nucleus | 0.0097 | 0.0030 | 0.0070 | 0.0028 | 0.0114 | 0.0025 | 1 | 0.0019 | 1 | 0.0049 | 1 | 0.0005 | 3 |
| 121 | raphe linear | 0.0020 | 0.0031 | 0.0015 | 0.0027 | 0.0040 | 0.0025 | 0 | 0.2321 | 0 | 0.2742 | 1 | 0.0225 | 1 |
| 122 | raphe magnus | 0.0068 | 0.0045 | -0.0001 | 0.0025 | 0.0076 | 0.0058 | 1 | 0.0283 | 0 | 0.9383 | 1 | 0.0431 | 2 |
| 123 | raphe obscurus nucleus | -0.0006 | 0.0102 | -0.0010 | 0.0011 | -0.0016 | 0.0103 | 0 | 0.8978 | 0 | 0.0941 | 0 | 0.7521 | 0 |
| 124 | red nucleus | 0.0046 | 0.0029 | 0.0009 | 0.0060 | 0.0056 | 0.0039 | 1 | 0.0252 | 0 | 0.7431 | 1 | 0.0310 | 2 |
| 125 | retrosplenial caudal ctx | 0.0048 | 0.0048 | 0.0062 | 0.0126 | 0.0122 | 0.0143 | 0 | 0.0876 | 0 | 0.3323 | 0 | 0.1291 | 0 |

*FIG. 16 (Continued)*

| 126 | retrosplenial rostral ctx | 0.0033 | 0.0021 | 0.0016 | 0.0051 | 0.0072 | 0.0041 | 1 | 0.0234 | 0 | 0.5284 | 1 | 0.0173 | 2 |
|-----|---------------------------|--------|--------|--------|--------|--------|--------|---|--------|---|--------|---|--------|---|
| 127 | reticular nucleus | 0.0093 | 0.0049 | 0.0042 | 0.0032 | 0.0104 | 0.0036 | 1 | 0.0137 | 1 | 0.0405 | 1 | 0.0030 | 3 |
| 128 | reticular nucleus midbrain | 0.0046 | 0.0018 | 0.0011 | 0.0009 | 0.0066 | 0.0018 | 1 | 0.0052 | 1 | 0.0427 | 1 | 0.0011 | 3 |
| 129 | reticulotegmental nucleus | -0.0009 | 0.0047 | -0.0003 | 0.0029 | 0.0020 | 0.0050 | 0 | 0.6770 | 0 | 0.8119 | 0 | 0.4286 | 0 |
| 130 | primary somatosensory ctx barrel f | 0.0046 | 0.0023 | 0.0011 | 0.0008 | 0.0048 | 0.0022 | 1 | 0.0104 | 1 | 0.0364 | 1 | 0.0080 | 3 |
| 131 | primary somatosensory ctx forelimb | 0.0054 | 0.0033 | 0.0015 | 0.0012 | 0.0062 | 0.0036 | 1 | 0.0217 | 0 | 0.0516 | 1 | 0.0189 | 2 |
| 132 | primary somatosensory ctx hindlimb | 0.0048 | 0.0022 | 0.0003 | 0.0021 | 0.0054 | 0.0035 | 1 | 0.0078 | 0 | 0.7794 | 1 | 0.0253 | 2 |
| 133 | primary somatosensory ctx jaw | 0.0055 | 0.0019 | 0.0025 | 0.0011 | 0.0072 | 0.0018 | 1 | 0.0031 | 1 | 0.0068 | 1 | 0.0009 | 3 |
| 134 | primary somatosensory ctx shoulder | 0.0032 | 0.0045 | 0.0008 | 0.0010 | 0.0035 | 0.0039 | 0 | 0.1855 | 0 | 0.1391 | 0 | 0.1149 | 0 |
| 135 | primary somatosensory ctx trunk | 0.0047 | 0.0021 | 0.0007 | 0.0015 | 0.0051 | 0.0027 | 1 | 0.0080 | 0 | 0.3882 | 1 | 0.0134 | 2 |
| 136 | primary somatosensory ctx upper li | 0.0038 | 0.0032 | 0.0018 | 0.0008 | 0.0058 | 0.0028 | 0 | 0.0587 | 1 | 0.0082 | 1 | 0.0100 | 2 |
| 137 | secondary somaotsensory ctx | 0.0031 | 0.0023 | 0.0009 | 0.0010 | 0.0047 | 0.0019 | 1 | 0.0399 | 0 | 0.1418 | 1 | 0.0056 | 2 |
| 138 | suprachiasmatic nucleus | 0.0037 | 0.0140 | 0.0071 | 0.0058 | 0.0099 | 0.0079 | 0 | 0.5868 | 0 | 0.0525 | 1 | 0.0486 | 1 |
| 139 | substantia innominata | 0.0066 | 0.0087 | 0.0027 | 0.0045 | 0.0076 | 0.0092 | 0 | 0.1624 | 0 | 0.2511 | 0 | 0.1377 | 0 |
| 140 | simple lobule cerebellum | 0.0045 | 0.0042 | 0.0019 | 0.0018 | 0.0063 | 0.0045 | 0 | 0.0725 | 0 | 0.0702 | 1 | 0.0345 | 1 |
| 141 | substantia nigra compacta | 0.0037 | 0.0061 | 0.0021 | 0.0039 | 0.0038 | 0.0044 | 0 | 0.2456 | 0 | 0.3030 | 0 | 0.1272 | 0 |
| 142 | substantia nigra reticularis | 0.0028 | 0.0023 | 0.0023 | 0.0018 | 0.0050 | 0.0023 | 0 | 0.0561 | 1 | 0.0432 | 1 | 0.0089 | 2 |
| 143 | supraoptic nucleus | 0.0067 | 0.0059 | 0.0018 | 0.0062 | 0.0094 | 0.0119 | 0 | 0.0655 | 0 | 0.5584 | 0 | 0.1508 | 0 |
| 144 | solitary tract nucleus | 0.0023 | 0.0009 | 0.0018 | 0.0050 | 0.0047 | 0.0011 | 1 | 0.0054 | 0 | 0.4758 | 1 | 0.0006 | 2 |
| 145 | bed nucleus stria terminalis | 0.0095 | 0.0026 | 0.0038 | 0.0023 | 0.0110 | 0.0025 | 1 | 0.0013 | 1 | 0.0218 | 1 | 0.0006 | 3 |
| 146 | subthalamic nucleus | 0.0058 | 0.0052 | 0.0006 | 0.0063 | 0.0053 | 0.0051 | 0 | 0.0668 | 0 | 0.8479 | 0 | 0.0816 | 0 |
| 147 | superior colliculus | 0.0047 | 0.0047 | 0.0039 | 0.0062 | 0.0085 | 0.0084 | 0 | 0.0891 | 0 | 0.2341 | 0 | 0.0859 | 0 |
| 148 | sub coeruleus nucleus | 0.0054 | 0.0025 | 0.0009 | 0.0012 | 0.0049 | 0.0029 | 1 | 0.0090 | 0 | 0.1733 | 1 | 0.0189 | 2 |
| 149 | supramammillary nucleus | 0.0001 | 0.0055 | 0.0097 | 0.0076 | 0.0070 | 0.0115 | 0 | 0.9656 | 1 | 0.0468 | 0 | 0.2457 | 1 |
| 150 | temporal ctx | -0.0006 | 0.0060 | 0.0033 | 0.0059 | 0.0031 | 0.0088 | 0 | 0.8311 | 0 | 0.2848 | 0 | 0.4684 | 0 |

**FIG. 16 (Continued)**

| | | | | | | | | | | | | | |
|---|---|---|---|---|---|---|---|---|---|---|---|---|---|
| 151 | triangular septal nucleus | 0.0060 | 0.0031 | 0.0047 | 0.0017 | 0.0105 | 0.0019 | 1 | 0.0115 | 1 | 0.0034 | 1 | 0.0003 | 3 |
| 152 | tenia tecta ctx | -0.0024 | 0.0061 | 0.0041 | 0.0013 | 0.0006 | 0.0053 | 0 | 0.4343 | 1 | 0.0024 | 0 | 0.8181 | 1 |
| 153 | olfactory tubercles | 0.0041 | 0.0062 | 0.0086 | 0.0031 | 0.0074 | 0.0080 | 0 | 0.2155 | 1 | 0.0033 | 0 | 0.1088 | 1 |
| 154 | trapezoid body | 0.0039 | 0.0033 | 0.0019 | 0.0046 | 0.0031 | 0.0025 | 0 | 0.0573 | 0 | 0.4117 | 1 | 0.0487 | 1 |
| 155 | Ventricle | 0.0089 | 0.0061 | 0.0055 | 0.0060 | 0.0121 | 0.0066 | 1 | 0.0310 | 0 | 0.1079 | 1 | 0.0147 | 2 |
| 156 | visual 1 ctx | 0.0018 | 0.0044 | 0.0020 | 0.0037 | 0.0044 | 0.0062 | 0 | 0.4086 | 0 | 0.2938 | 0 | 0.1844 | 0 |
| 157 | visual 2 ctx | 0.0042 | 0.0025 | 0.0030 | 0.0045 | 0.0067 | 0.0053 | 1 | 0.0203 | 0 | 0.2017 | 1 | 0.0469 | 2 |
| 158 | ventral anterior thalamic nucleus | 0.0080 | 0.0050 | 0.0039 | 0.0023 | 0.0090 | 0.0025 | 1 | 0.0231 | 1 | 0.0183 | 1 | 0.0012 | 3 |
| 159 | cochlear nucleus | 0.0029 | 0.0043 | 0.0025 | 0.0030 | 0.0057 | 0.0039 | 0 | 0.2094 | 0 | 0.1443 | 1 | 0.0314 | 1 |
| 160 | vestibular nucleus | 0.0037 | 0.0032 | 0.0034 | 0.0021 | 0.0060 | 0.0030 | 0 | 0.0646 | 1 | 0.0204 | 1 | 0.0110 | 2 |
| 161 | ventrolateral thalamic nucleus | 0.0055 | 0.0019 | 0.0036 | 0.0018 | 0.0066 | 0.0028 | 1 | 0.0032 | 1 | 0.0112 | 1 | 0.0059 | 3 |
| 162 | ventral lateral striatum | 0.0066 | 0.0025 | 0.0031 | 0.0015 | 0.0073 | 0.0026 | 1 | 0.0040 | 1 | 0.0106 | 1 | 0.0032 | 3 |
| 163 | ventromedial thalamic nucleus | 0.0060 | 0.0041 | 0.0054 | 0.0033 | 0.0088 | 0.0051 | 1 | 0.0296 | 1 | 0.0216 | 1 | 0.0184 | 3 |
| 164 | ventral medial nucleus | 0.0086 | 0.0061 | 0.0059 | 0.0058 | 0.0129 | 0.0067 | 1 | 0.0343 | 0 | 0.0835 | 1 | 0.0126 | 2 |
| 165 | ventral medial striatum | 0.0068 | 0.0042 | 0.0019 | 0.0022 | 0.0074 | 0.0033 | 1 | 0.0229 | 0 | 0.1285 | 1 | 0.0071 | 2 |
| 166 | ventral orbital ctx | 0.0045 | 0.0029 | 0.0024 | 0.0014 | 0.0069 | 0.0038 | 1 | 0.0262 | 1 | 0.0171 | 1 | 0.0155 | 3 |
| 167 | ventral pallidum | 0.0066 | 0.0044 | 0.0049 | 0.0026 | 0.0078 | 0.0029 | 1 | 0.0288 | 1 | 0.0141 | 1 | 0.0036 | 3 |
| 168 | ventral posteriolateral thalamic n | 0.0077 | 0.0048 | 0.0043 | 0.0042 | 0.0089 | 0.0055 | 1 | 0.0230 | 0 | 0.0820 | 1 | 0.0219 | 2 |
| 169 | ventral posteriolmedial thalamic n | 0.0067 | 0.0043 | 0.0047 | 0.0021 | 0.0094 | 0.0062 | 1 | 0.0254 | 1 | 0.0078 | 1 | 0.0265 | 3 |
| 170 | ventral subiculum | 0.0044 | 0.0017 | 0.0028 | 0.0013 | 0.0069 | 0.0029 | 1 | 0.0045 | 1 | 0.0080 | 1 | 0.0058 | 3 |
| 171 | ventral tegmental area | 0.0016 | 0.0052 | 0.0030 | 0.0031 | 0.0034 | 0.0025 | 0 | 0.5293 | 0 | 0.1034 | 1 | 0.0413 | 1 |
| 172 | White Matter | 0.0050 | 0.0020 | 0.0034 | 0.0009 | 0.0069 | 0.0018 | 1 | 0.0049 | 1 | 0.0010 | 1 | 0.0011 | 3 |
| 173 | White Matter | 0.0050 | 0.0020 | 0.0057 | 0.0028 | 0.0085 | 0.0030 | 1 | 0.0052 | 1 | 0.0113 | 1 | 0.0034 | 3 |
| 174 | zona incerta | 0.0043 | 0.0034 | 0.0046 | 0.0031 | 0.0068 | 0.0022 | 1 | 0.0471 | 1 | 0.0299 | 1 | 0.0024 | 3 |
| - | whole brain | 0.0045 | 0.0026 | 0.0028 | 0.0010 | 0.0064 | 0.0024 | 1 | 0.0175 | 1 | 0.0041 | 1 | 0.0042 | 3 |
| | | | | | | | | | 88 | | 82 | | 125 | |

*FIG. 16 (Continued)*

| Region Num | Region Nam | M1 | std | M2 | std | M3 | std | h1 | p1 | h2 | p2 | h3 | p3 | |
|---|---|---|---|---|---|---|---|---|---|---|---|---|---|---|
| 1 | 10th cerebellar lobule | 0.0065 | 0.0205 | 0.0066 | 0.0083 | 0.0090 | 0.0160 | 0 | 0.5165 | 0 | 0.1534 | 0 | 0.2771 | 0 |
| 2 | 1st cerebellar lobule | 0.0015 | 0.0105 | -0.0147 | 0.0314 | 0.0001 | 0.0184 | 0 | 0.7661 | 0 | 0.3552 | 0 | 0.9933 | 0 |
| 3 | 2nd cerebellar lobule | -0.0033 | 0.0180 | -0.0077 | 0.0253 | -0.0051 | 0.0186 | 0 | 0.7029 | 0 | 0.5331 | 0 | 0.5730 | 0 |
| 4 | 3rd cerebellar lobule | 0.0048 | 0.0038 | -0.0017 | 0.0047 | 0.0057 | 0.0130 | 1 | 0.0481 | 0 | 0.4684 | 0 | 0.3871 | 1 |
| 5 | 4th cerebellar lobule | -0.0067 | 0.0231 | 0.0089 | 0.0151 | 0.0070 | 0.0122 | 0 | 0.5509 | 0 | 0.2607 | 0 | 0.2691 | 0 |
| 6 | 5th cerebellar lobule | 0.0013 | 0.0026 | 0.0024 | 0.0038 | 0.0045 | 0.0031 | 0 | 0.3173 | 0 | 0.2329 | 1 | 0.0311 | 1 |
| 7 | motor trigeminal nucleus | 0.0099 | 0.0132 | 0.0040 | 0.0095 | 0.0158 | 0.0208 | 0 | 0.1663 | 0 | 0.3963 | 0 | 0.1643 | 0 |
| 8 | root of trigeminal nerve | 0.0117 | 0.0092 | -0.0017 | 0.0088 | 0.0078 | 0.0062 | 1 | 0.0460 | 0 | 0.6843 | 1 | 0.0475 | 2 |
| 9 | 6th cerebellar lobule | -0.0085 | 0.0212 | 0.0078 | 0.0054 | 0.0035 | 0.0025 | 0 | 0.4221 | 1 | 0.0311 | 1 | 0.0347 | 2 |
| 10 | 7th cerebellar lobule | 0.0001 | 0.0120 | 0.0009 | 0.0163 | 0.0100 | 0.0115 | 0 | 0.9922 | 0 | 0.9107 | 0 | 0.1249 | 0 |
| 11 | facial nucleus | 0.0062 | 0.0096 | 0.0080 | 0.0141 | 0.0060 | 0.0153 | 0 | 0.2208 | 0 | 0.2713 | 0 | 0.4319 | 0 |
| 12 | 8th cerebellar lobule | 0.0042 | 0.0171 | -0.0166 | 0.0270 | 0.0074 | 0.0103 | 0 | 0.6086 | 0 | 0.2404 | 0 | 0.1867 | 0 |
| 13 | 9th cerebellar lobule | -0.0160 | 0.0201 | -0.0069 | 0.0167 | -0.0046 | 0.0166 | 0 | 0.1499 | 0 | 0.4076 | 0 | 0.5727 | 0 |
| 14 | anterior thalamic nuclei | -0.0024 | 0.0231 | -0.0019 | 0.0045 | 0.0036 | 0.0065 | 0 | 0.8246 | 0 | 0.4034 | 0 | 0.2832 | 0 |
| 15 | anterior amygdaloid nucleus | 0.0157 | 0.0075 | 0.0099 | 0.0145 | 0.0069 | 0.0161 | 1 | 0.0094 | 0 | 0.2022 | 0 | 0.3903 | 1 |
| 16 | accumbens core | 0.0079 | 0.0039 | 0.0132 | 0.0226 | 0.0108 | 0.0081 | 1 | 0.0108 | 0 | 0.2625 | 1 | 0.0400 | 2 |
| 17 | accumbens shell | 0.0147 | 0.0342 | 0.0120 | 0.0180 | 0.0183 | 0.0281 | 0 | 0.3899 | 0 | 0.2107 | 0 | 0.2188 | 0 |
| 18 | anterior hypothalamic area | -0.0035 | 0.0321 | 0.0047 | 0.0191 | 0.0057 | 0.0069 | 0 | 0.8215 | 0 | 0.6123 | 0 | 0.1376 | 0 |
| 19 | anterior lobe pituitary | 0.0323 | 0.0309 | 0.0116 | 0.0505 | 0.0341 | 0.0395 | 0 | 0.0802 | 0 | 0.6348 | 0 | 0.1260 | 0 |
| 20 | anterior olfactory nucleus | 0.0033 | 0.0090 | 0.0029 | 0.0055 | 0.0061 | 0.0054 | 0 | 0.4558 | 0 | 0.2987 | 0 | 0.0647 | 0 |
| 21 | anterior pretectal nucleus | 0.0041 | 0.0290 | -0.0075 | 0.0249 | -0.0026 | 0.0221 | 0 | 0.7702 | 0 | 0.5359 | 0 | 0.8024 | 0 |
| 22 | arcuate nucleus | 0.0136 | 0.0815 | 0.0363 | 0.0735 | 0.0110 | 0.0222 | 0 | 0.7275 | 0 | 0.3314 | 0 | 0.3324 | 0 |
| 23 | auditory ctx | 0.0044 | 0.0058 | 0.0012 | 0.0062 | 0.0010 | 0.0072 | 0 | 0.1686 | 0 | 0.6796 | 0 | 0.7608 | 0 |
| 24 | basal amygdaloid nucleus | 0.0023 | 0.0113 | 0.0022 | 0.0066 | 0.0058 | 0.0109 | 0 | 0.6776 | 0 | 0.4913 | 0 | 0.2985 | 0 |
| 25 | CA1 dorsal | 0.0031 | 0.0041 | 0.0019 | 0.0047 | 0.0063 | 0.0016 | 0 | 0.1636 | 0 | 0.4163 | 1 | 0.0009 | 1 |

*FIG. 17*

| # | Region | | | | | | | | | | | | | |
|---|---|---|---|---|---|---|---|---|---|---|---|---|---|---|
| 26 | CA1 hippocampus ventral | -0.0029 | 0.0112 | -0.0006 | 0.0110 | -0.0006 | 0.0107 | 0 | 0.5935 | 0 | 0.9033 | 0 | 0.9075 | 0 |
| 27 | CA2 | 0.0121 | 0.0086 | 0.0097 | 0.0167 | 0.0145 | 0.0222 | 1 | 0.0346 | 0 | 0.2637 | 0 | 0.2169 | 1 |
| 28 | CA3 dorsal | 0.0021 | 0.0026 | 0.0008 | 0.0058 | -0.0016 | 0.0087 | 0 | 0.1381 | 0 | 0.7696 | 0 | 0.6949 | 0 |
| 29 | CA3 hippocampus ventral | 0.0019 | 0.0087 | 0.0084 | 0.0118 | 0.0147 | 0.0154 | 0 | 0.6589 | 0 | 0.1845 | 0 | 0.0994 | 0 |
| 30 | central amygdaloid nucleus | 0.0006 | 0.0202 | -0.0053 | 0.0272 | 0.0003 | 0.0298 | 0 | 0.9471 | 0 | 0.6851 | 0 | 0.9836 | 0 |
| 31 | anterior cingulate area | -0.0018 | 0.0095 | -0.0079 | 0.0092 | 0.0028 | 0.0081 | 0 | 0.6962 | 0 | 0.1266 | 0 | 0.4798 | 0 |
| 32 | central gray | 0.0017 | 0.0099 | -0.0005 | 0.0137 | 0.0003 | 0.0138 | 0 | 0.7266 | 0 | 0.9363 | 0 | 0.9607 | 0 |
| 33 | claustrum | 0.0021 | 0.0162 | 0.0031 | 0.0089 | 0.0017 | 0.0153 | 0 | 0.7840 | 0 | 0.4799 | 0 | 0.8125 | 0 |
| 34 | central medial thalamic nucleus | 0.0024 | 0.0224 | -0.0103 | 0.0181 | 0.0101 | 0.0223 | 0 | 0.8250 | 0 | 0.2705 | 0 | 0.3692 | 0 |
| 35 | cortical amygdaloid nucleus | 0.0162 | 0.0231 | -0.0008 | 0.0127 | 0.0067 | 0.0169 | 0 | 0.1923 | 0 | 0.8937 | 0 | 0.4261 | 0 |
| 36 | copula of the pyramis | 0.0052 | 0.0237 | -0.0089 | 0.0188 | 0.0040 | 0.0088 | 0 | 0.6469 | 0 | 0.3487 | 0 | 0.3604 | 0 |
| 37 | crus 1 of ansiform lobule | 0.0008 | 0.0062 | -0.0014 | 0.0045 | 0.0035 | 0.0085 | 0 | 0.7978 | 0 | 0.5175 | 0 | 0.4016 | 0 |
| 38 | crus 2 of ansiform lobule | 0.0007 | 0.0093 | 0.0044 | 0.0104 | 0.0048 | 0.0076 | 0 | 0.8752 | 0 | 0.3984 | 0 | 0.2316 | 0 |
| 39 | diagonal band of Broca | 0.0148 | 0.0329 | -0.0028 | 0.0433 | 0.0093 | 0.0125 | 0 | 0.3708 | 0 | 0.8937 | 0 | 0.1693 | 0 |
| 40 | dentate gyrus dorsal | -0.0044 | 0.0192 | -0.0029 | 0.0226 | 0.0087 | 0.0191 | 0 | 0.6324 | 0 | 0.7887 | 0 | 0.3652 | 0 |
| 41 | dentate gyrus ventral | 0.0096 | 0.0593 | -0.0007 | 0.0428 | 0.0057 | 0.0431 | 0 | 0.7344 | 0 | 0.9710 | 0 | 0.7841 | 0 |
| 42 | dorsal lateral striatum | 0.0026 | 0.0105 | -0.0004 | 0.0034 | -0.0002 | 0.0051 | 0 | 0.6122 | 0 | 0.7909 | 0 | 0.9301 | 0 |
| 43 | dorsal medial nucleus | 0.0251 | 0.0287 | 0.0384 | 0.0431 | 0.0217 | 0.0411 | 0 | 0.1219 | 0 | 0.1175 | 0 | 0.3024 | 0 |
| 44 | dorsal medial striatum | 0.0038 | 0.0081 | 0.0015 | 0.0043 | 0.0046 | 0.0074 | 0 | 0.3510 | 0 | 0.4886 | 0 | 0.2354 | 0 |
| 45 | dorsomedial tegmental area | -0.0002 | 0.0160 | -0.0181 | 0.0210 | 0.0144 | 0.0128 | 0 | 0.9790 | 0 | 0.1262 | 0 | 0.0659 | 0 |
| 46 | DPGi | -0.0077 | 0.0287 | -0.0117 | 0.0269 | -0.0021 | 0.0078 | 0 | 0.5818 | 0 | 0.3845 | 0 | 0.5761 | 0 |
| 47 | dorsal raphe | 0.0155 | 0.0158 | -0.0012 | 0.0269 | 0.0251 | 0.0151 | 0 | 0.0932 | 0 | 0.9256 | 1 | 0.0207 | 1 |
| 48 | subiculum dorsal | 0.0024 | 0.0211 | 0.0115 | 0.0261 | 0.0097 | 0.0202 | 0 | 0.8101 | 0 | 0.3815 | 0 | 0.3442 | 0 |
| 49 | extended amydala | 0.0178 | 0.0136 | 0.0072 | 0.0139 | 0.0032 | 0.0141 | 1 | 0.0422 | 0 | 0.3133 | 0 | 0.6348 | 1 |
| 50 | ectorhinal ctx | 0.0591 | 0.1297 | -0.0055 | 0.0413 | -0.0128 | 0.0720 | 0 | 0.3662 | 0 | 0.7812 | 0 | 0.7123 | 0 |

*FIG. 17 (Continued)*

| | | | | | | | | | | | | | |
|---|---|---|---|---|---|---|---|---|---|---|---|---|---|
| 51 | endopiriform nucleus | 0.0072 | 0.0117 | -0.0043 | 0.0083 | 0.0147 | 0.0132 | 0 | 0.2413 | 0 | 0.3120 | 0 | 0.0677 | 0 |
| 52 | entorhinal ctx | -0.0016 | 0.0068 | -0.0024 | 0.0097 | 0.0022 | 0.0101 | 0 | 0.6181 | 0 | 0.6047 | 0 | 0.6508 | 0 |
| 53 | external plexiform layer | 0.0115 | 0.0092 | 0.0081 | 0.0059 | 0.0046 | 0.0095 | 1 | 0.0494 | 1 | 0.0380 | 0 | 0.3432 | 2 |
| 54 | flocculus cerebellum | 0.0132 | 0.0225 | 0.0086 | 0.0218 | 0.0089 | 0.0095 | 0 | 0.2614 | 0 | 0.4293 | 0 | 0.1049 | 0 |
| 55 | frontal association ctx | -0.0018 | 0.0089 | -0.0011 | 0.0070 | -0.0010 | 0.0130 | 0 | 0.6741 | 0 | 0.7335 | 0 | 0.8712 | 0 |
| 56 | gigantocellular reticular nucleus | -0.0017 | 0.0037 | 0.0034 | 0.0067 | 0.0006 | 0.0020 | 0 | 0.3522 | 0 | 0.3145 | 0 | 0.5146 | 0 |
| 57 | glomerular layer | 0.0063 | 0.0071 | -0.0082 | 0.0227 | -0.0080 | 0.0165 | 0 | 0.1187 | 0 | 0.4618 | 0 | 0.3400 | 0 |
| 58 | globus pallidus | 0.0017 | 0.0293 | 0.0036 | 0.0168 | 0.0084 | 0.0143 | 0 | 0.9013 | 0 | 0.6606 | 0 | 0.2600 | 0 |
| 59 | granular cell layer | 0.0051 | 0.0051 | 0.0018 | 0.0040 | 0.0056 | 0.0094 | 0 | 0.0875 | 0 | 0.3716 | 0 | 0.2499 | 0 |
| 60 | habenula nucleus | -0.0065 | 0.0440 | -0.0071 | 0.0488 | 0.0167 | 0.0676 | 0 | 0.7576 | 0 | 0.7603 | 0 | 0.6095 | 0 |
| 61 | intercalated amygdaloid nucleus | 0.0303 | 0.0406 | 0.0379 | 0.0514 | 0.0154 | 0.0615 | 0 | 0.1701 | 0 | 0.1746 | 0 | 0.6059 | 0 |
| 62 | inferior colliculus | 0.0142 | 0.0179 | 0.0065 | 0.0135 | 0.0103 | 0.0105 | 0 | 0.1509 | 0 | 0.3402 | 0 | 0.0938 | 0 |
| 63 | infralimbic ctx | 0.0017 | 0.0118 | -0.0160 | 0.0183 | 0.0059 | 0.0159 | 0 | 0.7672 | 0 | 0.1212 | 0 | 0.4532 | 0 |
| 64 | insular ctx | 0.0055 | 0.0050 | 0.0042 | 0.0073 | 0.0092 | 0.0069 | 0 | 0.0698 | 0 | 0.2687 | 1 | 0.0416 | 1 |
| 65 | interposed nucleus | 0.0025 | 0.0199 | 0.0145 | 0.0243 | -0.0042 | 0.0247 | 0 | 0.7949 | 0 | 0.2536 | 0 | 0.7235 | 0 |
| 66 | inferior olivary complex | 0.0102 | 0.0201 | 0.0073 | 0.0168 | 0.0027 | 0.0296 | 0 | 0.3178 | 0 | 0.3851 | 0 | 0.8505 | 0 |
| 67 | interpeduncular nucleus | 0.0004 | 0.0141 | 0.0146 | 0.0351 | 0.0207 | 0.0296 | 0 | 0.9538 | 0 | 0.4058 | 0 | 0.1932 | 0 |
| 68 | lateral amygdaloid nucleus | -0.0110 | 0.0105 | 0.0093 | 0.0243 | -0.0128 | 0.0327 | 0 | 0.0783 | 0 | 0.4433 | 0 | 0.4320 | 0 |
| 69 | Lat | 0.0196 | 0.0191 | 0.0280 | 0.0326 | 0.0174 | 0.0158 | 0 | 0.0834 | 0 | 0.1271 | 0 | 0.0697 | 0 |
| 70 | locus ceruleus | -0.0006 | 0.0293 | 0.0135 | 0.0238 | 0.0146 | 0.0199 | 0 | 0.9629 | 0 | 0.2730 | 0 | 0.1764 | 0 |
| 71 | lateral dorsal thalamic nucleus | -0.0020 | 0.0330 | -0.0139 | 0.0420 | 0.0230 | 0.0179 | 0 | 0.8981 | 0 | 0.4995 | 1 | 0.0453 | 1 |
| 72 | lateral geniculate | -0.0038 | 0.0236 | -0.0117 | 0.0269 | 0.0066 | 0.0248 | 0 | 0.7357 | 0 | 0.3840 | 0 | 0.5817 | 0 |
| 73 | lateral hypothalamus | 0.0121 | 0.0165 | 0.0091 | 0.0213 | 0.0134 | 0.0164 | 0 | 0.1779 | 0 | 0.3935 | 0 | 0.1423 | 0 |
| 74 | lemniscal nucleus | -0.0076 | 0.0389 | -0.0108 | 0.0363 | 0.0150 | 0.0144 | 0 | 0.6856 | 0 | 0.5430 | 0 | 0.0809 | 0 |
| 75 | lateral orbital ctx | 0.0048 | 0.0050 | 0.0071 | 0.0090 | 0.0093 | 0.0052 | 0 | 0.0966 | 0 | 0.1557 | 1 | 0.0165 | 1 |

*FIG. 17 (Continued)*

EP 4 741 014 A2

| | | | | | | | | | | | | | |
|---|---|---|---|---|---|---|---|---|---|---|---|---|---|
| 76 | lateral posterior thalamic nucleus | 0.0174 | 0.0184 | 0.0222 | 0.0277 | 0.0292 | 0.0427 | 0 | 0.1028 | 0 | 0.1478 | 0 | 0.2013 | 0 |
| 77 | lateral preoptic area | -0.0014 | 0.0319 | -0.0047 | 0.0155 | 0.0003 | 0.0201 | 0 | 0.9242 | 0 | 0.5317 | 0 | 0.9769 | 0 |
| 78 | lateral septal nucleus | 0.0099 | 0.0061 | 0.0047 | 0.0097 | 0.0099 | 0.0067 | 1 | 0.0227 | 0 | 0.3342 | 1 | 0.0294 | 2 |
| 79 | primary motor ctx | 0.0070 | 0.0050 | 0.0029 | 0.0032 | 0.0068 | 0.0042 | 1 | 0.0349 | 0 | 0.1116 | 1 | 0.0218 | 2 |
| 80 | secondary motor ctx | 0.0013 | 0.0051 | 0.0040 | 0.0105 | 0.0034 | 0.0090 | 0 | 0.6042 | 0 | 0.4397 | 0 | 0.4513 | 0 |
| 81 | magnocellular preoptic nucleus | 0.0023 | 0.0277 | 0.0063 | 0.0211 | 0.0021 | 0.0217 | 0 | 0.8621 | 0 | 0.5397 | 0 | 0.8367 | 0 |
| 82 | medial dorsal thalamic nucleus | 0.0038 | 0.0114 | 0.0011 | 0.0082 | 0.0096 | 0.0236 | 0 | 0.5023 | 0 | 0.7772 | 0 | 0.4133 | 0 |
| 83 | medial amygdaloid nucleus | 0.0020 | 0.0348 | 0.0041 | 0.0379 | 0.0044 | 0.0237 | 0 | 0.9037 | 0 | 0.8230 | 0 | 0.7011 | 0 |
| 84 | medial cerebellar nucleus fastigia | 0.0013 | 0.0114 | -0.0091 | 0.0176 | -0.0068 | 0.0242 | 0 | 0.8167 | 0 | 0.3115 | 0 | 0.5618 | 0 |
| 85 | medial geniculate | 0.0129 | 0.0183 | 0.0066 | 0.0235 | 0.0212 | 0.0129 | 0 | 0.1906 | 0 | 0.5613 | 1 | 0.0210 | 1 |
| 86 | medial mammillary nucleus | -0.0104 | 0.0346 | -0.0030 | 0.0310 | 0.0033 | 0.0343 | 0 | 0.5378 | 0 | 0.8404 | 0 | 0.8391 | 0 |
| 87 | median raphe nucleus | 0.0188 | 0.0204 | 0.0102 | 0.0058 | 0.0177 | 0.0205 | 0 | 0.1083 | 1 | 0.0167 | 0 | 0.1254 | 1 |
| 88 | medial orbital ctx | -0.0222 | 0.0307 | 0.0016 | 0.0229 | -0.0005 | 0.0272 | 0 | 0.1818 | 0 | 0.8829 | 0 | 0.9711 | 0 |
| 89 | medial preoptic area | 0.0097 | 0.0182 | 0.0127 | 0.0084 | 0.0106 | 0.0095 | 0 | 0.2995 | 1 | 0.0278 | 0 | 0.0670 | 1 |
| 90 | medial pretectal area | 0.0131 | 0.0596 | 0.0214 | 0.0434 | 0.0040 | 0.0615 | 0 | 0.6485 | 0 | 0.3312 | 0 | 0.8905 | 0 |
| 91 | medial septum | -0.0005 | 0.0165 | 0.0030 | 0.0142 | 0.0029 | 0.0206 | 0 | 0.9453 | 0 | 0.6639 | 0 | 0.7702 | 0 |
| 92 | neural lobe pituitary | 0.0173 | 0.0695 | 0.0286 | 0.0465 | 0.0141 | 0.0652 | 0 | 0.6068 | 0 | 0.2407 | 0 | 0.6544 | 0 |
| 93 | olivary nucleus | -0.0031 | 0.0091 | -0.0050 | 0.0346 | 0.0063 | 0.0148 | 0 | 0.4820 | 0 | 0.7608 | 0 | 0.3924 | 0 |
| 94 | paraventricular nuclus | 0.0200 | 0.0198 | 0.0226 | 0.0224 | 0.0045 | 0.0241 | 0 | 0.0865 | 0 | 0.0868 | 0 | 0.6967 | 0 |
| 95 | periaqueductal gray thalamus | 0.0086 | 0.0112 | 0.0065 | 0.0084 | 0.0093 | 0.0089 | 0 | 0.1593 | 0 | 0.1586 | 0 | 0.0789 | 0 |
| 96 | parabrachial nucleus | -0.0010 | 0.0266 | 0.0045 | 0.0343 | 0.0151 | 0.0461 | 0 | 0.9362 | 0 | 0.7818 | 0 | 0.5054 | 0 |
| 97 | PCRt | 0.0034 | 0.0142 | -0.0023 | 0.0057 | -0.0008 | 0.0015 | 0 | 0.6240 | 0 | 0.4213 | 0 | 0.3371 | 0 |
| 98 | parafascicular thalamic nucleus | 0.0067 | 0.0081 | 0.0034 | 0.0190 | 0.0087 | 0.0129 | 0 | 0.1409 | 0 | 0.7125 | 0 | 0.2032 | 0 |
| 99 | paraflocculus cerebellum | 0.0076 | 0.0059 | 0.0068 | 0.0044 | 0.0065 | 0.0084 | 1 | 0.0446 | 1 | 0.0255 | 0 | 0.1589 | 2 |
| 100 | posterior hypothalamic area | 0.0003 | 0.0318 | 0.0016 | 0.0165 | -0.0106 | 0.0414 | 0 | 0.9856 | 0 | 0.8350 | 0 | 0.5968 | 0 |

*FIG. 17 (Continued)*

| | | | | | | | | | | | | | |
|---|---|---|---|---|---|---|---|---|---|---|---|---|---|
| 101 | pineal gland | 0.0403 | 0.0913 | 0.0148 | 0.0738 | -0.0413 | 0.1593 | 0 | 0.3794 | 0 | 0.6770 | 0 | 0.5935 | 0 |
| 102 | caudal piriform ctx | 0.0045 | 0.0043 | 0.0038 | 0.0070 | 0.0085 | 0.0056 | 0 | 0.0764 | 0 | 0.2957 | 1 | 0.0273 | 1 |
| 103 | rostral piriform ctx | 0.0046 | 0.0047 | 0.0031 | 0.0064 | 0.0042 | 0.0089 | 0 | 0.0920 | 0 | 0.3374 | 0 | 0.3532 | 0 |
| 104 | premammillary nucleus | 0.0152 | 0.0414 | 0.0076 | 0.0525 | 0.0419 | 0.0516 | 0 | 0.4571 | 0 | 0.7615 | 0 | 0.1434 | 0 |
| 105 | paramedian lobule | 0.0086 | 0.0166 | 0.0009 | 0.0049 | 0.0005 | 0.0075 | 0 | 0.3112 | 0 | 0.6983 | 0 | 0.8780 | 0 |
| 106 | pontine nuclei | -0.0027 | 0.0040 | -0.0106 | 0.0177 | 0.0065 | 0.0036 | 0 | 0.2125 | 0 | 0.2513 | 1 | 0.0164 | 1 |
| 107 | pontine reticular nucleus caudal | 0.0027 | 0.0046 | 0.0009 | 0.0042 | 0.0067 | 0.0051 | 0 | 0.2581 | 0 | 0.6565 | 1 | 0.0419 | 1 |
| 108 | pontine reticular nucleus oral | 0.0064 | 0.0144 | 0.0111 | 0.0193 | 0.0068 | 0.0190 | 0 | 0.3805 | 0 | 0.2672 | 0 | 0.4682 | 0 |
| 109 | posterior thalamic nucleus | -0.0012 | 0.0089 | -0.0042 | 0.0084 | 0.0075 | 0.0092 | 0 | 0.7735 | 0 | 0.3301 | 0 | 0.1426 | 0 |
| 110 | periolivary nucleus | 0.0059 | 0.0186 | -0.0073 | 0.0146 | 0.0062 | 0.0133 | 0 | 0.5149 | 0 | 0.3234 | 0 | 0.3591 | 0 |
| 111 | prerubral field | 0.0106 | 0.0138 | 0.0097 | 0.0403 | -0.0056 | 0.0492 | 0 | 0.1597 | 0 | 0.6180 | 0 | 0.8115 | 0 |
| 112 | principal sensory nucleus trigemin | -0.0015 | 0.0150 | 0.0062 | 0.0080 | 0.0073 | 0.0162 | 0 | 0.8296 | 0 | 0.1579 | 0 | 0.3684 | 0 |
| 113 | precuniform nucleus | 0.0116 | 0.0148 | 0.0090 | 0.0242 | 0.0010 | 0.0136 | 0 | 0.1558 | 0 | 0.4504 | 0 | 0.8718 | 0 |
| 114 | perirhinal ctx | -0.0146 | 0.0144 | -0.0151 | 0.0210 | -0.0137 | 0.0231 | 0 | 0.0866 | 0 | 0.1837 | 0 | 0.2550 | 0 |
| 115 | prelimbic ctx | 0.0092 | 0.0135 | 0.0068 | 0.0092 | 0.0161 | 0.0102 | 0 | 0.2006 | 0 | 0.1736 | 1 | 0.0239 | 1 |
| 116 | parietal ctx | 0.0090 | 0.0123 | 0.0054 | 0.0051 | 0.0123 | 0.0074 | 0 | 0.1782 | 0 | 0.0769 | 1 | 0.0203 | 1 |
| 117 | pedunculopontine tegmental area | -0.0052 | 0.0079 | -0.0025 | 0.0135 | 0.0112 | 0.0171 | 0 | 0.2163 | 0 | 0.6956 | 0 | 0.2167 | 0 |
| 118 | paraventricular nucleus | -0.0057 | 0.0306 | -0.0118 | 0.0210 | 0.0294 | 0.0358 | 0 | 0.6959 | 0 | 0.2772 | 0 | 0.1402 | 0 |
| 119 | retrochiasmatic nucleus | 0.0048 | 0.0147 | 0.0171 | 0.0593 | 0.0167 | 0.0294 | 0 | 0.5070 | 0 | 0.5543 | 0 | 0.2729 | 0 |
| 120 | reuniens nucleus | 0.0052 | 0.0073 | -0.0001 | 0.0088 | 0.0142 | 0.0132 | 0 | 0.1823 | 0 | 0.9827 | 0 | 0.0735 | 0 |
| 121 | raphe linear | 0.0082 | 0.0218 | 0.0041 | 0.0220 | 0.0083 | 0.0239 | 0 | 0.4485 | 0 | 0.6970 | 0 | 0.4807 | 0 |
| 122 | raphe magnus | 0.0175 | 0.0185 | -0.0056 | 0.0275 | 0.0241 | 0.0246 | 0 | 0.1014 | 0 | 0.6743 | 0 | 0.0936 | 0 |
| 123 | raphe obscurus nucleus | -0.0035 | 0.0491 | 0.0096 | 0.0118 | -0.0064 | 0.0159 | 0 | 0.8802 | 0 | 0.1412 | 0 | 0.4166 | 0 |
| 124 | red nucleus | 0.0034 | 0.0272 | -0.0083 | 0.0251 | 0.0092 | 0.0416 | 0 | 0.7958 | 0 | 0.5023 | 0 | 0.6475 | 0 |
| 125 | retrosplenial caudal ctx | -0.0022 | 0.0249 | 0.0086 | 0.0432 | -0.0050 | 0.0513 | 0 | 0.8501 | 0 | 0.6801 | 0 | 0.8388 | 0 |

*FIG. 17 (Continued)*

EP 4 741 014 A2

| # | Region | | | | | | | | | | | | |
|---|---|---|---|---|---|---|---|---|---|---|---|---|---|
| 126 | retrosplenial rostral ctx | 0.0015 | 0.0185 | 0.0063 | 0.0162 | 0.0043 | 0.0103 | 0 | 0.8678 | 0 | 0.4376 | 0 | 0.3984 | 0 |
| 127 | reticular nucleus | 0.0118 | 0.0056 | 0.0015 | 0.0043 | 0.0064 | 0.0050 | 1 | 1.0090 | 0 | 0.4691 | 1 | 1.0459 | 2 |
| 128 | reticular nucleus midbrain | 0.0045 | 0.0093 | 0.0014 | 0.0031 | 0.0046 | 0.0071 | 0 | 0.3436 | 0 | 0.3663 | 0 | 0.2175 | 0 |
| 129 | reticulotegmental nucleus | 0.0095 | 0.0207 | 0.0035 | 0.0248 | 0.0056 | 0.0257 | 0 | 0.3647 | 0 | 0.7650 | 0 | 0.6517 | 0 |
| 130 | primary somatosensory ctx barrel f | 0.0047 | 0.0053 | 0.0000 | 0.0037 | 0.0023 | 0.0041 | 0 | 0.1168 | 0 | 0.9972 | 0 | 0.2754 | 0 |
| 131 | primary somatosensory ctx forelimb | 0.0008 | 0.0040 | 0.0116 | 0.0208 | 0.0159 | 0.0256 | 0 | 0.6930 | 0 | 0.2809 | 0 | 0.2364 | 0 |
| 132 | primary somatosensory ctx hindlimb | 0.0007 | 0.0122 | -0.0006 | 0.0062 | 0.0034 | 0.0058 | 0 | 0.9072 | 0 | 0.8278 | 0 | 0.2642 | 0 |
| 133 | primary somatosensory ctx jaw | 0.0098 | 0.0095 | 0.0028 | 0.0033 | 0.0077 | 0.0063 | 0 | 0.0815 | 0 | 0.1253 | 0 | 0.0515 | 0 |
| 134 | primary somatosensory ctx shoulder | 0.0015 | 0.0086 | 0.0062 | 0.0154 | 0.0065 | 0.0094 | 0 | 0.7120 | 0 | 0.4210 | 0 | 0.1962 | 0 |
| 135 | primary somatosensory ctx trunk | -0.0095 | 0.0072 | -0.0142 | 0.0188 | -0.0030 | 0.0069 | 1 | 1.0425 | 0 | 0.1675 | 0 | 0.3859 | 1 |
| 136 | primary somatosensory ctx upper li | 0.0042 | 0.0048 | 0.0010 | 0.0041 | 0.0044 | 0.0049 | 0 | 0.1240 | 0 | 0.6279 | 0 | 0.1125 | 0 |
| 137 | secondary somaotsensory ctx | 0.0091 | 0.0131 | 0.0002 | 0.0049 | 0.0081 | 0.0070 | 0 | 0.1963 | 0 | 0.9252 | 0 | 0.0614 | 0 |
| 138 | suprachiasmatic nucleus | 0.0112 | 0.0212 | 0.0019 | 0.0631 | -0.0007 | 0.0292 | 0 | 0.3039 | 0 | 0.9508 | 0 | 0.9618 | 0 |
| 139 | substantia innominata | 0.0191 | 0.0322 | 0.0004 | 0.0523 | 0.0214 | 0.0375 | 0 | 0.2543 | 0 | 0.9874 | 0 | 0.2704 | 0 |
| 140 | simple lobule cerebellum | 0.0032 | 0.0030 | 0.0026 | 0.0110 | 0.0043 | 0.0030 | 0 | 0.0766 | 0 | 0.6227 | 1 | 0.0331 | 1 |
| 141 | substantia nigra compacta | 0.0146 | 0.0298 | 0.0128 | 0.0461 | 0.0397 | 0.0496 | 0 | 0.3356 | 0 | 0.5686 | 0 | 0.1483 | 0 |
| 142 | substantia nigra reticularis | -0.0049 | 0.0242 | -0.0033 | 0.0049 | 0.0047 | 0.0049 | 0 | 0.6776 | 0 | 0.2057 | 0 | 0.0994 | 0 |
| 143 | supraoptic nucleus | 0.0373 | 0.0557 | 0.0195 | 0.0368 | 0.0123 | 0.0368 | 0 | 0.2086 | 0 | 0.3018 | 0 | 0.4968 | 0 |
| 144 | solitary tract nucleus | 0.0028 | 0.0097 | -0.0011 | 0.0180 | 0.0088 | 0.0082 | 0 | 0.5498 | 0 | 0.9007 | 0 | 0.0728 | 0 |
| 145 | bed nucleus stria terminalis | 0.0065 | 0.0026 | -0.0027 | 0.0157 | 0.0093 | 0.0079 | 1 | 0.0051 | 0 | 0.7156 | 1 | 0.0587 | 1 |
| 146 | subthalamic nucleus | 0.0163 | 0.0471 | 0.0073 | 0.0421 | 0.0130 | 0.0392 | 0 | 0.4818 | 0 | 0.7186 | 0 | 0.4982 | 0 |
| 147 | superior colliculus | -0.0016 | 0.0089 | 0.0005 | 0.0028 | 0.0044 | 0.0065 | 0 | 0.7151 | 0 | 0.7362 | 0 | 0.2034 | 0 |
| 148 | sub coeruleus nucleus | 0.0082 | 0.0120 | -0.0003 | 0.0041 | 0.0093 | 0.0069 | 0 | 0.2015 | 0 | 0.8843 | 0 | 1.0382 | 1 |
| 149 | supramammillary nucleus | 0.0391 | 0.0635 | 0.0014 | 0.0699 | 0.1120 | 0.1122 | 0 | 0.2409 | 0 | 0.9675 | 0 | 0.0895 | 0 |
| 150 | temporal ctx | -0.0057 | 0.0336 | -0.0257 | 0.0483 | 0.0144 | 0.0264 | 0 | 0.7220 | 0 | 0.2994 | 0 | 0.2907 | 0 |

*FIG. 17 (Continued)*

| | | | | | | | | | | | | | |
|---|---|---|---|---|---|---|---|---|---|---|---|---|---|
| 151 | triangular septal nucleus | 0.0012 | 0.0184 | -0.0023 | 0.0248 | 0.0293 | 0.0230 | 0 | 0.8888 | 0 | 0.8468 | 1 | 0.0462 | 1 |
| 152 | tenia tecta ctx | 0.0013 | 0.0297 | 0.0076 | 0.0286 | 0.0033 | 0.0245 | 0 | 0.9279 | 0 | 0.5847 | 0 | 0.7761 | 0 |
| 153 | olfactory tubercles | 0.0184 | 0.0257 | 0.0216 | 0.0297 | 0.0182 | 0.0270 | 0 | 0.1835 | 0 | 0.1790 | 0 | 0.2046 | 0 |
| 154 | trapezoid body | 0.0118 | 0.0475 | 0.0037 | 0.0584 | 0.0221 | 0.0361 | 0 | 0.6065 | 0 | 0.8939 | 0 | 0.2435 | 0 |
| 155 | Ventricle | 0.0104 | 0.0169 | 0.0147 | 0.0184 | 0.0007 | 0.0100 | 0 | 0.2405 | 0 | 0.1487 | 0 | 0.8806 | 0 |
| 156 | visual 1 ctx | 0.0074 | 0.0105 | -0.0038 | 0.0146 | 0.0073 | 0.0172 | 0 | 0.1913 | 0 | 0.5946 | 0 | 0.3984 | 0 |
| 157 | visual 2 ctx | 0.0042 | 0.0104 | -0.0027 | 0.0126 | 0.0012 | 0.0063 | 0 | 0.4164 | 0 | 0.6617 | 0 | 0.7029 | 0 |
| 158 | ventral anterior thalamic nucleus | 0.0015 | 0.0240 | -0.0050 | 0.0115 | 0.0175 | 0.0249 | 0 | 0.8950 | 0 | 0.3867 | 0 | 0.1926 | 0 |
| 159 | cochlear nucleus | 0.0210 | 0.0148 | 0.0188 | 0.0147 | 0.0100 | 0.0205 | 1 | 0.0339 | 1 | 0.0458 | 0 | 0.3392 | 2 |
| 160 | vestibular nucleus | 0.0125 | 0.0252 | 0.0110 | 0.0264 | 0.0056 | 0.0275 | 0 | 0.3290 | 0 | 0.4024 | 0 | 0.6725 | 0 |
| 161 | ventrolateral thalamic nucleus | 0.0041 | 0.0093 | 0.0068 | 0.0171 | 0.0023 | 0.0068 | 0 | 0.3836 | 0 | 0.4205 | 0 | 0.4960 | 0 |
| 162 | ventral lateral striatum | 0.0082 | 0.0137 | 0.0051 | 0.0056 | 0.0084 | 0.0052 | 0 | 0.2515 | 0 | 0.1099 | 1 | 0.0222 | 1 |
| 163 | ventromedial thalamic nucleus | 0.0026 | 0.0107 | 0.0073 | 0.0169 | -0.0008 | 0.0084 | 0 | 0.6128 | 0 | 0.3911 | 0 | 0.8453 | 0 |
| 164 | ventral medial nucleus | 0.0169 | 0.0110 | 0.0217 | 0.0362 | 0.0304 | 0.0158 | 1 | 0.0263 | 0 | 0.2507 | 1 | 0.0125 | 2 |
| 165 | ventral medial striatum | 0.0038 | 0.0078 | 0.0035 | 0.0042 | 0.0061 | 0.0065 | 0 | 0.3385 | 0 | 0.1403 | 0 | 0.1015 | 0 |
| 166 | ventral orbital ctx | -0.0010 | 0.0184 | 0.0177 | 0.0288 | -0.0043 | 0.0279 | 0 | 0.9108 | 0 | 0.2419 | 0 | 0.7488 | 0 |
| 167 | ventral pallidum | 0.0018 | 0.0117 | 0.0061 | 0.0049 | 0.0132 | 0.0146 | 0 | 0.7430 | 0 | 0.0515 | 0 | 0.1141 | 0 |
| 168 | ventral posteriolateral thalamic n | 0.0188 | 0.0199 | 0.0088 | 0.0085 | 0.0184 | 0.0072 | 0 | 0.1022 | 0 | 0.0815 | 1 | 0.0047 | 1 |
| 169 | ventral posteriolmedial thalamic n | 0.0026 | 0.0118 | 0.0049 | 0.0040 | 0.0095 | 0.0110 | 0 | 0.6456 | 0 | 0.0527 | 0 | 0.1273 | 0 |
| 170 | ventral subiculum | 0.0156 | 0.0255 | 0.0132 | 0.0199 | 0.0260 | 0.0333 | 0 | 0.2422 | 0 | 0.2133 | 0 | 0.1562 | 0 |
| 171 | ventral tegmental area | 0.0079 | 0.0200 | -0.0024 | 0.0167 | -0.0063 | 0.0480 | 0 | 0.4267 | 0 | 0.7624 | 0 | 0.7843 | 0 |
| 172 | White Matter | 0.0042 | 0.0012 | 0.0027 | 0.0044 | 0.0058 | 0.0022 | 1 | 0.0016 | 0 | 0.2450 | 1 | 0.0040 | 2 |
| 173 | White Matter | 0.0180 | 0.0299 | 0.0157 | 0.0305 | 0.0230 | 0.0321 | 0 | 0.2494 | 0 | 0.3153 | 0 | 0.1834 | 0 |
| 174 | zona incerta | 0.0015 | 0.0151 | 0.0054 | 0.0082 | 0.0147 | 0.0091 | 0 | 0.8393 | 0 | 0.2101 | 1 | 0.0222 | 1 |
| - | whole brain | 0.0046 | 0.0020 | 0.0021 | 0.0007 | 0.0057 | 0.0019 | 1 | 0.0069 | 1 | 0.0028 | 1 | 0.0027 | 3 |
| | | | | | | | | 17 | | 7 | | 27 | | |

**FIG. 17 (Continued)**

EP 4 741 014 A2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62711251
- WO 2017019812 A

- WO 2017019182 A


**Non-patent literature cited in the description**

- Alzheimer's Association. 2018 Alzheimer's Disease Facts and Figures.. *Alzheimers Dement.*, 2018, vol. 14 (3), 367-429
- **ASSINI FL** ; **DUZZIONI M** ; **TAKAHASHI RN.** Object location memory in mice: Pharmacological validation and further evidence of hippocampal CA1 participation.. *Behav Brain Res.*, 2009, vol. 204 (1), 206-211
- **BARBIER EL** ; **LAMALLE L** ; **DÉCORPS M.** Methodology of brain perfusion imaging.. *J Magn Reson Imaging.*, 2001, vol. 13 (4), 496-520
- **BREMERICH J** ; **BILECEN D** ; **REIMER P.** MR angiography with blood pool contrast agents.. *Eur Radiol.*, 2007, vol. 17 (12), 3017-3024
- **BRUNSER AM** ; **HOPPE A** ; **ILLANES S et al.** Accuracy of diffusion-weighted imaging in the diagnosis of stroke in patients with suspected cerebral infarct.. *Stroke.*, 2013
- **CHEN, C.-C. V** ; **CHEN, Y.-C.** ; **HSIAO, H.-Y.** ; **CHANG, C.** ; **CHERN, Y.** Neurovascular abnormalities in brain disorders: highlights with angiogenesis and magnetic resonance imaging studies.. *J. Biomed Sci.*, 2013, vol. 20, 47
- **CHRISTEN T** ; **NI W** ; **QIU D et al.** High-resolution cerebral blood volume imaging in humans using the blood pool contrast agent ferumoxytol.. *Magn Reson Med.*, 2012, 705-710
- **CUNNINGHAM CH** ; **ARAI T** ; **YANG PC** ; **MCCONNELL M V.** ; **PAULY JM** ; **CONOLLY SM.** Positive contrast magnetic resonance imaging of cells labeled with magnetic nanoparticles.. *Magn Reson Med.*, 2005, vol. 53 (5), 999-1005
- **GHARAGOUZLOO CA** ; **MCMAHON PN** ; **SRIDHAR S.** Quantitative contrast-enhanced MRI with superparamagnetic nanoparticles using ultrashort time-to-echo pulse sequences.. *Magn Reson Med.*, 2015, vol. 74 (2), 431-441
- **GHARAGOUZLOO CA** ; **TIMMS L** ; **QIAO J et al.** Quantitative vascular neuroimaging of the rat brain using superparamagnetic nanoparticles: New insights on vascular organization and brain function.. *Neuroimage.*, 2017, vol. 163, 24-33

- **GREENBERG SM** ; **VERNOOIJ MW** ; **CORDONNIER C et al.** Cerebral microbleeds: a guide to detection and interpretation.. *Lancet Neurol.*, 2009
- **GUO S** ; **ZHOU Y** ; **XING C et al.** The Vasculome of the Mouse Brain.. *PLoS One.*, 2012
- **HUANG, Y.** ; **MUCKE, L.** Alzheimer mechanisms and therapeutic strategies.. *Cell*, 2012, vol. 148, 1204-1222
- **KIM SM** ; **KIM MJ** ; **RHEE HY et al.** Regional cerebral perfusion in patients with Alzheimer's disease and mild cognitive impairment: effect of APOE Epsilon4 allele.. *Neuroradiology.*, 2013, vol. 55 (1), 25-34
- **LARKIN MC** ; **LYKKEN C** ; **TYE LD** ; **WICKELGREN JG** ; **FRANK LM.** Hippocampal output area CA1 broadcasts a generalized novelty signal during an object-place recognition task.. *Hippocampus.*, 2014, vol. 24 (7), 773-783
- **LI L** ; **JIANG Q** ; **ZHANG L et al.** Angiogenesis and improved cerebral blood flow in the ischemic boundary area detected by MRI after administration of sildenafil to rats with embolic stroke.. *Brain Res.*, 2007, vol. 1132 (1), 185-192
- **MANDEVILLE JB.** IRON fMRI measurements of CBV and implications for BOLD signal.. *Neuroimage.*, 2012, vol. 62 (2), 1000-1008
- **MCLAY RN** ; **FREEMAN SM** ; **HARLAN RE** ; **IDE CF** ; **KASTIN AJ** ; **ZADINA JE.** Aging in the hippocampus: Interrelated actions of neurotrophins and glucocorticoids.. *Neurosci Biobehav Rev.*, 1997, vol. 21 (5), 615-629
- **MUEHE AM** ; **FENG D** ; **VON EYBEN R et al.** Safety Report of Ferumoxytol for Magnetic Resonance Imaging in Children and Young Adults.. *Invest Radiol.*, 2016, vol. 51 (4), 221-227
- **PARDO J** ; **URIARTE M** ; **CÓNSOLE GM et al.** Insulin-like growth factor-I gene therapy increases hippocampal neurogenesis, astrocyte branching and improves spatial memory in female aging rats.. *Eur J Neurosci.*, 2016, vol. 44 (4), 2120-2128

- **REIJMER YD** ; **VAN VELUW SJ** ; **GREENBERG SM.** Ischemic brain injury in cerebral amyloid angiopathy.. *J Cereb Blood Flow Metab.*, 2016
- **REMPP KA** ; **BRIX G** ; **WENZ F** ; **BECKER CR** ; **GÜCKEL F** ; **LORENZ WJ.** Quantification of regional cerebral blood flow and volume with dynamic susceptibility contrast-enhanced MR imaging.. *Radiology.*, 1994, vol. 193, 637-641
- **SCHABEL MC** ; **PARKER DL.** Uncertainty and bias in contrast concentration measurements using spoiled gradient echo pulse sequences.. *Phys Med Biol.*, 2008, vol. 53 (9), 2345-2373
- **SEPPENWOOLDE JH** ; **VIERGEVER MA** ; **BAKKER CJG.** Passive tracking exploiting local signal conservation: The white marker phenomenon.. *Magn Reson Med.*, 2003, vol. 50 (4), 784-790
- **SHI Y** ; **WARDLAW JM.** Update on cerebral small vessel disease: a dynamic whole-brain disease.. *BMJ.*, 2016, vol. 1 (3), 83-92
- **SQUIRE LR.** Memory and the hippocampus: A synthesis from findings with rats, monkeys, and humans.. *Psychol Rev.*, 1992, vol. 99 (2), 195-231
- **STUBER M** ; **GILSON WD** ; **SCHÄR M et al.** Positive contrast visualization of iron oxide-labeled stem cells using inversion-recovery with ON-resonant water suppression (IRON).. *Magn Reson Med.*, 2007, vol. 58, 1072-1077
- **TROPRÈS I** ; **GRIMAULT S** ; **VAETH A et al.** Vessel size imaging.. *Magn Reson Med.*, 2001, vol. 45, 397-408
- **WALKER-SAMUEL S** ; **LEACH MO** ; **COLLINS DJ.** Reference tissue quantification of DCE-MRI data without a contrast agent calibration.. *Phys Med Biol.*, 2007, vol. 52, 589-601
- **WARDLAW JM** ; **SMITH EE** ; **BIESSELS GJ et al.** Neuroimaging standards for research into small vessel disease and its contribution to ageing and neurodegeneration.. *Lancet Neurol.*, 2013, vol. 12 (8), 822-838
- **WEY H-Y** ; **DESAI VR** ; **DUONG TQ.** A review of current imaging methods used in stroke research.. *Neurol Res.*, 2013
- Dementia.. *WHO Fact sheet*, May 2017
- **YANKEELOV T** ; **GORE J.** Dynamic contrast enhanced magnetic resonance imaging in oncology: theory, data acquisition, analysis, and examples.. *Curr Med Imaging Rev.*, 2009, vol. 3 (2), 91-107
- **ZLOKOVIC, B. V.** Neurovascular pathways to neurodegeneration in Alzheimer's disease and other disorders.. *Nat. Rev. Neurosci.*, 2011